# EUROPEAN PATENT APPLICATION

(11) **EP 2 363 711 A1**
(43) Date of publication of application: **07.09.2011**
(21) Application number: 11159423.0
(22) Date of filing: 29.01.2007
(51) Int. Cl.: G01N 33/574

(54) **Methods for identifying patients with an increased likelihood of having ovarian cancer and compositions therefor**

(30) Priority: 27.01.2006 US 762760 P
(62) Divisional of application: 07717460.5
(71) Applicant: Tripath Imaging, Inc., Burlington, NC 27215 (US)
(72) Inventor: Fischer, Timothy J., Raleigh, NC 27613 (US); Malinowski , Douglas P., Hillsborough, NC 27278 (US); He, Qin, Raleigh, NC 27613 (US); Whitehead, Clark M., Apex, NC 27502 (US); Cheek, Robert L., Mebane, NC 27302 (US); Groelke, John W., Raleigh, NC 27613 (US)
(74) Representative: Bentham, Andrew

(57) **Abstract**

Screening methods for identifying patients with an increased likelihood of having ovarian cancer are provided. The screening methods involve the detection of expression of a plurality of biomarkers in a body sample, wherein the biomarkers are HE4 and CA125 and overexpression of at least one of the biomarkers is indicative of an increased likelihood of having ovarian cancer. The methods may further comprise detection of the expression of the biomarker glycodelin. The screening methods may also further comprise a two-step analysis. In some aspects of the invention, expression of a biomarker is detected at the protein level using a biomarker-specific antibody or at the nucleic acid level using nucleic acid hybridization techniques. Kits for practicing the methods of the invention are further provided.

## Description

### FIELD OF THE INVENTION

The present invention relates to methods and compositions for identifying women having an increased likelihood of having ovarian cancer.

### BACKGROUND OF THE INVENTION

Ovarian cancer represents a heterogeneous group of diseases that affect women on a global basis. There are several forms of ovarian cancer which include epithelial cancer, germ-line cancer of the ovaries and ovarian stromal cancer. Epithelial ovarian cancer represents the most common form of the disease. Approximately 5-10% of epithelial ovarian cancer represents a hereditary form of the disease and three common patterns are recognized: ovarian cancer alone; ovarian and breast cancer linked to BRAC1 and BRCA2 genetic linkage on chromosomes 17q21 and 13q12 respectively; and ovarian and colon cancer. The most important risk factor for ovarian cancer is a first degree relative with the disease (e.g., a mother, sister or daughter with ovarian cancer). See, for example, Patridge et al. (1999) CA-A Cancer Journal for Clinicians 49:297-320In 2005, there were an estimated 22,000 new cases of ovarian cancer diagnoses and 16,000 deaths from ovarian cancer. See generally *American Cancer Society* website at www.cancer.org; *Rational Cancer Institute* website at www.cancer.gov. Ovarian cancer is a disease that primarily affects post-menopausal women with the median age for diagnosis at 63 years of age. However, the disease can affect women at all age groups. National Cancer Institute *Surveillance, Epidemiology, and End Result (SEER) Program* at www.seer.cancer.gov.

The classification of ovarian cancer stage is based upon the extent of localization versus spread of the disease beyond the ovaries. Stage 1 ovarian cancer is confined to one or both of the ovaries. Stage 2 disease involves a tumor in one or both ovaries with pelvic extension. In Stage 3 ovarian cancer, a tumor is present in or both ovaries with microscopically confirmed peritoneal metastasis outside the pelvis and/or regional lymph node metastasis. Stage 4 ovarian cancer is characterized by distant metastasis beyond the peritoneal cavity. Ovarian cancer is generally diagnosed in an advance stage of the disease due to the lack of specific clinical symptoms that would indicate the presence of small tumors. For women under the age of 50, less than 40% of ovarian cancers are detected when tumors are localized to one or both ovaries and when disease prognosis is best. For women over the age of 50, that number drops to less than 15%. Approximately 68% of women of all age groups afflicted with ovarian cancer are not diagnosed until distant metastasis is present. See generally National Cancer Institute *Surveillance, Epidemiology, and End Results (SEER) Program* at www.seer.cancer.gov.

Ovarian cancer spreads via local shedding from the ovarian epithelium into the peritoneal cavity followed by implantation on the peritoneum and local invasion of the bowel and bladder. The presence of lymph node involvement in ovarian cancer is evident in all stages of diagnosed ovarian cancer. The percentage of positive lymph nodes increases significantly with progression of the disease (i.e., Stage 1,24%; Stage 2, 50%, Stage 3, 74%; Stage 4, 73%). *Id.*

The survival of patients with ovarian cancer is a function of the stage at which the disease is diagnosed, with the 5-year survival decreasing with advanced disease. More than 90% of women diagnosed with ovarian cancer in Stage 1 survive for at least 5 years following diagnosis. The 5-year survival rate drops to less than 30% when the disease is not diagnosed until Stage 4 (i.e., distant metastasis). Id.

Epithelial ovarian cancer is the most common form of the disease. There are four recognized major histological classes of epithelial ovarian cancer and include serous, endometrioid, clear cell, and mucinous subtypes. The pathogenesis of ovarian cancer is poorly understood but is believed to arise from ovarian surface epithelium. See Bell (2005) Mod. Pathol.18 (Supp12):S19-32. Life factors that provide the greatest reduction in risk of ovarian cancer include multiparity, use of oral contraceptives, and breast feeding, all of which prevent ovulation. Because ovulation results in epithelial damage, followed by repair and possible inflammatory responses, repetition of this process throughout a woman's reproductive life without interruption appears to lead to cell damage and to increase the risk of ovarian cancer. See, for example, Ness et al. (1999) J. Natl. Cancer Inst. 91:1459-1467. However, there is no recognized, stepwise progression of ovarian cancer through defined precursor lesions, such as those recognized for both cervical carcinoma and colon cancer. Hence, considerable research has been directed at understanding the molecular basis for ovarian cancer and to understand the basic differences between the various Histological subtypes of ovarian cancer. These studies have utilized gene expression analysis to provide this understanding and have identified a series of potential biomarkers for evaluation in diagnostic applications. See for example Ono et al. (2000) Cancer Res. 60:5007-11; Welsh et al. (2001) Proc. Natl. Acad. Sci. USA 98:1176-1181; Donninger et al. (2004) Oncogene 23:8065-8077; and Lee et al. (2004) In.t; J. Oncol. 24(4):847-851.

Ovarian cancer is often detected with the presentation of overt clinical symptoms, most notably the presentation of abdominal pain, an adnexal mass, abdominal bloating, and urinary urgency. As such, the detection of ovarian cancer is often detected at an advanced stage, where the prognosis and clinical outcome is poor. Detection of ovarian cancer at an early stage (i.e., Stage 1) results in approximately 90% cure rate using standard surgery and chemotherapy; hence there is a clinical need to detect ovarian cancer at an early stage where treatment will be most effective. Unfortunately, current screening methods to detect early stage ovarian cancer are insufficient. The current practice for ovarian cancer screening employs the use of CA125 and transvaginal ultrasound (sonogaphy). Rising serum levels of CA125 are associated with ovarian cancer and subsequent utilization of transvaginal ultrasound helps detect the presence of ovarian cancer. Confirmation of ovarian disease is based upon invasive procedures such as laprotomy. However, the use of CA125 is ineffective for general population screening due to issues of limited sensitivity, limited specificity, and a poor positive predictive value of <3%. Bast (2003) J Clin Oncol. 21(10 Suppl):200-205. As a result, there is no consensus on the recommendations for generally screening for ovarian cancer in the asymptomatic patient population. See National Cancer Institute Web Site at www.cancer.gov. For high risk patients, the generally accepted procedures for the detection of ovarian cancer include the use of pelvic examinations, the use of CA125 serum testing, and transvaginal ultrasound (sonography). Patridge et al. (1999) CA-A Cancer Journal for Clinicians 49:297-320.

CA125 is a well characterized tumor marker normally expressed on the surface of epithelial cells and is often detected in the serum of normal patients at 35U/mL. Elevated serum levels of CA125 (>35U/mL) are often detected in approximately 85% of ovarian cancer patients; the remaining 15% of ovarian cancer patients have normal serum levels of CA125. Furthermore, CA125 is elevated in only 50% of stage 1 ovarian cancer patients, thereby limiting its clinical utility in the early detection of ovarian cancer. However, elevated serum levels of CA125 are used for the monitoring of disease recurrence following therapeutic intervention and this represents the currently approved use for CA125 by the FDA. In addition, elevated serum levels of CA125 are predictive of future detectable ovarian cancer.

The low prevalence rates of ovarian cancer in the general population create significant challenges for the development of a screening test that would promote early detection of the disease. Screening methods for diseases with low prevalence rates such as ovarian cancer often result in a high ratio of false positives to true positives that limits the clinical utility of such screening programs. Given the significant risks associated with surgical exploration for possible ovarian cancer, a clinically useful screening test should refer to surgery no more than 10 women for every woman who actually has ovarian cancer (i.e., a positive predictive value (PPV) of at least 10%). Skates et al. (2004) J. Clin. Oncol. 22:4059-4066. PPV is highly dependent upon the prevalence rates for a particular disease or condition and will shift dramatically as a result of differences in disease prevalence. Therefore, with low prevalence diseases, such as ovarian cancer, screening diagnostic tests with a relatively low PPV still have significant clinical utility. Potential ovarian cancer screening programs must be adjusted for the low prevalence of ovarian cancer and assessed for biomarker performance and clinical need. See, for example, Skates et al. (2004) J. Clin. Oncol. 22:4059-4066; Bast et al. (2005) Int. J. Gynecol. Cancer 15:274-281; and Rosen et al. (2005) Gyn. Oncol. 99:267-277. Despite efforts to identify a biomarker or panel of biomarkers for the detection, particularly early detection, of ovarian cancer, no adequate screening or diagnostic test that satisfies clinical needs currently exists. Currently available methods, such as detection of CA125, exhibit unacceptably high false-positive rates.

The current recommendations from the National Cancer Institute state that "there is insufficient evidence to establish that screening for ovarian cancer with serum markers such as CA125, transvaginal ultrasound or pelvic examinations would result in a decrease in mortality from ovarian cancer" *(*NCI Summary of Evidence (Level 4, 5); dated February 2005). In light of the serious risk of false-positives with currently available screening techniques, the NCI has not supported institution of general screening procedures for ovarian cancer. As such, no standardized screening test exists for ovarian cancer, despite the fact that early diagnosis significantly improves 5-year survival rates.

Therefore, a significant need exists in the art for reliable methods and compositions that are capable of specifically identifying women that have ovarian cancer. In particular, screening methods for identifying patients with an increased likelihood of having ovarian cancer are needed. Women identified as having an increased likelihood of having ovarian cancer could be selected for more aggressive diagnostic methods to definitively determine if they presently have the disease. Moreover, such screening methods could be performed in the general female patient population on a routine basis to facilitate the detection of ovarian cancer in the early stages of the disease when prognosis and disease outcome are best.

### BRIEF SUMMARY OF THE INVENTION

Screening methods for identifying patients with an increased likelihood of having ovarian cancer are provided. The methods of the invention generally comprise detecting in a patient body sample expression of a plurality of biomarkers that are selectively overexpressed in ovarian cancer. Overexpression of the biomarkers is indicative of an increased likelihood that the patient has ovarian cancer. The methods of the invention may comprise, for example, a "two-step" analysis, wherein a first assay step is performed to detect the expression of a first biomarker or panel of biomarkers. If the first biomarker or panel of biomarkers is overexpressed, a second assay step is performed to detect the expression of a second biomarker or panel of biomarkers. Overexpression of the first and second biomarkers or panels of biomarkers is indicative of an increased likelihood that the patient has ovarian cancer. The first assay step may be designed to enrich the patient population under review by eliminating a large percentage of women that are "true negatives." The second assay step is typically intended to rule out those patients in the enriched population that do not presently have ovarian cancer by eliminating additional true negative patients from the enriched population. These assay steps may be performed as a single test encompassing both assay steps or as two distinct tests, wherein each test comprises one of the assay steps. A single biomarker or panel of biomarkers may be used in each analysis step to achieve the desired values for sensitivity, specificity, negative predictive value (NPV), and positive predictive value (FPV). Algorithms may be developed to combine particular biomarkers in the different assay steps to achieve the desired sensitivity, specificity, NPV, and PPV.

In other aspects of the invention, a screening method for identifying patients with an increased likelihood of having ovarian cancer comprises performing a "single-step" or "one-step" assay in which the overexpression of a plurality of biomarkers that are selectively overexpressed in ovarian cancer is assessed in a patient body sample. Overexpression of these biomarkers is indicative of an increased likelihood of the patient having ovarian cancer. In contrast to the two-step screening method described above, the single-step screening assay does not require performing a first assay step to detect overexpression of a particular biomarker(s) followed by a second assay step to assess if a second biomarker(s) is also overexpressed in order to identify a patient with an increased likelihood of having ovarian cancer.

A patient that is identified by the screening methods of the invention as having an increased likelihood of having ovarian cancer may be subjected to further diagnostic tests to definitively determine if the patient has ovarian cancer. Patients that are classified as having an increased likelihood of having ovarian cancer in accordance with the methods disclosed herein, but that are determined not to currently have the disease, are generally monitored on a regular basis for the development of ovarian cancer. Moreover, the present methods may be used to screen the general female patient population for ovarian cancer on a routine basis. Thus, the screening methods of the invention may permit the diagnosis of ovarian cancer at earlier stages of the disease when prognosis is significantly better. Kits for practicing the screening methods of the invention are also provided.

Biomarker expression can be assessed at the protein or nucleic acid level. In some embodiments, biomarker expression is detected at the protein level using biomarker-specific antibodies. Expression of the biomarkers of the invention can also be detected by nucleic acid-based techniques, including, for example, hybridization and RT-PCR. Biomarker expression can be assessed in a variety of body samples, including but not limited to blood (e.g., whole blood, blood serum, blood having platelets removed, etc.), lymph, ascitic fluids, urine, gynecological fluids (e.g., ovarian, fallopian, and uterine secretion, menses, etc.), biopsies, and fluids obtained during laparoscopy.

Methods for diagnosing ovarian cancer in a patient are also encompassed by the present invention. The diagnostic methods generally comprise detecting in a body sample the expression of a plurality of biomarkers that are selectively overexpressed in ovarian cancer. Overexpression of the plurality of biomarkers is indicative of the presence of ovarian cancer. Kits for practicing the diagnostic methods of then invention are also provided.

Methods for assessing the efficacy of a particular therapy for ovarian cancer in a patient are also disclosed herein. The invention is further directed to a method for monitoring the regression or progression of ovarian lancer in a patient.

### BRIEF DESCRIPTION OF THE DRAWINGS

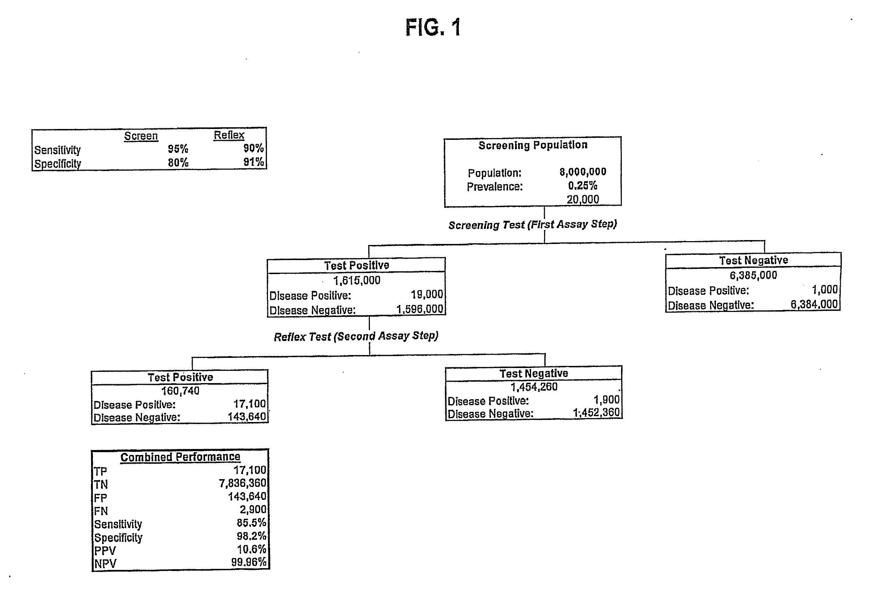

Figure 1 provides a schematic representation of an exemplary "two-step" screening test for identifying patients with an increased likelihood of having ovarian cancer. The 8 million female patients screened in this example represent the high-risk, asymptomatic U.S. patient population, as defined herein below. In the first assay step, a significant number of true negatives are eliminated from further testing, thereby leaving an enriched population for further analysis in the second assay step. The second assay step further rules out additional patients that are true negatives in order to identify those women with the highest risk of having ovarian cancer. Additional details regarding the two-step screening method are provided in the text.
Figure 2 provides the Receiver Operating Characteristic (ROC) plots for HE4 obtained with samples from patients over the age of 55 (A) and with all patient samples. Additional experimental details are provided in Example 2.
Figure 3 provides the ROC plots for inhibin A (INH) obtained with samples from patients over the age of 55 (A) and with all patient samples. Additional experimental details are provided in Example 2.
Figure 4 provides the ROC plot for prolactin obtained with all patient samples.
   Additional experimental details are provided in Example 2.
Figure 5 provides the ROC plot for PLAU-R obtained with all patient samples.
   Additional experimental details are provided in Example 2.
Figure 6 provides the ROC plots for glycodelin (GLY) obtained with samples from patients over the age of 55 (A) and with all patient samples. Additional experimental details are provided in Example 2.
Figure 7 provides the ROC plot for SLPI obtained with all patient samples.
   Additional experimental details are provided in Example 2.
Figure 8 provides the ROC plot for CTHRC1 obtained with all patient samples. Additional experimental details are provided in Example 2.
Figure 9 provides the ROC plot for PAI-1 obtained with all patient samples.
   Additional experimental details are provided in Example 2.
Figure 10 provides the ROC plot for KLK-10 obtained with all patient samples. Additional experimental details are provided in Example 2.
Figure 11 provides the ROC plots for CA125 obtained with samples from patients over the age of 55 (A) and with all patient samples. Additional experimental details are provided in Example 2.
Figure 12 provides the ROC plot for KLK-6 obtained with all patient samples.
   Additional experimental details are provided in Example 2.
Figure 13 provides the ROC plot for Muc-1 (MU) obtained with all patient samples. Additional experimental details are provided in Example 2.
Figure 14 provides the ROC plot for MMP-7 (MM) obtained with all patient samples. Additional experimental details are provided in Example 2.

### DETAILED DESCRIPTION OF THE INVENTION

The present invention provides screening methods and compositions for identifying patients with ovarian cancer. The screening methods generally comprise detecting the expression of a plurality of biomarkers in a body sample, particularly a blood sample, more particularly a serum sample, from the patient. Overexpression of the biomarkers used in the practice of the invention is indicative of an increased likelihood of the presence of ovarian cancer. In particular screening methods of the invention, a two-step analysis is used to identify patients having an increased likelihood of having ovarian cancer. The first assay step is performed to detect the expression of a first biomarker or panel of biomarkers in a patient body sample. If the first biomarker or panel of biomarkers is determined to be overexpressed in the sample, a second assay step is performed to detect the expression of a second biomarker or panel of biomarkers. Overexpression of the first and second biomarkers or panels of biomarkers is indicative of an increased likelihood that the patient has ovarian cancer. In certain embodiments, antibodies are used to detect biomarker protein expression. In other aspects of the invention, when the expression of a panel of biomarkers is detected, overexpression of only a subset of the biomarkers analyzed may be sufficient to be indicative of an increased likelihood of the patient having ovarian cancer.

Although the invention is not limited to a particular mechanism, the first assay step is generally designed to enrich the true-positive patient population (i.e., on a percentage basis) by eliminating a large number of true negatives from further testing. The first assay step may employ a higher sensitivity and negative predictive values (NPV) in order to achieve the desired enrichment of the true-positive patient population. The second assay step is typically intended to rule out those patients in the enriched population that do not presently have ovarian cancer, that is, to eliminate additional true negatives from the enriched population. The second assay step may employ a higher specificity and positive predictive value (PPV), while maintaining a reasonable sensitivity, to further eliminate true negatives from the enriched population of true-positive patients. A schematic representation of the two-step screening test and the results that can be obtained with this method is provided in Figure 1.

One of skill in the art will recognize that these assay steps may be performed as a single screening test encompassing both assay steps or as two distinct tests, wherein each test encompasses one of the assay steps. A single biomarker or panel of biomarkers may be used in each assay step to achieve the desired values for sensitivity, specificity, NPV, and PPV. Algorithms may be developed to select particular biomarkers or combinations of biomarkers to achieve the desired sensitivity, specificity, NPV, and PPV for each assay step and the combined screening test comprising both assay steps.

In addition to the two-step analysis described herein above, a screening method for identifying patients with an increased likelihood of having ovarian cancer comprises performing a "single-step" or "one-step" screening method or assay in which the expression of a plurality of biomarkers that are selectively overexpressed in ovarian cancer is assessed in a patient body sample. Overexpression of at least one biomarker(s) is indicative of an increased likelihood of the patient having ovarian cancer. In particular aspects of the invention, the single or one-step screening method comprises detecting expression of a plurality of biomarkers, including, for example, HE4, CA125, glycodelin, MMP-7, Muc-1, PAI-1, CTHRC1, inhibin A, PLAU-R, prolactin, KLK-10, KLK-6, SLPI, and alpha-1 anti-trypsin, in abody sample, wherein overexpression of at least one, particularly two, more particularly three, of the biomarkers is indicative of an increased likelihood of having ovarian cancer. In particular embodiments of the single-step screening method of the invention, expression of glycodelin, HE4, and CA125 is assessed and overexpression of at least one, two, or three of the biomarkers is indicative of an increased likelihood of having ovarian cancer. See, for example, Table 54 and Experimental Example 4. One of skill in the art will appreciate that in contrast to the two-step screening method described above, the single-step screening assay does not require performing a first assay step to detect overexpression of a particular biomarker or panel of biomarkers followed by a second assay step to assess if a second biomarker or panel of biomarkers is also overexpressed in order to identify a patient with an increased likelihood of having ovarian cancer. As such, a first assay step to enrich the population for "true-positive" patients and a second assay step to rule out those patients from the enriched population that do not actually have ovarian cancer ("false positives") is not required in the single-step screening method described herein.

The level of expression of a particular biomarker that is sufficient to constitute "overexpression" will vary depending on the specific biomarker used. In particular embodiments of the invention, a "threshold level" of expression is established for a particular biomarker, wherein expression levels above this value are deemed overexpression. A variety of statistical and mathematical methods for establishing the threshold level of expression are known in the art. A threshold expression level for a particular biomarker may be selected, for example, based on data from Receiver Operating Characteristic (ROC) plots, as described in Examples 2 and 3, or on compilations of data from normal patient samples (i.e., a normal patient population). For example, the threshold expression level may be established at the mean expression level plus two times the standard deviation, based on analysis of samples from normal patients not afflicted with ovarian cancer. One of skill in the art will appreciate that these threshold expression levels can be varied, for example, by moving along the ROC plot for a particular biomarker, to obtain different values for sensitivity, specificity, positive predictive value (PPV), and negative predictive value (NPV), thereby affecting overall assay performance.

A patient that is identified as having an increased likelihood of having ovarian cancer in accordance with the disclosed methods may be subj ected to further diagnostic testing to definitively determine if the patient has ovarian cancer. "Further diagnostic testing" includes but is not limited to pelvic examination, transvaginal ultrasound, CT scan, MRI, laparotomy, laparoscopy, and biopsy. Such diagnostic methods are well known in the art. Moreover, patients classified as having an increased likelihood of having ovarian cancer that are determined by further diagnostic testing not to currently have ovarian cancer may be closely monitored on a regular basis for the development of ovarian cancer. Monitoring of such patients may include but is not limited to periodic pelvic examination, transvaginal ultrasound, CT scan, and MRI. A physician of ordinary skill in the art will appreciate appropriate techniques for monitoring patients for the development of ovarian cancer. By identifying and monitoring patients having an increased likelihood of having ovarian cancer, the screening methods of the invention may permit the detection of ovarian cancer at an earlier stage of the disease, particularly Stage 1 or Stage 2, when prognosis and disease outcome are greatly improved.

In particular embodiments, antibodies are used to detect biomarker expression at the protein level. In other aspects of the invention, biomarker expression is detected at the nucleic acid level. Kits for practicing the screening methods of the invention, including the two-step screening method, are further provided.

By "ovarian cancer" is intended those conditions classified by post exploratory laparotomy as premalignant pathology, malignant pathology, and cancer (FIGO Stages 1-4). Staging and classification of ovarian cancer are described in detail above. "Early-stage ovarian cancer" refers to those disease states classified as Stage 1 or Stage 2 carcinoma. Early detection of ovarian cancer significantly increases 5-year survival rates. The term "screening method" refers to strategies to identify patients that have an increased likelihood of having ovarian cancer so that such patients can be selected for more aggressive diagnostic methods to definitively determine if the patients have ovarian cancer. The "screening methods" or "diagnostic screening methods" of the invention are generally not intended to definitively diagnose a patient as having or not having ovarian cancer. Rather, such methods are intended to identify women having an increased likelihood of having ovarian cancer so that these women may be definitively diagnosed using other methods (e.g., pelvic examination, transvaginal ultrasound, CT scan, MRI, laparotomy, laparoscopy, and biopsy of tissue samples). Regimens may also be instituted for monitoring patients identified as having an increased likelihood of having ovarian cancer by the present methods but that are determined not to currently have the disease.

The screening methods of the invention may be performed on a case-by-case basis or as a periodic routine screening test for the general female population. In some embodiments, the screening methods for identifying patients with an increased likelihood of having ovarian cancer may be viewed as comparable to Pap smears for the identification of patients having an increased likelihood of having cervical cancer. As used herein, "identifying patients with an increased likelihood of having ovarian cancer" is intended methods for detecting those females that are more likely to have ovarian cancer. An "increased likelihood of having ovarian cancer" is intended to mean that patients who are determined in accordance with the present methods to exhibit overexpression of particular biomarkers are more likely to have ovarian cancer than those patients who do not.

"Diagnosing ovarian cancer" is intended to include, for example, diagnosing or detecting the presence of ovarian cancer, monitoring the progression of the disease, and identifying or detecting cells or samples that are indicative of ovarian cancer. The terms diagnosing, detecting, and identifying ovarian cancer are used interchangeably herein. Definitive diagnosis of ovarian cancer will generally comprise performing a biopsy on a tissue sample from the patient.

The methods of the present invention permit superior assessment of the likelihood of having ovarian cancer when compared with proposed screening methods currently known in the art (e.g., measurement of CA125 levels). As used herein, "specificity" refers to the level at which a method of the invention can accurately identify samples that have been confirmed as nonmalignant by exploratory laparotomy (i.e., true negatives). That is, specificity is the proportion of disease negatives that are test-negative. In a clinical study, specificity is calculated by dividing the number of true negatives by the sum of true negatives and false positives. By "sensitivity" is intended the level at which a method of the invention can accurately identify samples that have been laparotomy-confumed as positive for ovarian cancer (i.e., true positives). Thus, sensitivity is the proportion of disease positives that are test-positive. Sensitivity is calculated in a clinical study by dividing the number of true positives by the sum of true positives and false negatives. The sensitivity of the disclosed methods for the detection of ovarian cancer is at least about 70%, particularly at least about 80%, more particularly at least about 90, 91, 92, 93, 94, 95, 96, 97, 98, 99% or more. Furthermore, the specificity of the present methods is at least about 70%, particularly at least about 80%, most particularly at least about 90, 91, 92, 93, 94, 95, 96, 97, 98, 99% or more.

The term "positive predictive value" or "PPV" refers to the percentage of patients with a positive test result in the methods of the invention who actually have ovarian cancer. PPV is calculated in a clinical study by dividing the number of true positives by the sum of true positives and false positives. PPV is highly dependent upon the prevalence rates for a particular disease or condition and will shift dramatically as a result of differences in disease prevalence. Therefore, with low prevalence diseases, such as ovarian cancer, screening tests with a relatively low PPV still have significant clinical utility. In contrast, a disease with high prevalence rates would require a higher PPV to be clinically useful See, for example, Skates et al. (2004) J. Clin. Oncol. 22:4059-4066; Bast et al. (2005) Int. J. Gynecol. Cancer 15:274-281; Rosen et al. (2005) Gyn. Oncol. 99:267-277; and Pepe (2004) The Statistical Evaluation of Medical Tests for Classification and Prediction (Oxford University Press), all of which are herein incorporated by reference in their entirety. The PPV for the present methods of identifying patients with an increased likelihood of having ovarian cancer is generally at least about 7, 8, 9, 10, 15, 20, 25, 30% or more. In some embodiments, the PPV of a method of the invention is at least about 10%. A PPV of at least about 10% for a diagnostic screening method is considered in the art to be of clinical utility. See Skates *et al., supra.* The "negative predictive value" or "NPV" of a test is the percentage of patients with a negative test result who actually do not have ovarian cancer. NPV is calculated in a clinical study by dividing the number of true negatives by the sum of true negatives and false negatives. The NPV for the present methods of identifying patients with an increased likelihood of having ovarian cancer is generally at least about 80%, particularly at least about 90%, more particularly at least about 91,92,93,94,95,96, 97,98, 99,99.1,99.2,99.3, 99.4, 99.5, 99.6, 99.7, 99.8, 99.9% or more. In some embodiments, the NPV of a method of the invention for is at least about 99%. One of skill in the art will appreciate that the PPV and NPV values for the methods of the invention are based on a prevalence-adjusted population and are reflective of the prevalence rates of ovarian cancer within the U. S. female population.

A "biomarker" is any gene or protein whose level of expression in a tissue or cell is altered compared to that of a normal or healthy cell or tissue. Biomarkers of the invention are selective for ovarian cancer. By "selectively overexpressed in ovarian cancer" is intended that the biomarker of interest is overexpressed in ovarian cancer but is not overexpressed in conditions classified as nonmalignant, benign, and other conditions that are not considered to be clinical disease. Thus, detection of the biomarkers of the invention permits the differentiation of samples indicative of an increased likelihood of having ovarian cancer or the presence of ovarian cancer from normal samples (i.e., samples from patients that are ovarian-cancer free) and samples that are indicative of nonmalignant and benign proliferation. Biomarkers of the invention may be referred to herein interchangeably as "ovarian cancer biomarkers," "markers.," or "ovarian cancer markers."

The biomarkers of the invention include genes and proteins. Such biomarkers include DNA comprising the entire or partial sequence of the nucleic acid sequence encoding the biomarker, or the complement of such a sequence. The biomarker nucleic acids also include RNA comprising the entire or partial sequence of any of the nucleic acid sequences of interest. A biomarker protein is a protein encoded by or corresponding to a DNA biomarker of the invention. A biomarker protein comprises the entire or partial amino acid sequence of any of the biomarker proteins or polypeptides. Fragments and variants of biomarker genes and proteins are also encompassed by the present invention. By "fragment" is intended a portion of the polynucleotide or a portion of the amino acid sequence and hence protein encoded thereby. Polynucleotides that are fragments of a biomarker nucleotide sequence generally comprise at least 10,15,20, 50,75, 100, 150, 200, 250, 300, 350, 400, 450, 500, 550, 600, 650, 700, 800, 900, 1,000, 1,100, 1,200, 1,300, or 1,400 contiguous nucleotides, or up to the number of nucleotides present in a full-length biomarker polynucleotide disclosed herein. A fragment of a biomarker polynucleotide will generally encode at least 15, 25, 30, 50, 100, 150, 200, or 250 contiguous amino acids, or up to the total number of amino acids present in a full-length biomarker protein of the invention. "Variant" is intended to mean substantially similar sequences. Generally, variants of a particular biomarker of the invention will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that biomarker as determined by sequence alignment programs.

The biomarkers of the invention include any gene or protein that is selectively overexpressed in ovarian cancer, as defined herein above, and may comprise known biomarkers as well as those presently unknown in the art. In particular embodiments, biomarkers are secreted proteins or proteins that are predicted to encode membranous proteins with transmembrane segments and extracellular domains. Biomarkers of interest include BB4, CA125, glycodelin, MMP-7, Muc-1, PAI-1, CTHRC1, inhibin, PLAU-R, prolactin, KLK-10, KLK-6, and SLPI, alpha-1 anti-trypsin (AAT), Imp-2, FLJ10546, FLJ23499, MGC13057, SPON1, S100A1, SLC39A4, TACSTD2, MBG2, HETKL27 (MAL2), Cox-1, protein kinase C-iota, cadherin-6, ADPRT, matriptase, folate receptor, claudin 4, mesothelin, aquaporin 5, cofilin 1, gelsolin, clusterin, alpha tetranectin, vitronectin. pregnancy-associated plasma protein-A (PAPP-A), and folistatin. Biomarkers of particular interest include but are not limited to HE4, CA125, Glycodelin, MMP-7, Muc-1, PAI-1, CTHRC1, inhibin, PLAU-R, prolactin, KLK-10, KLK-6, SLPI, and alpha-1 anti-trypsin (AAT). Biomarkers of more particular interest include HE4, glycodelin, MMP-7, SLPI, PLAU-R, Muc-1, inhibin A, and PAI-1.

HB4 is a protein that was first observed in human epididymis tissue, and the name "HE4" is an abbreviation of "Human Epididymis Protein 4". Subsequent studies have shown that HE4 protein is also present in the female reproductive tract and other epithelial tissues. The HE4 gene resides on human chromosome 20q12-13.1, and the 20q12 chromosome region has been found to be frequently amplified in ovarian carcinomas. Studies have shown that HE4 is expressed by ovarian carcinoma cells. The protein is N-glycosylated and is secreted extracellularly. See, for example, Drapin et al. (2005) Cancer Research 65(6): 2162-9; Hellström et al. (2003) Cancer Research 63: 3695-3700; and Bingle et al. (2002) Oncogene 21: 2768-2773.

CA-125 is a high molecular weight, cell surface glycoprotein detected in the serum of a large proportion of patients with ovarian epithelial cancer (OBC). However, while the percentage is high (75-90%) in advanced stages of this disease, it is only elevated in 50% of the patients with Stage 1 disease. Use of CA-125 as a marker for OEC is problematic because the molecule is also expressed in a number of normal and pathological conditions including menstruation, pregnancy, endometriosis, inflammatory diseases and other types of cancer. Improved sensitivity and specificity for OEC has been reported among post menopausal women. See, for example, Bast et al. (1998) Int'l J. Biological Markers 13:170-187; and Moss et al. (2005) J. Clin. Pathol. 58:308-312.

Glycodelin is a member of the lipocalin superfamily with several distinctive actions in cell recognition and differentiation principally in the reproductive axis. Previously, this glycoprotein has been named progesterone-associated endometrial protein (PAEP) and placental protein 14 (PP-14). The name change was in part initiated because glycodelin is not synthesized by the endometrium or placenta. Glycodelin has been purified from amniotic fluid as 28kDa molecule in SDS gels and reported to be synthesized by the normal ovary and by malignant ovarian tumors. Its presence has been reported in serum. See generally Seppala et al. (2002) Endocrine Reviews 23:401-430; Pala et al. (1997) J. Chromatography B 704:25-34; Meerit Kamarainen et al. (1996)Amer. J. Pathology 148: 1435-1443.

Proteins of the matrix metalloproteinase (MMP) family are involved in the breakdown of extracellular matrix in normal physiological processes, such as embryonic development, reproduction, and tissue remodeling, as well as in disease processes, such as arthritis and metastasis. Most MMP's are secreted as inactive proproteins which are activated when cleaved by extracellular proteinases. The enzyme encoded by the MMP-7 (matrilysin) gene degrades proteoglycans, fibronectin, elastin and casein and differs from most MMP family members in that it lacks a conserved C-terminal protein domain. The enzyme is involved in wound healing, and studies in mice suggest that it regulates the activity of defensins in intestinal mucosa. The MMP-7 gene is part of a cluster of MMP genes which localize to chromosome 11q22.3. MMP-7 is expressed in epithelial cells of normal and diseased tissue. It is known to be expressed in tumors of the breast, colon, and prostate, among others. It is abundant in ovarian carcinoma cells, but not detectable by IHC in normal ovarian epithelial tissue.

Muc-1 (mucin1, EMA, PEM, episialin) is a large cell surface mucin glycoprotein expressed by most glandular and ductal epithelial cells and some hematopoietic cell lineages. Functionally, Muc-1 is believed be involved with cell protection and lubrication, and may play a role in cell adhesion, and/or cell signaling. The Muc-1 gene contains seven exons and produces several different alternatively spliced variants. The major expressed form of Muc-1 uses all seven exons and is a type 1 transmembrane protein with a large extracellular tandem repeat domain. The tandem repeat domain is highly O-glycosylated and alterations in glycosylation have been shown in epithelial cancer cells. Muc-1 has been found to be elevated in many types of cancer, most notably with advanced breast cancer. Standing alone as a serum-based marker, it would have little specificity, but could be utilized as a prognostic aid, in conjunction with other, more specific markers.

PAI-1 (i.e., plasminogen activator inhibitor type 1) is a serine or cysteine proteinase inhibitor. The PAI-1 mRNA is 2876 bp in length, and the encoded protein is 402 amino acids long. The calculated molecular weight is 42,769 Da, whereas the affinity-purified protein is reported to be approximately 50,000 Da, as determined by SDS gel electrophoresis. PAI-1 plays a role in inhibiting extracellular matrix degradation by PLAU and is a putative unregulated c-Myc target gene. PAI-1 also plays a key role in controlling coagulation and tissue remodeling. PAI-1 limits the production of plasmin and serves to keep fibrinolysis in check. Some physiological functions involving the inhibition of plasmin by PAI-1 include ovulation, cell migration, and epithelial cell differentiation. High PAI-1 levels in cancer indicates poor prognosis for survival of many human cancers, including breast and lung cancers. Pappot et al. (Nov. 29, 2005) Lung Cancer [Epub ahead of print]; Chazaud et al. (2002) American Journal of Pathology 160:237-246.

Collagen triple helix repeat containing 1 (CTHRC1) was identified in a screen for differentially expressed sequences in balloon-injured versus normal arteries. In studies by Pyagay *et al.,* CTHRC1 expression was not detectable in normal arteries. However, on injury it was transiently expressed by fibroblasts of the remodeling adventitia and by smooth muscle cells of the neointima. It was also found in the matrix of calcifying human atherosclerotic plaques. CTHRC1 is a secreted 28-kDa protein that is glycosylated and highly conserved from lower chordates to mammals. A short collagen motif with 12 Gly-X-Y repeats appears to be responsible for trimerization of the protein and this renders the molecule susceptible to cleavage by collagenase. Cthrc1 mRNA expression levels are increased in response to transforming growth factor-beta and bone morphogenetic protein-4. Cell migration assays performed with CTHRC1-overexpressing fibroblasts and smooth muscle cells demonstrate that increased CTHRC1 levels are associated with enhanced migratory ability. Furthermore, CTHRC1 overexpression caused a dramatic reduction in collagen type I mRNA and protein levels. The data of Pyagay *et al.* indicate that the novel molecule CTHRC1 is transiently expressed in the arterial wall in response to injury where it may contribute to vascular remodeling by limiting collagen matrix deposition and promoting cell migration. Pyagay et al. (2005) Circ. Res. 96(2):261-8.

Inhibins are protein hormones that belong to the transforming growth factor β superfamily and are heterodimers consisting of α and β subunits joined by disulfide bonds. The 2 forms of inhibins (i.e., A and B) differ in the type of β subunit (i.e., βA or βB) linked to the α subunit. In women of reproductive age, inhibins are known to be secreted by granulosa cells of the ovary and circulate in blood. Inhibins vary in concentration through the menstrual cycle and during pregnancy, and impact pituitary FSH production, gametogenesis and gestational events. In post menopausal women inhibins fall to very low levels. Recent publications indicate that inhibins are diagnostic markers for ovarian cancer. See for example Robertson et al. (2002) Mol. Cell Endocrinol. 191:97-103; EI-Shalakany et al. (2004) J. Obstet. Gynaecol. Res. 30: 155-161.

Plasminogen activator urokinase-receptor (PLAU-R or UPAR) is a cell surface glycoprotein with a molecular weight of approximately 60 kDa that is attached by its carboxy-terminal end to the cell membrane by GPI linkage. PLAU-R serves as a specific receptor for the serine protease urokinase plasminogen activator (uPA) that is involved in basement membrane/extracellular matrix remodeling in both normal and pathological processes. Soluble PLAU-R, released from the cell surface, has been reported to be at elevated concentrations in serum in several types of human cancer including colorectal, breast and ovarian cancer. See for example Sier et al. (1998) Cancer Res. 58:1843-1849; and Begum et al. (2004) Anticancer Research 24:1981-1986.

Prolactin is a 198-amino acid, 23kDa protein hormone secreted in significant quantities by the anterior pituitary gland. In concert with estrogen, prolactin plays an important role in the initiation of mammary gland growth and in lactation. In addition, prolactin is thought to have a significant role in cell growth and immune function. A recent report using a commercially available ELISA kit reported that prolactin levels in serum were significantly elevated in ovarian cancer. See generally Mor et al. (2005) Proc. Natl Acad Sci. 102: 7677-7682.

Kallikreins are a subgroup of serine proteases having diverse physiological functions. They are clustered as a group on chromosome 19q13. Growing evidence suggests that many kallikreins are implicated in carcinogenesis and some have potential as novel cancer and other disease biomarkers.

KLK-6 is one of the fifteen kallikrein subfamily members located in a cluster on chromosome 19. The encoded enzyme is regulated by steroid hormones. In tissue culture, the enzyme has been found to generate amyloidogenic fragments from the amyloid precursor protein, suggesting a potential for involvement in Alzheimer's disease. KLK-6 has been verified as a secreted protein, and found to be elevated in ovarian cancer patients. Thus, it has been hypothesized to have value as a serological marker for this disease.

The human kallikrein 10 gene (KLK10, also known as normal epithelial cell specific 1 gene (NES-1)) is a member of the kallikrein gene family. The gene product is a secreted serine protease whose concentration in various biological fluids is known to be altered in some disease states. In particular, KLK10 is expressed in epithelial cells of the ovary and this expression has been reported to be elevated in serum of patients with ovarian cancer. Thus, KLK10 may serve as a serum biomarker for ovarian cancer. See for example Liu-Ying et al. (2003) Cancer Res. 63:907-811; Yousef and Diamandis (2003) Thromb Haemost. 90:7-16.

Secretory Leukocyte Protease Inhibitor (SLPI) is a protein that was first isolated in human parotid gland secretions. Subsequent studies have shown that the SLPI protein is also present in saliva and numerous mucosal surfaces such as those of the lung, nasal passages, cervix, and seminal vesicles.

SLPI is believed to act as a defense against chronic lung ailments because it is a potent inhibitor of neutrophil elastase, whose presence is heightened in chronic inflammatory lung diseases and which can destroy many of the components of lung tissue. SLPI also inhibits the release of histamine from mast cells, so it is also active in the allergic response. Protection of fetal membranes and cervical tissue is also thought to be a function of SLPI.

The gene which encodes the SLPI protein has been found to be up-regulated in ovarian cancer, and a significant difference has been found between the elevated levels of SLPI in the serum of patients with malignant ovarian cancer as opposed to the levels found in patients with benign ovarian cysts or normal patients. The SLPI protein has a mass of 12 kDa, is non-glycosylated, hydrophobic, and cationic. See generally Hollander et al. (2003) Cancer Cell International 3:14; Helmig et al. (1995) Eur. J. Obstet. Gynecol. Reprod. Biol. 59(1):95-101; Hough et al. (2001) Cancer Research 61: 3869-3876; and Tsukishiro et al. (2005) Gynecologic Oncology 96: 516-519.

Alpha-1 anti-trypsin (AAT) is an acute phase protein synthesized by the liver and the principal serum inhibitor of proteolytic enzymes such as trypsin, chymotrypsin, plasmin and thrombin. In an inflammatory reaction the serum concentration of AAT may be elevated as much as 3-4 fold. The molecule exists as a number of genetic variants with a monomer molecular weight of 40-50 kDa. The mean normal serum concentration (mg/ml) has been reported to be 2.21±0.35 and 2.14±0.37. Interest in AAT as an ovarian cancer biomarker resulted from an in-house 2D gel electrophoresis/mass spec. study that indicated that ART was elevated in the serum of ovarian cancer patients. See for example Song et al. (1994) J. Affect. Disorders 30:283-288; Ledue et al. (1993) Clin Chim. Acta 223: 73-28.

Although the above biomarkers have been discussed in detail, any biomarker whose overexpression is selective for ovarian cancer can be used to practice the invention, including biomarkers known in the art and those not yet identified. Such biomarkers include genes and proteins that are, for example, involved in defects in DNA replication/cell cycle control, cell growth and proliferation, escape from apoptosis, angiogenesis or lymphogenesis, or the mechanisms of cancer cell motility and invasion.

Of particular interest are biomarkers that are selectively overexpressed in early-stage ovarian cancer. By "selectively overexpressed in early-stage ovarian cancer" is intended that the biomarker of interest is overexpressed in stage 1 or stage 2 ovarian cancer states but is not overexpressed in normal samples or in conditions classified as nonmalignant, benign, and other conditions that are not considered to be clinical disease. One of skill in the art will appreciate that early-stage ovarian cancer biomarkers include those genes and proteins specific for ovarian cancer that are initially overexpressed in stage 1 or stage 2 and whose overexpression persists throughout the advanced stages of the disease, as well as biomarkers that are only overexpressed in stage 1 or stage 2 ovarian cancer. Detection of expression of biomarkers that are selectively overexpressed in early-stage ovarian cancer may permit the earlier detection and diagnosis of ovarian cancer and, accordingly, improve patient prognosis.

The methods of the invention comprise detecting the expression of a plurality of biomarkers. As used herein, a "plurality" of biomarkers refers to 2, 3, 4, 5, 6, 7, 8, 9, 10 or more biomarkers. In particular, when the two-step screening method of the invention is used to identify patients having an increased likelihood of having ovarian cancer, a plurality of biomarkers may refer to the detection of at least one biomarker during the first assay step and at least one additional biomarker during the second assay step. One of skill in the art will also recognize that a panel of biomarkers can be used to identify patients with an increased likelihood of having ovarian cancer in accordance with the present methods. A panel of biomarkers may comprise any number or combination of biomarkers of interest. In some embodiments, a panel comprising a plurality of biomarkers selected from the group consisting of HE4, CA125, Glycodelin, MMP-7, Muc-1, PAI-1, CTHRC1, inhibin, PLAU-R, prolactin, KLK-10, KLK-6, and SLPI, alpha-1 anti-trypsin (art). In other embodiments, a panel of biomarkers selected from the subset of biomarkers comprising CA125, HE4, glycodelin, MMP-7, SLPI, PLAU-R, Muc-1, inhibin A, and PAI-1 is provided.

Any female patient or patient population may be assessed using the screening and diagnostic methods of the invention. For example, the methods disclosed herein may be performed on the general female patient population, as well as on the narrower population of post-menopausal women. The term "post-menopausal" is understood by those of skill in the art. In particular embodiments, post-menopausal generally refers to, for example, women over the age of 55. In particular embodiments, the screening methods are performed routinely (e.g., annually, every two years, etc.) on the general female population. Regular screening of patients may begin, for example, at the onset of menses, at age 30, or at the beginning of menopause. Screening of the high-risk patient population, as defined herein below, will typically be performed on a routine basis independent of patient age. Patients who are both asymptomatic and symptomatic (i.e., displaying characteristic symptoms of ovarian cancer, such as pelvic or abdominal pain or swelling) can be assessed for an increased likelihood of having ovarian using the screening and diagnostic methods of the invention. Women that are at a low-risk of developing ovarian and those that are considered high-risk based on clinical and family history risk factors may also be assessed using the present methods. Patients considered "high-risk" based on such clinical and family history risk factors include but are not limited to patients living with breast cancer, colon cancer, or breast/ovarian syndrome, women with a first-degree relative with ovarian cancer (e.g., mother, daughter, or sister), patients positive for at least one breast cancer gene (BRCA 1 or 2), and women suffering from HNPCC (i.e., Hereditary non-polyposis colorectal cancer).

In one aspect of the invention, the target population for the screening and diagnostic methods is the group of asymptomatic patients classified as high-risk on the basis of, for example, the above-referenced clinical and familial risk factors. This high-risk, asymptomatic population represents more than 8 million women in the U.S. It is recognized that the methods, compositions, and kits of the invention will be of particular utility to patients having an enhanced risk of developing ovarian cancers and to their physicians. Patients recognized in the art as having an increased risk of developing ovarian cancers include, for example, patients having a familial history of ovarian cancer and patients of advancing age (i.e., typically women over 55 years of age).

A number of clinical conditions or characteristics not directly related to ovarian cancer may exist in the patient populations tested in accordance with the methods of the invention, thereby interfering with the results of the screening and diagnostic methods disclosed herein. Such clinical conditions and characteristics are referred to herein as "interfering substances and pathologies" and include but are not limited to pregnancy (first trimester), breast cancer, chronic hepatitis, colon cancer, oral contraceptive therapy, coronary artery disease, deep vein thrombosis, diabetes, endometriosis, hormone replacement therapy, menstruation, multiple myeloma, ovarian cysts/polycystic disease, polymyalgia, polymyositis, rectal cancer, rheumatoid arthritis, systemic lupus rythematosus (SLB), and warfarin treatment. Additional exemplary interfering pathologies include uterine conditions (e.g., myomas, adenomyosis, and endometrial cancer), ovarian conditions (e.g., benign growths such as functional cysts, theca-lutein cysts, pregnancy luteoma, sclerocystic ovaries, serous cystadenoma, mucinous cystadenoma, cystic teratoma, fibroma, thecoma, and Brenner tumor; neoplasms; and malignant conditions such as cystadenocarcinoma and adenocarcinoma), fallopian tube conditions (e.g., tubo-ovarian abscess, hydrosaplinx, parovarian cyst, ectopic pregnancy, and cancer of the fallopian tubes), bowel conditions (e.g., distention with gas and/or feces, diverticulitis, ileitis, appendicitis, and colon cancer), and other miscellaneous conditions (e.g., distended bladder, pelvic kidney, urachal cyst, abdominal wall hematoma, abdominal wall abscess, and retroperitoneal neoplasms, such as lymphoma, sarcoma, and teratoma. In particular embodiments, the biomarkers, threshold expression levels, and mathematical models used in the screening and diagnostic methods described herein will be selected so as to minimize the effects of interfering substances and pathologies on the performance (i.e., the specificity, sensitivity, PPV, and NPV) of the claimed methods.

By "body sample" is intended any sampling of cells, tissues, or bodily fluids from a patient in which expression of a biomarker can be detected. Examples of such body samples include but are not limited to blood (e.g., whole blood, blood serum, blood having platelets removed, etc.), lymph, ascitic fluids, urine, gynecological fluids (e.g., ovarian, fallopian, and uterine secretion, menses, etc.), biopsies, and fluids obtained during laparoscopy. Body samples may be obtained from a patient by a variety of techniques including, for example, by venipuncture, by scraping or swabbing an area, or by using a needle to aspirate bodily fluids or tissues. Methods for collecting various body samples are well known in the art. In particular embodiments, the body sample comprises blood or serum. The present inventors have recognized that the methods for the collection and storage of blood samples, more particularly serum samples, affect the performance of the methods disclosed herein. Several studies indicate that body sample, particularly serum sample, collection and storage methods are critical to achieve acceptable assay performance. See, for example, Diamandis (2004) J. Natl. Cancer Institute 95:353-356 and Thavasu et al. (1992) J. Immunol. Meth. 153:115-124. which is herein incorporated reference in its entirety. An exemplary method for serum collection and storage is provided in Example 1.

Any methods available in the art for the detection biomarker expression can be used to practice the invention. The expression of a biomarker of the invention can be detected on a nucleic acid level or a protein level. In order to determine overexpression, the body sample to be examined may be compared with a corresponding body sample that originates from a healthy person. That is, the "normal" level of expression is the level of expression of the biomarker in a body sample from a patient that is not afflicted with ovarian cancer. Such a sample can be present in standardized form. In some embodiments, determination of biomarker overexpression requires no comparison between the body sample and a corresponding body sample that originates from a normal person.

Any biomarker or combination of biomarkers of the invention, as well as any known ovarian cancer biomarkers, may be used in the methods, compositions, and kits of the present invention. In general, it is preferable to use biomarkers for which the difference between the level of expression of the biomarker in a body sample from a patient afflicted with ovarian cancer and the level of expression of the same biomarker in a "normal" body sample (i.e., from an ovarian cancer free patient) is as great as possible. Although this difference can be as small as the limit of detection of the method for assessing expression of the biomarker, it is preferred that the difference be at least greater than the standard error of the assessment method, and optimally a difference of at least 2, 3, 4, 5, 6, 7, 8, 9, 10, 15, 20, 25-fold or greater than the level of expression of the same biomarker in a normal body sample. The "normal" level of expression of a biomarker of the invention may be determined by assessing expression of the biomarker in a body sample obtained from a non-ovarian cancer afflicted patient. Alternatively, and particularly as further information becomes available as a result of the routine performance of the methods described herein, average values for biomarker expression levels may be used.

As described above, in some aspects of the invention, a threshold level of expression of a particular biomarker, above which the biomarker is considered to be overexpressed in a patient sample, may be established. Methods for selecting the threshold value include but are not limited to analysis of the ROC plot for the biomarker or analysis of the biomarker expression level for a normal patient population. Exemplary threshold or "cutoff" values include (1) the mean expression level plus two times the standard deviation, as determined from a population of normal patient samples and (2) expression levels selected from the ROC curve that represent the highest value of sensitivity plus specificity. The threshold level of expression for a particular biomarker may be determined relative to the expression level in a normal patient population. Persons of skill in the art will appreciate that other methods for selecting appropriate threshold expression values can be used to practice the invention.

Methods for detecting biomarkers of the invention comprise any methods that determine the quantity or the presence of the biomarkers either at the nucleic acid or protein level. Such methods are well known in the art and include but are not limited to western blots, northern blots, southern blots, BLISA, immunoprecipitation, immunofluorescence, flow cytometry, immunocytochemistry, multiplex bead-based immunoassays, nucleic acid hybridization techniques, nucleic acid reverse transcription methods, and nucleic acid amplification methods. In particular embodiments, overexpression of a biomarker is detected on a protein level using, for example, antibodies that are directed against specific biomarker proteins. These antibodies can be used in various methods such as Western blot, ELISA, multiplex bead-based immunoassay, immunoprecipitation, or immunocytochemistry techniques. The multiplex bead-based assays used to practice the present invention include but are not limited to the Luminex technology described in U.S. Patent Nos. 6,599,331, 6,592,822, and 6,268,222, all of which are herein incorporated by reference in their entirety. In particular embodiments, the Luminex LabMAP© system is utilized, as described in International Publication No. WO 2005/016126, which is herein incorporated by reference it its entirety.

In some embodiments of the invention, antibodies specific for biomarker proteins are utilized to detect the expression of a plurality of biomarker proteins in a body sample in order to identify patients with an increased likelihood of having ovarian cancer. The method comprises obtaining a body sample from a patient, particularly a serum sample, contacting the body sample with antibodies directed to a plurality of biomarkers that are selectively overexpressed in ovarian cancer, and detecting antibody binding to determine if the biomarkers are overexpressed in the patient sample. In the screening methods of the invention, overexpression of the biomarkers is indicative of an increased likelihood of having ovarian cancer. Patients classified by the present screening methods as having an increased likelihood of having ovarian cancer are subjected to further diagnostic testing to detect the presence of ovarian cancer. Female patients identified by the present screening methods for ovarian cancer that are determined to not be currently suffering from ovarian cancer are regularly monitored in order to potentially detect ovarian cancer at an earlier stage.

As described above, in particular aspects of the invention, the screening methods for identifying patients with an increased likelihood of having ovarian cancer may comprise a two-step analysis. That is, the method comprises performing a first assay step comprising detecting the expression of a first biomarker or a first panel of biomarkers in a body sample and determining if the first biomarker or panel of biomarkers is overexpressed. If a positive result is obtained in the first assay step (i.e., the first biomarker or panel of biomarkers is overexpressed in the body sample), the method further comprises performing a second assay step comprising detecting the expression of a second biomarker or a second panel of biomarkers and determining if the second biomarker or panel of biomarkers is overexpressed. Overexpression of both the first and second biomarkers or panels of biomarkers (i.e., a positive result in both assay steps) is indicative of an increased likelihood of having ovarian cancer. In particular embodiments, detection of expression of the biomarkers in the first and second assay steps is performed at the protein level and comprises contacting the body sample, more particularly a serum sample, with antibodies specific for the particular biomarkers of interest and detecting antibody binding. In certain methods, biomarker protein expression is detected using an ELISA or multiplex bead-based immunoassay format.

In the "two-step" screening methods of the invention, the biomarker or panel of biomarkers used in the first step may be employed to maximize sensitivity and NPV. That is, the first screening step may be designed to maximize the number of true negatives classified as negative by the methods of the invention, thereby eliminating a significant percentage of true negatives from further analysis. Therefore, the patient population classified as positive by the first assay step will be enriched in true positive patients. The second screening step in the two-step methods may be designed to maximize PPV and specificity, while maintaining a reasonable sensitivity, in order to identify women with the highest likelihood of having ovarian cancer. Specificity, sensitivity, PPV, and NPV values may be determined for each individual assay step in the method, as described above. When a two-step screening method is used, combined sensitivity, specificity, PPV, and NPV values may determined for the method as a whole. In particular embodiments, the two-step method for identifying patients having an increased likelihood of having ovarian cancer will have a combined sensitivity of at least 90%, a combined specificity of at least 98%, a combined PPV of at least 10%, and a combined NPV of at least 99.9%. One of skill in the art will further appreciate that while two-step screening methods have been described in detail, similar screening methods comprising 3, 4, 5, or more assay steps are also encompassed by the present invention. Such follow-on assay steps may rule out other diseases, such as cardiovascular conditions, from the presence of ovarian cancer.

In some embodiments of the invention, algorithms or mathematical models may be applied to develop "test rules" for determining when a patient sample is "positive" (i.e., indicative of an increased likelihood of having ovarian cancer). For example, when expression of HE4, CA125, PLAU-R, glycodelin, Muc-1, and PAI-1 is detected, the screening test is considered positive if (1) any three of HE4, CAI25, glycodelin, PAI-1, and PLAU-R are positive (i.e., overexpressed) or if (2) HE4 and CA125 are both overexpressed; otherwise, the test is considered negative. In a further example, the test is considered positive if (1) any three of HE4, CA125, glycodelin, MMP-7, and PLAU-R are positive (i.e., overexpressed) or if (2) HE4 is overexpressed and also any one of CA125, glycodelin, MMP-7, or PLAU-R is positive; otherwise, the test is deemed negative. One of skill in the art will appreciate that a variety of such test rules can be-developed and applied in the present methods for identifying patients with an increased likelihood of having ovarian cancer such as logistical regression. Other mathematical models that can be applied are described in Zhou et al. (2002) Statistical Methods in Diagnostic Medicine (Wiley, New York), which is herein incorporated by reference in ts entirety.

In other aspects of the invention, methods for diagnosing ovarian cancer in a patient comprise obtaining a body sample from a patient, contacting the body sample with antibodies directed to a plurality of biomarkers that are selectively overexpressed in ovarian cancer, and detecting antibody binding to determine if the biomarkers are overexpressed in the patient sample. In the diagnostic methods of the invention, overexpression of the biomarkers is indicative of the presence of ovarian cancer.

In still other embodiments of the invention, methods for assessing the efficacy of a therapy for treating ovarian cancer in a patient are provided. Such methods typically comprise comparing the level of expression of a plurality of biomarkers of the invention in a first patient body sample procured prior to the initiation of therapy with that from a second sample obtained following administration of at least a portion of the therapy. A significantly lower level of expression of the biomarkers in the second patient sample relative to that of the first sample obtained prior to the initiation of the therapy is a positive indication of the efficacy of the therapy for treating ovarian cancer in the patient, whereas a significantly higher level of expression of the biomarkers in the second sample is a negative indication of the efficacy of the therapy. As used herein, a "positive indication of the efficacy of the therapy" means that the therapy is producing beneficial results in the treatment of ovarian cancer (e.g., tumor regression, etc.). A "negative indication of the efficacy of the therapy" is intended to mean that the therapy is not having beneficial effects with respect to treatment of ovarian cancer. A negative indication of the efficacy of the particular treatment may be related to, for example, the dosage. At higher dosages, the therapy may be efficacious.

One of skill in the art will recognize that in these methods the term "therapy" includes any therapy for treating ovarian cancer, including but not limited to chemotherapy, radiation therapy, surgical removal of tumor tissue, gene therapy, and biologic therapy. The methods of the invention may be used to evaluate a patient before, during, and after therapy to evaluate, for example, a reduction in tumor burden.

The invention additionally provides a monitoring method for assessing the regression or progression of ovarian cancer in a patient comprising detecting in a first patient sample at a first time point the level of expression of a plurality of biomarkers of the invention, repeating this analysis with a second patient sample obtained at a later time point, and comparing the level of expression of the biomarkers at the two time points. A significantly higher level of expression of the biomarkers in the patient body sample at the later time point indicates that the ovarian cancer has progressed, whereas a significantly lower level of expression is an indication that the ovarian cancer has regressed. As used herein, "regression of ovarian cancer" is intended to mean that the condition of the patient with respect to ovarian cancer has improved, as characterized by, for example, decreased tumor size. "Progression of ovarian cancer" means that the condition of the patient with respect to ovarian cancer has worsened, as characterized by, for example, increased tumor size, metastasis, etc. The meanings of the terms "regression" and "progression" with respect to disease states will be understood by those of skill in the art.

In certain aspects of the invention, biomarker expression is detected at the protein level using antibodies. The terms "antibody" and "antibodies" broadly encompass naturally occurring forms of antibodies and recombinant antibodies such as single-chain antibodies, chimeric and humanized antibodies and multi-specific antibodies as well as fragments and derivatives of all of the foregoing, which fragments and derivatives have at least an antigenic binding site. Antibody derivatives may comprise a protein or chemical moiety conjugated to the antibody.

"Antibodies" and "immunoglobulins" (Igs) are glycoproteins having the same structural characteristics. While antibodies exhibit binding specificity to an antigen, immunoglobulins include both antibodies and other antibody-like molecules that lack antigen specificity. Polypeptides of the latter kind are, for example, produced at low levels by the lymph system and at increased levels by myelomas.

The term "antibody" is used in the broadest sense and covers fully assembled antibodies, antibody fragments that can bind antigen ( e.g., Fab', F'(ab)₂, Fv, single chain antibodies, diabodies), and recombinant peptides comprising the foregoing.

The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Antibody fragments" comprise a portion of an intact antibody, preferably the antigen-binding or variable region of the intact antibody. Examples of antibody fragments include Fab, Fab', F(ab')2, and Fv fragments; diabodies; linear antibodies (Zapata et al. (1995) Protein Eng. 8(10):1057-1062); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize 35 readily. Pepsin treatment yields an F(ab')2 fragment that has two antigen-combining sites and is still capable of cross-linking antigen.

"Fv" is the minimum antibody fragment that contains a complete antigen recognition and binding site. In a two-chain Fv species, this region consists of a dimer of one heavy- and one light-chain variable domain in tight, non-covalent association. In a single-chain Fv species, one heavy- and one light-chain variable domain can be covalently linked by flexible peptide linker such that the light and heavy chains can associate in a "dimeric" structure analogous to that in a two-chain Fv species. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (C_{H}1) of the heavy chain. Fab fragments differ from Fab' fragments by the addition of a few residues at the carboxy terminus of the heavy chain CH2 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments that have hinge cysteines between them.

Polyclonal antibodies can be prepared by immunizing a suitable subject (e.g., rabbit, goat, mouse, or other mammal) with a biomarker protein immunogen. The antibody titer in the immunized subject can be monitored over time by standard techniques, such as with an enzyme linked immunosorbent assay (ELISA) using immobilized biomarker protein. At an appropriate time after immunization, e.g., when the antibody titers are highest, antibody-producing cells can be obtained from the subject and used to prepare monoclonal antibodies by standard techniques, such as the hybridoma technique originally described by Kohler and Milstein (1975) Mature 256:495-497, the human B cell hybridoma technique (Kozbor et al. (1983) Immunol. Today 4:72), the EBV-hybridoma technique (Cole et al. (1985) in Monoclonal Antibodies and Cancer- Therapy, ed. Reisfeld and Sell (Alan R. Liss, Inc., Now York, NY), pp. 77-96) or trioma techniques. The technology for producing hybridomas is well known *(see* generally Coligan et al., eds. (1994) Current Protocols in Immunology (John Wiley & Sons, Inc., New York, NY); Galfre et al. (1977) Nature 266:550-52; Kenneth (1980) in Monoclonal Antibodies: A New Dimension In Biological Analyses (Plenum Publishing Corp., NY); and Lerner (1981) Yale J. Biol. Med., 54:387-402).

Alternative to preparing monoclonal antibody-secreting hybridomas, a monoclonal antibody can be identified and isolated by screening a recombinant combinatorial immunoglobulin library (e.g., an antibody phage display library) with a biomarker protein to thereby isolate immunoglobulin library members that bind the biomarkers protein. Kits for generating and screening phage display libraries are commercially available (e.g., the Pharmacia *Recombinant Phage Antibody System,* Catalog No. 27-9400-01; and the Stratagene *SurfzAP9 Phage Display Kit,* Catalog No. 240512). Additionally, examples of methods and reagents particularly amenable for use in generating and screening antibody display library can be found in, for example, U.S. Patent No. 5,223,409; PCT Publication Nos. WO 92/18619; WO 91/17271; WO 92/20791; WO 92/15679; 93/01288; WO 92/02047; 92/09690; and 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J. 12:725-734.

The compositions of the invention further comprise monoclonal antibodies and variants and fragments thereof that specifically bind to biomarker proteins of interest. The monoclonal antibodies may be labeled with a detectable substance as described below to facilitate biomarker protein detection in the sample. Such antibodies find use in practicing the methods of the invention. Monoclonal antibodies having the binding characteristics of the antibodies disclosed herein are also encompassed by the present invention. Compositions further comprise antigen-binding variants and fragments of the monoclonal antibodies, hybridoma cell lines producing these antibodies, and isolated nucleic acid molecules encoding the amino acid sequences of these monoclonal antibodies.

Antibodies having the binding characteristics of a monoclonal antibody of the invention are also provided. "Binding characteristics" or "binding specificity" when used in reference to an antibody means that the antibody recognizes the same or similar antigenic epitope as a comparison antibody. Examples of such antibodies include, for example, an antibody that competes with a monoclonal antibody of the invention in a competitive binding assay. One of skill in the art could determine whether an antibody competitively interferes with another antibody using standard methods.

By "epitope" is intended the part of an antigenic molecule to which an antibody is produced and to which the antibody will bind. Epitopes can comprise linear amino acid residues (i.e., residues within the epitope are arranged sequentially one after another in a linear fashion), nonlinear amino acid residues (referred to herein as "nonlinear epitopes"; these epitopes are not arranged sequentially), or both linear and nonlinear amino acid residues. Typically epitopes are short amino acid sequences, e.g. about five amino acids in length. Systematic techniques for identifying epitopes are known in the art and are described, for example, in U.S. Pat. No. 4,708,871. Briefly, a set of overlapping oligopeptides derived from the antigen may be synthesized and bound to a solid phase array of pins, with a unique oligopeptide on each pin. The array of pins may comprise a 96-well microtiter plate, permitting one to assay all 96 oligopeptides simultaneously, e.g., for binding to a biomarker-specifc monoclonal antibody. Alternatively, phage display peptide library kites (New England BioLabs) are currently commercially available for epitope mapping. Using these methods, the binding affinity for every possible subset of consecutive amino acids may be determined in order to identify the epitope that a given antibody binds. Epitopes may also be identified by inference when epitope length peptide sequences are used to immunize animals from which antibodies are obtained. Epitopes may also be defined by carbohydrate side chains present as either N-linked or O-linked oligosaccharides present on glycoproteins.

Antigen-binding fragments and variants of the monoclonal antibodies disclosed herein are further provided. Such variants will retain the desired binding properties of the parent antibody. Methods for making antibody fragments and variants are generally available in the art For example, amino acid sequence variants of a monoclonal antibody described herein, can be prepared by mutations in the cloned DNA sequence encoding the antibody of interest. Methods for mutagenesis and nucleotide sequence alterations are well known in the art. See, for example, Walker and Gaastra, eds. (1983) Techniques in Molecular Biology (MacMillan Publishing Company, New York); Kunkel (1985) Proa Natl. Acad. Sci. USA 82:488-492; Kunkel et al. (1987) Methods Enzymol. 154:367-382; Sambrook et al. (1989) Molecular Cloning: A Laboratory Manual (Cold Spring Harbor, New York); U.S. Patent No. 4,873,192; and the references cited therein; herein incorporated by reference. Guidance as to appropriate amino acid substitutions that do not affect biological activity of the polypeptide of interest may be found in the model of Dayhoff et al. (1978) in Atlas of Protein Sequence and Structure (Natl. Biomed. Res. Found., Washington, D.C.), herein incorporated by reference. Conservative substitutions, such as exchanging one amino acid with another having similar properties, may be preferred. Examples of conservative substitutions include, but are not limited to, Gly⇔Ala, Val⇔Ile⇔Leu, Asp⇔Glu, Lys⇔Arg, Asn<=>Gln, and Phe⇔Trp⇔Tyr.

In constructing variants of the antibody polypeptide of interest, modifications are made such that variants continue to possess the desired activity, i.e., similar binding affinity to the biomarker. Obviously, any mutations made in the DNA encoding the variant polypeptide must not place the sequence out of reading frame and preferably will not create complementary regions that could produce secondary mRNA structure. See EP Patent Application Publication No. 75,444.

Preferably, variants of a reference biomarker antibody have amino acid sequences that have at least 70% or 75% sequence identity, preferably at least 80% or 85% sequence identity, more preferably at least 90%, 91%, 92%, 93%, 94% or 95% sequence identity to the amino acid sequence for the reference antibody molecule, or to a shorter portion of the reference antibody molecule. More preferably, the molecules share at least 96%, 97%, 98% or 99% sequence identity. For purposes of the present invention, percent sequence identity is determined using the Smith-Waterman homology search algorithm using an affine gap search with a gap open penalty of 12 and a gap extension penalty of 2, BLOSUM matrix of 62. The Smith-Waterman homology search algorithm is taught in Smith and Waterman (1981) Adv. Appl. Math. 2:482-489. A variant may, for example, differ from the reference antibody by as few as 1 to 15 amino acid residues, as few as 1 to 10 amino acid residues, such as 6-10, as few as 5, as few as 4, 3, 2, or even 1 amino acid residue.

With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues, and may be 30, 40, 50, or more amino acid residues. Corrections for sequence identity associated with conservative residue substitutions or gaps can be made (see Smith-Waterman homology search algorithm).

One of skill in the art will recognize that optimization of reagents and conditions, for example, antibody titer and parameters for detection of antigen-antibody binding, is needed to maximize the signal to noise ratio for a particular antibody. Antibody concentrations that maximize specific binding to the biomarkers of the invention and minimize non-specific binding (or "background") will be determined. In particular embodiments, appropriate antibody titers are determined by initially testing various antibody dilutions on patient serum samples. The design of assays to optimize antibody titer and detection conditions is standard and well within the routine capabilities of those of ordinary skill in the art. Some antibodies require additional optimization to reduce background and/or to increase specificity and sensitivity.

Furthermore, one of skill in the art will recognize that the concentration of a particular antibody used to practice the methods of the invention will vary depending on such factors as time for binding, level of specificity of the antibody for the biomarker protein, and method of body sample preparation. Moreover, when multiple antibodies are used in a single sample, the required concentration may be affected by the order in which the antibodies are applied to the sample, i.e., simultaneously as a cocktail or sequentially as individual antibody reagents. Furthermore, the detection chemistry used to visualize antibody binding to a biomarker of interest must also be optimized to produce the desired signal to noise ratio.

Detection of antibody binding can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include ¹²⁵I, ¹³¹I, ³⁵S, or ³H_{.}

The antibodies used to practice the invention are selected to have high specificity for the biomarker proteins of interest. Methods for making antibodies and for selecting appropriate antibodies are known in the art. See, for example, Celis, ed. (in press) Cell Biology & Laboratory Handbook, 3rd edition (Academic Press, New York), which is herein incorporated in its entirety by reference. In some embodiments, commercial antibodies directed to specific biomarker proteins may be used to practice the invention. The antibodies of the invention may be selected on the basis of desirable staining of cytological, rather than histological, samples. That is, in particular embodiments the antibodies are selected with the end sample type (i.e., serum samples) in mind and for binding specificity.

In other embodiments, the expression of a biomarker of interest is detected at the nucleic acid level. Nucleic acid-based techniques for assessing expression are well known in the art and include, for example, determining the level of biomarker mRNA in a body sample. Many expression detection methods use isolated RNA. Any RNA isolation technique that does not select against the isolation of mRNA can be utilized for the purification of RNA from serum samples (see, e.g., Ausubel et al., ed., (1987-1999) Current Protocols in Molecular Biology (John Wiley & Sons, New York). Additionally, large numbers of blood, serum, or tissue samples can readily be processed using techniques well known to those of skill in the art, such as, for example, the single-step RNA isolation process of Chomczynski (1989, U.S. Pat. No. 4,843,155).

The term "probe" refers to any molecule that is capable of selectively binding to a specifically intended target biomolecule, for example, a nucleotide transcript or a protein encoded by or corresponding to a biomarker. Probes can be synthesized by one of skill in the art, or derived from appropriate biological preparations. Probes may be specifically designed to be labeled. Examples of molecules that can be utilized as probes include, but are not limited to, RNA, DNA, proteins, antibodies, and organic molecules.

Isolated mRNA can be used in hybridization or amplification assays that include, but are not limited to, Southern or Northern analyses, polymerase chain reaction analyses and probe arrays. One method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to an mRNA or genomic DNA encoding a biomarker of the present invention. Hybridization of an mRNA with the probe indicates that the biomarker in question is being expressed.

In one embodiment, the mRNA is immobilized on a solid surface and contacted with a probe, for example by running the isolated mRNA on an agarose gel and transferring the mRNA from the gel to a membrane, such as nitrocellulose. In an alternative embodiment, the probe(s) are immobilized on a solid surface and the mRNA is contacted with the probe(s), for example, in an Affymetrix gene chip array. A skilled artisan can readily adapt know mRNA detection methods for use in detecting the level of mRNA encoded by the biomarkers of the present invention.

An alternative method for determining the level of biomarker mRNA in a sample involves the process of nucleic acid amplification, e.g., by RT-PCR (the experimental embodiment set forth in Mullis, 1987, U.S. Pat. No. 4,683,202), ligase chain reaction (Barany (1991) Proc. Natl. Acad. Set. USA 88:1899;193), self sustained sequence replication (Guatelli et al. (1990) Pro. Natl. Acad. Sci. USA 87:1874-1878), transcriptional amplification system (Kwoh et al. (1989) Proc. Natl. Acad. Sci. USA 86:1173-1177), Q-Beta Replicase (Lizardi et al. (1988) Bio/Technology 6:1197), rolling circle replication (Lizardi et al., U.S. Pat. No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. In particular aspects of the invention, biomarker expression is assessed by quantitative fluorogenic RT-PCR (i.e., the TaqMan® System). Such methods typically utilize pairs of oligonucleotide primers that are specific for the biomarker of interest. Methods for designing oligonucleotide primers specific for a known sequence are well known in the art.

Biomarker expression levels of RNA may be monitored using a membrane blot (such as used in hybridization analysis such as Northern, Southern, dot, and the like), or microwells, sample tubes, gels, beads or fibers (or any solid support comprising bound nucleic acids). See U.S. Patent Nos. 5,770,722, 5,874,219, 5,744,305, 5,677,195 and 5,445,934, which are incorporated herein by reference. The detection of biomarker expression may also comprise using nucleic acid probes in solution.

In one embodiment of the invention, microarrays are used to detect biomarker expression. Microarrays are particularly well suited for this purpose because of the reproducibility between different experiments. DNA microarrays provide one method for the simultaneous measurement of the expression levels of large numbers of genes. Each array consists of a reproducible pattern of capture probes attached to a solid support Labeled RNA or DNA is hybridized to complementary probes on the array and then detected by laser scanning. Hybridization intensities for each probe on the array are determined and converted to a quantitative value representing relative gene expression levels. See, U.S. Pat Nos. 6,040,138, 5,800,992 and 6,020,135, 6,033,860, and 6,344,316, which are incorporated herein by reference. High-density oligonucleotide arrays are particularly useful for determining the gene expression profile for a large number of RNA's in a sample.

Techniques for the synthesis of these arrays using mechanical synthesis methods are described in, e.g., U.S. Patent No. 5,384,261, incorporated herein by reference in its entirety for all purposes. Although a planar array surface is preferred, the array may be fabricated on a surface of virtually any shape or even a multiplicity of surfaces. Arrays may be peptides or nucleic acids on beads, gels, polymeric surfaces, fibers such as fiber optics, glass or any other appropriate substrate, see U.S. Pat. Nos. 5,770,358, 5,789,162, 5,708,153, 6,040,193 and 5,800,992, each of which is hereby incorporated in its entirety for all purpose. Arrays may be packaged in such a manner as to allow for diagnostics or other manipulation of an all-inclusive device. *See,* for example, U.S. Pat. Nos. 5,856,174 and 5,922,591 herein incorporated by reference.

In one approach, total mRNA isolated from the sample is converted to labeled cRNA and then hybridized to an oligonucleotide array. Each sample is hybridized to a separate array. Relative transcript levels may be calculated by reference to appropriate controls present on the array and in the sample.

Kits for practicing the screening and diagnostic methods of the invention are further provided. By "kit" is intended any manufacture (e.g., a package or a container) comprising at least one reagent, e.g., an antibody, a nucleic acid probe, etc. for specifically detecting the expression of a biomarker of the invention. The kit may be promoted, distributed, or sold as a unit for performing the methods of the present invention. Additionally, the kits may contain a package insert describing the kit and methods for its use.

In particular embodiments, kits for use in screening methods for identifying patients with an increased likelihood of having ovarian cancer comprise a plurality of antibodies directed to biomarker proteins of interest, particularly wherein the biomarkers are selected from the group consisting of HE4, CA125, glycodelin, MMP-7, Muc-1, Pat- 1, CTHRC1, inhibin A, PLAU-R, prolactin, KLK-10, KLK-6, SLPI, and alpha-1 anti-trypsin. Chemicals for the detection of antibody binding to the biomarker may also be included in the kit. Other reagents for detecting expression of biomarkers using antibodies in an ELISA or multiplex bead-based immunoassay format may be further included in a kit of the invention. In other embodiments, kits for use in the screening methods disclosed herein comprise nucleic acid probes for the detection of expression of a plurality of biomarkers of interest in a body sample, particularly wherein the biomarkers are selected from the group consisting of HE4, CA125, glycodelin, MMP-7, Muc-1, Pat-1, CTHRC1, inhibin A, PLAU-R, prolactin, KLK-10, KLK-6, SLPI, and alpha-1 anti-trypsin.

In certain aspects of the invention, kits for performing the two-step screening method for identifying patients having an increased likelihood of having ovarian cancer are provided. Kits may be designed to perform the two-step screening method as two separate tests. Such kits include reagents for performing a single assay step (i.e., the first or second assay step). In other embodiments, the kits comprise reagents so that the two-step method may be performed as a single test (i.e., include reagents for both the first and second assay steps). Further kits encompassed by the invention find use in practicing the single-step screening method described herein above. The kits may further comprise descriptions of algorithms or mathematical models to be applied with the one-step or two-step screening methods, as well as automated platforms that implement the algorithms/mathematical models with little or no human intervention. The kits may be further provided such that each step in the two-step method is performed in an automated, semi-automated, or manual fashion.

Kits for diagnosing ovarian cancer are also provided. Such kits may include antibodies or nucleic acid probes that are specific for detection of a plurality of biomarker that are selectively overexpressed in ovarian cancer, more particularly HB4, CA125, glycodelin, MMP-7, Muc-1, PAI-1, CTHRC1, inhibin A, PLAU-R, prolactin, KLK-10, KLK-6, SLPI, and alpha- anti-trypsin.

One of skill in the art will further appreciate that any or all steps in the screening and diagnostic methods of the invention could be implemented by personnel or, alternatively, performed in an automated fashion. That is, the methods can be performed in an automated, semi-automated, or manual fashion. Furthermore, the methods disclosed herein can also be combined with other methods known or later developed to permit a more accurate identification of patients having an increased likelihood of having ovarian cancer or a more reliable diagnosis of ovarian cancer.

The article "a" and "an" are used herein to refer to one or more than one (i.e., to at least one) of the grammatical object of the article. By way of example, "an element" means one or more element.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation:

### EXPERIMENTAL

### Example 1: Exemplary Method for Collection of Blood, Processing of Blood Into Serum. Serum Storage Conditions and Serum Shipping Conditions

### Blood Collection

A sufficient quantity of blood was drawn by venipuncture into a Becton Diclcinson Vacutainer red-topped collection tubes containing no additive (catalog number 366430). The lot number of the blood collection tube was recorded. Whole blood was not refrigerated or frozen. Processing of the whole blood to serum began immediately after collection of the blood.

### Serum Preparation Procedure

Blood collection tubes were placed upright in a rack and stored at room temperature. The blood was allowed to clot for a minimum of 60 minutes and a maximum of 90 minutes from time of collection. Blood was thoroughly clotted before centrifugation. The time required for complete clotting for each sample was recorded.

After blood was thoroughly clotted, the samples were centrifuged at 1300xg for 10 minutes using either a swing-head or fixed-angle centrifuge rotor. Tubes were carefully removed from the centrifuge to avoid disturbing the red blood cells on the bottom.

The stopper was removed from each tube without disturbing the cell pellet. The serum from each donor was combined into a labeled tube using a disposable transfer pipette without disturbing the cell layer or allowing any cells into the pipette. If cells were disturbed during transfer, the samples were re-centrifuged as described above.

Serum samples from each donor were mixed by gently inverting the tube five times. One mL aliquots were placed in labeled tubes and frozen at -20°C to -80°C. Serum was not stored at room temperature for more than two hours or at 2-8°C for more than eight hours prior to freezing.

### Example 2: Ovarian Biomarker Selection Study - Individual Biomarker Analysis

The purpose of the biomarker selection study was to evaluate the initial clinical utility of a set of fourteen candidate ovarian cancer biomarkers. The premise of the study was to be able to identify and quantitate the amount of each biomarker in a patient's serum. Biomarker protein expression in serum samples was detected using co*mmercial or novel biomarker-specific antibodies in an ELISA format. Fourteen target biomarkers were analyzed, as described below in Table 1:

**Table 1. Candidate markers**

| | |
|---|---|
| HE4 | CA125 |
| SLPI | Glycodelin |
| KLK6 | MMP7 |
| KLK 10 | PLAU-R |
| CTHRC1 | Inhibin |
| PAI-1 | Prolactin |
| MUC1 | Alpha-1 anti-lrypsin |

### Tar get populati.on and demographic of the study

In evaluations of this type, it is essential that the samples tested are representative of the target clinical population and that normal controls are demographically matched. A total of 900 patient serum samples were analyzed in this study. 200 of the samples were from ovarian cancer patients in various stages of the disease (i.e., the 200 samples were evenly split among Stage 1, 2, 3 ovarian cancer samples). A total of 500 normal sera were used in the study. 104 of the normal serum samples were from post-menopausal women (set as women over the age of 55 for the present study) to establish the baseline levels of each biomarker and to calculate the appropriate threshold cut-off values. The remaining 396 normal serum samples were evenly split between pre-menopausal women under the age of 55 and post-menopausal women over the age of 55. This distribution permitted the investigation of any age or hormone-related patient distributions. The remaining 200 samples in the study were collected from donors with various conditions/diseases, as described herein above (i.e., "interfering conditions" or "interfering pathologies"). The inclusion of these samples allowed for the analysis of any interfering pathologies that may affect individual biomarker performance. Summaries of the patient populations analyzed and study demographics are provided in Tables 2-4.

**Table 2: Demographics of sample cases in the study**

| Characteristics | Ovarian Cancer | Study Normals | Interfering Pathologies | Threshold Normals |
|---|---|---|---|---|
| Total number of samples | 200 | 396 | 200 | 104 |
| Age at diagnosis (years) | | | | |
| Mean (std) | 55.5 (11.5) | 55.3 (9.7) | 57.0(18.3) | 63.0 (6.9) |
| Range | 19-81 | 40-84 | 20-97 | 41-80 |
| Age group distribution | | | | |
| <=55 | 100 (50%) | 201 (50.8%) | 92 (46%) | 2 (1.9%) |
| >55 | 100(50%) | 195 (49.2%) | 108 (54%) | 102 (98.1%) |
| | | | | |
| Race, n | | | | |
| African-American | 10(5%) | 3 (1%) | 12 (6%) | 0 |
| Caucasian | 183(92%) | 389 (99%) | 185(92.5%) | 98 (100%) |
| Other | 6(3%) | 1(<1%) | 3(1.5%) | 0 |
| Missing | 1 | 3 | 0 | 6 |

**Table 3: Clinical Characteristics of Ovarian Cancer Patients in Study**

| | Frequency | Percent |
|---|---|---|
| Ovarian Cancer Stage, n | 200 | |
| 1 | 67 | 33.5% |
| 2 | 66 | 33% |
| 3 | 67 | 33.5% |
| Histological Classification, n | 197 | |
| | | |
| Clear Cell | 10 | 5% |
| Endometrioid | 39 | 19% |
| Serous | 23 | 12% |
| Mucinous | 36 | 18% |
| Adenocarcinoma | 49 | 25% |
| Papillary | 25 | 13% |
| Unclassified | 13 | 7% |
| Stromal Sarcoma | 2 1% | |

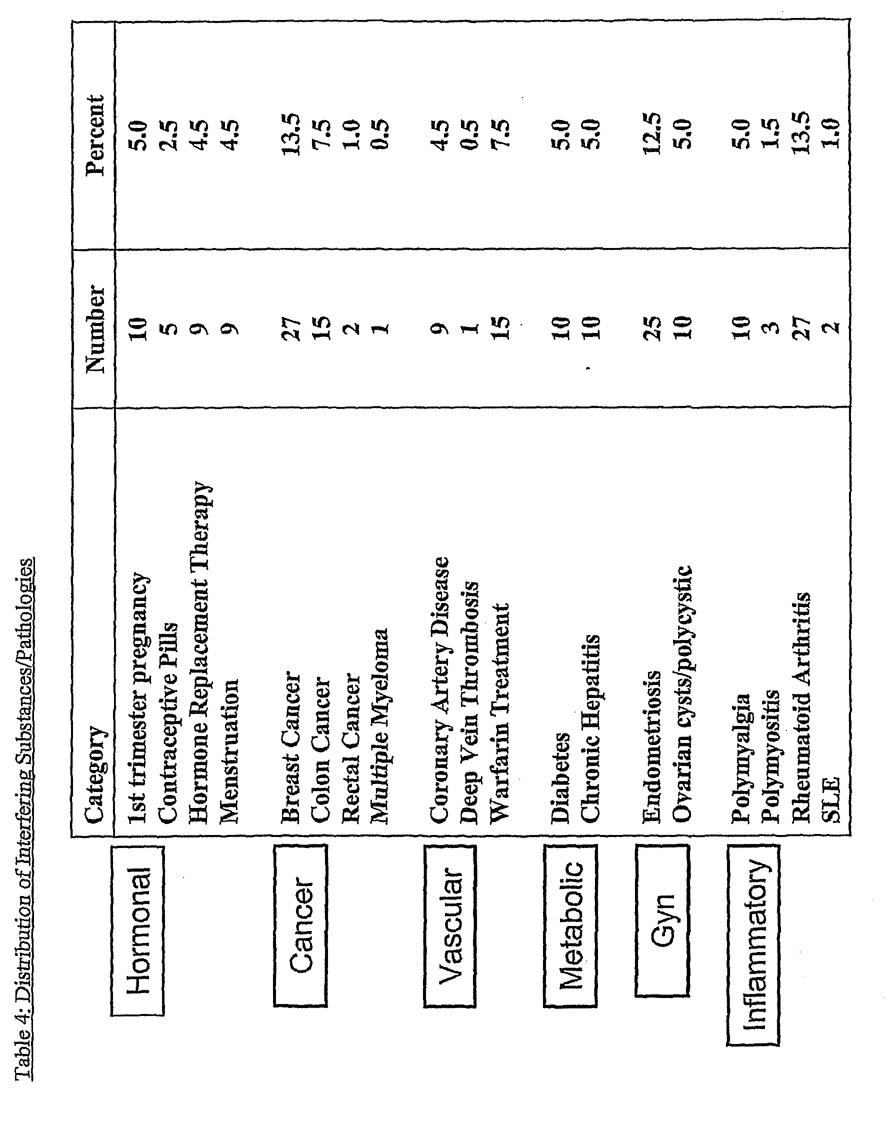

### Gener-al Description of Automation and Validation Analysis for the Study

All 900 sera samples were processed in duplicate for each of the 14 markers. An automated assay system with barcode tracking was utilized in generating the marker selection data. The assays run in this marker selection study were all completed using a Tecan Evo automated robotic liquid handler. The use of this platform allowed all 900 samples to be processed for each marker in a single experimental run (one run per day). The precision of the automated platform was verified before any study samples were processed.

All of the ELISA assays were processed for this study using a buffered solution as the diluent for the standard curve. This was done to standardize, as much as possible, the protocols for each of the assays.

The normal sera used in this study were obtained from community blood collection centers compliant with local IRB customs. While these sera can be considered 'normal' it must be noted that these donors were not screened by a medical professional to confirm their disease free or normal status

### Detection of Expression of HE4 in Serum Samples (A Representative Example) Materials and :Methods

### A. Coating of assay plates:

ELISA 96-well plates were coated with 100 µl/well of the primary antibody, anti-HE4 monoclonal 90.1 #6, at 2 µg/ml in PBS, then the plates were incubated at 4°C overnight. The next day, the plates were washed once with PBS, then 250µl/well of PBS-3% BSA was added to all wells, and the plates were incubated for 2 hours at 30°C. The plates were then emptied and dried in a vacuum oven for 2 hours at room temperature. Then they were heat-sealed inside a mylar foil bag along with a desiccant pack and stored at 4°C until used in an assay.

### B. Assay Methods

The foil bags containing the assay plates were warmed to room temperature immediately prior to use in the assay. The serum samples were diluted 1:4 into PBS-1% bovine Serum-0.05% Tween 20-1 mg/ml mouse IgG. The HE4 antigen protein was diluted to 100 ng/ml, then serially diluted two-fold into PBS-1% Bovine Serum-0.05% Tween 20-1 mg/ml Mouse IgG, then all the individual standard curve samples were further diluted 1:4 into the buffer so that they would be diluted the same as the serum samples.

The diluted sera samples and standard curve samples were added to the anti-The4 coated assay plates in 100 µl/well volumes. Then the plates were incubated for 2 hours at 30°C. The plates were next washed 5x with 250µl/well of PBS-0.05% Tween 20.

The secondary antibody, anti-HE4 monoclonal 71.1#1.13-HRP, was diluted 1:16,000 into PBS-1% bovine IgG-0.05% Tween 20-1 1mg/ml mouse IgG. The secondary antibody solution was then added to the aspirated plates in 100 µl/well volumes. The plates were incubated for 1 hour at 30°C.

The plates were washed 5x with 250 µl/well of PBS-0.05% Tween 20. The developing solution, TMB(3,3',5,5'- Tetramethylbenzidine) was warmed to room temperature prior to use, and then the TMB was added to the aspirated plates in 100 µl/well volumes.

The plates were incubated for 10 minutes at room temperature and then the Stop solution, 2N H2SO4, was added to the plates (containing the TMB) in 100 µl/well volumes.

The plates were incubated for 10 minutes at room temperature, and then they were read on the Molecular Devices SpectraMax plate reader at 450 nm, with a reference wavelength of 650 nm, using SoftMax Pro software.

The data was saved as SoftMax Pro files, and also exported as Text files for use with MS Excel.

Controls:
Serum used as the High Control: Uniglobe # 72372
Serum used as the Low Control: Uniglobe # 72404
Buffer Control used was PBS-1% Bovine Serum-0.05% Tween 20- 1 mg/ml Mouse IgG

The CV's for the controls across all 25 plates are summarized in Table 5.

**Table 5: The CV's for the Controls Across All 25 Plates**

| Standards point per point CV% across all 25 plates | | | |
|---|---|---|---|
| ng/ml | Mean OD | SD | CV% |
| 100 | 2,614 | 0.145 | 5.6 |
| 50 | 1.681 | 0.101 | 6,0 |
| 25 | 0.931 | 0.075 | 8.1 |
| 12.5 | 0.510 | Q.043 . | 8.4 |
| 6.25 | 0.263 | 0.027 | 10.4 |
| 3.13 | 0.139 | 0.014 | 10.0 |
| 1.56 | 0.076 | 0.008 | 10.5 |
| 0.78 | 0.044 | 0.012 | 27.6 |

### Results (Standard Curves)

A standard curve for HE4 was prepared by diluting the BB4 protein in PBS/1% bovine serum/0.05% Tween 20-1 mg/mL of mouse IgG. Preparation of such standard curves is well known in the art. Standard curves and the levels or concentrations of the biomarkers in all serum samples were produced for the remaining biomarkers, essentially as described above for HE4, with variations that could be appreciated and implemented by one of skill in the art. Standard curve ranges and curve fit equations for the biomarkers analyzed (i. e., HE4, glycodelin, SLPI, PLAU-R, MUC-1, Pat-1, MMP-7, inhibin, CA125, CTHRC1, KLK-6; KLK-10, alpha-1 anti-trypsin, and prolactin) are presented below:

### HE4

- Standard Curve experimental range = 0.78 nglm1 to 100 ng/ml, in serial two-fold dilutions
- Curve fit = third order polynomial, y = -1L-07x³ - 0.0001 x² + 0.0402x + 0.0157 ; R²= 0.9999
- The standard curve samples and the serum samples were then both diluted 1:4 to run in the assay; since both the serum samples and the standard curve samples were diluted in the same manner there was no need to use a dilution factor during data analysis to determine the concentrations of the unknown serum samples.

### Glycodelin

- Standard Curve experimental range = 3 ng/ml to 100 ng/ml, in serial two-fold dilutions
- Curve fit = quadratic, y = -0.0003x² + 0.0478x + 0.0952 ; R² = 0.9851
- The serum samples were run in the assay undiluted, so no dilution factor was used in the data analysis.

### SLPI

- Standard Curve experimental range = 2.38 ng/ml to 305 ng/ml, in serial two-fold dilutions
- Curve fit = third order polynomial, y = 7E-08x³ - 7E-05x² + 0.0237x - 0.039 ;
   R²= 0.9997
- The serum samples were diluted 1:6.1 in an early step of the automated sample preparation technique. Then those diluted serum samples were further diluted 1:6.6 in another automated step in order to achieve a total dilution of 1:40. Those 1:40 diluted samples were then run in the assay.
- The standard curve samples were prepared at the concentrations stated above (experimental range), then those standard curve samples were all further diluted 1:6.1, and those 1:6.1 diluted samples were then run in the assay.
- Since there was a discrepancy between the dilution of the serum samples (1:40) as compared to the dilution of the standard curve samples (1:6.1), all the serum sample concentration values obtained from the standard curve were multiplied by a dilution factor of "6.6" to correct for the discrepancy (1:40 = 1:6.1 x 1:6.6).

### uPar (PLAU-R)

- Standard Curve experimental range = 62.5 pg/ml to 4000 pg/ml, in serial two-fold dilutions
- Curve fit = linear, y=0.0006x+0.0522; R²= 0.9977
- The serum samples were diluted 1:6 in an early step of the automated sample preparation technique. Then those diluted serum samples were further diluted 1:3 in another automated step in order to achieve a total dilution of 1: 18. Those 1:18 diluted samples were then run in the assay.
- The standard curve samples were prepared at the concentrations stated above (experimental range), then those standard curve samples were all further diluted 1:3, and those 1:3 diluted samples were then run in the assay.
- Since there was a discrepancy between the dilution of the serum samples (1:18) as compared to the dilution of the standard curve samples (1:3), all the serum sample concentration values obtained from the standard curve were multiplied by a dilution factor of "6" to correct for the discrepancy (1:18 = 1:3 x 1:6).

### MUC-1

- Standard Curve experimental range = 0.3 U/ml to 600 U/ml, in serial threefold dilutions
- Curve fit = third order polynomial, y = 3E-07x³- 0.0001x²+ 0.0236x + 0.0371 ; R²=1.0
- The standard curve samples and the serum samples were then both diluted 1:2 to run in the assay; since both the serum samples and the standard curve samples were diluted in the same manner there was no need to use a dilution factor during data analysis to determine the concentrations of the unknown serum samples.

### PAI-1

- Standard Curve experimental range = 0.78 ng/ml to 100 ng/ml, in serial two-fold dilutions
- Curve fit = linear, y = 0.0139x + 0.0151; R²= 0.9996
- The standard curve samples and the serum samples were then both diluted 1:4 to run in the assay; since both the serum samples and the standard curve samples were diluted in the same manner there was no need to use a dilution factor during data analysis to determine the concentrations of the unknown serum samples.

### MMP-7

- Standard Curve experimental range = 0.16 ng/ml to 20 ng/ml, in serial two-fold dilutions
- Curve fit = third order polynomial, y = 0.000 1x³ - 0.0046x² + 0.1578x + 0.0936; R²= 0.9999
- The standard curve samples and the serum samples were then both diluted 1:2 to run in the assay; since both the serum samples and the standard curve samples were diluted in the same manner there was no need to use a dilution factor during data analysis to determine the concentrations of the unknown serum samples.

### Inhibin

- Standard Curve experimental range =10 pg/ml to 1000 pg/ml, in serial two-fold dilutions
- Curve fit = linear, y = 0.0007x + 0.0136; R² = 0.9997
- The standard curve samples and the serum samples were then both diluted 1:3 to run in the assay; since both the serum samples and the standard curve samples were diluted in the same manner there was no need to use a dilution factor during data analysis to determine the concentrations of the unknown serum samples.

### CA125

- Standard Curve experimental range =15 U/ml to 400 U/ml, in serial two-fold dilutions
- Curve fit = linear, y = 0.0021x + 0.0892 ; R²= 0.9986
- The serum samples were run in the assay undiluted, so no dilution factor was used in the data analysis.

### CTHRC 1

- Standard Curve experimental range = 0.78 ng/ml to 100 ng/ml, in serial two-fold dilutions
- Curve fit = linear, y = 0.0261x + 0.042; R² = 0.9991
- The standard curve samples and the serum samples were then both diluted 1:2 to run in the assay; since both the serum samples and the standard curve samples were diluted in the same manner there was no need to use a dilution factor during data analysis to determine the concentrations of the unknown serum samples.

### ILK-6

- Standard Curve experimental range = 0.78 ng/ml to 100 ng/ml, in serial two-fold dilutions
- Curve fit = third order polynomial, y = -4E-07x³ + 0.0001x² + 0.0133x + 0.0662 ;
   R²= 1.0
- The standard curve samples and the serum samples were then both diluted 1:2 to run in the assay; since both the serum samples and the standard curve samples were diluted in the same manner there was no need to use a dilution factor during data analysis to determine the concentrations of the unknown serum samples.

### KLK-10

- Standard Curve experimental range = ng/ml to 80 ng/ml, in serial two-fold dilutions
- Curve fit = linear, y = 0.0758x - 0.0312 ; R²= 0.9976
- The standard curve samples and the serum samples were then both diluted 1:4 to run in the assay; since both the serum samples and the standard curve samples were diluted in the same manner there was no need to use a dilution factor during data analysis to determine the concentrations of the unknown serum samples.

### Alpha 1 anti-trypsin

- Standard Curve experimental range = 3.3 ng/ml to 90 ng/ml, in serial two-fold dilutions
- Curve fit = quadratic, y = -0.0002x² + 0.0412x + 0.0592 ; R²= 0.9997
- The serum samples were diluted 1:250,000, and then those diluted samples were run in the assay. The standard curve samples were serially diluted as stated above (experimental range), and then those samples were run in the assay.
- Since there was a discrepancy between the dilution of the serum samples (1:250,000) as compared to the dilution of the standard curve samples (undiluted beyond the serial dilutions), all the serum sample concentration values obtained from the standard curve were multiplied by a dilution factor of "250,000" to correct for the discrepancy.

### Prolactin

- Standard Curve experimental range = 2 ng/ml to 240 ng/ml, in serial two-fold dilutions
- Curve fit = linear, y = 0.0049x + 0.03 63 ; R2 = 0.9917
- The standard curve samples and the serum samples were then both diluted 1:5 to run in the assay; since both the serum samples and the standard curve samples were diluted in the same manner there was no need to use a dilution factor during data analysis to determine the concentrations of the unknown serum samples.

### Results - Individual Marker Performance

Among the 500 normal cases used in the present study, 104 normal cases were used as control group to select the threshold cutoff point. Table 6 provides the mean, standard deviation, range, mean+2*std, mean+3*std based on these 104 threshold normal cases for each marker. Table 7 and Table 8 provide the mean standard deviation, range, mean+2*std in all normal study cases (396 cases) and with normal study cases from patients over the age of 55 (195 or less). The mean and standard deviations were very similar between the normal threshold control cases and normal study cases for patients over the age of 55 for each biomarker except for markers HE4 and Inhibin. See Tables 6 and 8.

**Table 6: Mean and STD of Bach Biomarker in 104 Normal Control Cases**

| Variable | N | Mean | Std Dev | Min | Max | Mean+2*std | Mean+3*std | Best Cutoff |
|---|---|---|---|---|---|---|---|---|
| HB4 | 104 | 1.7 | 0.91 | 0.5 | 6.6 | 3.5 | 4.4 | 2.2 |
| CA125 | 104 | 6.9 | 6.42 | 0.6 | 42.6 | 19.7 | 26.1 | 20 |
| GLY | 104 | 1.8 | 2.75 | 0.0 | 18.6 | 7.4 | 10.1 | 5 |
| MMP | 104 | 3.4 | 1.05 | 1.9 | 7.0 | 5.5 | 6.5 | 4 |
| Mucl | 104 | 12.5 | 10.71 | 0.1 | 51.8 | 33.9 | 44.6 | 12 |
| PAI | 104 | 74.8 | 24.92 | 16.4 | 143.2 | 124.7 | | 95 |
| CTRHC1 | 104 | 3.2 | 1.16 | 1.2 | 6.2 | 5.5 | | 4 |
| INH | 104 | 0.6 | 2.80 | 0.0 | 26.9 | 6.2 | 9.0 | 2 |
| Plau-R | 104 | 1945.4 | 534.03 | 1056.00 | 4248.0 | 3013.4 | 3547.5 | 2399.9 |
| PROLAC | 104 | 10.2 | 10.94 | 3.1 | 102.2 | 32.0 | 43.0 | 11 |
| IlLK10 | 104 | 0.8 | 0.51 | 0.3 | 2.3 | 1.8 | | 0.9 |
| KLK6 | 104 | 2.2 | 2.39 | 0.3 | 16.3 | 7.0 | 9.4 | 5 |
| SLPI | 104 | 65.7 | 14.38 | 40.2 | 134.5 | 94.5 | 108.9 | 65 |

**Table 7: Mean and STD ofEach Biomarker in 396 Normal Study Cases**

| Variable | N | Mean | Std Dev | Min | Max | Meau+2*std |
|---|---|---|---|---|---|---|
| HE4 | 396 | 1.6 | 2.1 | 0.2 | 36.4 | 5.8* |
| CA125 | 396 | 10.4 | 14.2 | 1.7 | 212.4 | 38.7* |
| GLY | 396 | 5.0 | 10.2 | 0.0 | 98.4 | 25.3* |
| MMP | 396 | 3.0 | 1.1 | 0.8 | 10.4 | 5.1 |
| Mucl | 396 | 8.3 | 8.2 | 0.0 | 52.1 | 24.7 |
| PAI | 396 | 73.2 | 28.0 | 18.8 | 145.7 | 129.2 |
| CTRHCl | 396 | 2.9 | 1.2 | 0.4 | 8.7 | 5.2 |
| T1VH | 360 | 11.3 | 28.1 | 0.0 | 265.4 | 67.5* |
| PLAUR | 396 | 2086.0 | 782.0 | 822.0 | 6384.00 | 3650 |
| PROLAC | 396 | 11.6 | 9.5 | 2.0 | 138.0 | 30.7 |
| KLK10 | 396 | 0.9 | 0.4 | 0.2 | 2.5 | 1.7 |
| KLK6 | 396 | 1.7 | 1.7 | 0.1 | 18.0 | 5.1 |
| SLPI | 396 | 59.6 | 18.0 | 31.3 | 305.0 | 95.6 |
| * The values in norma cases were very different from the values in normal control cases, which means these markers might be sensitive due to age effect. | | | | | | |

**Table 8: Mean and STD of Each Biomarker in Normal Study Cases (Patients Over the Age of 55)**

| Variable | N | Mean | Std Dev | Min | Max | Mean+2*std |
|---|---|---|---|---|---|---|
| HE4 | 195 | 2.0 | 2.8 | 0.2 | 36.4 | 7.6* |
| CA125 | 194 | 10.6 | 5.6 | 2.3 | 35.7 | 21.9 |
| GLY | 195 | 1.7 | 2.3 | 0.0 | 24.8 | 6.3 |
| MMP | 195 | 3.2 | 1.2 | 0.8 | 10.4 | 5.6 |
| Mucl | 195 | 8.9 | 8.4 | 0.0 | 36.6 | 25.7 |
| PAI | 195 | 75.6 | 27.9 | 19.1 | 145.7 | 131.3 |
| CTRHCl | 195 | 3.2 | 1.1 | 1.2 | 7.1 | 5.4 |
| INH | 177 | 5.2 | 26.6 | 0.0 | 265.4 | 58.5** |
| PLAUR | 195 | 2209.5 | 752.1 | 1122.0 | 5100.0 | 3713.7 |
| PROLAC | 195 | 10.8 | 11.1 | 2.0 | 138.0 | 32.9 |
| KLK10 | 195 | 0.9 | 0.4 | 0.2 | 2.4 | 1.7 |
| KLK6 | 195 | 1.8 | 2.0 | 0.1 | 18.0 | 5.7 |
| SLPI | 195 | 60.3 | 14.2 | 31.3 | 126.9 | 88.6 |
| * The standard deviation was larger in the normal study cases than in the normal control cases for patients over the age of 55 | | | | | | |
| ** The mean and standard deviation were larger in the normal study cases than in the normal control cases for patients over the age of 55 | | | | | | |

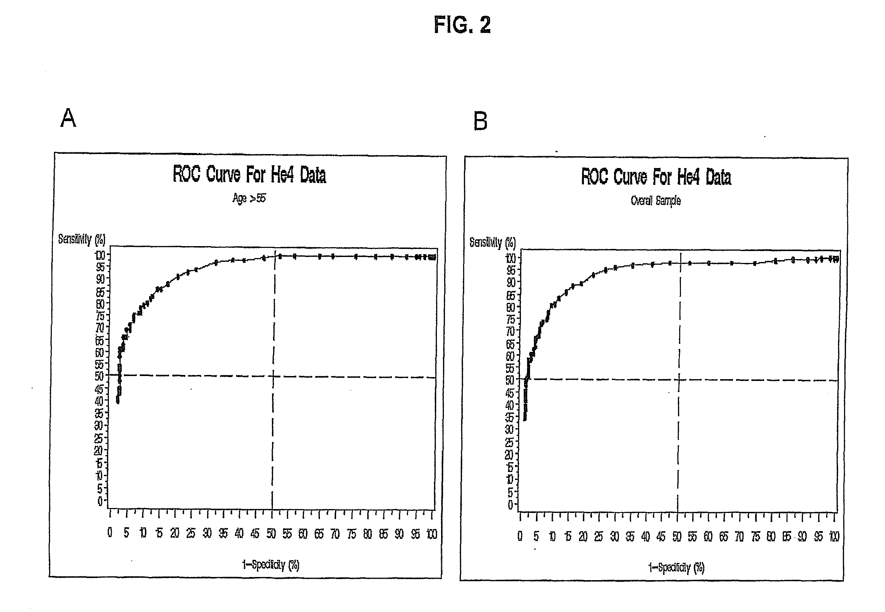

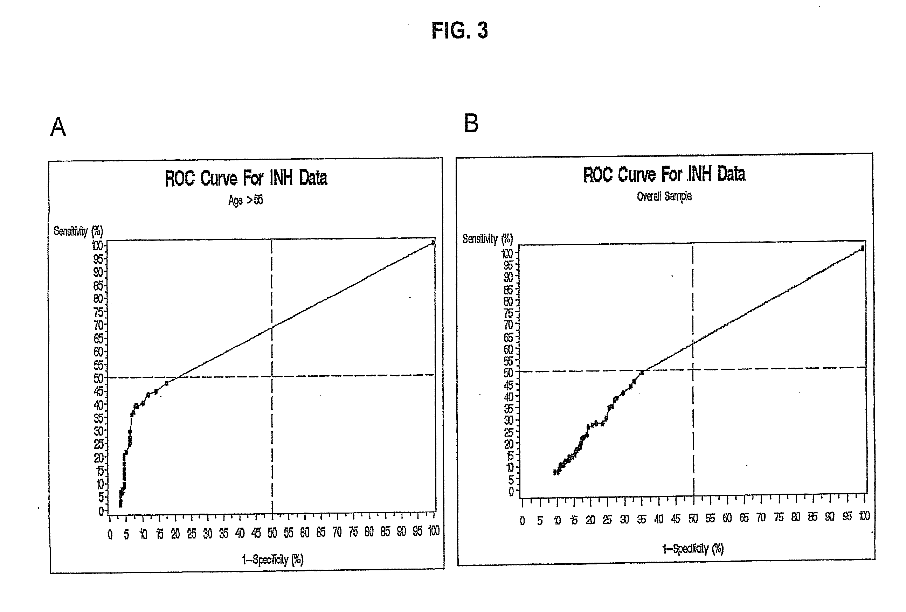

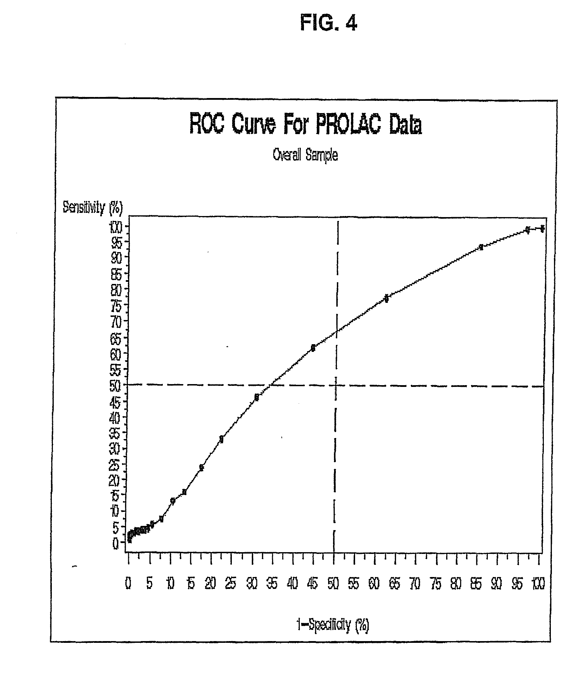

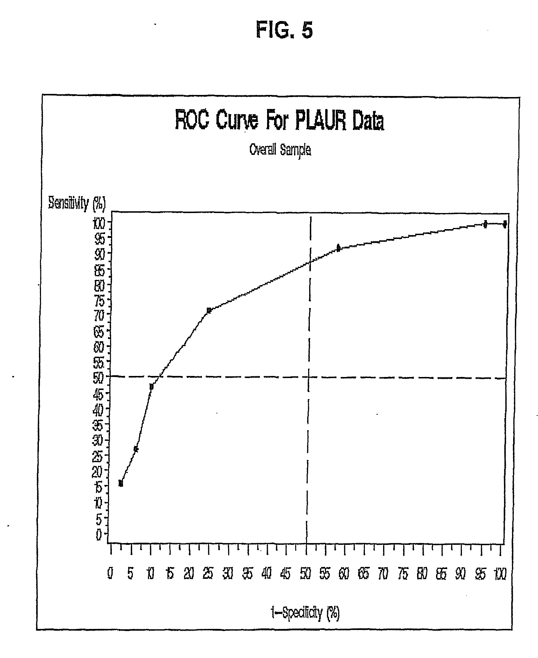

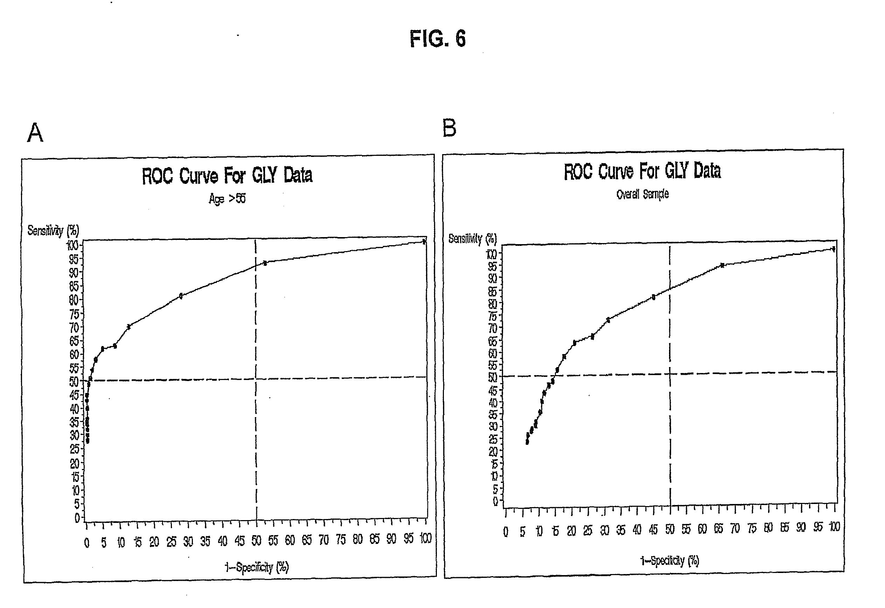

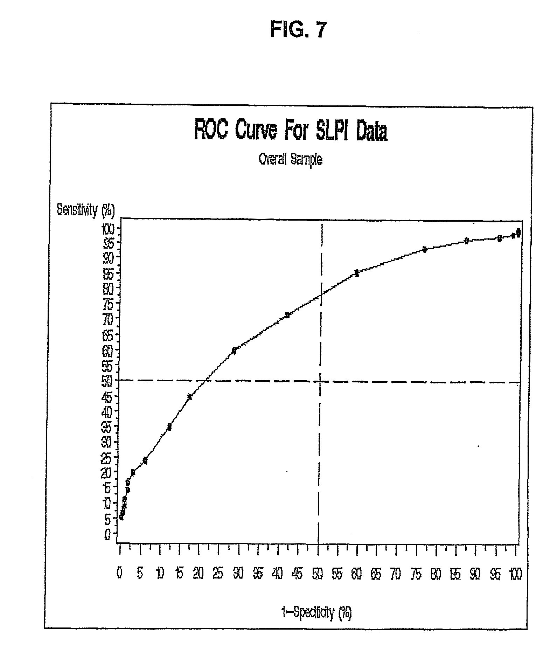

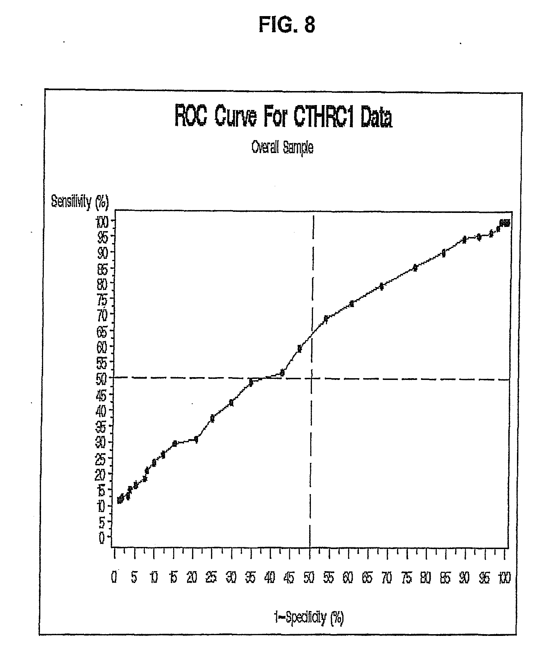

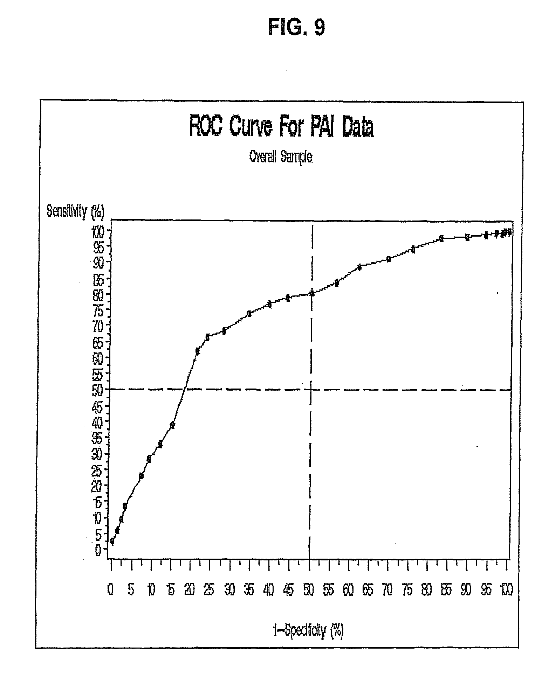

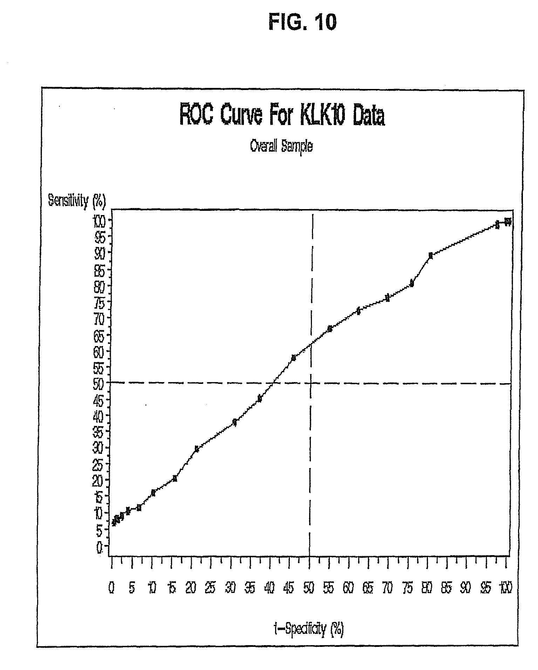

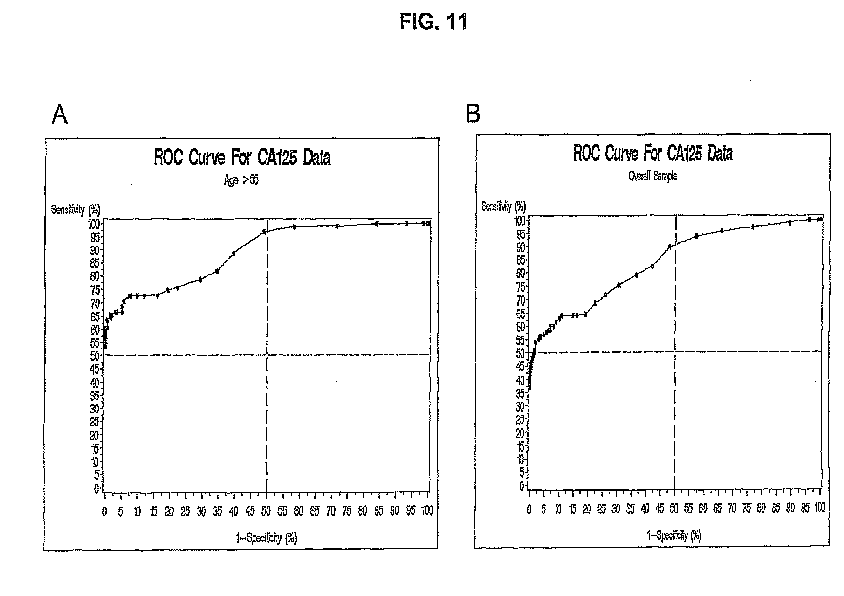

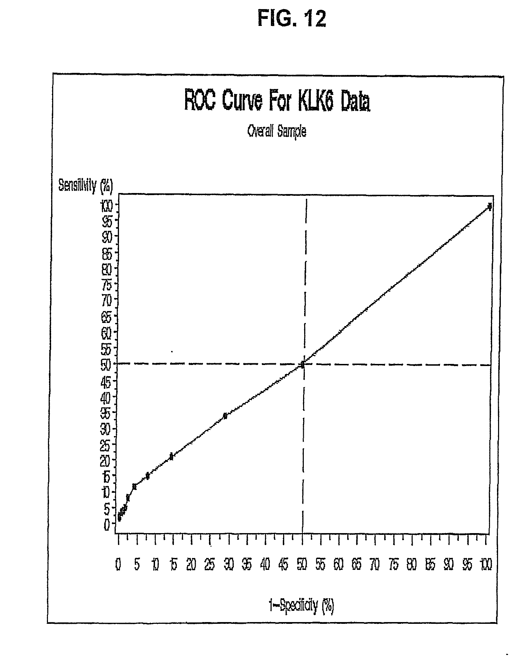

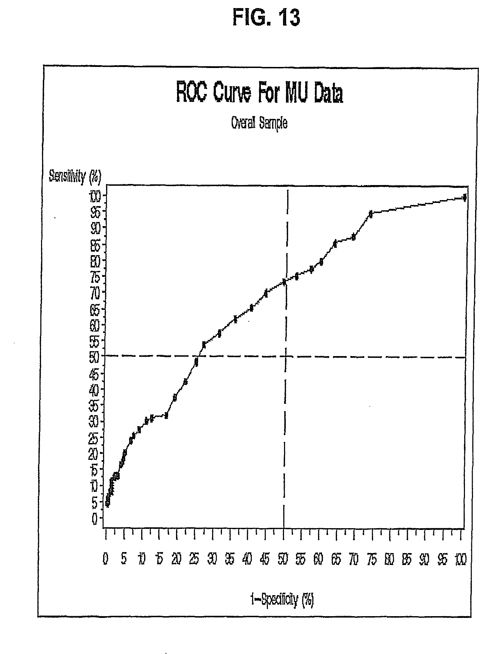

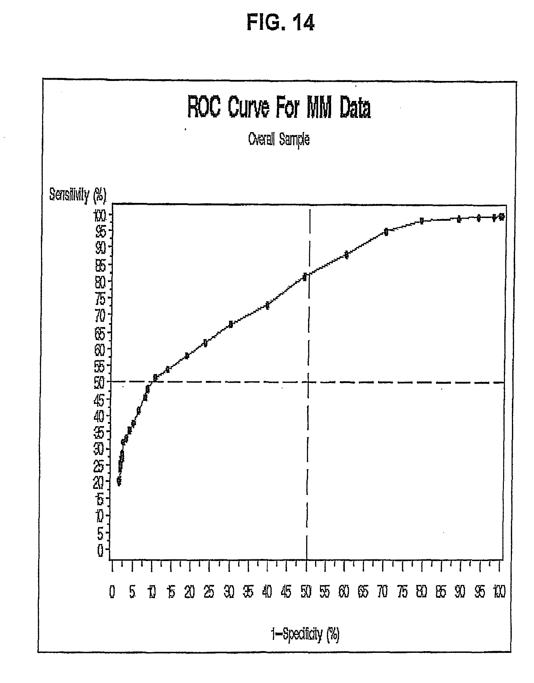

For each individual marker, the ROC curve was obtained. The ROC curve only consists of the 396 normal cases and the 200 ovarian cancer cases. The 104 normal cases as control and 200 interfering substance/pathology cases were not included in this analysis. The ROC curves for HE4, inhibin A, prolactin, PLAU-R, glycodelin, SLPI, CTHRCI, P.AT-1, KLK-10, CA125, KLK-6, MUC-1, and MMP-7 are presented in Figures 2-14, respectively.

Also, the percentage of positive and negative samples were determined for each marker by age group, by cancer stage, and by interfering substance/pathology category. These determinations were based on two kinds of cutoff points: (1) mean+2*std, and (2) the best cutoff from the ROC curve. The best cutoff points were selected from the ROC curve with the highest value of sensitivity plus specificity. The best cutoff point was listed in Table 6. Summaries of the positive results for each biomarker by age group, cancer stage, and interfering substance/pathology category are presented in tables 9 to 47.

### Biomarker: HE4

**Table 9: Summary by Age Group for HE4**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 57 | 63.33 | 98 | 90.74 | 41 | 45.56 | 81 | 75.00 |
| | **Negative** | 33 | 36.67 | 10 | 9.26 | 49 | 54.44 | 27 | 25.00 |
| | **Total** | 90 | 100.00 | 108 | 100.0 0 | 90 | 100.00 | 1as | 100.00 |
| **NORMAL** | **Positive** | 15 | 7.46 | 39 | 20.00 | 3 | 1.49 | 13 | 6.67 |
| | **Negative** | 186 | 92.54 | 156 | 80.00 | 198 | 98.51 | 182 | 93.33 |
| | **Total** | 201 | 100.00 | 195 | 100.0 0 | 201 | 100.00 | 195 | 100.00 |
| **OvCA** | **Positive** | 81 | 81.00 | 91 | 91.00 | 58 | 58.00 | 75 | 75.00 |
| | **Negative** | 19 | 19.00 | 9 | 9.00 | 42 | 42.00 | 25 | 25.00 |
| | **Total** | 100 | 100.00 | 100 | 100.0 0 | 100 | 100.00 | 100 | 100.00 |

**Table 10: Summary by Interfering Substance/Pathology Category for HE4**

| | | Best Cutoff from ROC plot | | Mean+2*std | |
|---|---|---|---|---|---|
| Group | Result | N | % | N | % |
| Tumors | Positive | 3 8 | 84.44 | 27 | 60.00 |
| | Negative | 7 | 15.56 | 18 | 40.00 |
| | Total | 45 | 100.00 | 45 | 100.00 |
| Metabolic Disorder | Positive | 20 | 100.00 | 20 | 100.00 |
| | Negative | 0 | 0.00 | 0 | 0.00 |
| | Total | 20 | 100.00 | 20 | 100.00 |
| Hormonal Fluctuation | Positive | 23 | 69.70 | 16 | 48.48 |
| | Negative | 10 | 30.30 | 17 | 51.52 |
| | Total | 33 | 100.00 | 33 | 100.00 |
| Gynecologic Disorder | Positive | 17 | 48.57 | 12 | 34.29 |
| | Negative | 18 | 51.43 | 23 | 65.71 |
| | Total | 35 | 100.00 | 35 | 100.00 |
| Vascular Disorder | Positive | 22 | 88.00 | 19 | 76.00 |
| | Negative | 3 | 12.00 | 6 | 24.00 |
| | Total | 25 | 100.00 | 25 | 100.00 |
| Chronic Immuno. Disorder | Positive | 37 | 88.10 | 29 | 69 05 |
| | Negative | 5 | 11.90 | 13 | 30.95 |
| | Total | 42 | 100.00 | 42 | 100.00 |

**Table 11: Summary by Cancer Stage for HE4**

| | | Best Cutoff from ROC plot | | Mean+2*std | |
|---|---|---|---|---|---|
| Stage | Result | N | % | N | % |
| **1** | **Positive** | 57 | 85.07 | 42 | 62.69 |
| | **Negative** | 10 | 14.93 | 25 | 37.31 |
| | **Total** | 67 | 100.00 | 67 | 100.00 |
| **2** | **Positive** | 53 | 80.30 | 41 | 62.1.2 |
| | **Negative** | 13 | 19.70 | 25 | 37.88 |
| | **Total** | 66 | 100.00 | 66 | 100.00 |
| **3** | **Positive** | 62 | 92.54 | 50 | 74.63 |
| | **Negative** | 5 | 7.46 | 17 | 25.37 |

### Biomarker Muc-1

**Table 12: Summary by Age Group for Muc-1**

| | | Best Cutoff from ROC plot | | | | Mean+2*std | | | |
|---|---|---|---|---|---|---|---|---|---|
| | | Age <=55 | | Age>55 | | Age <=55 | | Apse>55 | |
| **Group** | **Result** | N | % | N | % | N | % | N | % |
| **IP** | **Positive** | 38 | 42.22 | 53 | 49.07 | 4 | 4.44 | 9 | 8.33 |
| | **Negative** | 52 | 57.78 | 55 | 50.93 | 86 | 95.56 | 99 | 91.67 |
| | **Total** | 90 | 100.00 | 108 | 100.00 | 90 | 100.00 | 108 | 100.00 |
| **NORMAL** | **Positive** | 49 | 24.38 | 58 | 29.74 | 2 | 1.00 | 4 | 2.05 |
| | **Negative** | 152 | 75.62 | 137 | 70.26 | 199 | 99.00 | 191 | 97.95 |
| | **Total** | 201 | 100.00 | 195 | 100.00 | 201 | 100.00 | 195 | 100.00 |
| **OvCA** | **Positive** | 56 | 56.00 | 52 | 52.00 | 4 | 4.00 | 12 | 12.00 |
| | **Negative** | 44 | 44.00 | 48 | 48.00 | 96 | 96.00 | 88 | 88.00 |
| | **Total** | 100 | 100.00 | 100 | 100.00 | 100 | 100.00 | 100 | 100.00 |

**Table 13: Summary by Interfering Substance/Pathology Category for Muc-1**

| | | Best Cutoff from ROC plot | | Mean+2*std | |
|---|---|---|---|---|---|
| Group | Result | N | % | N | % |
| Tumors | Positive | 20 | 44.44 | 3 | 6.67 |
| | Negative | 25 | 55.56 | 42 | 93.33 |
| | Total | 45 | 100.00 | 45 | 100.00 |
| Metabolic Disorder | Positive | 8 | 40.00 | 1 | 5.00 |
| | Negative | 12 | 60.00 | 19 | 95.00 |
| | Total | 20 | 100.00 | 20 | 100.00 |
| Hormonal Fluctuation | Positive | 14 | 42.42 | 1 | 3.03 |
| | Negative | 19 | 57.58 | 32 | 96.97 |
| | Total | 33 | 100.00 | 33 | 100.00 |
| Gynecologic Disorder | Positive | 17 | 48.57 | 1 | 2.86 |
| | Negative | 18 | 51.43 | 34 | 97.14 |
| | Total | 35 | 100.00 | 35 | 100.00 |
| Vascular Disorder | Positive | 11 | 44.00 | 2 | 8.00 |
| | Negative | 14 | 56.00 | 23 | 92.00 |
| | Total | 25 | 100.00 | 25 | 100.00 |
| Chronic Immuno. Disorder | Positive | 22 | 52.38 | 5 | 11.90 |
| | Negative | 20 | 47.62 | 37 | 88.1.0 |
| | Total | 42 | 100.00 | 42 | 100.00 |

**Table 14: Summary by Cancer Stage for Muc- 1**

| | | Best Cutoff from ROC plot | | Mean+2*std | |
|---|---|---|---|---|---|
| Stage | Result | N | % | N | % |
| **1** | **Positive** | 44 | 65.67 | 6 | 8.96 |
| | **Negative** | 23 | 34.33 | 61 | 91.04 |
| | **Total** | 67 | 100.00 | 67 | 100.00 |
| **2** | **Positive** | 27 | 40.91 | 3 | 4.55 |
| | **Negative** | 39 | 59.09 | 63 | 95.45 |
| | **Total** | 66 | 100.00 | 66 | 100.00 |
| **3** | **Positive** | 37 | 55.22 | 7 | 10.45 |
| | **Negative** | 30 | 44.78 | 60 | 89.55 |
| | **Total** | 67 | 100.00 | 67 | 100.00 |

### Biomarker: KLK-6

**Table I5: Summary by Age Group for KLK-6**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 5 | 5.56 | 8 | 7.41 | 4 | 4.44 | 4 | 3.70 |
| | **Negative** | 85 | 94.44 | 100 | 92.59 | 86 | 95.56 | 104 | 96.30 |
| | **Total** | 90 | 100.00 | 108 | 100.00 | 90 | 100.00 | 108 | 100.00 |
| **NORMAL** | **Positive** | 6 | 2.99 | 11 | 5.64 | 2 | 1.00 | 5 | 2.56 |
| | **Negative** | 195 | 97.01 | 184 | 94.36 | 199 | 99.00 | 190 | 97.44 |
| | **Total** | 201 | 100.00 | 195 | 100.00 | 201 | 100.00 | 195 | 100.00 |
| **OvCA** | **Positive** | 10 | 10.00 | 13 | 13.00 | 4 | 4.00 | 6 | 6.00 |
| | **Negative** | 90 | 90.00 | 87 | 87.00 | 96 | 96.00 | 94 | 94.00 |
| | **Total** | 100 | 100.00 | 100 | 100.00 | 100 | 100.00 | 100 | 100.00 |

**Table 16: Summary by Interfering Substance/Pathology Category for KLK-6**

| | | Best Cutoff from ROC plot | | Mean+2*std | |
|---|---|---|---|---|---|
| Group | Result | N | % | N | % |
| Tumors | Positive | 5 | 11.11 | 4 | 8.89 |
| | Negative | 40 | 88.89 | 41 | 91.11 |
| | Total | 45 | 100.00 | 45 | 100.00 |
| Metabolic Disorder | Positive | 0 | 0.00 | 0 | 0.00 |
| | Negative | 20 | 100.00 | 20 | 100.00 |
| | Total | 20 | 100.00 | 20 | 100.00 |
| Hormonal Fluctuation | Positive | 0 | 0.00 | 0 | 0.00 |
| | Negative | 33 | 100.00 | 33 | 100.00 |
| | Total | 33 | 100.00 | 33 | 100.00 |
| Gynecologic Disorder | Positive | 3 | 8.57 | 2 | 5.71 |
| | Negative | 32 | 91.43 | 33 | 94.29 |
| | Total | 35 | 100.00 | 35 | 100.00 |
| Vascular Disorder | Positive | 3 | 12.00 | 1 | 4.00 |
| | Negative | 22 | 88.00 | 24 | 96.00 |
| | Total | 25 | 100.00 | 25 | 100.00 |
| Chronic Immuno. Disorder | Positive | 2 | 4.76 | 1 | 2.38 |
| | Negative | 40 | 95.24 | 41 | 97.6 |
| | Total | 42 | 100.00 | 42 | 100.00 |

**Table 17: Summary by Cancer Stage Group for KLK-6**

| | | Best Cutoff from ROC plot | | Mean+2*std | |
|---|---|---|---|---|---|
| Stage | Result | N | % | N | % |
| **1** | **Positive** | 10 | 14.93 | 4 | 5.97 |
| | **Negative** | 57 | 85.07 | 63 | 94.03 |
| | **Total** | 67 | 100.00 | 67 | 100.00 |
| **2** | **Positive** | 7 | 10.61 | 3 | 4.55 |
| | **Negative** | 59 | 89.39 | 63 | 95.45 |
| | **Total** | 66 | 100.00 | 66 | 100.00 |
| **3** | **Positive** | 6 | 8.96 | 3 | 4.48 |
| | **Negative** | 61 | 91.04 | 64 | 95.52 |
| | **Total** | 67 | 100.00 | 67 | 100.00 |

### Biomarker: KLK-10

**Table 18: Summary by Age Group for KLK-10**

| | | Best Cutoff from RQC | | | | plot Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 56 | 62.22 | 51 | 47.22 | 7 | 7.78 | 6 | 5.56 |
| | **Negative** | 34 | 37.78 | 57 | 52.78 | 83 | 92.22 | 102 | 94.44 |
| | **Total** | 90 | 100.00 | 108 | 100.00 | 90 | 100.00 | 108 | 100.00 |
| **NORMAL** | **Positive** | 92 | 45.77 | 89 | 45.64 | 4 | 1.99 | 2 | 1.03 |
| | **Negative** | 109 | 54.23 | 106 | 54.36 | 197 | 98.01 | 193 | 98.97 |
| | **Total** | 201 | 100.00 | 195 | 100.00 | 201 | 100.00 | 195 | 100.00 |
| **OvCA** | **Positive** | 63 | 63.00 | 53 | 53.00 | 9 | 9.00 | 7 | 7.00 |
| | **Negative** | 37 | 37.00 | 47 | 47.00 | 91 | 91.00 | 93 | 93.00 |
| | **Total** | 100 | 100.00 | 100 | 100.00 | 100 | 100.00 | 100 | 100.00 |

**Table 19: Summary by Interfering Substance/Pathology Group for KLK-10**

| | | Best Cutoff from ROC plot | | Mean+2*std | |
|---|---|---|---|---|---|
| Group | Result | N | % | N | % |
| Tumors | Positive | 28 | 62.22 | 3 | 6.67 |
| | Negative | 17 | 37.78 | 42 | 93.33 |
| | Total | 45 | 100.00 | 45 | 100.00 |
| Metabolic Disorder | Positive | 10 | 50.00 | 0 | 0.00 |
| | Negative | 10 | 50.00 | 20 | 100.00 |
| | Total | 20 | 100.00 | 20 | 100.00 |
| Hormonal Fluctuation | Positive | 14 | 42.42 | 0 | 0.00 |
| | Negative | 19 | 57.58 | 33 | 100.00 |
| | Total | 33 | 100.00 | 33 | 100.00 |
| Gynecologic Disorder | Positive | 23 | 65.71 | 3 | 8.57 |
| | Negative | 12 | 34.29 | 32 | 91.43 |
| | Total | 35 | 100.00 | 35 | 100.00 |
| Vascular Disorder | Positive | 11 | 44.00 | 1 | 4.00 |
| | Negative | 14 | 56.00 | 24 | 96.00 |
| | Total | 25 | 100.00 | 25 | 100.00 |
| Chronic Immuno. Disorder | Positive | 23 | 54.76 | 6 | 14.29 |
| | Negative | 19 | 45.24 | 36 | 85.71 |
| | Total | 42 | 100.00 | 42 | 100.00 |

**Table 20: Summary hy Cancer Stage Group for KLK- 10**

| | | Best Cutoff from ROC plot | | Mean+2*std | |
|---|---|---|---|---|---|
| Stage | Result | N | % | N | % |
| **1** | **Positive** | 37 | 55.22 | 8 | 11.94 |
| | **Negative** | 30 | 44.78 | 59 | 88.06 |
| | **Total** | 67 | 100.00 | 67 | 100.00 |
| **2** | **Positive** | 37 | 56.06 | 3 | 4.55 |
| | **Negative** | 29 | 43.94 | 63 | 95.45 |
| | **Total** | 66 | 100.00 | 66 | 100.00 |
| **3** | **Positive** | 42 | 62.69 | 5 | 7.46 |
| | **Negative** | 25 | 37.31 | 62 | 92.54 |
| | **Total** | 67 | 100.00 | 67 | 100.00 |

### Biomarker: PM-1

**Table 21: Summary by Age Group for PAI-1**

| | | Best Cutoff from ROC plots | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 52 | 57.78 | 67 | 62.04 | 12 | 1.3.33 | 10 | 9.26 |
| | **Negative** | 38 | 42.22 | 41 | 37.96 | 78 | 86.67 | 98 | 90.74 |
| | **Total** | 90 | 100.00 | 108 | 100.00 | 90 | 100.00 | 108 | 100.00 |
| **NORMAL** | **Positive** | 43 | 21.39 | 52 | 26.67 | 5 | 2.49 | 10 | 5.13 |
| | **Negative** | 158 | 78.61 | 143 | 73.33 | 196 | 97.51 | 185 | 94.87 |
| | **Total** | 201 | 100.00 | 195 | 100.00 | 201 | 100.00 | 195 | 100.00 |
| **OvCA** | **Positive** | 71 | 71.00 | 62 | 62.00 | 19 | 19.00 | 11 | 11.00 |
| | **Negative** | 29 | 29.00 | 38 | 38.00 | 81 | 81.00 | 89 | 89.00 |
| | **Total** | 100 | 100.00 | 100 | 100.00 | 100 | 100.00 | 100 | 100.00 |

**Table 22: Summary by, Interfering Substance/Pathology Group for PAI-1**

| | | Best Cutoff from ROC plot | | Mean+2*std | |
|---|---|---|---|---|---|
| Group | Result | N | % | N | % |
| Tumors | Positive | 17 | 37.78 | 4 | 8.89 |
| | Negative | 28 | 62.22 | 41 | 91.11 |
| | Total | 45 | 100.00 | 45 | 100.00 |
| Metabolic Disorder | Positive | 16 | 80.00 | 4 | 20.00 |
| | Negative | 4 | 20.00 | 16 | 80.00 |
| | Total | 20 | 100.00 | 20 | 100.00 |
| Hormonal Fluctuation | Positive | 24 | 72.73 | 5 | 15.15 |
| | Negative | 9 | 27.27 | 28 | 84.85 |
| | Total | 33 | 100.00 | 33 | 100.00 |
| Gynecologic Disorder | Positive | 17 | 48.57 | 2 | 5.71 |
| | Negative | 18 | 51.43 | 33 | 94.29 |
| | Total | 35 | 100.00 | 35 | 100.00 |
| Vascular Disorder | Positive | 17 | 68.00 | 1 | 4.00 |
| | Negative | 8 | 32.00 | 24 | 96.00 |
| | Total | 25 | 100.00 | 25 | 100.00 |
| Chronic Immuno. Disorder | Positive | 30 | 71.43 | 6 | 14.29 |
| | Negative | 12 | 28.57 | 36 | 85.71 |
| | Total | 42 | 100.00 | 42 | 100.00 |

**Table 23: Summary by Cancer Stage Group for PAI-1**

| | | Best Cutoff from ROC plot | | Mean+2*std | |
|---|---|---|---|---|---|
| Stage | Result | N | % | N | % |
| 1 | Positive | 54 | 80.60 | 15 | 22.39 |
| | **Negative** | 13 | 19.40 | 52 | 77.61 |
| | | | | | |
| | **Total** | 67 | 100.00 | 67 | 100.00 |
| **2** | **Positive** | 33 | 50.00 | 4 | 6.06 |
| | **Negative** | 33 | 50.00 | 62 | 93.94 |
| | | | | | |
| | **Total** | 66 | 100.00 | 66 | 100.00 |
| **3** | **Positive** | 46 | 68.66 | 11 | 16.42 |
| | **Negative** | 21 | 31.34 | 56 | 83.58 |
| | | | | | |
| | **Total** | 67 | 100.00 | 67 | 100.0 |

### Biomarker: CTHRC1

**Table 24: Summary by Age Group for CTHRC1**

| | | Best Cutoff from ROC plot | | | | Mean+2*std | | | |
|---|---|---|---|---|---|---|---|---|---|
| | | Age <=55 | | Age>55 | | Age<=55 | | Age>55 | |
| **Group** | **Result** | N | % | N | % | N | % | N | % |
| **IP** | **Positive** | 12 | 13.33 | 35 | 32.41 | 6 | 6.67 | 11 | 10.19 |
| | **Negative** | 78 | 86.67 | 73 | 67.59 | 84 | 93.33 | 97 | 89.81 |
| | **Total** | 90 | 100.00 | 108 | 100.00 | 90 | 100.00 | 108 | 100.00 |
| **NORMAL** | **Positive** | 20 | 9.95 | 40 | 20.51 | 4 | 1.99 | 4 | 2.05 |
| | **Negative** | 181 | 90.05 | 155 | 79.49 | 197 | 98.01 | 191 | 97.95 |
| | **Total** | 201 | 100.00 | 195 | 100.00 | 201 | 100.00 | 195 | 100.00 |
| **OvCA** | **Positive** | 24 | 24.00 | 35 | 35.00 | 10 | 10.00 | 15 | 15.00 |
| | **Negative** | 76 | 76.00 | 65 | 65.00 | 90 | 90.00 | 85 | 85.00 |
| | **Total** | 100 | 100.00 | 100 | 100.00 | 100 | 100.00 | 100 | 100.00 |

**Table 25: Summary by Interfering SubstancelPathologv Group for CTHRC1**

| | | Best Cutoff from ROC plot | | Mean+2*std | |
|---|---|---|---|---|---|
| Group | Result | N | % | N | % |
| Tumors | Positive | 13 | 28.89 | 7 | 15.56 |
| | Negative | 32 | 71.11 | 38 | 84.44 |
| | Total | 45 | 100.00 | 45 | 100.00 |
| Metabolic Disorder | Positive | 2 | 10.00 | 0 | 0.00 |
| | Negative | 18 | 90.00 | 20 | 100.00 |
| | Total | 20 | 100.00 | 20 . | 100.00 |
| Hormonal Fluctuation | Positive | 1 | 3.03 | 1 | 3.03 |
| | Negative | 32 | 96.97 | 32 | 96.97 |
| | Total | 33 | 100.00 | 33 | 100.00 |
| Gynecologic Disorder | Positive | 2 | 5.71 | 0 | 0.00 |
| | Negative | 33 | 94.29 | 35 | 100.00 |
| | Total | 35 | 100.00 | 35 | 100.00 |
| Vascular Disorder | Positive | 11 | 44.00 | 5 | 20.00 |
| | Negative | 14 | 56.00 | 20 | 80.00 |
| | Total | 25 | 100.00 | 25 | 100.00 |
| Chronic Immuno. Disorder | Positive | 18 | 42.86 | 4 | 9.52 |
| | Negative | 24 | 57.14 | 38 | 90.48 |
| | Total | 42 | 100.00 | 42 | 100.00 |

**Table 26: Summary by Cancer Stage Group for CTHRC1**

| | | Best Cutoff from ROC plot | | Mean+2*std | |
|---|---|---|---|---|---|
| Stage | Result | N | % | N | % |
| **1** | **Positive** | 16 | 23.88 | 7 | 10.45 |
| | **Negative** | 51 | 76.12 | 60 | 89.55 |
| | **Total** | 67 | 100.00 | 67 | 100.00 |
| **2** | **Positive** | 19 | 28.79 | 8 | 12.12 |
| | **Negative** | 47 | 71.21 | 58 | 87.88 |
| | **Total** | 66 | 100.00 | 66 | 100.00 |
| **3** | **Positive** | 24 | 35.82 | 10 | 14.93 |
| | **Negative** | 43 | 64.18 | 57 | 85.07 |
| | **Total** | 67 | 100.00 | 67 | 100.00 |

### Biomarker: SLPI

**Table 27: Summary by Age Group for SLPI**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 27 | 30.00 | 61 | 56.48 | 4 | 4.44 | 23 | 21.30 |
| | **Negative** | 63 | 70.00 | 47 | 43.52 | 86 | 95.56 | 85 | 78.70 |
| | **Total** | 90 | 100.00 | 108 | 100.00 | 90 | 100.00 | 108 | 100.00 |
| **NORMAL** | **Positive** | 49 | 24.38 | 63 | 32.31 | 4 | 1.99 | 3 | 1.54 |
| | **Negative** | 152 | 75.62 | 132 | 67.69 | 197 | 98.01 | 192 | 98.46 |
| | **Total** | 201 | 100.00 | 195 | 100.00 | 201 | 100.00 | 195 | 100.00 |
| **OvCA** | **Positive** | 53 | 53.00 | 67 | 67.00 | 14 | 14.00 | 16 | 16.00 |
| | **Negative** | 47 | 47.00 | 33 | 33.00 | 86 | 86.00 | 84 | 84.00 |
| | **Total** | 100 | 100.00 | 100 | 100.00 | 100 | 100.00 | 100 | 100.00 |

**Table 28: Summary by Interfering Substance/Pathology Group for SLPI**

| | | Best Cutoff from ROC plot | | Mean+2*std | |
|---|---|---|---|---|---|
| Group | Result | N | % | N | % |
| Tumors | Positive | 20 | 44.44 | 7 | 15.56 |
| | Negative | 25 | 55.56 | 38 | 84.44 |
| | Total | 45 | 100.00 | 45 | 100.00 |
| Metabolic Disorder | Positive | 10 | 50.00 | 1 | 5.00 |
| | Negative | 10 | 50.00 | 19 | 95.00 |
| | Total | 20 | 100.00 | 20 | 100.00 |
| Hormonal Fluctuation | Positive | 6 | 18.18 | 0 | 0.00 |
| | Negative | 27 | 81.82 | 33 | 100.00 |
| | Total | 33 | 100.00 | 33 | 100.00 |
| Gynecologic Disorder | Positive | 12 | 34.29 | 4 | 11.43 |
| | Negative | 23 | 65.71 | 31 | 88.57 |
| | Total | 35 | 100.00 | 35 | 100.00 |
| Vascular Disorder | Positive | 16 | 64.00 | 9 | 36.00 |
| | Negative | 9 | 36.00 | 16 | 64.00 |
| | Total | 25 | 100.00 | 25 | 100.00 |
| Chronic Immuno. Disorder | Positive | 26 | 61.90 | 7 | 16.67 |
| | Negative | 16 | 38.10 | 35 | 83.33 |
| | Total | 42 | 100.00 | 42 | 100.00 |

**Table 29: Summary by Cancer Stage Group for SLPI**

| | | Best Cutoff from ROC plot | | Mean+2*std | |
|---|---|---|---|---|---|
| Stage | Result | N | % | N | % |
| **1** | **Positive** | 34 | 50.75 | 9 | 13.43 |
| | **Negative** | 33 | 49.25 | 58 | 86.57 |
| | **Total** | 67 | 100.00 | 67 | 100.00 |
| **2** | **Positive** | 38 | 57.58 | 4 | 6.06 |
| | **Negative** | 28 | 42.42 | 62 | 93.94 |
| | **Total** | 66 | 100.00 | 66 | 100.00 |
| **3** | **Positive** | 48 | 71.64 | 17 | 25.37 |
| | **Negative** | 19 | 28.36 | 50 | 74.63 |
| | **Total** | 67 | 100.00 | 67 | 100.00 |

### Biomarker: Inhibin A

**Table 30: Summary by Age Group for Inhibin**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 53 | 58.89 | 11 | 10.19 | 48 | 53.33 | 7 | 6.48 |
| | **Negative** | 37 | 41.11 | 97 | 89.81 | 42 | 46.67 | 101 | 93.52 |
| | **Total** | 90 | 100.00 | 108 | 100.00 | 90 | 100.00 | 108 | 100.00 |
| **NORMAL** | **Positive** | 94 | 46.77 | 25 | 12.82 | 83 | 41.29 | 14 | 7.18 |
| | **Negative** | 107 | 53.23 | 170 | 87.18 | 118 | 58.71 | 181 | 92.82 |
| | **Total** | 201 | 100.00 | 195 | 100.00 | 201 | 100.00 | 195 | 100.00 |
| **OvCA** | **Positive** | 42 | 42.00 | 41 | 41.00 | 32 | 32.00 | 36 | 36.00 |
| | **Negative** | 58 | 58.00 | 59 | 59.00 | 68 | 68.00 | 64 | 64.00 |
| | **Total** | 100 | 100.00 | 100 | 100.00 | 100 | 100.00 | 100 | 100.00 |

**Table 31: Summary by Interfering Substance/Pathology Group for Inhibin**

| | | Best Cutoff from ROC plot | | Mean+2*std | |
|---|---|---|---|---|---|
| Group | Result | N | % | N | % |
| Tumors | Positive | 4 | 8.89 | 2 | 4.44 |
| | Negative | 41 | 91.11 | 43 | 95.56 |
| | Total | 45 | 100.00 | 45 | 100.00 |
| Metabolic Disorder | Positive | 8 | 40.00 | 8 | 40.00 |
| | Negative | 12 | 60.00 | 12 | 60.00 |
| | Total | 20 | 100.00 | 20 | 100.00 |
| Hormonal Fluctuation | Positive | 16 | 48.48 | 16 | 48.48 |
| | Negative | 17 | 51.52 | 17 | 51.52 |
| | Total | 33 | 100.00 | 33 | 100.00 |
| Gynecologic Disorder | Positive | 27 | 77.14 | 25 | 71.43 |
| | Negative | 8 | 22.86 | 10 | 28.57 |
| | Total | 35 | 100.00 | 35 | 100.00 |
| Vascular Disorder | Positive | 3 | 12.00 | 2 | 8.00 |
| | Negative | 22 | 88.00 | 23 | 92.00 |
| | Total | 25 | 100.00 | 25 | 100.00 |
| Chronic Immuno. Disorder | Positive | 8 | 19.05 | 4 | 9.52 |
| | Negative | 34 | 80.95 | 38 | 90.48 |
| | Total | 42 | 100.00 | 42 | 100.00 |

**Table 32: Summary by Cancer Stage Group for Inhibin**

| | | Best Cutoff from ROC plot | | Mean+2*std | |
|---|---|---|---|---|---|
| Stage | Result | N | % | N | % |
| **1** | **Positive** | 25 | 37.31 | 20 | 29.85 |
| | **Negative** | 42 | 62.69 | 47 | 70.15 |
| | **Total** | 67 | 100.00 | 67 | 100.00 |
| **2** | **Positive** | 35 | 53.03 | 32 | 48.48 |
| | **Negative** | 31 | 46.97 | 34 | 51.52 |
| | **Total** | 66 | 100.00 | 66 | 100.00 |
| 3 | Positive | 23 | 34.33 | 16 | 23.88 |
| | Negative | 44 | 65.67 | 51 | 76.12 |
| | Total | 67 | 100.00 | 67 | 100.00 |

### Biomarker: Glycodelin

**Table 33: Summary by Age Group for Glycodelin**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 53 | 58.89 | 18 | 16.67 | 36 | 40.00 | 10 | 9.26 |
| | **Negative** | 37 | 41.11 | 90 | 83.33 | 54 | 60.00 | 98 | 90.74 |
| | **Total** | 90 | 100.00 | 108 | 100.00 | 90 | 100.00 | 108 | 100.00 |
| **NORMAL** | **Positive** | 73 | 36.32 | 10 | 5.13 | 56 | 27.86 | 3 | 1.54 |
| | **Negative** | 128 | 63.68 | 185 | 94.87 | 145 | 72.14 | 192 | 98.46 |
| | **Total** | 201 | 100.00 | 195 | 100.00 | 201 | 100.00 | 195 | 100.00 |
| **OvCA** | **Positive** | 65 | 65.00 | 62 | 62.00 | 49 | 49.00 | 52 | 52.00 |
| | **Negative** | 35 | 35.00 | 38 | 38.00 | 51 | 51.00 | 48 | 48.00 |
| | **Total** | 100 | 100.00 | 100 | 100.00 | 100 | 100.00 | 100 | 100.00 |

**Table 34: Summary by Interfering Substance/Patholog-y Group for Glycodelin**

| | | Best Cutoff from ROC plot | | Mean+2*std | |
|---|---|---|---|---|---|
| Group | Result | N | % | N | % |
| Tumors | Positive | 13 | 28.89 | 6 | 13.33 |
| | Negative | 32 | 71.11 | 39 | 86.67 |
| | Total | 45 | 100.00 | 45 | 100.00 |
| Metabolic Disorder | Positive | 9 | 45.00 | 5 | 25.00 |
| | Negative | 11 | 55.00 | 15 | 75.00 |
| | Total | 20 | 100.00 | 20 | 100.00 |
| Hormonal Fluctuation | Positive | 18 | 54.55 | 15 | 45.45 |
| | Negative | 15 | 45.45 | 18 | 54.55 |
| | Total | 33 | 100.00 | 33 | 100.00 |
| Gynecologic Disorder | Positive | 21 | 60.00 | 14 | 40.00 |
| | Negative | 14 | 40.00 | 21 | 60.00 |
| | Total | 35 | 100.00 | 35 | 100.00 |
| Vascular Disorder | Positive | 1 | 4.00 | 1 | 4.00 |
| | Negative | 24 | 96.00 | 24 | 96.00 |
| | Total | 25 | 100.00 | 25 | 100.00 |
| Chronic Immuno. Disorder | Positive | 9 | 21.43 | 5 | 11.90 |
| | Negative | 33 | 78.57 | 37 | 88.10 |
| | Total | 42 | 100.00 | 42 | 100.00 |

**Table 35: Summary by Cancer Stage Group for Glycodelin**

| | | Best Cutoff from ROC plot | | Mean+2*std | |
|---|---|---|---|---|---|
| Stage | Result | N | % | N | % |
| **1** | **Positive** | 43 | 64.18 | 34 | 50.75 |
| | **Negative** | 24 | 35.82 | 33 | 49.25 |
| | **Total** | 67 | 100.00 | 67 | 100.00 |
| **2** | **Positive** | 41 | 62.12 | 37 | 56.06 |
| | **Negative** | 25 | 37.88 | 29 | 43.94 |
| | **Total** | 66 | 100.00 | 66 | 100.00 |
| **3** | **Positive** | 43 | 64.18 | 30 | 44.78 |
| | **Negative** | 24 | 35.82 | 37 | 55.22 |
| | **Total** | 67 | 100.00 | 67 | 100.00 |

### Biomarker: MMP-7

**Table 36: Summary by Age Group for MMP-7**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 19 | 21.11 | 78 | 72.22 | 9 | 10.00 | 44 | 40.74 |
| | **Negative** | 71 | 78.89 | 30 | 27.78 | 81 | 90.00 | 64 | 59.26 |
| | **Total** | 90 | 100.00 | 108 | 100.00 | 90 | 100.00 | 108 | 100.00 |
| **NORMAL** | **Positive** | 12 | 5.97 | 30 | 15.38 | 2 | 1.00 | 7 | 3.59 |
| | **Negative** | 189 | 94.03 | 165 | 84.62 | 199 | 99.00 | 188 | 96.41 |
| | **Total** | 201 | 100.00 | 195 | 100.00 | 201 | 100.00 | 195 | 100.00 |
| **OvCA** | **Positive** | 50 | 50.00 | 53 | 53.00 | 28 | 28.00 | 33 | 33.00 |
| | **Negative** | 50 | 50.00 | 47 | 47.00 | 72 | 72.00 | 67 | 67.00 |
| | **Total** | 100 | 100.00 | 100 | 100.00 | 100 | 100.00 | 100 | 100.00 |

**Table 37: Summary by Interfering Substance/Pathology Group for MMP-7**

| | | Best Cutoff from ROC plot | | Mean+2*std | |
|---|---|---|---|---|---|
| Group | Result | N | % | N | % |
| Tumors | Positive | 27 | 60.00 | 15 | 33.33 |
| | Negative | 18 | 40.00 | 30 | 66.67 |
| | Total | 45 | 100.00 | 45 | 100.00 |
| Metabolic Disorder | Positive | 8 | 40.00 | 5 | 25.00 |
| | Negative | 12 | 60.00 | 15 | 75.00 |
| | Total | 20 | 100.00 | 20 | 100.00 |
| Hormonal Fluctuation | Positive | 3 | 9.09 | 0 | 0.00 |
| | Negative | 30 | 90.91 | 33 | 100.00 |
| | Total | 33 | 100.00 | 33 | 100.00 |
| Gynecologic Disorder | Positive | 8 | 22.86 | 3 | 8.57 |
| | Negative | 27 | 77.14 | 32 | 91.43 |
| | Total | 35 | 100.00 | 35 | 100.00 |
| Vascular Disorder | Positive | 22 | 88.00 | 10 | 40.00 |
| | Negative | 3 | 12.00 | 15 | 60.00 |
| | Total | 25 | 100.00 | 25 | 100.00 |
| Chronic Immuno. Disorder | Positive | 29 | 69.05 | 20 | 47.62 |
| | Negative | 13 | 30.95 | 22 | 52.38 |
| | Total | 42 | 100.00 | 42 | 100.00 |

**Table 38: Summary by Cancer Stage Group for MMP-7**

| | | Best Cutoff from ROC plot | | Mean+2*std | |
|---|---|---|---|---|---|
| Stage | Result | N | % | N | % |
| **1** | **Positive** | 33 | 49.25 | 20 | 29.85 |
| | **Negative** | 34 | 50.75 | 47 | 70.15 |
| | **Total** | 67 | 100.00 | 67 | 100.00 |
| **2** | **Positive** | 28 | 42.42 | 11 | 16.67 |
| | **Negative** | 38 | 57.58 | 55 | 83.33 |
| | **Total** | 66 | 100.00 | 66 | 100.00 |
| **3** | **Positive** | 42 | 62.69 | 30 | 44.78 |
| | **Negative** | 25 | 37.31 | 37 | 55.22 |
| | **Total** | 67 | 100.00 | 67 | 100.00 |

### Biomarker: PLAU-R

**Table 39: Summary by Age Group for PLAU-R**

| | | Best Cutoff from ROC plot | | | | Mean+2*std | | | |
|---|---|---|---|---|---|---|---|---|---|
| | | Age<=55 | | Age>55 | | Age<=55 | | Age>55 | |
| **Group** | **Result** | N | % | N | % | N | % | N | % |
| **IP** | **Positive** | 50 | 55.56 | 71 | 65.74 | 24 | 26.67 | 56 | 51.85 |
| | **Negative** | 40 | 44.44 | 37 | 34.26 | 66 | 73.33 | 52 | 48.15 |
| | **Total** | 90 | 100.00 | 108 | 100.00 | 90 | 100.00 | 108 | 100.00 |
| **NORMAL** | **Positive** | 35 | 17.41 | 61 | 31.28 | 16 | 7.96 | 23 | 11.79 |
| | **Negative** | 166 | 82.59 | 134 | 68.72 | 185 | 92.04 | 172 | 88.21 |
| | **Total** | 201 | 100.00 | 195 | 100.00 | 201 | 100.00 | 195 | 100.00 |
| **OvCA** | **Positive** | 71 | 71.00 | 72 | 72.00 | 44 | 44.00 | 50 | 50.00 |
| | **Negative** | 29 | 29.00 | 28 | 28.00 | 56 | 56.00 | 50 | 50.00 |
| | **Total** | 100 | 100.00 | 100 | 100.00 | 100 | 100.00 | 100 | 100.00 |

**Table 40: Summary by Interfering Substance/Pathology Group for PLAU-R**

| | | Best Cutoff from ROC plot | | Mean+2*std | |
|---|---|---|---|---|---|
| Group | Result | N | % | N | % |
| Tumors | Positive | 30 | 66.67 | 20 | 44.44 |
| | Negative | 15 | 33.33 | 25 | 55.56 |
| | Total | 45 | 100.00 | 45 | 100.00 |
| Metabolic Disorder | Positive | 13 | 65.00 | 11 | 55.00 |
| | Negative | 7 | 35.00 | 9 | 45.00 |
| | Total | 20 | 100.00 | 20 | 100.00 |
| Hormonal Fluctuation | Positive | 13 | 39.39 | 1 | 3.03 |
| | Negative | 20 | 60.61 | 32 | 96.97 |
| | Total | 33 | 100.00 | 33 | 100.00 |
| Gynecologic Disorder | Positive | 15 | 42.86 | 9 | 25.71 |
| | Negative | 20 | 57.14 | 26 | 74.29 |
| | Total | 35 | 100.00 | 35 | 100.00 |
| Vascular Disorder | Positive | 18 | 72.00 | 11 | 44.00 |
| | Negative | 7 | 28.00 | 14 | 56.00 |
| | Total | 25 | 100.00 | 25 | 100.00 |
| Chronic Immuno. Disorder | Positive | 32 | 76.19 | 28 | 66.67 |
| | Negative | 10 | 23.81 | 14 | 33.33 |
| | Total | 42 | 100.00 | 42 | 100.00 |

**Table 41: Summary by Cancer Stage Group for PLAU-R**

| | | Best Cutoff from ROC plot | | Mean+2*std | |
|---|---|---|---|---|---|
| Stage | Result | N | % | N | % |
| **1** | **Positive** | 53 | 79.10 | 30 | 44.78 |
| | **Negative** | 14 | 20.90 | 37 | 55.22 |
| | **Total** | 67 | 100.00 | 67 | 100.00 |
| **2** | **Positive** | 40 | 60.61 | 29 | 43.94 |
| | **Negative** | 26 | 39.39 | 37 | 56.06 |
| | **Total** | 66 | 100.00 | 66 | 100.00 |
| **3** | **Positive** | 50 | 74.63 | 35 | 52.24 |
| | **Negative** | 17 | 25.37 | 32 | 47.76 |
| | **Total** | 67 | 100.00 | 67 | 100.00 |

### Biomarker: Prolactin

**Table 42: Summary by Age Group for Prolactin**

| | | Best Cutoff from ROC plot | | | | Mean+2*std | | | |
|---|---|---|---|---|---|---|---|---|---|
| | | Age <=55 | | Age>55 | | Age <=55 | | Age>55 | |
| **Group** | **Result** | N | % | N | % | N | % | N | % |
| **IP** | **Positive** | 50 | 55.56 | 49 | 45.37 | 9 | 10.00 | 6 | 5.56 |
| | **Negative** | 40 | 44.44 | 59 | 54.63 | 81 | 90.00 | 102 | 94.44 |
| | **Total** | 90 | 100.00 | 108 | 100.00 | 90 | 100.00 | 108 | 100.00 |
| **NORMAL** | **Positive** | 92 | 45.77 | 54 | 27.69 | 6 | 2.99 | 3 | 1.54 |
| | **Negative** | 109 | 54.23 | 141 | 72.31 | 195 | 97.01 | 192 | 98.46 |
| | **Total** | 201 | 100.00 | 195 | 100.00 | 201 | 100.00 | 195 | 100.00 |
| **OvCA** | **Positive** | 64 | 64.00 | 48 | 48.00 | 5 | 5.00 | 2 | 2.00 |
| | **Negative** | 36 | 36.00 | 52 | 52.00 | 95 | 95.00 | 98 | 98.00 |
| | **Total** | 100 | 100.00 | 100 | 100.00 | 100 | 100.00 | 100 | 100.00 |
| | | | | | | | | | |

**Table 43: Summary by Interfering Substance/Pathology Group for Prolactin**

| | | Best Cutoff from ROC plot | | Mean+2*std | |
|---|---|---|---|---|---|
| Group | Result | N | % | N | % |
| Tumors | Positive | 17 | 37.78 | 3 | 6.67 |
| | Negative | 28 | 62.22 | 42 | 93.33 |
| | Total | 45 | 100.00 | 45 | 100.00 |
| Metabolic Disorder | Positive | 4 | 20.00 | 2 | 10.00 |
| | Negative | 16 | 80.00 | 18 | 90.00 |
| | Total | 20 | 100.00 | 20 | 100.00 |
| Hormonal Fluctuation | Positive | 23 | 69.70 | 4 | 12.12 |
| | Negative | 10 | 30.30 | 29 | 87.88 |
| | Total | 33 | 100.00 | 33 | 100.00 |
| Gynecologic Disorder | Positive | 18 | 51.43 | 3 | 8.57 |
| | Negative | 17 | 48.57 | 32 | 91.43 |
| | Total | 35 | 100.00 | 35 | 100.00 |
| Vascular Disorder | Positive | 13 | 52.00 | 2 | 8.00 |
| | Negative | 12 | 48.00 | 23 | 92.00 |
| | Total | 25 | 100.00 | 25 | 100.00 |
| Chronic Immuno. Disorder | Positive | 26 | 61.90 | 2 | 4.76 |
| | Negative | 16 | 38.10 | 40 | 95.24 |
| | Total | 42 | 100.00 | 42 | 100.00 |

**Table 44: Summary by Cancer Stage Group for Prolactin**

| | | Best Cutoff from ROC plot | | Mean+2*std | |
|---|---|---|---|---|---|
| Stage | Result | N | % | N | % |
| **1** | **Positive** | 42 | 62.69 | 2 | 2.99 |
| | **Negative** | 25 | 37.31 | 65 | 97.01 |
| | **Total** | 67 | 100.00 | 67 | 100.0 0 |
| **2** | **Positive** | 30 | 45.45 | 2 | 3.03 |
| | **Negative** | 36 | 54.55 | 64 | 96.97 |
| | **Total** | 66 | 100.00 | 66 | 100.0 0 |
| **3** | **Positive** | 40 | 59.70 | 3 | 4.48 |
| | **Negative** | 27 | 40.30 | 64 | 95.52 |
| | **Total** | 67 | 100.00 | 67 | 100.0 0 |

### Biomarker: CA125 (Lab Corp Data)

**Table 45: Summary by Age Group for CA125 (LabCorp Data)**

| | | Cutoff at 35 | | | |
|---|---|---|---|---|---|
| | | Age <=55 | | Age>55 | |
| **Group** | **Result** | N | % | N | % |
| **IP** | **Positive** | 13 | 14.94 | 6 | 5.66 |
| | **Negative** | 74 | 85.06 | 100 | 94.34 |
| | **Total** | 87 | 100.00 | 106 | 100.00 |
| **NORMAL** | **Positive** | 5 | 2.51 | 1 | 0.52 |
| | **Negative** | 194 | 97.49 | 193 | 99.48 |
| | **Total** | 199 | 100.00 | 194 | 100.00 |
| **OvCA** | **Positive** | 39 | 39.39 | 60 | 60.61 |
| | **Negative** | 60 | 60.61 | 39 | 39.39 |
| | **Total** | 99 | 100.00 | 99 | 100.00 |

**Table 46: Summary by Interfering Substance/Pathology Group for CA125 (LabCorp data)**

| | | Cutoff at 35 | |
|---|---|---|---|
| Group | Result | N | % |
| Tumors | Positive | 5 | 11.11 |
| | Negative | 40 | 88.89 |
| | Total | 45 | 100.00 |
| Metabolic Disorder | Positive | 1 | 5.56 |
| | Negative | 17 | 94.44 |
| | Total | 18 | 100.00 |
| Hormonal Fluctuation | Positive | 7 | 21.21 |
| | Negative | 26 | 78.79 |
| | Total | 33 | 100.00 |
| Gynecologic Disorder | Positive | 4 | 12.50 |
| | Negative | 28 | 87.50 |
| | Total | 32 | 100.00 |
| Vascular Disorder | Positive | 0 | 0.00 |
| | Negative | 25 | 100.00 |
| | Total | 25 | 100.00 |
| Chronic Immuno. Disorder | Positive | 2 | 4.76 |
| | Negative | 40 | 95.24 |
| | Total | 42 | 100.00 |

**Table 47: Summary by Cancer Stage Group for CA 125 (LabCorp data)**

| | | Cutoff at 35 | |
|---|---|---|---|
| Stage | Result | N | % |
| **1** | **Positive** | 24 | 35.82 |
| | **Negative** | 43 | 64.18 |
| | **Total** | 67 | 100.00 |
| **2** | **Positive** | 37 | 56.06 |
| | **Negative** | 29 | 43.94 |
| | **Total** | 66 | 100.00 |
| **3** | **Positive** | 38 | 58.46 |
| | **Negative** | 27 | 41.54 |
| | **Total** | 65 | 100.00 |

Summaries of the sensitivity of the thirteen biomarkers analyzed are presented in Tables 48-50, based on tumor stage, normal 2-standard deviation limits, or "best fit," respectively.

**Table 48: Individual marker sensitivity summary by tumor stage (Based on best cutoff)**

| Marker | Stage 1 % pos | Stage 2 % pos | Stage 3 % pos |
|---|---|---|---|
| HE4 | 85.1 | 80.3 | 92.5 |
| Glycodelin | 64.2 | 62.1 | 64.2 |
| MMP7 | 49.3 | 42.4 | 62.7 |
| SLPI | 50.8 | 57.6 | 71.6 |
| PAI-1 | 80.6 | 50.0 | 68.7 |
| MUC-1 | 65.7 | 40.9 | 55.2 |
| CA125 | 35.8 | 56.1 | 58.5 |
| Plau-R | 79.1 | 60.6 | 74.6 |
| Inhibin A | 37.3 | 53.0 | 34.3 |
| CTHRC1 | 23.9 | 28.8 | 35.8 |
| KLK6 | 14.9 | 10.6 | 9.0 |
| KLK10 | 55.2 | 56.1 | 62.7 |
| Prolactin | 62.7 | 45.5 | 59.7 |

**Table 49: Individual Marker Sensitivity Summary (based on Normal 2SD limit)**

| Marker | Sensitivity | Specificity |
|---|---|---|
| HE4 | 66.5 | 96 |
| Glycodelin | 50.5 | 85.3 |
| MMP7 | 30.5 | 97.7 |
| SLPI | 15.0 | 98.3 |
| Plau-R | 47.0 | 90.1 |
| MUC1 | 8.0 | 98.5 |
| Inhibin A | 34.0 | 75.8 |
| PAI-1 | 15.0 | 96.2 |
| CA125 (>=35u/ml) | 50.0 | 98.5 |
| CTHRC1 | 12.5 | 98.0 |
| KLK6 | 5.0 | 98.2 |
| KLK10 | 8.0 | 98.5 |
| Prolactin | 3.5 | 97.7 |

**Table 50: Individual Marker Sensitivity Summary (based on best cutoff)**

| Marker | Sensitivity | Specificity |
|---|---|---|
| HE4 | 86.0 | 86.3 |
| Glycodelin | 63.5 | 79.0 |
| MMP7 | 51.5 | 89.4 |
| SLPI | 60.0 | 71.7 |
| Plau-R | 71.5 | 77.2 |
| MUC1 | 54.0 | 73.0 |
| Inhibin A | 40.7 | 70.3 |
| PAI-1 | 66.5 | 76.0 |
| CA125 (>=35u/ml) | 50.5 | 98.2 |
| CTHRC1 | 29.5 | 84.8 |
| KLK6 | 11.5 | 95.7 |
| KLK10 | 58.0 | 54.3 |
| Prolactin | 56.0 | 63.1 |

### Example 3: Ovarian Biomarker Selection Study - Multiple Biomarker Analysis

The logistic regression method was used for selection of optimal biomarkers for this study. Using logistic regression, stepwise selection starts off by finding the biomarker that produces the largest R-square value with the dependent variable. Then, given that the 'best' biomarker is in the model, logistic regression finds that next best biomarker in terms of adding to the R-square. This process of finding a set of biomarkers that adds to the R-square is stopped when biomarkers can no longer add to the R-square, in accordance with certain statistical criteria. These sets of biomarkers are not unique because some of the biomarkers have the same predictive performance.

Based on one selected model, a ROC plot can be produced, which provides the relationship between the sensitivity and specificity rates. Therefore, an appropriate specificity and sensitivity can be chosen in order to obtain an expected PPV with accepted sensitivity.

Tables 51 and 52 provide several examples involving selection of a set of biomarkers with a higher specificity and an appropriate sensitivity with a PPV of approximately 10% and a combined sensitivity rate around 70%-80%, under the assumption that the population is 8,000,000 and the disease prevalence is 0.25%. Different target values for sensitivity, specificity, NPV and PPV can be obtained by selecting different assay cutoffs, different biomarker combinations, and different rules for combining the biomarkers. Biomarker performance was analyzed using the biomarker values as continuous variables (Table 51) and as categorical variables (Table 52).

By way of definition, when two assay steps are performed, the first assay step may be referred to as the "screen test," and the second step may be referred to as the "reflex test." "Best cutoff point" refers to the value obtained from the ROC plot for a particular biomarker that produces the best sensitivity and specificity. "Mean+2*std" refers to the average expression level plus twice the standard deviation, based on analysis of normal samples from patients not afflicted with ovarian cancer.

### Biomarker Performance as Continuous Variables

**Table 51: Estimated Sensitivity, Specificity, PPV, and NPV Adjusted by Disease Prevalence for Different Models (Biomarker values as continuous variables)**

| Example | Marker Selected and rule | Screen/ Reflex Test Sensitivity | Screen/ Reflex Test Specificity | Combined Performance* | | | |
|---|---|---|---|---|---|---|---|
| | | | | Sensitivity | Specificity | PPV | NPV |
| Age>55 | | | | | | | |
| 1 | Screen Test: If HE4 <=1.8 then test is negative | 90.2% | 62.2% | | | | |
| | Reflex Test: If HE4 > 1.8 then the following marker selected: CA125, GLY, PAI, Plau-R (actual value used**) | 87% | 96% | 78.474% | 98.4880% | 11.5105% | 99.9453% |
| 2† | Marker selected: HE4, CA125, GLY, MMP-7, PAI, Plau-R (actual value used) | 81.7% | 98% | 81.7% | 98% | 9.2873% | 99.9532% |
| *Assume the population=8,000,000; prevalence=0.25%, estimated cancer cases=20,000 in the population. **Actual value refers to the actual biomarker expression level obtained in the test †Although all listed biomarkers were assayed in a single step, additional algorithms were applied to obtain the values for sensitivity, specificity, PPV, and NPV indicated in the table. | | | | | | | |

### Biomarker Performance as Categorical Variables

**Table 52: Estimated Sensitivity, Specificity, PPV, and NPV Adjusted by Disease Prevalence for Different Models (Biomarker Values as Categorical Variables)**

| Example | Marker Selected and rule | Screen/Reflex Test Sensitivity | Screen/ Reflex Test Specificity | Combined Performance* | | | |
|---|---|---|---|---|---|---|---|
| | | | | Sensitivity | Specificity | PPV | NPV |
| Overall Sample | | | | | | | |
| 1 | Screen Test: If HE4 <=1.8 then test is negative | 91.9% | 73% | | | | |
| | | | | | | | |
| | Reflex Test: If HE4 >1.8 then the following marker selected: CA125, MUC1, GLY, PAI-1, Plau-R (Best cutoff points used) | 80% | 92.5% | 73.52% | 98.704% | 12.4479% | 99.9328% |
| | | | | | | | |

| Age>55 | | | | | | | |
|---|---|---|---|---|---|---|---|
| 1 | Screen Test: If HE4 <=1.8 then test is negative | 90.2% | 62.2% | | | | |
| | | | | | | | |
| | Reflex Test: If HE4 >1.8 then the following marker selected (best cutoff points used) : CA125, MUC-1, MMP-7, Plau-R, GLY | 78% | 95.9% | 70.356% | 98.4502% | 10.2154% | 99.9246% |

| Example | Marker Selected and rule | Screen/ Reflex Test Sensitivity | Screen/ Reflex Test Specificity | Combined Performance* | | | |
|---|---|---|---|---|---|---|---|
| | | | | Sensitivity | Specificity | PPV | NPV |
| 2 | Screen Test: If HE4 <=1.8 then test is negative | 90.2% | 62.2% | | | | |
| | | | | | | | |
| | Reflex Test: If HE4 >1.8 then the following marker selected (Mean+2*std cutoff points used): CA125, MUC-1, MMP-7, Plau-R, Inhibin | 82% | 95.8% | 73.964% | 98.4124% | 10.4555% | 99.9337% |
| 3† | Marker selected: PLAU-R, GLY, HE4, CA125, MMP-7, PAI-1 (Best cutoff points used) | 80.8% | 98.5% | 80.8% | 98.5% | 11.8946% | 99.9512% |
| 4† | Marker selected: PLAU-R, GLY, HE4, CA125, MMP-7 (Mean+2*std cutoff points used) | 75% | 99% | 75% | 99% | 15.8228% | 99.9368% |
| *: CA125 cutoff>=35 in either best cutoff or mean+2std cases **Assume the population=8,000,000; prevalence=0,25%, the estimate cancer cases=20,000 in the population. † Although all listed biomarkers were assayed in a single step, additional algorithms were applied to obtain the values for sensitivity, specificity, PPV, and NPV indicated in the table. | | | | | | | |

### Sensitivity, Specificity, PPV, and NPV Results Obtained with Application of "Test Rules "

In the current study, the biomarkers HE4, CA125, PLAU-R, glycodelin, Muc-1, PAI-1, MMP-7, and inhibin were the most frequently selected markers based on marker performance either as continuous or categorical variables. In contrast to the statistical methods described above, Table 53 provides the estimated sensitivity, specificity, PPV, and NPV (adjusted by disease prevalence) based on two test rules: (1) if any two of the above biomarkers are positive (i.e., overexpressed), then the test is declared positive or (2) if any 3 of the above biomarkers are positive (i.e., overexpressed), the test is declared positive. Contrary to the results presented above in Tables 51 and 52, though, the application of either test rule did not produce a high specificity rate, which in turn lead to an unacceptably low PPV (<1.5%) when adjusted for the low prevalence rate of ovarian cancer. The application of "test rules," such as those described herein, is representative of the current state of the art in the identification of patients having an increased risk of ovarian cancer.

**Table 53: Estimated Sensitivity, Specificity, PPV, and NPV Adjusted by Disease Prevalence Based on Test Rules**

| Example | Marker Selected and rule | Performance* | | | |
|---|---|---|---|---|---|
| | | Sensitivity | Specificity | PPV | NPV |
| Overall Sample | | | | | |
| 1 | Any of two following markers are positive: HE4, CA125, Plau-R, GLY, MUC-1, PAI-1, MMP-7, Inhibin (best cutoff points used) | 97.475% | 51.654% | 0.5028% | 99.9878% |
| 2 | Any of 3 following markers are positive: HE4, CA125, Plau-R, GLY, MUC-1, PAI-1, MMP-7, Inhibin (best cutoff points used) | 91.414% | 78.372% | 1.0482% | 99.9726% |

| Age >55 Year Older | | | | | |
|---|---|---|---|---|---|
| 1 | Any of two following markers are positive: HE4, CA125, Plau-R, GLY, MUC-1, PAI-1, MMP-7, Inhibin (best cutoff points used) | 96.970% | 56.186% | 0.5516% | 99.9865% |
| 2 | Any of following markers are positive: HE4, CA125, Plau-R, GLY, MUC-1, PAI-1, MMP-7, Inhibin (best cutoff points used) | 94.949% | 78.866% | 1.1135% | 99.9840% |
| * CA125 cutoff>=35 in either best cutoff or mean+2std cases **Assume the population=8,000,000; prevalence=0.25%, estimated cancer cases=20,000 in the population. | | | | | |

### Example 4: Ovarian Biomarker Selection Study- Multiple Biomarker Analysis Using One-Step Screening Method'

Expression of the biomarkers HE4, CA125, and glycodelin and combinations thereof for all patients and patient age over 55 was assessed using a one-step screening method, as described herein above. In particular, Table 54 provides the results for a one-step algorithm for patients over age 55. The test algorithm was based on a linear logistic regression model, and the actual value of each marker was used in the model. Linear logistic regression models (and other algorithms) are routine and well known in the art. See, for example, Fleiss (2003) Statistical Methods for Rates and Proportions (3^{rd} Ed.), which is herein incorporated by reference in its entirety. The algorithm offered 76.8% sensitivity at 96.4% specificity. A test outcome defined as positive was based on the following linear logistic regression model: In(P/(1-P))= - 4.2391 + 0.0888*CA125 + 0.1790*HB4 + 0.2349*GLY, where P is the conditional probability of ovarian cancer given CA125, HE4, and glycodelin being overexpressed in the patient body sample. A similar result was obtained with CA125 determined using manufacture's recommended cutoff value of ≥ 35u/mL.

**Table 54: Sensitivity and specificity of single-step screening assay to distinguish ovarian cancer (all stages) from controls in patients over age 55**

| Marker | Variable | Algorithm | Sensitivity | Specificity |
|---|---|---|---|---|
| HE4, CA125, GLYCODELIN | Actual value | 1 Step: based on linear logistic model | 76.8% | 96.4% |
| HE4, CA125, GLYCODELIN | Actual value, except CA125 | 1 Step: based on linear logistic model | 71.4% | 95.4% |

### Example 5: Bootstrap Method of Analysis

### Validation of Study Methods

A bootstrap method of analysis was also conducted to assure a robust estimate of sensitivity and specificity for the above studies given that an independent and unique set of cancer sera was not available to validate the model. The bootstrap method consisted of artificially creating 1000 random datasets with replacement selection from the study sample set. A set of the most frequently selected markers in the study based on optimal performance was selected: HB4, CA125, PLAU-R, Glycodelin, MUC1 and PAI-1. A "test" was defined as follows: Test is positive if either (1) HB4 is positive and any one of CA125, Glycodelin, MMP-7, PLAU-R is positive or (2) HE4 is negative but CA125, Glycodelin, MMP-7, PLAU-R are all positive; otherwise, the "test" is negative. From the 1000 random samples, the mean/standard deviation and 95% confidence interval of the sensitivity/specificity of this defined test were obtained.

**Table 55: Biomarker Sequence Information**

| | **Amino Acid Sequence** | | **Nucleotide Sequence** | |
|---|---|---|---|---|
| **Biomarker Name** | **Accession No.** | **Sequence Identifier** | **Accession No.** | **Sequence Identifier** |
| HE4 (Isoform 1) | NP 006094 | SEQ ID NO:1 | NM 006103 | SEQ ID NO:2 |
| HE4 (Isoform 3) | NP 542771 | SEQ ID NO:3 | NM 080733 | SEQ ID NO:4 |
| HE4 (Isoform 4) | NP 542772 | SEQ ID NO:5 | NM 080734 | SEQ ID NO:6 |
| HE4 (Isoform 2) | NP 542774 | SEQ ID NO:7 | NM 080736 | SEQ ID NO:8 |
| HE4 (Isoform 5) | NP 542773 | SEQ ID NO:9 | NM 080735 | SEQ ID NO:10 |
| KLK6 (Variant A) | NP 002765 | SEQ ID NO:11 | NM 002774 | SEQ ID NO:12 |
| KLK6 (Variant B) | NP 001012982 | SEQ ID NO:13 | NM 001012964 | SEQ ID NO:14 |
| KLK6 (Variant C) | NP 001012983 | SEQ ID NO:15 | NM 001012965 | SEQ ID NO:16 |
| KLK (variant D) | NP 001012984 | SEQ ID NO:17 | NM 001012966 | SEQ ID NO:18 |
| KLK10 (Variant 1) | NP 002767 | SEQ ID NO:19 | NM 002776 | SEQ ID NO:20 |
| KLK10 (Variant 2) | NP 665895 | SEQ ID NO:21 | NM 145888 | SEQ ID NO:22 |
| Glycodelin (Variant 1) | NP 001018059 | SEQ ID NO:23 | NM 001018409 | SEQ ID NO:24 |
| Glycodelin (Variant 2) | NP 002562 | SEQ ID NO:25 | NM 002571 | SEQ ID NO:26 |
| PAI-1 | NP 000593 | SEQ ID NO:27 | NM 000602 | SEQ ID NO:28 |
| Muc-1 (Variant 1) | NP_002447 | SEQ ID NO:29 | NM 002456 | SEQ ID NO:30 |
| Muc-1 (Variant 2) | NP 001018016 | SEQ ID NO:31 | NM 001018016 | SEQ ID NO:32 |
| Muc-1 (Variant 3) | NP 001018017 | SEQ ID NO:33 | NM 001018017 | SEQ ID NO:34 |
| Muc-1 (Variant 4) | NP 001018021 | SEQ ID NO:35 | NM 001018021 | SEQ ID NO:36 |
| Alpha-1 anti-trypsin | NP 000286 | SEQ ID NO:37 | NM 000295 | SEQ ID NO:38 |
| Alpha-1 anti-trypsin | NP_001002235 | SEQ ID NO:39 | NM 001002235 | SEQ ID NO:40 |
| Alpha-1 anti-trypsin | NP 001002236 | SEQ ID NO:41 | NM 001002236 | SEQ ID NO:42 |
| PLAUR (Variant 1) | NP 002650 | SEQ ID NO:43 | NM 002659 | SEQ ID NO:44 |
| PLAUR (Variant 2) | NP 001005376 | SEQ ID NO:45 | NM 001005376 | SEQ ID NO:46 |
| PLAUR (Variant 3) | NP 001005377 | SEQ ID NO:47 | NM 001005377 | SEQ ID NO:48 |
| CTHRC1 | NP 612464 | SEQ ID NO:49 | NM 138455 | SEQ ID NO:50 |
| Inhibin (INHA) | NP 002182 | SEQ ID NO:51 | NM 002191 | SEQ ID NO:52 |
| Inhibin (INHBB) | NP 002184 | SEQ ID NO:53 | NM 002193 | SEQ ID NO:54 |
| Inhibin (INHBA) | NP 002183 | SEQ ID NO:55 | NM 002192 | SEQ ID NO:56 |
| CA125 (Muc-16) | NP 078966 | SEQ ID NO:57 | NM 024690 | SEQ ID NO:58 |
| **MMP-7** | NP 002414 | SEQ ID NO:59 | NM 002423 | SEQ ID NO:60 |
| Prolactin | NP 000939 | SEQ ID NO:61 | NM 000948 | SEQ ID NO:62 |
| SLPI | NP 003055 | SEQ ID NO:63 | NM 003064 | SEQ ID NO:64 |

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended embodiments.

### ASPECTS OF THE INVENTON

1. A screening method for identifying patients with an increased likelihood of having ovarian cancer, the method comprising:
   a) performing a first assay step comprising detecting the expression of a first biomarker or first panel of biomarkers in a body sample and determining if the first biomarker or the first panel of biomarkers is overexpressed; and
   b) performing a second assay step if the first biomarker or the first panel of biomarkers is determined to be overexpressed in step (a), the second assay step comprising detecting the expression of a second biomarker or a second panel of biomarkers in the body sample and determining if the second biomarker or second panel of biomarkers is overexpressed; wherein overexpression of both the first and second biomarkers or panels of biomarkers is indicative of an increased likelihood of the patient having ovarian cancer.
2. The method of aspect 1, wherein patients identified as having an increased likelihood of having ovarian cancer are subjected to additional diagnostic testing to determine if the patient has ovarian cancer, wherein the additional diagnostic testing is selected from the group consisting of pelvic examination, transvaginal ultrasound, CT scan, MRI, laparotomy, laparoscopy, and tissue sample biopsy.
3. The method of aspect 1, wherein patients identified as having an increased likelihood of having ovarian cancer that are determined not to currently have ovarian cancer are further monitored on a regular basis for the development of ovarian cancer.
4. The method of aspect 1, wherein the method is used to detect early-stage ovarian cancer.
5. The method of aspect 1, wherein a threshold level of expression for each biomarker is determined by analyzing a Receiver Operating Characteristic (ROC) plot for each biomarker, wherein an expression level above a threshold level indicates that a particular biomarker is overexpressed.
6. The method of aspect 5, wherein the threshold level of expression for each biomarker is determined relative to the expression level in a normal patient population.
7. The method of aspect 1, wherein the patients screened in accordance with the method of claim 1 are asymptomatic and have a family history of ovarian cancer or clinical risk factors indicating a high-risk for developing ovarian cancer.
8. The method of aspect 1, wherein the screening method is performed in a population of post-menopausal female patients.
9. The method of aspect 1, wherein the body sample is a blood or serum sample.
10. The method of aspect 1, wherein expression of the first and second biomarkers or panels of biomarkers is detected at the protein level using antibodies.
11. The method of aspect 10, wherein expression of the first and second biomarker proteins or panels of biomarker proteins is detected using an ELISA format or a multiplex bead-based immunoassay.
12. The method of aspect 1, wherein expression of the first and second biomarkers or panels of biomarkers is detected at the nucleic acid level using RT- PCR.
13. The method of aspect 1, wherein the biomarkers are selected from the group consisting of HE4, CA125, glycodelin, MMP-7, Muc-1, PAI-1, CTHRCl, inhibin A, PLAU-R, prolactin, KLK-IO, KLK-6, SLP15 and alpha-1 anti-trypsin.
14. The method of aspect 13, wherein the biomarkers are selected from the group consisting of CA125, HE4, glycodelin, MMP-7, SLPI, PLAU-R, Muc-1, inhibin A, and PAI-I.
15. The method of aspect 1, wherein the sensitivity of the first assay step is at least about 85%, and wherein the specificity of the first assay step is at least about 75%.
16. The method of aspect 1, wherein the sensitivity of the second assay step is at least about 80%, and wherein the specificity of the second assay step is at least about 85%.
17. The method of aspect 1, wherein the combined sensitivity of the first and second assay steps is at least about 70%, and wherein the combined specificity of the first and second screening tests is at least about 95%.
18. The method of aspect 1, wherein the negative predictive value (NPV) of the combined screening method comprising the first and second assay steps is at least about 99%.
19. The method of aspect 1, wherein the PPV is at least about 8%.
20. The method of aspect 1, wherein the combined screening test comprising the first and second assay steps has a sensitivity of at least about 90%, a specificity of at least about 98%, an NPV of at least about 99.9%, and a PPV of at least about 10%.
21. The method of aspect 1, wherein the first biomarker is HE4 and the second panel of biomarkers is selected from the group consisting of a panel comprising CA125, glycodelin, Muc-1, PAI-I, and PLAU-R, a panel comprising CA125 and PAI-I, a panel comprising CA125, glycodelin, PAI-I, and MMP-7, a panel comprising CA125, glycodelin, PAI-I, and PLAU-R, a panel comprising CA125, glycodelin, PAI-I, and PLAU-R, a panel comprising glycodelin, Muc-1, PLAU-R, and inhibin A, a panel comprising CA125, Muc-1, glycodelin, PAI-I, and PLAU-R, and a panel comprising CA125, MMP-7, glycodelin, and PLAU-R.
22. The method of aspect 1, wherein the screening method is performed in an automated, semi-automated, or manual fashion.
23. The method of aspect 1, wherein the first and second assay steps are performed as a single test or as individual tests, wherein the first assay step comprises a first test, and wherein the second assay step comprises a second test.
24. A screening method for identifying patients with an increased likelihood of having ovarian cancer, the method comprising:
   a) detecting the expression of a plurality of biomarkers in a body sample; and
   b) applying a mathematical model to the biomarker expression level data to identify patients with an increased likelihood of having ovarian cancer.
25. The method of aspect 24, wherein the mathematical model is selected from the group consisting of a logistic stepwise regression model for selection of the best biomarkers and the receiving operating characteristic (ROC) curve used for selection of a best cutoff point for a biomarker.
26. The method of aspect 24, wherein the method is used to detect early- stage ovarian cancer.
27. The method of aspect 24, wherein the patients screened are asymptomatic and have a family history of ovarian cancer or clinical risk factors indicating a high-risk for developing ovarian cancer.
28. The method of aspect 24, wherein the screening method is performed in a population of post-menopausal female patients.
29. The method of aspect 24, wherein the body sample is a blood or serum sample.
30. The method of aspect 24, wherein the plurality of biomarkers is selected from the group consisting of HE4, CA125, glycodelin, MMP-7, Muc-1, PAI-1, CTHRC1, inhibin A, PLAU-R, prolactin, KLK-10, KLK-6, SLPI, and alpha-1 antitrypsin.
31. The method of aspect 30, wherein the plurality of biomarkers is selected from the group consisting of HE4, CA125, and glycodelin.
32. A single-step screening method for identifying patients with an increased likelihood of having ovarian cancer, the method comprising detecting the expression of at least three biomarkers selected from the group consisting of HE4, CA125, glycodelin, MMP-7, Muc-1, PAI-I, CTHRC1, inhibin A, PLAU-R, prolactin, KLK-10, KLK-6, SLPI, and alpha-1 anti-trypsin glycodelin, HE4, and CA125 in a body sample, wherein overexpression of at least one of the biomarkers is indicative of the patient having an increased likelihood of having ovarian cancer.
33. The method of aspect 32, wherein overexpression of at least one of the biomarkers selected from the group consisting of HE4, glycodelin, and CA125 is indicative of the patient having an increased likelihood of having ovarian cancer.
34. A kit comprising a plurality of antibodies, wherein each of said antibodies specifically binds to a distinct biomarker protein that is selectively overexpressed in ovarian cancer, wherein said biomarker proteins are selected from the group consisting of HE4, CA125, glycodelin, MMP-7, Muc-1, PAI-I, CTHRC1, inhibin A, PLAU-R, prolactin, KLK-10, KLK-6, SLPI, and alpha-1 anti-trypsin.
35. The kit of aspect 34, wherein said biomarker proteins are selected from the group consisting of CA125, HE4, glycodelin, MMP-7, SLPI, PLAU-R, Muc-1, inhibin A, and PAI-I.
36. The kit of aspect 34, wherein said kit further comprises chemicals for the detection of antibody binding to said biomarker proteins.
37. A kit for performing the screening method of aspect 1 as a single test, wherein the test comprises the first and second assay steps.
38. A kit for performing the first or second assay step of the method of aspect 1.

## Claims

1. A single-step screening method for identifying patients with an increased likelihood of having ovarian cancer, the method comprising detecting the expression level of at least two biomarker proteins in a body sample, wherein the biomarker proteins are HE4 and CA125, and wherein overexpression of at least one of the biomarker proteins is indicative of the patient having an increased likelihood of having ovarian cancer.

2. The method of claim 1, wherein said method further comprises detecting the expression of the biomarker glycodelin, wherein overexpression of at least one of the biomarkers is indicative of the patient having an increased likelihood of having ovarian cancer.

3. The method of claim 2, wherein overexpression of all three biomarkers is indicative of the patient having an increased likelihood of having ovarian cancer.

4. A screening method for identifying patients with an increased likelihood of having ovarian cancer, wherein said method comprises:
a) performing a first assay step comprising detecting the expression of HE4 in a body sample and determining if HE4 is overexpressed; and
b) performing a second assay step if HE4 is determined to be overexpressed in step (a), the second assay step comprising detecting the expression of the biomarker CA125, and determining if the biomarker CA125 is overexpressed;
wherein overexpression of HE4 and CA125 is indicative of an increased likelihood of the patient having ovarian cancer.

5. The method of claim 4, wherein said second assay step further comprises detecting the expression of the biomarker glycodelin, and determining if the biomarker glycodelin is overexpressed, wherein overexpression of HE4, glycodelin, and CA125 is indicative of an increased likelihood of the patient having ovarian cancer.

6. The method of claim 4 or 5, wherein the first and second assay steps are performed as a single test or as individual tests, wherein the first assay step comprises a first test, and wherein the second assay step comprises a second test.

7. The method of any one of claims 1-6, wherein:
(i) patients identified as having an increased likelihood of having ovarian cancer are subjected to additional diagnostic testing to determine if the patient has ovarian cancer, wherein the additional diagnostic testing is selected from the group consisting of pelvic examination, transvaginal ultrasound, CT scan, MRI, laparotomy, laparoscopy, and tissue sample biopsy, or
(ii) patients identified as having an increased likelihood of having ovarian cancer that are determined not to currently have ovarian cancer are further monitored on a regular basis for the development of ovarian cancer, or
(iii) the method is used to detect early-stage ovarian cancer, or
(iv) a threshold level of expression for each biomarker is determined by analyzing a Receiver Operating Characteristic (ROC) plot for each biomarker, wherein an expression level above a threshold level indicates that a particular biomarker is overexpressed; or
(v) the patients screened in accordance with the method of claim 1 are asymptomatic and have a family history of ovarian cancer or clinical risk factors indicating a high-risk for developing ovarian cancer, or
(vi) the screening method is performed in a population of post-menopausal female patients, or
(vii) the body sample is a blood or serum sample, or
(viii) expression of the biomarkers is detected at the protein level using antibodies, or
(ix) expression of the biomarkers is detected at the nucleic acid level using RT-PCR.

8. The method of claim 7(iv), wherein the threshold level of expression for each biomarker is determined relative to the expression level in a normal patient population.

9. The method of claim 7(viii), wherein expression of the biomarker proteins is detected using an ELISA format or a multiplex bead-based immunoassay.

10. The method of any one of claims 1-6, wherein:
(i) the sensitivity is at least about 85%, and wherein the specificity is at least about 75%, or the sensitivity is at least about 80%, and wherein the specificity is at least about 85%, or
(ii) the sensitivity is at least about 70%, and wherein the specificity is at least about 95%, or
(iii) the negative predictive value (NPV) of the screening method is at least about 99%, or
(iv) the PPV is at least about 8%, or
(iv) the screening test has a sensitivity of at least about 90%, a specificity of at least about 98%, an NPV of at least about 99.9%, and a PPV of at least about 10%.

11. A screening method for identifying patients with an increased likelihood of having ovarian cancer, the method comprising:
a) detecting the expression of a plurality of biomarkers in a body sample, wherein the biomarkers are HE4 and CA125; and
b) applying a mathematical model to the biomarker expression level data to identify patients with an increased likelihood of having ovarian cancer.

12. The method of claim 11, wherein the biomarkers are HE4, CA125, and glycodelin.

13. The method of claim 11 or 12, wherein:
(i) the mathematical model is selected from the group consisting of a logistic stepwise regression model for selection of the best biomarkers and the receiving operating characteristic (ROC) curve used for selection of a best cutoff point for a biomarker, or
(ii) the method is used to detect early-stage ovarian cancer; or
(iii) the patients screened are asymptomatic and have a family history of ovarian cancer or clinical risk factors indicating a high-risk for developing ovarian cancer, or
(iv) the screening method is performed in a population of post-menopausal female patients, or
(v) the body sample is a blood or serum sample.

14. A kit comprising:
(i) an antibody that binds to HE4,
(ii) an antibody that binds to CA125, and
optionally further comprising chemicals for the detection of antibody binding to said biomarker proteins.

15. The kit of claim 14, wherein said kit further comprises an antibody that binds to glycodelin.


SEQUENCE LISTING

<110> TRIPATH IMAGING, INC.

<120> METHODS FOR IDENTIFYING PATIENTS WITH AN INCREASED LIKELIHOOD OF
HAVING OVARIAN CANCER AND COMPOSITIONS THERFOR

<130> N.105504A

<150> EP 07717460.5
<151> 2007-01-29

<150> US 60/762,760
<151> 2006-01-27

<160> 64

<170> FastSEQ for Windows Version 4.0

<210> 1
<211> 124
<212> PRT
<213> Homo sapiens

<400> 1
Met Pro Ala Cys Arg Leu Gly Pro Leu Ala Ala Ala Leu Leu Leu Ser
 1               5                  10                  15
Leu Leu Leu Phe Gly Phe Thr Leu Val Ser Gly Thr Gly Ala Glu Lys
            20                  25                  30
Thr Gly Val Cys Pro Glu Leu Gln Ala Asp Gln Asn Cys Thr Gln Glu
        35                  40                  45
Cys Val Ser Asp Ser Glu Cys Ala Asp Asn Leu Lys Cys Cys Ser Ala
    50                  55                  60
Gly Cys Ala Thr Phe Cys Ser Leu Pro Asn Asp Lys Glu Gly Ser Cys
65                  70                  75                  80
Pro Gln Val Asn Ile Asn Phe Pro Gln Leu Gly Leu Cys Arg Asp Gln
                85                  90                  95
Cys Gln Val Asp Ser Gln Cys Pro Gly Gln Met Lys Cys Cys Arg Asn
            100                 105                 110
Gly Cys Gly Lys Val Ser Cys Val Thr Pro Asn Phe
        115                 120

<210> 2
<211> 570
<212> DNA
<213> Homo sapiens

<400> 2
cacctgcacc ccgcccgggc atagcaccat gcctgcttgt cgcctaggcc cgctagccgc 60
cgccctcctc ctcagcctgc tgctgttcgg cttcacccta gtctcaggca caggagcaga 120
gaagactggc gtgtgccccg agctccaggc tgaccagaac tgcacgcaag agtgcgtctc 180
ggacagcgaa tgcgccgaca acctcaagtg ctgcagcgcg ggctgtgcca ccttctgctc 240
tctgcccaat gataaggagg gttcctgccc ccaggtgaac attaactttc cccagctcgg 300
cctctgtcgg gaccagtgcc aggtggacag ccagtgtcct ggccagatga aatgctgccg 360
caatggctgt gggaaggtgt cctgtgtcac tcccaatttc tgagctccag ccaccaccag 420
gctgagcagt gaggagagaa agtttctgcc tggccctgca tctggttcca gcccacctgc 480
cctccccttt ttcgggactc tgtattccct cttgggctga ccacagcttc tccctttccc 540
aaccaataaa gtaaccactt tcagcaaaaa                                   570

<210> 3
<211> 79
<212> PRT
<213> Homo sapiens

<400> 3
Met Pro Ala Cys Arg Leu Gly Pro Leu Ala Ala Ala Leu Leu Leu Ser

 1               5                   10                  15
Leu Leu Leu Phe Gly Phe Thr Leu Val Ser Gly Thr Gly Ala Glu Lys
            20                  25                  30
Thr Gly Val Cys Pro Glu Leu Gln Ala Asp Gln Asn Cys Thr Gln Glu
        35                  40                  45
Cys Val Ser Asp Ser Glu Cys Ala Asp Asn Leu Lys Cys Cys Ser Ala
    50                  55                  60
Gly Cys Ala Thr Phe Cys Ser Leu Pro Asn Gly Gln Leu Ala Glu
65                  70                  75

<210> 4
<211> 694
<212> DNA
<213> Homo sapiens

<400> 4
cacctgcacc ccgcccgggc atagcaccat gcctgcttgt cgcctaggcc cgctagccgc 60
cgccctcctc ctcagcctgc tgctgttcgg cttcacccta gtctcaggca caggagcaga 120
gaagactggc gtgtgccccg agctccaggc tgaccagaac tgcacgcaag agtgcgtctc 180
ggacagcgaa tgcgccgaca acctcaagtg ctgcagcgcg ggctgtgcca ccttctgctc 240
tctgcccaat ggccaactgg ctgagtgatt cgaagaaagt gaggaatcct ccctggacac 300
tgtatcgccc ttcgtcgtct ttcagtcaat ctcttccact ctaaggattg agtgagcgcg 360
agctggggac tctctcaaag ataaggaggg ttcctgcccc caggtgaaca ttaactttcc 420
ccagctcggc ctctgtcggg accagtgcca ggtggacagc cagtgtcctg gccagatgaa 480
atgctgccgc aatggctgtg ggaaggtgtc ctgtgtcact cccaatttct gaggtccagc 540
caccaccagg ctgagcagtg aggagagaaa gtttctgcct ggccctgcat ctggttccag 600
cccacctgcc ctcccctttt tcgggactct gtattccctc ttgggctgac cacagcttct 660
ccctttccca accaataaag taaccacttt cagc                              694

<210> 5
<211> 76
<212> PRT
<213> Homo sapiens

<400> 5
Met Pro Ala Cys Arg Leu Gly Pro Leu Ala Ala Ala Leu Leu Leu Ser
 1               5                   10                  15
Leu Leu Leu Phe Gly Phe Thr Leu Val Ser Asp Lys Glu Gly Ser Cys
            20                  25                  30
Pro Gln Val Asn Ile Asn Phe Pro Gln Leu Gly Leu Cys Arg Asp Gln
        35                  40                  45
Cys Gln Val Asp Ser Gln Cys Pro Gly Gln Met Lys Cys Cys Arg Asn
    50                  55                  60
Gly Cys Gly Lys Val Ser Cys Val Thr Pro Asn Phe
65                  70                  75

<210> 6
<211> 421
<212> DNA
<213> Homo sapiens

<400> 6
cacctgcacc ccgcccgggc atagcaccat gcctgcttgt cgcctaggcc cgctagccgc 60
cgccctcctc ctcagcctgc tgctgttcgg cttcacccta gtctcagata aggagggttc 120
ctgcccccag gtgaacatta actttcccca gctcggcctc tgtcgggacc agtgccaggt 180
ggacagccag tgtcctggcc agatgaaatg ctgccgcaat ggctgtggga aggtgtcctg 240
tgtcactccc aatttctgag gtccagccac caccaggctg agcagtgagg agagaaagtt 300
tctgcctggc cctgcatctg gttccagccc acctgccctc ccctttttcg ggactctgta 360
ttccctcttg ggctgaccac agcttctccc tttcccaacc aataaagtaa ccactttcag 420
c                                                                  421

<210> 7
<211> 102
<212> PRT
<213> Homo sapiens

<400> 7
Met Pro Ala Cys Arg Leu Gly Pro Leu Ala Ala Ala Leu Leu Leu Ser
1               5                   10                  15
Leu Leu Leu Phe Gly Phe Thr Leu Val Ser Gly Thr Gly Ala Glu Lys
            20                  25                  30
Thr Gly Val Cys Pro Glu Leu Gln Ala Asp Gln Asn Cys Thr Gln Glu
        35                  40                  45
Cys Val Ser Asp Ser Glu Cys Ala Asp Asn Leu Lys Cys Cys Ser Ala
    50                  55                  60
Gly Cys Ala Thr Phe Cys Ser Leu Pro Asn Ala Leu Phe His Trp His
65                  70                  75                  80
Leu Lys Thr Arg Arg Leu Trp Glu Ile Ser Gly Pro Arg Pro Arg Arg
                85                  90                  95
Pro Thr Trp Asp Ser Ser
            100

<210> 8
<211> 358
<212> DNA
<213> Homo sapiens

<400> 8
cacctgcacc ccgcccgggc atagcaccat gcctgcttgt cgcctaggcc cgctagccgc 60
cgccctcctc ctcagcctgc tgctgttcgg cttcacccta gtctcaggca caggagcaga 120
gaagactggc gtgtgccccg agctccaggc tgaccagaac tgcacgcaag agtgcgtctc 180
ggacagcgaa tgcgccgaca acctcaagtg ctgcagcgcg ggctgtgcca ccttctgctc 240
tctgcccaat gcactgttcc actggcacct aaagacacgg aggctctggg agatttctgg 300
ccctaggcca cgaaggccca cttgggactc aagctgaggt cctgtgattc catttggg 358

<210> 9
<211> 73
<212> PRT
<213> Homo sapiens

<400> 9
Met Leu Gln Val Gln Val Asn Leu Pro Val Ser Pro Leu Pro Thr Tyr
1               5                   10                  15
Pro Tyr Ser Phe Phe Tyr Pro Asp Lys Glu Gly Ser Cys Pro Gln Val
            20                  25                  30
Asn Ile Asn Phe Pro Gln Leu Gly Leu Cys Arg Asp Gln Cys Gln Val
        35                  40                  45
Asp Ser Gln Cys Pro Gly Gln Met Lys Cys Cys Arg Asn Gly Cys Gly
    50                  55                  60
Lys Val Ser Cys Val Thr Pro Asn Phe
65                  70

<210> 10
<211> 411
<212> DNA
<213> Homo sapiens

<400> 10
agcccagtga ggggcagtgg gggggccatg ctgcaggtac aagttaatct ccctgtatcg 60
cctctgccca cttaccctta ctcctttttc tacccagata aggagggttc ctgcccccag 120
gtgaacatta actttcccca gctcggcctc tgtcgggacc agtgccaggt ggacagccag 180
tgtcctggcc agatgaaatg ctgccgcaat ggctgtggga aggtgtcctg tgtcactccc 240
aatttctgag gtccagccac caccaggctg agcagtgagg agagaaagtt tctgcctggc 300
cctgcatctg gttccagccc acctgccctc ccctttttcg ggactctgta ttccctcttg 360
ggctgaccac agcttctccc tttcccaacc aataaagtaa ccactttcag c 411

<210> 11
<211> 244
<212> PRT
<213> Homo sapiens

<400> 11

Met Lys Lys Leu Met Val Val Leu Ser Leu Ile Ala Ala Ala Trp Ala
1               5                   10                  15
Glu Glu Gln Asn Lys Leu Val His Gly Gly Pro Cys Asp Lys Thr Ser
            20                  25                  30
His Pro Tyr Gln Ala Ala Leu Tyr Thr Ser Gly His Leu Leu Cys Gly
        35                  40                  45
Gly Val Leu Ile His Pro Leu Trp Val Leu Thr Ala Ala His Cys Lys
    50                  55                  60
Lys Pro Asn Leu Gln Val Phe Leu Gly Lys His Asn Leu Arg Gln Arg
65                  70                  75                  80
Glu Ser Ser Gln Glu Gln Ser Ser Val Val Arg Ala Val Ile His Pro
                85                  90                  95
Asp Tyr Asp Ala Ala Ser His Asp Gln Asp Ile Met Leu Leu Arg Leu
            100                 105                 110
Ala Arg Pro Ala Lys Leu Ser Glu Leu Ile Gln Pro Leu Pro Leu Glu
        115                 120                 125
Arg Asp Cys Ser Ala Asn Thr Thr Ser Cys His Ile Leu Gly Trp Gly
    130                 135                 140
Lys Thr Ala Asp Gly Asp Phe Pro Asp Thr Ile Gln Cys Ala Tyr Ile
145                 150                 155                 160
His Leu Val Ser Arg Glu Glu Cys Glu His Ala Tyr Pro Gly Gln Ile
                165                 170                 175
Thr Gln Asn Met Leu Cys Ala Gly Asp Glu Lys Tyr Gly Lys Asp Ser
            180                 185                 190
Cys Gln Gly Asp Ser Gly Gly Pro Leu Val Cys Gly Asp His Leu Arg
        195                 200                 205
Gly Leu Val Ser Trp Gly Asn Ile Pro Cys Gly Ser Lys Glu Lys Pro
    210                 215                 220
Gly Val Tyr Thr Asn Val Cys Arg Tyr Thr Asn Trp Ile Gln Lys Thr
225                 230                 235                 240
Ile Gln Ala Lys

<210> 12
<211> 1527
<212> DNA
<213> Homo sapiens

<400> 12
ggcggacaaa gcccgattgt tcctgggccc tttccccatc gcgcctgggc ctgctcccca 60
gcccggggca ggggcggggg ccagtgtggt gacacacgct gtagctgtct ccccggctgg 120
ctggctcgct ctctcctggg gacacagagg tcggcaggca gcacacagag ggacctacgg 180
gcagctgttc cttcccccga ctcaagaatc cccggaggcc cggaggcctg cagcaggagc 240
ggccatgaag aagctgatgg tggtgctgag tctgattgct gcagcctggg cagaggagca 300
gaataagttg gtgcatggcg gaccctgcga caagacatct cacccctacc aagctgccct 360
ctacacctcg ggccacttgc tctgtggtgg ggtccttatc catccactgt gggtcctcac 420
agctgcccac tgcaaaaaac cgaatcttca ggtcttcctg gggaagcata accttcggca 480
aagggagagt tcccaggagc agagttctgt tgtccgggct gtgatccacc ctgactatga 540
tgccgccagc catgaccagg acatcatgct gttgcgcctg gcacgcccag ccaaactctc 600
tgaactcatc cagccccttc ccctggagag ggactgctca gccaacacca ccagctgcca 660
catcctgggc tggggcaaga cagcagatgg tgatttccct gacaccatcc agtgtgcata 720
catccacctg gtgtcccgtg aggagtgtga gcatgcctac cctggccaga tcacccagaa 780
catgttgtgt gctggggatg agaagtacgg gaaggattcc tgccagggtg attctggggg 840
tccgctggta tgtggagacc acctccgagg ccttgtgtca tggggtaaca tcccctgtgg 900
atcaaaggag aagccaggag tctacaccaa cgtctgcaga tacacgaact ggatccaaaa 960
aaccattcag gccaagtgac cctgacatgt gacatctacc tcccgaccta ccaccccact 1020
ggctggttcc agaacgtctc tcacctagac cttgcctccc ctcctctcct gcccagctct 1080
gaccctgatg cttaataaac gcagcgacgt gagggtcctg attctccctg gttttacccc 1140
agctccatcc ttgcatcact ggggaggacg tgatgagtga ggacttgggt cctcggtctt 1200
acccccacca ctaagagaat acaggaaaat cccttctagg catctcctct ccccaaccct 1260
tccacacgtt tgatttcttc ctgcagaggc ccagccacgt gtctggaatc ccagctccgc 1320
tgcttactgt cggtgtcccc ttgggatgta cctttcttca ctgcagattt ctcacctgta 1380
agatgaagat aaggatgata cagtctccat aaggcagtgg ctgttggaaa gatttaaggt 1440
ttcacaccta tgacatacat ggaatagcac ctgggccacc atgcactcaa taaagaatga 1500
attttattat gaaaaaaaaa aaaaaaa                                     1527

<210> 13

<211> 244
<212> PRT
<213> Homo sapiens

<400> 13
Met Lys Lys Leu Met Val Val Leu Ser Leu Ile Ala Ala Ala Trp Ala
1               5                   10                  15
Glu Glu Gln Asn Lys Leu Val His Gly Gly Pro Cys Asp Lys Thr Ser
            20                  25                  30
His Pro Tyr Gln Ala Ala Leu Tyr Thr Ser Gly His Leu Leu Cys Gly
        35                  40                  45
Gly Val Leu Ile His Pro Leu Trp Val Leu Thr Ala Ala His Cys Lys
    50                  55                  60
Lys Pro Asn Leu Gln Val Phe Leu Gly Lys His Asn Leu Arg Gln Arg
65                  70                  75                  80
Glu Ser Ser Gln Glu Gln Ser Ser Val Val Arg Ala Val Ile His Pro
                85                  90                  95
Asp Tyr Asp Ala Ala Ser His Asp Gln Asp Ile Met Leu Leu Arg Leu
            100                 105                 110
Ala Arg Pro Ala Lys Leu Ser Glu Leu Ile Gln Pro Leu Pro Leu Glu
        115                 120                 125
Arg Asp Cys Ser Ala Asn Thr Thr Ser Cys His Ile Leu Gly Trp Gly
    130                 135                 140
Lys Thr Ala Asp Gly Asp Phe Pro Asp Thr Ile Gln Cys Ala Tyr Ile
145                 150                 155                 160
His Leu Val Ser Arg Glu Glu Cys Glu His Ala Tyr Pro Gly Gln Ile
                165                 170                 175
Thr Gln Asn Met Leu Cys Ala Gly Asp Glu Lys Tyr Gly Lys Asp Ser
            180                 185                 190
Cys Gln Gly Asp Ser Gly Gly Pro Leu Val Cys Gly Asp His Leu Arg
        195                 200                 205
Gly Leu Val Ser Trp Gly Asn Ile Pro Cys Gly Ser Lys Glu Lys Pro
    210                 215                 220
Gly Val Tyr Thr Asn Val Cys Arg Tyr Thr Asn Trp Ile Gln Lys Thr
225                 230                 235                 240
Ile Gln Ala Lys

<210> 14
<211> 1527
<212> DNA
<213> Homo sapiens

<400> 14
aggacgttcc agaagcatct ggggacagaa ccagcctctt ccagggaggc ctgggagctg 60
ggggtgtgtg tctggcagtc cctgcagccc tgggctctgc ggcccctgcg tcctccgctt 120
ggctctgcca ctgcatctga gtgtcttctc tcctcacggc tccccgcatt tctaactctt 180
tctgcctcct cgtctcaaag ctgttccttc ccccgactca agaatccccg gaggcccgga 240
ggcctgcagc aggagcggcc atgaagaagc tgatggtggt gctgagtctg attgctgcag 300
cctgggcaga ggagcagaat aagttggtgc atggcggacc ctgcgacaag acatctcacc 360
cctaccaagc tgccctctac acctcgggcc acttgctctg tggtggggtc cttatccatc 420
cactgtgggt cctcacagct gcccactgca aaaaaccgaa tcttcaggtc ttcctgggga 480
agcataacct tcggcaaagg gagagttccc aggagcagag ttctgttgtc cgggctgtga 540
tccaccctga ctatgatgcc gccagccatg accaggacat catgctgttg cgcctggcac 600
gcccagccaa actctctgaa ctcatccagc cccttcccct ggagagggac tgctcagcca 660
acaccaccag ctgccacatc ctgggctggg gcaagacagc agatggtgat ttccctgaca 720
ccatccagtg tgcatacatc cacctggtgt cccgtgagga gtgtgagcat gcctaccctg 780
gccagatcac ccagaacatg ttgtgtgctg gggatgagaa gtacgggaag gattcctgcc 840
agggtgattc tgggggtccg ctggtatgtg gagaccacct ccgaggcctt gtgtcatggg 900
gtaacatccc ctgtggatca aaggagaagc caggagtcta caccaacgtc tgcagataca 960
cgaactggat ccaaaaaacc attcaggcca agtgaccctg acatgtgaca tctacctccc 1020
gacctaccac cccactggct ggttccagaa cgtctctcac ctagaccttg cctcccctcc 1080
tctcctgccc agctctgacc ctgatgctta ataaacgcag cgacgtgagg gtcctgattc 1140
tccctggttt taccccagct ccatccttgc atcactgggg aggacgtgat gagtgaggac 1200
ttgggtcctc ggtcttaccc ccaccactaa gagaatacag gaaaatccct tctaggcatc 1260
tcctctcccc aacccttcca cacgtttgat ttcttcctgc agaggcccag ccacgtgtct 1320
ggaatcccag ctccgctgct tactgtcggt gtccccttgg gatgtacctt tcttcactgc 1380

agatttctca cctgtaagat gaagataagg atgatacagt ctccataagg cagtggctgt 1440
tggaaagatt taaggtttca cacctatgac atacatggaa tagcacctgg gccaccatgc 1500
actcaataaa gaatgaattt tattatg 1527

<210> 15
<211> 137
<212> PRT
<213> Homo sapiens

<400> 15
Met Leu Leu Arg Leu Ala Arg Pro Ala Lys Leu Ser Glu Leu Ile Gln
1               5                   10                  15
Pro Leu Pro Leu Glu Arg Asp Cys Ser Ala Asn Thr Thr Ser Cys His
            20                  25                  30
Ile Leu Gly Trp Gly Lys Thr Ala Asp Gly Asp Phe Pro Asp Thr Ile
        35                  40                  45
Gln Cys Ala Tyr Ile His Leu Val Ser Arg Glu Glu Cys Glu His Ala
    50                  55                  60
Tyr Pro Gly Gln Ile Thr Gln Asn Met Leu Cys Ala Gly Asp Glu Lys
65                  70                  75                  80
Tyr Gly Lys Asp Ser Cys Gln Gly Asp Ser Gly Gly Pro Leu Val Cys
                85                  90                  95
Gly Asp His Leu Arg Gly Leu Val Ser Trp Gly Asn Ile Pro Cys Gly
            100                 105                 110
Ser Lys Glu Lys Pro Gly Val Tyr Thr Asn Val Cys Arg Tyr Thr Asn
        115                 120                 125
Trp Ile Gln Lys Thr Ile Gln Ala Lys
    130                 135

<210> 16
<211> 1495
<212> DNA
<213> Homo sapiens

<400> 16
aggacgttcc agaagcatct ggggacagaa ccagcctctt ccagggaggc ctgggagctg 60
ggggtgtgtg tctggcagtc cctgcagccc tgggctctgc ggcccctgcg tcctccgctt 120
ggctctgcca ctgcatctga gtgtcttctc tcctcacggc tccccgcatt tctaactctt 180
tctgcctcct cgtctcaaag ctgttccttc ccccgactca agaatccccg gaggcccgga 240
ggcctgcagc agcctgggca gaggagcaga ataagttggt gcatggcgga ccctgcgaca 300
agacatctca cccctaccaa gctgccctct acacctcggg ccacttgctc tgtggtgggg 360
tccttatcca tccactgtgg gtcctcacag ctgcccactg caaaaaaccg aatcttcagg 420
tcttcctggg gaagcataac cttcggcaaa gggagagttc ccaggagcag agttctgttg 480
tccgggctgt gatccaccct gactatgatg ccgccagcca tgaccaggac atcatgctgt 540
tgcgcctggc acgcccagcc aaactctctg aactcatcca gccccttccc ctggagaggg 600
actgctcagc caacaccacc agctgccaca tcctgggctg gggcaagaca gcagatggtg 660
atttccctga caccatccag tgtgcataca tccacctggt gtcccgtgag gagtgtgagc 720
atgcctaccc tggccagatc acccagaaca tgttgtgtgc tggggatgag aagtacggga 780
aggattcctg ccagggtgat tctgggggtc cgctggtatg tggagaccac ctccgaggcc 840
ttgtgtcatg gggtaacatc ccctgtggat caaaggagaa gccaggagtc tacaccaacg 900
tctgcagata cacgaactgg atccaaaaaa ccattcaggc caagtgaccc tgacatgtga 960
catctacctc ccgacctacc accccactgg ctggttccag aacgtctctc acctagacct 1020
tgcctcccct cctctcctgc ccagctctga ccctgatgct taataaacgc agcgacgtga 1080
gggtcctgat tctccctggt tttaccccag ctccatcctt gcatcactgg ggaggacgtg 1140
atgagtgagg acttgggtcc tcggtcttac ccccaccact aagagaatac aggaaaatcc 1200
cttctaggca tctcctctcc ccaacccttc cacacgtttg atttcttcct gcagaggccc 1260
agccacgtgt ctggaatccc agctccgctg cttactgtcg gtgtcccctt gggatgtacc 1320
tttcttcact gcagatttct cacctgtaag atgaagataa ggatgataca gtctccataa 1380
ggcagtggct gttggaaaga tttaaggttt cacacctatg acatacatgg aatagcacct 1440
gggccaccat gcactcaata aagaatgaat tttattatga aaaaaaaaaa aaaaa 1495

<210> 17
<211> 40
<212> PRT
<213> Homo sapiens

<400> 17

Met Lys Lys Leu Met Val Val Leu Ser Leu Ile Ala Ala Gly Ile Phe
1               5                   10                  15
Arg Ser Ser Trp Gly Ser Ile Thr Phe Gly Lys Gly Arg Val Pro Arg
            20                  25                  30
Ser Arg Val Leu Leu Ser Gly Leu
        35                  40

<210> 18
<211> 1386
<212> DNA
<213> Homo sapiens

<400> 18
aggacgttcc agaagcatct ggggacagaa ccagcctctt ccagggaggc ctgggagctg 60
ggggtgtgtg tctggcagtc cctgcagccc tgggctctgc ggcccctgcg tcctccgctt 120
ggctctgcca ctgcatctga gtgtcttctc tcctcacggc tccccgcatt tctaactctt 180
tctgcctcct cgtctcaaag ctgttccttc ccccgactca agaatccccg gaggcccgga 240
ggcctgcagc aggagcggcc atgaagaagc tgatggtggt gctgagtctg attgctgcag 300
gaatcttcag gtcttcctgg ggaagcataa ccttcggcaa agggagagtt cccaggagca 360
gagttctgtt gtccgggctg tgatccaccc tgactatgat gccgccagcc atgaccagga 420
catcatgctg ttgcgcctgg cacgcccagc caaactctct gaactcatcc agccccttcc 480
cctggagagg gactgctcag ccaacaccac cagctgccac atcctgggct ggggcaagac 540
agcagatggt gatttccctg acaccatcca gtgtgcatac atccacctgg tgtcccgtga 600
ggagtgtgag catgcctacc ctggccagat cacccagaac atgttgtgtg ctggggatga 660
gaagtacggg aaggattcct gccagggtga ttctgggggt ccgctggtat gtggagacca 720
cctccgaggc cttgtgtcat ggggtaacat cccctgtgga tcaaaggaga agccaggagt 780
ctacaccaac gtctgcagat acacgaactg gatccaaaaa accattcagg ccaagtgacc 840
ctgacatgtg acatctacct cccgacctac caccccactg gctggttcca gaacgtctct 900
cacctagacc ttgcctcccc tcctctcctg cccagctctg accctgatgc ttaataaacg 960
cagcgacgtg agggtcctga ttctccctgg ttttacccca gctccatcct tgcatcactg 1020
gggaggacgt gatgagtgag gacttgggtc ctcggtctta cccccaccac taagagaata 1080
caggaaaatc ccttctaggc atctcctctc cccaaccctt ccacacgttt gatttcttcc 1140
tgcagaggcc cagccacgtg tctggaatcc cagctccgct gcttactgtc ggtgtcccct 1200
tgggatgtac ctttcttcac tgcagatttc tcacctgtaa gatgaagata aggatgatac 1260
agtctccata aggcagtggc tgttggaaag atttaaggtt tcacacctat gacatacatg 1320
gaatagcacc tgggccacca tgcactcaat aaagaatgaa ttttattatg aaaaaaaaaa 1380
aaaaaa                                                            1386

<210> 19
<211> 276
<212> PRT
<213> Homo sapiens

<400> 19
Met Arg Ala Pro His Leu His Leu Ser Ala Ala Ser Gly Ala Arg Ala
1               5                   10                  15
Leu Ala Lys Leu Leu Pro Leu Leu Met Ala Gln Leu Trp Ala Ala Glu
            20                  25                  30
Ala Ala Leu Leu Pro Gln Asn Asp Thr Arg Leu Asp Pro Glu Ala Tyr
        35                  40                  45
Gly Ser Pro Cys Ala Arg Gly Ser Gln Pro Trp Gln Val Ser Leu Phe
    50                  55                  60
Asn Gly Leu Ser Phe His Cys Ala Gly Val Leu Val Asp Gln Ser Trp
65                  70                  75                  80
Val Leu Thr Ala Ala His Cys Gly Asn Lys Pro Leu Trp Ala Arg Val
                85                  90                  95
Gly Asp Asp His Leu Leu Leu Leu Gln Gly Glu Gln Leu Arg Arg Thr
            100                 105                 110
Thr Arg Ser Val Val His Pro Lys Tyr His Gln Gly Ser Gly Pro Ile
        115                 120                 125
Leu Pro Arg Arg Thr Asp Glu His Asp Leu Met Leu Leu Lys Leu Ala
    130                 135                 140
Arg Pro Val Val Leu Gly Pro Arg Val Arg Ala Leu Gln Leu Pro Tyr
145                 150                 155                 160
Arg Cys Ala Gln Pro Gly Asp Gln Cys Gln Val Ala Gly Trp Gly Thr
                165                 170                 175
Thr Ala Ala Arg Arg Val Lys Tyr Asn Lys Gly Leu Thr Cys Ser Ser

                180                 185                 190
Ile Thr Ile Leu Ser Pro Lys Glu Cys Glu Val Phe Tyr Pro Gly Val
            195                 200                 205
Val Thr Asn Asn Met Ile Cys Ala Gly Leu Asp Arg Gly Gln Asp Pro
    210                 215                 220
Cys Gln Ser Asp Ser Gly Gly Pro Leu Val Cys Asp Glu Thr Leu Gln
225                 230                 235                 240
Gly Ile Leu Ser Trp Gly Val Tyr Pro Cys Gly Ser Ala Gln His Pro
                245                 250                 255
Ala Val Tyr Thr Gln Ile Cys Lys Tyr Met Ser Trp Ile Asn Lys Val
            260                 265                 270
Ile Arg Ser Asn
        275

<210> 20
<211> 1580
<212> DNA
<213> Homo sapiens

<400> 20
catcctgcca cccctagcct tgctggggac gtgaaccctc tccccgcgcc tgggaagcct 60
tcttggcacc gggacccgga gaatccccac ggaagccagt tccaaaaggg atgaaaaggg 120
ggcgtttcgg gcactgggag aagcctgtat tccagggccc ctcccagagc aggaatctgg 180
gacccaggag tgccagcctc acccacgcag atcctggcca tgagagctcc gcacctccac 240
ctctccgccg cctctggcgc ccgggctctg gcgaagctgc tgccgctgct gatggcgcaa 300
ctctgggccg cagaggcggc gctgctcccc caaaacgaca cgcgcttgga ccccgaagcc 360
tatggctccc cgtgcgcgcg cggctcgcag ccctggcagg tctcgctctt caacggcctc 420
tcgttccact gcgcgggtgt cctggtggac cagagttggg tgctgacggc cgcgcactgc 480
ggaaacaagc cactgtgggc tcgagtaggg gatgaccacc tgctgcttct tcagggagag 540
cagctccgcc ggaccactcg ctctgttgtc catcccaagt accaccaggg ctcaggcccc 600
atcctgccaa ggcgaacgga tgagcacgat ctcatgttgc tgaagctggc caggcccgta 660
gtgctggggc cccgcgtccg ggccctgcag cttccctacc gctgtgctca gcccggagac 720
cagtgccagg ttgctggctg gggcaccacg gccgcccgga gagtgaagta caacaagggc 780
ctgacctgct ccagcatcac tatcctgagc cctaaagagt gtgaggtctt ctaccctggc 840
gtggtcacca acaacatgat atgtgctgga ctggaccggg gccaggaccc ttgccagagt 900
gactctggag gccccctggt ctgtgacgag accctccaag gcatcctctc gtggggtgtt 960
tacccctgtg gctctgccca gcatccagct gtctacaccc agatctgcaa atacatgtcc 1020
tggatcaata aagtcatacg ctccaactga tccagatgct acgctccagc tgatccagat 1080
gttatgctcc tgctgatcca gatgcccaga ggctccatcg tccatcctct tcctccccag 1140
tcggctgaac tctccccttg tctgcactgt tcaaacctct gccgccctcc acacctctaa 1200
acatctcccc tctcacctca ttcccccacc tatccccatt ctctgcctgt actgaagctg 1260
aaatgcagga agtggtggca aaggtttatt ccagagaagc caggaagccg gtcatcaccc 1320
agcctctgag agcagttact ggggtcaccc aacctgactt cctctgccac tccctgctgt 1380
gtgactttgg gcaagccaag tgccctctct gaacctcagt ttcctcatct gcaaaatggg 1440
aacaatgacg tgcctacctc ttagacatgt tgtgaggaga ctatgatata acatgtgtat 1500
gtaaatcttc atggtgattg tcatgtaagg cttaacacag tgggtggtga gttctgacta 1560
aaggttacct gttgtcgtga                                              1580

<210> 21
<211> 276
<212> PRT
<213> Homo sapiens

<400> 21
Met Arg Ala Pro His Leu His Leu Ser Ala Ala Ser Gly Ala Arg Ala
1               5                   10                  15
Leu Ala Lys Leu Leu Pro Leu Leu Met Ala Gln Leu Trp Ala Ala Glu
            20                  25                  30
Ala Ala Leu Leu Pro Gln Asn Asp Thr Arg Leu Asp Pro Glu Ala Tyr
        35                  40                  45
Gly Ser Pro Cys Ala Arg Gly Ser Gln Pro Trp Gln Val Ser Leu Phe
    50                  55                  60
Asn Gly Leu Ser Phe His Cys Ala Gly Val Leu Val Asp Gln Ser Trp
65                  70                  75                  80
Val Leu Thr Ala Ala His Cys Gly Asn Lys Pro Leu Trp Ala Arg Val
                85                  90                  95
Gly Asp Asp His Leu Leu Leu Leu Gln Gly Glu Gln Leu Arg Arg Thr

                100                 105                 110
Thr Arg Ser Val Val His Pro Lys Tyr His Gln Gly Ser Gly Pro Ile
        115                 120                 125
Leu Pro Arg Arg Thr Asp Glu His Asp Leu Met Leu Leu Lys Leu Ala
    130                 135                 140
Arg Pro Val Val Leu Gly Pro Arg Val Arg Ala Leu Gln Leu Pro Tyr
145                 150                 155                 160
Arg Cys Ala Gln Pro Gly Asp Gln Cys Gln Val Ala Gly Trp Gly Thr
                165                 170                 175
Thr Ala Ala Arg Arg Val Lys Tyr Asn Lys Gly Leu Thr Cys Ser Ser
            180                 185                 190
Ile Thr Ile Leu Ser Pro Lys Glu Cys Glu Val Phe Tyr Pro Gly Val
        195                 200                 205
Val Thr Asn Asn Met Ile Cys Ala Gly Leu Asp Arg Gly Gln Asp Pro
    210                 215                 220
Cys Gln Ser Asp Ser Gly Gly Pro Leu Val Cys Asp Glu Thr Leu Gln
225                 230                 235                 240
Gly Ile Leu Ser Trp Gly Val Tyr Pro Cys Gly Ser Ala Gln His Pro
                245                 250                 255
Ala Val Tyr Thr Gln Ile Cys Lys Tyr Met Ser Trp Ile Asn Lys Val
            260                 265                 270
Ile Arg Ser Asn
        275


<210> 22
<211> 1443
<212> DNA
<213> Homo sapiens

<400> 22
accagcggca gaccacaggc agggcagagg cacgtctggg tcccctccct ccttcctatc 60
ggcgactccc aggatcctgg ccatgagagc tccgcacctc cacctctccg ccgcctctgg 120
cgcccgggct ctggcgaagc tgctgccgct gctgatggcg caactctggg ccgcagaggc 180
ggcgctgctc ccccaaaacg acacgcgctt ggaccccgaa gcctatggct ccccgtgcgc 240
gcgcggctcg cagccctggc aggtctcgct cttcaacggc ctctcgttcc actgcgcggg 300
tgtcctggtg gaccagagtt gggtgctgac ggccgcgcac tgcggaaaca agccactgtg 360
ggctcgagta ggggatgacc acctgctgct tcttcaggga gagcagctcc gccggaccac 420
tcgctctgtt gtccatccca agtaccacca gggctcaggc cccatcctgc caaggcgaac 480
ggatgagcac gatctcatgt tgctgaagct ggccaggccc gtagtgctgg ggccccgcgt 540
ccgggccctg cagcttccct accgctgtgc tcagcccgga gaccagtgcc aggttgctgg 600
ctggggcacc acggccgccc ggagagtgaa gtacaacaag ggcctgacct gctccagcat 660
cactatcctg agccctaaag agtgtgaggt cttctaccct ggcgtggtca ccaacaacat 720
gatatgtgct ggactggacc ggggccagga cccttgccag agtgactctg gaggccccct 780
ggtctgtgac gagaccctcc aaggcatcct ctcgtggggt gtttacccct gtggctctgc 840
ccagcatcca gctgtctaca cccagatctg caaatacatg tcctggatca ataaagtcat 900
acgctccaac tgatccagat gctacgctcc agctgatcca gatgttatgc tcctgctgat 960
ccagatgccc agaggctcca tcgtccatcc tcttcctccc cagtcggctg aactctcccc 1020
ttgtctgcac tgttcaaacc tctgccgccc tccacacctc taaacatctc ccctctcacc 1080
tcattccccc acctatcccc attctctgcc tgtactgaag ctgaaatgca ggaagtggtg 1140
gcaaaggttt attccagaga agccaggaag ccggtcatca cccagcctct gagagcagtt 1200
actggggtca cccaacctga cttcctctgc cactccctgc tgtgtgactt tgggcaagcc 1260
aagtgccctc tctgaacctc agtttcctca tctgcaaaat gggaacaatg acgtgcctac 1320
ctcttagaca tgttgtgagg agactatgat ataacatgtg tatgtaaatc ttcatggtga 1380
ttgtcatgta aggcttaaca cagtgggtgg tgagttctga ctaaaggtta cctgttgtcg 1440
tga                                                               1443

<210> 23
<211> 180
<212> PRT
<213> Homo sapiens

<400> 23
Met Leu Cys Leu Leu Leu Thr Leu Gly Val Ala Leu Val Cys Gly Val
1               5                   10                  15
Pro Ala Met Asp Ile Pro Gln Thr Lys Gln Asp Leu Glu Leu Pro Lys
            20                  25                  30
Leu Ala Gly Thr Trp His Ser Met Ala Met Ala Thr Asn Asn Ile Ser

35                  40                  45
Leu Met Ala Thr Leu Lys Ala Pro Leu Arg Val His Ile Thr Ser Leu
    50                  55                  60
Leu Pro Thr Pro Glu Asp Asn Leu Glu Ile Val Leu His Arg Trp Glu
65                  70                  75                  80
Asn Asn Ser Cys Val Glu Lys Lys Val Leu Gly Glu Lys Thr Glu Asn
                85                  90                  95
Pro Lys Lys Phe Lys Ile Asn Tyr Thr Val Ala Asn Glu Ala Thr Leu
            100                 105                 110
Leu Asp Thr Asp Tyr Asp Asn Phe Leu Phe Leu Cys Leu Gln Asp Thr
        115                 120                 125
Thr Thr Pro Ile Gln Ser Met Met Cys Gln Tyr Leu Ala Arg Val Leu
    130                 135                 140
Val Glu Asp Asp Glu Ile Met Gln Gly Phe Ile Arg Ala Phe Arg Pro
145                 150                 155                 160
Leu Pro Arg His Leu Trp Tyr Leu Leu Asp Leu Lys Gln Met Glu Glu
                165                 170                 175
Pro Cys Arg Phe
            180

<210> 24
<211> 857
<212> DNA
<213> Homo sapiens

<400> 24
catccctctg gctccagagc tcagagccac ccacagccgc agccatgctg tgcctcctgc 60
tcaccctggg cgtggccctg gtctgtggtg tcccggccat ggacatcccc cagaccaagc 120
aggacctgga gctcccaaag ttggcaggga cctggcactc catggccatg gcgaccaaca 180
acatctccct catggcgaca ctgaaggccc ctctgagggt ccacatcacc tcactgttgc 240
ccacccccga ggacaacctg gagatcgttc tgcacagatg ggagaacaac agctgtgttg 300
agaagaaggt ccttggagag aagactgaga atccaaagaa gttcaagatc aactatacgg 360
tggcgaacga ggccacgctg ctcgatactg actacgacaa tttcctgttt ctctgcctac 420
aggacaccac cacccccatc cagagcatga tgtgccagta cctggccaga gtcctggtgg 480
aggacgatga gatcatgcag ggattcatca gggctttcag gcccctgccc aggcacctat 540
ggtacttgct ggacttgaaa cagatggaag agccgtgccg tttctaggtg agctcctgcc 600
tggtcctgcc tcctggctca cctccgcctc caggaagacc agactcccac ccttccacac 660
ctccagagca gtgggacttc ctcctgccct ttcaaagaat aaccacagct cagaagacga 720
tgacgtggtc atctgtgtcg ccatcccctt cctgctgcac acctgcacca cggccatggg 780
gaggctgctc cctgggggca gagtctctgg cagaggttat taataaaccc ttggagcatg 840
aaaaaaaaaa aaaaaaa                                                857

<210> 25
<211> 180
<212> PRT
<213> Homo sapiens

<400> 25
Met Leu Cys Leu Leu Leu Thr Leu Gly Val Ala Leu Val Cys Gly Val
1               5                   10                  15
Pro Ala Met Asp Ile Pro Gln Thr Lys Gln Asp Leu Glu Leu Pro Lys
            20                  25                  30
Leu Ala Gly Thr Trp His Ser Met Ala Met Ala Thr Asn Asn Ile Ser
        35                  40                  45
Leu Met Ala Thr Leu Lys Ala Pro Leu Arg Val His Ile Thr Ser Leu
    50                  55                  60
Leu Pro Thr Pro Glu Asp Asn Leu Glu Ile Val Leu His Arg Trp Glu
65                  70                  75                  80
Asn Asn Ser Cys Val Glu Lys Lys Val Leu Gly Glu Lys Thr Glu Asn
                85                  90                  95
Pro Lys Lys Phe Lys Ile Asn Tyr Thr Val Ala Asn Glu Ala Thr Leu
            100                 105                 110
Leu Asp Thr Asp Tyr Asp Asn Phe Leu Phe Leu Cys Leu Gln Asp Thr
        115                 120                 125
Thr Thr Pro Ile Gln Ser Met Met Cys Gln Tyr Leu Ala Arg Val Leu
    130                 135                 140
Val Glu Asp Asp Glu Ile Met Gln Gly Phe Ile Arg Ala Phe Arg Pro

145                 150                 155                 160
Leu Pro Arg His Leu Trp Tyr Leu Leu Asp Leu Lys Gln Met Glu Glu
                165                 170                 175
Pro Cys Arg Phe
            180

<210> 26
<211> 828
<212> DNA
<213> Homo sapiens

<400> 26
catccctctg gctccagagc tcagagccac ccacagccgc agccatgctg tgcctcctgc 60
tcaccctggg cgtggccctg gtctgtggtg tcccggccat ggacatcccc cagaccaagc 120
aggacctgga gctcccaaag ttggcaggga cctggcactc catggccatg gcgaccaaca 180
acatctccct catggcgaca ctgaaggccc ctctgagggt ccacatcacc tcactgttgc 240
ccacccccga ggacaacctg gagatcgttc tgcacagatg ggagaacaac agctgtgttg 300
agaagaaggt ccttggagag aagactgaga atccaaagaa gttcaagatc aactatacgg 360
tggcgaacga ggccacgctg ctcgatactg actacgacaa tttcctgttt ctctgcctac 420
aggacaccac cacccccatc cagagcatga tgtgccagta cctggccaga gtcctggtgg 480
aggacgatga gatcatgcag ggattcatca gggctttcag gcccctgccc aggcacctat 540
ggtacttgct ggacttgaaa cagatggaag agccgtgccg tttctagctc acctccgcct 600
ccaggaagac cagactccca cccttccaca cctccagagc agtgggactt cctcctgccc 660
tttcaaagaa taaccacagc tcagaagacg atgacgtggt catctgtgtc gccatcccct 720
tcctgctgca cacctgcacc acggccatgg ggaggctgct ccctgggggc agagtctctg 780
gcagaggtta ttaataaacc cttggagcat gaaaaaaaaa aaaaaaaa          828

<210> 27
<211> 402
<212> PRT
<213> Homo sapiens

<400> 27

<210> 28
<211> 2876
<212> DNA
<213> Homo sapiens

<400> 28
gaattcctgc agctcagcag ccgccgccag agcaggacga accgccaatc gcaaggcacc 60
tctgagaact tcaggatgca gatgtctcca gccctcacct gcctagtcct gggcctggcc 120
cttgtctttg gtgaagggtc tgctgtgcac catcccccat cctacgtggc ccacctggcc 180
tcagacttcg gggtgagggt gtttcagcag gtggcgcagg cctccaagga ccgcaacgtg 240
gttttctcac cctatggggt ggcctcggtg ttggccatgc tccagctgac aacaggagga 300
gaaacccagc agcagattca agcagctatg ggattcaaga ttgatgacaa gggcatggcc 360
cccgccctcc ggcatctgta caaggagctc atggggccat ggaacaagga tgagatcagc 420
accacagacg cgatcttcgt ccagcgggat ctgaagctgg tccagggctt catgccccac 480
ttcttcaggc tgttccggag cacggtcaag caagtggact tttcagaggt ggagagagcc 540
agattcatca tcaatgactg ggtgaagaca cacacaaaag gtatgatcag caacttgctt 600
gggaaaggag ccgtggacca gctgacacgg ctggtgctgg tgaatgccct ctacttcaac 660
ggccagtgga agactccctt ccccgactcc agcacccacc gccgcctctt ccacaaatca 720
gacggcagca ctgtctctgt gcccatgatg gctcagacca acaagttcaa ctatactgag 780
ttcaccacgc ccgatggcca ttactacgac atcctggaac tgccctacca cggggacacc 840
ctcagcatgt tcattgctgc cccttatgaa aaagaggtgc ctctctctgc cctcaccaac 900
attctgagtg cccagctcat cagccactgg aaaggcaaca tgaccaggct gccccgcctc 960
ctggttctgc ccaagttctc cctggagact gaagtcgacc tcaggaagcc cctagagaac 1020
ctgggaatga ccgacatgtt cagacagttt caggctgact tcacgagtct ttcagaccaa 1080
gagcctctcc acgtcgcgca ggcgctgcag aaagtgaaga tcgaggtgaa cgagagtggc 1140
acggtggcct cctcatccac agctgtcata gtctcagccc gcatggcccc cgaggagatc 1200
atcatggaca gacccttcct ctttgtggtc cggcacaacc ccacaggaac agtccttttc 1260
atgggccaag tgatggaacc ctgaccctgg ggaaagacgc cttcatctgg gacaaaactg 1320
gagatgcatc gggaaagaag aaactccgaa gaaaagaatt ttagtgttaa tgactctttc 1380
tgaaggaaga gaagacattt gccttttgtt aaaagatggt aaaccagatc tgtctccaag 1440
accttggcct ctccttggag gacctttagg tcaaactccc tagtctccac ctgagaccct 1500
gggagagaag tttgaagcac aactccctta aggtctccaa accagacggt gacgcctgcg 1560
ggaccatctg gggcacctgc ttccacccgt ctctctgccc actcgggtct gcagacctgg 1620
ttcccactga ggccctttgc aggatggaac tacggggctt acaggagctt ttgtgtgcct 1680
ggtagaaact atttctgttc cagtcacatt gccatcactc ttgtactgcc tgccaccgcg 1740
gaggaggctg gtgacaggcc aaaggccagt ggaagaaaca ccctttcatc tcagagtcca 1800
ctgtggcact ggccacccct ccccagtaca ggggtgctgc aggtggcaga gtgaatgtcc 1860
cccatcatgt ggcccaactc tcctggcctg gccatctccc tccccagaaa cagtgtgcat 1920
gggttatttt ggagtgtagg tgacttgttt actcattgaa gcagatttct gcttcctttt 1980
atttttatag gaatagagga agaaatgtca gatgcgtgcc cagctcttca ccccccaatc 2040
tcttggtggg gaggggtgta cctaaatatt tatcatatcc ttgcccttga gtgcttgtta 2100
gagagaaaga gaactactaa ggaaaataat attatttaaa ctcgctccta gtgtttcttt 2160
gtggtctgtg tcaccgtatc tcaggaagtc cagccacttg actggcacac acccctccgg 2220
acatccagcg tgacggagcc cacactgcca ccttgtggcc gcctgagacc ctcgcgcccc 2280
ccgcgccccc cgcgcccctc tttttcccct tgatggaaat tgaccataca atttcatcct 2340
ccttcagggg atcaaaagga cggagtgggg ggacagagac tcagatgagg acagagtggt 2400
ttccaatgtg ttcaatagat ttaggagcag aaatgcaagg ggctgcatga cctaccagga 2460
cagaactttc cccaattaca gggtgactca cagccgcatt ggtgactcac ttcaatgtgt 2520

catttccggc tgctgtgtgt gagcagtgga cacgtgaggg gggggtgggt gagagagaca 2580
ggcagctcgg attcaactac cttagataat atttctgaaa acctaccagc cagagggtag 2640
ggcacaaaga tggatgtaat gcactttggg aggccaaggc gggaggattg cttgagccca 2700
ggagttcaag accagcctgg gcaacatacc aagacccccg tctctttaaa aatatatata 2760
ttttaaatat acttaaatat atatttctaa tatctttaaa tatatatata tattttaaag 2820
accaatttat gggagaattg cacacagatg tgaaatgaat gtaatctaat agaagc 2876

<210> 29
<211> 273
<212> PRT
<213> Homo sapiens

<400> 29
Met Thr Pro Gly Thr Gln Ser Pro Phe Phe Leu Leu Leu Leu Leu Thr
1               5                   10                  15
Val Leu Thr Val Val Thr Gly Ser Gly His Ala Ser Ser Thr Pro Gly
            20                  25                  30
Gly Glu Lys Glu Thr Ser Ala Thr Gln Arg Ser Ser Val Pro Ser Ser
        35                  40                  45
Thr Glu Lys Asn Ala Leu Ser Thr Gly Val Ser Phe Phe Phe Leu Ser
    50                  55                  60
Phe His Ile Ser Asn Leu Gln Phe Asn Ser Ser Leu Glu Asp Pro Ser
65                  70                  75                  80
Thr Asp Tyr Tyr Gln Glu Leu Gln Arg Asp Ile Ser Glu Met Phe Leu
                85                  90                  95
Gln Ile Tyr Lys Gln Gly Gly Phe Leu Gly Leu Ser Asn Ile Lys Phe
            100                 105                 110
Arg Pro Gly Ser Val Val Val Gln Leu Thr Leu Ala Phe Arg Glu Gly
        115                 120                 125
Thr Ile Asn Val His Asp Val Glu Thr Gln Phe Asn Gln Tyr Lys Thr
    130                 135                 140
Glu Ala Ala Ser Arg Tyr Asn Leu Thr Ile Ser Asp Val Ser Val Ser
145                 150                 155                 160
Asp Val Pro Phe Pro Phe Ser Ala Gln Ser Gly Ala Gly Val Pro Gly
                165                 170                 175
Trp Gly Ile Ala Leu Leu Val Leu Val Cys Val Leu Val Ala Leu Ala
            180                 185                 190
Ile Val Tyr Leu Ile Ala Leu Ala Val Cys Gln Cys Arg Arg Lys Asn
        195                 200                 205
Tyr Gly Gln Leu Asp Ile Phe Pro Ala Arg Asp Thr Tyr His Pro Met
    210                 215                 220
Ser Glu Tyr Pro Thr Tyr His Thr His Gly Arg Tyr Val Pro Pro Ser
225                 230                 235                 240
Ser Thr Asp Arg Ser Pro Tyr Glu Lys Val Ser Ala Gly Asn Gly Gly
                245                 250                 255
Ser Ser Leu Ser Tyr Thr Asn Pro Ala Val Ala Ala Thr Ser Ala Asn
            260                 265                 270
Leu

<210> 30
<211> 1209
<212> DNA
<213> Homo sapiens

<400> 30
acctctcaag cagccagcgc ctgcctgaat ctgttctgcc ccctccccac ccatttcacc 60
accaccatga caccgggcac ccagtctcct ttcttcctgc tgctgctcct cacagtgctt 120
acagttgtta cgggttctgg tcatgcaagc tctaccccag gtggagaaaa ggagacttcg 180
gctacccaga gaagttcagt gcccagctct actgagaaga atgctttgtc tactggggtc 240
tctttctttt tcctgtcttt tcacatttca aacctccagt ttaattcctc tctggaagat 300
cccagcaccg actactacca agagctgcag agagacattt ctgaaatgtt tttgcagatt 360
tataaacaag ggggttttct gggcctctcc aatattaagt tcaggccagg atctgtggtg 420
gtacaattga ctctggcctt ccgagaaggt accatcaatg tccacgacgt ggagacacag 480
ttcaatcagt ataaaacgga agcagcctct cgatataacc tgacgatctc agacgtcagc 540
gtgagtgatg tgccatttcc tttctctgcc cagtctgggg ctggggtgcc aggctggggc 600
atcgcgctgc tggtgctggt ctgtgttctg gttgcgctgg ccattgtcta tctcattgcc 660

ttggctgtct gtcagtgccg ccgaaagaac tacgggcagc tggacatctt tccagcccgg 720
gatacctacc atcctatgag cgagtacccc acctaccaca cccatgggcg ctatgtgccc 780
cctagcagta ccgatcgtag cccctatgag aaggtttctg caggtaatgg tggcagcagc 840
ctctcttaca caaacccagc agtggcagcc acttctgcca acttgtaggg gcacgtcgcc 900
cgctgagctg agtggccagc cagtgccatt ccactccact caggttcttc agggccagag 960
cccctgcacc ctgtttgggc tggtgagctg ggagttcagg tgggctgctc acagcctcct 1020
tcagaggccc caccaatttc tcggacactt ctcagtgtgt ggaagctcat gtgggcccct 1080
gagggctcat gcctgggaag tgttgtggtg gggctcccca ggaggactgg cccagagagc 1140
cctgagatag cggggatcct gaactggact gaataaaacg tggtctccca ctgcgccaaa 1200
aaaaaaaaa 1209

<210> 31
<211> 264
<212> PRT
<213> Homo sapiens

<400> 31
Met Thr Pro Gly Thr Gln Ser Pro Phe Phe Leu Leu Leu Leu Leu Thr
1               5                   10                  15
Val Leu Thr Ala Thr Thr Ala Pro Lys Pro Ala Thr Val Val Thr Gly
            20                  25                  30
Ser Gly His Ala Ser Ser Thr Pro Gly Gly Glu Lys Glu Thr Ser Ala
        35                  40                  45
Thr Gln Arg Ser Ser Val Pro Ser Ser Thr Glu Lys Asn Ala Phe Asn
    50                  55                  60
Ser Ser Leu Glu Asp Pro Ser Thr Asp Tyr Tyr Gln Glu Leu Gln Arg
65                  70                  75                  80
Asp Ile Ser Glu Met Phe Leu Gln Ile Tyr Lys Gln Gly Gly Phe Leu
                85                  90                  95
Gly Leu Ser Asn Ile Lys Phe Arg Pro Gly Ser Val Val Val Gln Leu
            100                 105                 110
Thr Leu Ala Phe Arg Glu Gly Thr Ile Asn Val His Asp Val Glu Thr
        115                 120                 125
Gln Phe Asn Gln Tyr Lys Thr Glu Ala Ala Ser Arg Tyr Asn Leu Thr
    130                 135                 140
Ile Ser Asp Val Ser Val Ser Asp Val Pro Phe Pro Phe Ser Ala Gln
145                 150                 155                 160
Ser Gly Ala Gly Val Pro Gly Trp Gly Ile Ala Leu Leu Val Leu Val
                165                 170                 175
Cys Val Leu Val Ala Leu Ala Ile Val Tyr Leu Ile Ala Leu Ala Val
            180                 185                 190
Cys Gln Cys Arg Arg Lys Asn Tyr Gly Gln Leu Asp Ile Phe Pro Ala
        195                 200                 205
Arg Asp Thr Tyr His Pro Met Ser Glu Tyr Pro Thr Tyr His Thr His
    210                 215                 220
Gly Arg Tyr Val Pro Pro Ser Ser Thr Asp Arg Ser Pro Tyr Glu Lys
225                 230                 235                 240
Val Ser Ala Gly Asn Gly Gly Ser Ser Leu Ser Tyr Thr Asn Pro Ala
                245                 250                 255
Val Ala Ala Thr Ser Ala Asn Leu
            260

<210> 32
<211> 1182
<212> DNA
<213> Homo sapiens

<400> 32
acctctcaag cagccagcgc ctgcctgaat ctgttctgcc ccctccccac ccatttcacc 60
accaccatga caccgggcac ccagtctcct ttcttcctgc tgctgctcct cacagtgctt 120
acagctacca cagcccctaa acccgcaaca gttgttacgg gttctggtca tgcaagctct 180
accccaggtg gagaaaagga gacttcggct acccagagaa gttcagtgcc cagctctact 240
gagaagaatg cttttaattc ctctctggaa gatcccagca ccgactacta ccaagagctg 300
cagagagaca tttctgaaat gtttttgcag atttataaac aagggggttt tctgggcctc 360
tccaatatta agttcaggcc aggatctgtg gtggtacaat tgactctggc cttccgagaa 420
ggtaccatca atgtccacga cgtggagaca cagttcaatc agtataaaac ggaagcagcc 480
tctcgatata acctgacgat ctcagacgtc agcgtgagtg atgtgccatt tcctttctct 540

gcccagtctg gggctggggt gccaggctgg ggcatcgcgc tgctggtgct ggtctgtgtt 600
ctggttgcgc tggccattgt ctatctcatt gccttggctg tctgtcagtg ccgccgaaag 660
aactacgggc agctggacat ctttccagcc cgggatacct accatcctat gagcgagtac 720
cccacctacc acacccatgg gcgctatgtg ccccctagca gtaccgatcg tagcccctat 780
gagaaggttt ctgcaggtaa tggtggcagc agcctctctt acacaaaccc agcagtggca 840
gccacttctg ccaacttgta ggggcacgtc gcccgctgag ctgagtggcc agccagtgcc 900
attccactcc actcaggttc ttcagggcca gagcccctgc accctgtttg ggctggtgag 960
ctgggagttc aggtgggctg ctcacagcct ccttcagagg ccccaccaat ttctcggaca 1020
cttctcagtg tgtggaagct catgtgggcc cctgagggct catgcctggg aagtgttgtg 1080
gtgggggctc ccaggaggac tggcccagag agccctgaga tagcggggat cctgaactgg 1140
actgaataaa acgtggtctc ccactgcgcc aaaaaaaaaa aa 1182

<210> 33
<211> 255
<212> PRT
<213> Homo sapiens

<400> 33
Met Thr Pro Gly Thr Gln Ser Pro Phe Phe Leu Leu Leu Leu Leu Thr
1               5                   10                  15
Val Leu Thr Val Val Thr Gly Ser Gly His Ala Ser Ser Thr Pro Gly
            20                  25                  30
Gly Glu Lys Glu Thr Ser Ala Thr Gln Arg Ser Ser Val Pro Ser Ser
        35                  40                  45
Thr Glu Lys Asn Ala Phe Asn Ser Ser Leu Glu Asp Pro Ser Thr Asp
    50                  55                  60
Tyr Tyr Gln Glu Leu Gln Arg Asp Ile Ser Glu Met Phe Leu Gln Ile
65                  70                  75                  80
Tyr Lys Gln Gly Gly Phe Leu Gly Leu Ser Asn Ile Lys Phe Arg Pro
                85                  90                  95
Gly Ser Val Val Val Gln Leu Thr Leu Ala Phe Arg Glu Gly Thr Ile
            100                 105                 110
Asn Val His Asp Val Glu Thr Gln Phe Asn Gln Tyr Lys Thr Glu Ala
        115                 120                 125
Ala Ser Arg Tyr Asn Leu Thr Ile Ser Asp Val Ser Val Ser Asp Val
    130                 135                 140
Pro Phe Pro Phe Ser Ala Gln Ser Gly Ala Gly Val Pro Gly Trp Gly
145                 150                 155                 160
Ile Ala Leu Leu Val Leu Val Cys Val Leu Val Ala Leu Ala Ile Val
                165                 170                 175
Tyr Leu Ile Ala Leu Ala Val Cys Gln Cys Arg Arg Lys Asn Tyr Gly
            180                 185                 190
Gln Leu Asp Ile Phe Pro Ala Arg Asp Thr Tyr His Pro Met Ser Glu
        195                 200                 205
Tyr Pro Thr Tyr His Thr His Gly Arg Tyr Val Pro Pro Ser Ser Thr
    210                 215                 220
Asp Arg Ser Pro Tyr Glu Lys Val Ser Ala Gly Asn Gly Gly Ser Ser
225                 230                 235                 240
Leu Ser Tyr Thr Asn Pro Ala Val Ala Ala Thr Ser Ala Asn Leu
                245                 250                 255

<210> 34
<211> 1155
<212> DNA
<213> Homo sapiens

<400> 34
acctctcaag cagccagcgc ctgcctgaat ctgttctgcc ccctccccac ccatttcacc 60
accaccatga caccgggcac ccagtctcct ttcttcctgc tgctgctcct cacagtgctt 120
acagttgtta cgggttctgg tcatgcaagc tctaccccag gtggagaaaa ggagacttcg 180
gctacccaga gaagttcagt gcccagctct actgagaaga atgcttttaa ttcctctctg 240
gaagatccca gcaccgacta ctaccaagag ctgcagagag acatttctga aatgtttttg 300
cagatttata aacaaggggg ttttctgggc ctctccaata ttaagttcag gccaggatct 360
gtggtggtac aattgactct ggccttccga gaaggtacca tcaatgtcca cgacgtggag 420
acacagttca atcagtataa aacggaagca gcctctcgat ataacctgac gatctcagac 480
gtcagcgtga gtgatgtgcc atttcctttc tctgcccagt ctggggctgg ggtgccaggc 540
tggggcatcg cgctgctggt gctggtctgt gttctggttg cgctggccat tgtctatctc 600

attgccttgg ctgtctgtca gtgccgccga aagaactacg ggcagctgga catctttcca 660
gcccgggata cctaccatcc tatgagcgag taccccacct accacaccca tgggcgctat 720
gtgcccccta gcagtaccga tcgtagcccc tatgagaagg tttctgcagg taatggtggc 780
agcagcctct cttacacaaa cccagcagtg gcagccactt ctgccaactt gtaggggcac 840
gtcgcccgct gagctgagtg gccagccagt gccattccac tccactcagg ttcttcaggg 900
ccagagcccc tgcaccctgt ttgggctggt gagctgggag ttcaggtggg ctgctcacag 960
cctccttcag aggccccacc aatttctcgg acacttctca gtgtgtggaa gctcatgtgg 1020
gcccctgagg gctcatgcct gggaagtgtt gtggtggggg ctcccaggag gactggccca 1080
gagagccctg agatagcggg gatcctgaac tggactgaat aaaacgtggt ctcccactgc 1140
gccaaaaaaa aaaaa 1155

<210> 35
<211> 96
<212> PRT
<213> Homo sapiens

<400> 35
Met Thr Pro Gly Thr Gln Ser Pro Phe Phe Leu Leu Leu Leu Leu Thr
1               5                   10                  15
Val Leu Thr Val Val Thr Gly Ser Gly His Ala Ser Ser Thr Pro Gly
            20                  25                  30
Gly Glu Lys Glu Thr Ser Ala Thr Gln Arg Ser Ser Val Pro Ser Ser
        35                  40                  45
Thr Glu Lys Asn Ala Ile Pro Ala Pro Thr Thr Thr Lys Ser Cys Arg
    50                  55                  60
Glu Thr Phe Leu Lys Cys Phe Cys Arg Phe Ile Asn Lys Gly Val Phe
65                  70                  75                  80
Trp Ala Ser Pro Ile Leu Ser Ser Gly Gln Asp Leu Trp Trp Tyr Asn
                85                  90                  95

<210> 36
<211> 1136
<212> DNA
<213> Homo sapiens

<400> 36
acctctcaag cagccagcgc ctgcctgaat ctgttctgcc ccctccccac ccatttcacc 60
accaccatga caccgggcac ccagtctcct ttcttcctgc tgctgctcct cacagtgctt 120
acagttgtta cgggttctgg tcatgcaagc tctaccccag gtggagaaaa ggagacttcg 180
gctacccaga gaagttcagt gcccagctct actgagaaga atgctatccc agcaccgact 240
actaccaaga gctgcagaga gacatttctg aaatgttttt gcagatttat aaacaagggg 300
gttttctggg cctctccaat attaagttca ggccaggatc tgtggtggta caattgactc 360
tggccttccg agaaggtacc atcaatgtcc acgacgtgga gacacagttc aatcagtata 420
aaacggaagc agcctctcga tataacctga cgatctcaga cgtcagcgtg agtgatgtgc 480
catttccttt ctctgcccag tctggggctg gggtgccagg ctggggcatc gcgctgctgg 540
tgctggtctg tgttctggtt gcgctggcca ttgtctatct cattgccttg gctgtctgtc 600
agtgccgccg aaagaactac gggcagctgg acatctttcc agcccgggat acctaccatc 660
ctatgagcga gtaccccacc taccacaccc atgggcgcta tgtgccccct agcagtaccg 720
atcgtagccc ctatgagaag gtttctgcag gtaatggtgg cagcagcctc tcttacacaa 780
acccagcagt ggcagccact tctgccaact tgtaggggca cgtcgcccgc tgagctgagt 840
ggccagccag tgccattcca ctccactcag gttcttcagg gccagagccc ctgcaccctg 900
tttgggctgg tgagctggga gttcaggtgg gctgctcaca gcctccttca gaggccccac 960
caatttctcg gacacttctc agtgtgtgga agctcatgtg ggcccctgag ggctcatgcc 1020
tgggaagtgt tgtggtgggg gctcccagga ggactggccc agagagccct gagatagcgg 1080
ggatcctgaa ctggactgaa taaaacgtgg tctcccactg cgccaaaaaa aaaaaa 1136

<210> 37
<211> 418
<212> PRT
<213> Homo sapiens

<400> 37

<210> 38
<211> 1607
<212> DNA
<213> Homo sapiens

<400> 38
aatgactcct ttcggtaagt gcagtggaag ctgtacactg cccaggcaaa gcgtccgggc 60
agcgtaggcg ggcgactcag atcccagcca gtggacttag cccctgtttg ctcctccgat 120
aactggggtg accttggtta atattcacca gcagcctccc ccgttgcccc tctggatcca 180
ctgcttaaat acggacgagg acagggccct gtctcctcag cttcaggcac caccactgac 240
ctgggacagt gaatcgacaa tgccgtcttc tgtctcgtgg ggcatcctcc tgctggcagg 300
cctgtgctgc ctggtccctg tctccctggc tgaggatccc cagggagatg ctgcccagaa 360
gacagataca tcccaccatg atcaggatca cccaaccttc aacaagatca cccccaacct 420
ggctgagttc gccttcagcc tataccgcca gctggcacac cagtccaaca gcaccaatat 480
cttcttctcc ccagtgagca tcgctacagc ctttgcaatg ctctccctgg ggaccaaggc 540
tgacactcac gatgaaatcc tggagggcct gaatttcaac ctcacggaga ttccggaggc 600
tcagatccat gaaggcttcc aggaactcct ccgtaccctc aaccagccag acagccagct 660

ccagctgacc accggcaatg gcctgttcct cagcgagggc ctgaagctag tggataagtt 720
tttggaggat gttaaaaagt tgtaccactc agaagccttc actgtcaact tcggggacac 780
cgaagaggcc aagaaacaga tcaacgatta cgtggagaag ggtactcaag ggaaaattgt 840
ggatttggtc aaggagcttg acagagacac agtttttgct ctggtgaatt acatcttctt 900
taaaggcaaa tgggagagac cctttgaagt caaggacacc gaggaagagg acttccacgt 960
ggaccaggtg accaccgtga aggtgcctat gatgaagcgt ttaggcatgt ttaacatcca 1020
gcactgtaag aagctgtcca gctgggtgct gctgatgaaa tacctgggca atgccaccgc 1080
catcttcttc ctgcctgatg aggggaaact acagcacctg gaaaatgaac tcacccacga 1140
tatcatcacc aagttcctgg aaaatgaaga cagaaggtct gccagcttac atttacccaa 1200
actgtccatt actggaacct atgatctgaa gagcgtcctg ggtcaactgg gcatcactaa 1260
ggtcttcagc aatggggctg acctctccgg ggtcacagag gaggcacccc tgaagctctc 1320
caaggccgtg cataaggctg tgctgaccat cgacgagaaa gggactgaag ctgctggggc 1380
catgttttta gaggccatac ccatgtctat cccccccgag gtcaagttca acaaaccctt 1440
tgtcttctta atgattgaac aaaataccaa gtctcccctc ttcatgggaa aagtggtgaa 1500
tcccacccaa aaataactgc ctctcgctcc tcaacccctc ccctccatcc ctggccccct 1560
ccctggatga cattaaagaa gggttgagct ggtccctgcc tgcaaaa 1607

<210> 39
<211> 418
<212> PRT
<213> Homo sapiens

<400> 39

<210> 40
<211> 1588
<212> DNA
<213> Homo sapiens

<400> 40
tgggcaggaa ctgggcactg tgcccagggc atgcactgcc tccacgcagc aaccctcaga 60
gtcctgagct gaaccaagaa ggaggagggg gtcgggcctc cgaggaaggc ctagccgctg 120
ctgctgccag gaattccagg ttggaggggc ggcaacctcc tgccagcctt caggccactc 180
tcctgtgcct gccagaagag acagagcttg aggagagctt gaggagagca ggaaaggaca 240
atgccgtctt ctgtctcgtg gggcatcctc ctgctggcag gcctgtgctg cctggtccct 300
gtctccctgg ctgaggatcc ccagggagat gctgcccaga agacagatac atcccaccat 360
gatcaggatc acccaacctt caacaagatc acccccaacc tggctgagtt cgccttcagc 420
ctataccgcc agctggcaca ccagtccaac agcaccaata tcttcttctc cccagtgagc 480
atcgctacag cctttgcaat gctctccctg gggaccaagg ctgacactca cgatgaaatc 540
ctggagggcc tgaatttcaa cctcacggag attccggagg ctcagatcca tgaaggcttc 600
caggaactcc tccgtaccct caaccagcca gacagccagc tccagctgac caccggcaat 660
ggcctgttcc tcagcgaggg cctgaagcta gtggataagt ttttggagga tgttaaaaag 720
ttgtaccact cagaagcctt cactgtcaac ttcggggaca ccgaagaggc caagaaacag 780
atcaacgatt acgtggagaa gggtactcaa gggaaaattg tggatttggt caaggagctt 840
gacagagaca cagtttttgc tctggtgaat tacatcttct ttaaaggcaa atgggagaga 900
ccctttgaag tcaaggacac cgaggaagag gacttccacg tggaccaggt gaccaccgtg 960
aaggtgccta tgatgaagcg tttaggcatg tttaacatcc agcactgtaa gaagctgtcc 1020
agctgggtgc tgctgatgaa atacctgggc aatgccaccg ccatcttctt cctgcctgat 1080
gaggggaaac tacagcacct ggaaaatgaa ctcacccacg atatcatcac caagttcctg 1140
gaaaatgaag acagaaggtc tgccagctta catttaccca aactgtccat tactggaacc 1200
tatgatctga agagcgtcct gggtcaactg ggcatcacta aggtcttcag caatggggct 1260
gacctctccg gggtcacaga ggaggcaccc ctgaagctct ccaaggccgt gcataaggct 1320
gtgctgacca tcgacgagaa agggactgaa gctgctgggg ccatgttttt agaggccata 1380
cccatgtcta tcccccccga ggtcaagttc aacaaaccct ttgtcttctt aatgattgaa 1440
caaaatacca agtctcccct cttcatggga aaagtggtga atcccaccca aaaataactg 1500
cctctcgctc ctcaacccct cccctccatc cctggccccc tccctggatg acattaaaga 1560
agggttgagc tggtccctgc ctgcaaaa                                    1588

<210> 41
<211> 418
<212> PRT
<213> Homo sapiens

<400> 41

<210> 42
<211> 1902
<212> DNA
<213> Homo sapiens

<400> 42
tgggcaggaa ctgggcactg tgcccagggc atgcactgcc tccacgcagc aaccctcaga 60
gtcctgagct gaaccaagaa ggaggagggg gtcgggcctc cgaggaaggc ctagccgctg 120
ctgctgccag gaattccagg ttggaggggc ggcaacctcc tgccagcctt caggccactc 180
tcctgtgcct gccagaagag acagagcttg aggagagctt gaggagagca ggaaagggcg 240
gcagtaagtc ttcagcatca ggcattttgg ggtgactcag taaatggtag atcttgctac 300
cagtggaaca gccactaagg attctgcagt gagagcagag ggccagctaa gtggtactct 360
cccagagact gtctgactca cgccaccccc tccaccttgg acacaggacg ctgtggtttc 420
tgagccaggt acaatgactc ctttcgcagc ctcccccgtt gcccctctgg atccactgct 480
taaatacgga cgaggacagg gccctgtctc ctcagcttca ggcaccacca ctgacctggg 540
acagtgaatc gacaatgccg tcttctgtct cgtggggcat cctcctgctg gcaggcctgt 600
gctgcctggt ccctgtctcc ctggctgagg atccccaggg agatgctgcc cagaagacag 660
atacatccca ccatgatcag gatcacccaa ccttcaacaa gatcaccccc aacctggctg 720
agttcgcctt cagcctatac cgccagctgg cacaccagtc caacagcacc aatatcttct 780
tctccccagt gagcatcgct acagcctttg caatgctctc cctggggacc aaggctgaca 840
ctcacgatga aatcctggag ggcctgaatt tcaacctcac ggagattccg gaggctcaga 900
tccatgaagg cttccaggaa ctcctccgta ccctcaacca gccagacagc cagctccagc 960
tgaccaccgg caatggcctg ttcctcagcg agggcctgaa gctagtggat aagtttttgg 1020
aggatgttaa aaagttgtac cactcagaag ccttcactgt caacttcggg gacaccgaag 1080
aggccaagaa acagatcaac gattacgtgg agaagggtac tcaagggaaa attgtggatt 1140
tggtcaagga gcttgacaga gacacagttt ttgctctggt gaattacatc ttctttaaag 1200
gcaaatggga gagacccttt gaagtcaagg acaccgagga agaggacttc cacgtggacc 1260
aggtgaccac cgtgaaggtg cctatgatga agcgtttagg catgtttaac atccagcact 1320
gtaagaagct gtccagctgg gtgctgctga tgaaatacct gggcaatgcc accgccatct 1380

tcttcctgcc tgatgagggg aaactacagc acctggaaaa tgaactcacc cacgatatca 1440
tcaccaagtt cctggaaaat gaagacagaa ggtctgccag cttacattta cccaaactgt 1500
ccattactgg aacctatgat ctgaagagcg tcctgggtca actgggcatc actaaggtct 1560
tcagcaatgg ggctgacctc tccggggtca cagaggaggc acccctgaag ctctccaagg 1620
ccgtgcataa ggctgtgctg accatcgacg agaaagggac tgaagctgct ggggccatgt 1680
ttttagaggc catacccatg tctatccccc ccgaggtcaa gttcaacaaa ccctttgtct 1740
tcttaatgat tgaacaaaat accaagtctc ccctcttcat gggaaaagtg gtgaatccca 1800
cccaaaaata actgcctctc gctcctcaac ccctcccctc catccctggc cccctccctg 1860
gatgacatta aagaagggtt gagctggtcc ctgcctgcaa aa 1902

<210> 43
<211> 335
<212> PRT
<213> Homo sapiens

<400> 43
Met Gly His Pro Pro Leu Leu Pro Leu Leu Leu Leu Leu His Thr Cys
1               5                   10                  15
Val Pro Ala Ser Trp Gly Leu Arg Cys Met Gln Cys Lys Thr Asn Gly
            20                  25                  30
Asp Cys Arg Val Glu Glu Cys Ala Leu Gly Gln Asp Leu Cys Arg Thr
        35                  40                  45
Thr Ile Val Arg Leu Trp Glu Glu Gly Glu Glu Leu Glu Leu Val Glu
    50                  55                  60
Lys Ser Cys Thr His Ser Glu Lys Thr Asn Arg Thr Leu Ser Tyr Arg
65                  70                  75                  80
Thr Gly Leu Lys Ile Thr Ser Leu Thr Glu Val Val Cys Gly Leu Asp
                85                  90                  95
Leu Cys Asn Gln Gly Asn Ser Gly Arg Ala Val Thr Tyr Ser Arg Ser
            100                 105                 110
Arg Tyr Leu Glu Cys Ile Ser Cys Gly Ser Ser Asp Met Ser Cys Glu
        115                 120                 125
Arg Gly Arg His Gln Ser Leu Gln Cys Arg Ser Pro Glu Glu Gln Cys
    130                 135                 140
Leu Asp Val Val Thr His Trp Ile Gln Glu Gly Glu Glu Gly Arg Pro
145                 150                 155                 160
Lys Asp Asp Arg His Leu Arg Gly Cys Gly Tyr Leu Pro Gly Cys Pro
                165                 170                 175
Gly Ser Asn Gly Phe His Asn Asn Asp Thr Phe His Phe Leu Lys Cys
            180                 185                 190
Cys Asn Thr Thr Lys Cys Asn Glu Gly Pro Ile Leu Glu Leu Glu Asn
        195                 200                 205
Leu Pro Gln Asn Gly Arg Gln Cys Tyr Ser Cys Lys Gly Asn Ser Thr
    210                 215                 220
His Gly Cys Ser Ser Glu Glu Thr Phe Leu Ile Asp Cys Arg Gly Pro
225                 230                 235                 240
Met Asn Gln Cys Leu Val Ala Thr Gly Thr His Glu Pro Lys Asn Gln
                245                 250                 255
Ser Tyr Met Val Arg Gly Cys Ala Thr Ala Ser Met Cys Gln His Ala
            260                 265                 270
His Leu Gly Asp Ala Phe Ser Met Asn His Ile Asp Val Ser Cys Cys
        275                 280                 285
Thr Lys Ser Gly Cys Asn His Pro Asp Leu Asp Val Gln Tyr Arg Ser
    290                 295                 300
Gly Ala Ala Pro Gln Pro Gly Pro Ala His Leu Ser Leu Thr Ile Thr
305                 310                 315                 320
Leu Leu Met Thr Ala Arg Leu Trp Gly Gly Thr Leu Leu Trp Thr
                325                 330                 335

<210> 44
<211> 1548
<212> DNA
<213> Homo sapiens

<400> 44
cagggccgag ccagcccctt caccaccagc cggccgcgcc ccgggaaggg aagtttgtgg 60
cggaggaggt tcgtacggga ggagggggag gcgcccacgc atctggggct gactcgctct 120

ttcgcaaaac gtctgggagg agtccctggg gccacaaaac tgcctccttc ctgaggccag 180
aaggagagaa gacgtgcagg gaccccgcgc acaggagctg ccctcgcgac atgggtcacc 240
cgccgctgct gccgctgctg ctgctgctcc acacctgcgt cccagcctct tggggcctgc 300
ggtgcatgca gtgtaagacc aacggggatt gccgtgtgga agagtgcgcc ctgggacagg 360
acctctgcag gaccacgatc gtgcgcttgt gggaagaagg agaagagctg gagctggtgg 420
agaaaagctg tacccactca gagaagacca acaggaccct gagctatcgg actggcttga 480
agatcaccag ccttaccgag gttgtgtgtg ggttagactt gtgcaaccag ggcaactctg 540
gccgggctgt cacctattcc cgaagccgtt acctcgaatg catttcctgt ggctcatcag 600
acatgagctg tgagaggggc cggcaccaga gcctgcagtg ccgcagccct gaagaacagt 660
gcctggatgt ggtgacccac tggatccagg aaggtgaaga agggcgtcca aaggatgacc 720
gccacctccg tggctgtggc taccttcccg gctgcccggg ctccaatggt ttccacaaca 780
acgacacctt ccacttcctg aaatgctgca acaccaccaa atgcaacgag ggcccaatcc 840
tggagcttga aaatctgccg cagaatggcc gccagtgtta cagctgcaag gggaacagca 900
cccatggatg ctcctctgaa gagactttcc tcattgactg ccgaggcccc atgaatcaat 960
gtctggtagc caccggcact cacgaaccga aaaaccaaag ctatatggta agaggctgtg 1020
caaccgcctc aatgtgccaa catgcccacc tgggtgacgc cttcagcatg aaccacattg 1080
atgtctcctg ctgtactaaa agtggctgta accacccaga cctggatgtc cagtaccgca 1140
gtggggctgc tcctcagcct ggccctgccc atctcagcct caccatcacc ctgctaatga 1200
ctgccagact gtggggaggc actctcctct ggacctaaac ctgaaatccc cctctctgcc 1260
ctggctggat ccgggggacc cctttgccct tccctcggct cccagcccta cagacttgct 1320
gtgtgacctc aggccagtgt gccgacctct ctgggcctca gttttcccag ctatgaaaac 1380
agctatctca caaagttgtg tgaagcagaa gagaaaagct ggaggaaggc cgtgggccaa 1440
tgggagagct cttgttatta ttaatattgt tgccgctgtt gtgttgttgt tattaattaa 1500
tattcatatt atttatttta tacttacata aagattttgt accagtgg 1548

<210> 45
<211> 281
<212> PRT
<213> Homo sapiens

<400> 45
Met Gly His Pro Pro Leu Leu Pro Leu Leu Leu Leu Leu His Thr Cys
1               5                   10                  15
Val Pro Ala Ser Trp Gly Leu Arg Cys Met Gln Cys Lys Thr Asn Gly
            20                  25                  30
Asp Cys Arg Val Glu Glu Cys Ala Leu Gly Gln Asp Leu Cys Arg Thr
        35                  40                  45
Thr Ile Val Arg Leu Trp Glu Glu Gly Glu Glu Leu Glu Leu Val Glu
    50                  55                  60
Lys Ser Cys Thr His Ser Glu Lys Thr Asn Arg Thr Leu Ser Tyr Arg
65                  70                  75                  80
Thr Gly Leu Lys Ile Thr Ser Leu Thr Glu Val Val Cys Gly Leu Asp
                85                  90                  95
Leu Cys Asn Gln Gly Asn Ser Gly Arg Ala Val Thr Tyr Ser Arg Ser
            100                 105                 110
Arg Tyr Leu Glu Cys Ile Ser Cys Gly Ser Ser Asp Met Ser Cys Glu
        115                 120                 125
Arg Gly Arg His Gln Ser Leu Gln Cys Arg Ser Pro Glu Glu Gln Cys
    130                 135                 140
Leu Asp Val Val Thr His Trp Ile Gln Glu Gly Glu Glu Gly Arg Pro
145                 150                 155                 160
Lys Asp Asp Arg His Leu Arg Gly Cys Gly Tyr Leu Pro Gly Cys Pro
                165                 170                 175
Gly Ser Asn Gly Phe His Asn Asn Asp Thr Phe His Phe Leu Lys Cys
            180                 185                 190
Cys Asn Thr Thr Lys Cys Asn Glu Gly Pro Ile Leu Glu Leu Glu Asn
        195                 200                 205
Leu Pro Gln Asn Gly Arg Gln Cys Tyr Ser Cys Lys Gly Asn Ser Thr
    210                 215                 220
His Gly Cys Ser Ser Glu Glu Thr Phe Leu Ile Asp Cys Arg Gly Pro
225                 230                 235                 240
Met Asn Gln Cys Leu Val Ala Thr Gly Thr His Glu Arg Ser Leu Trp
                245                 250                 255
Gly Ser Trp Leu Pro Cys Lys Ser Thr Thr Ala Leu Arg Pro Pro Cys
            260                 265                 270
Cys Glu Glu Ala Gln Ala Thr His Val
        275                 280

<210> 46
<211> 1437
<212> DNA
<213> Homo sapiens

<400> 46
cagggccgag ccagcccctt caccaccagc cggccgcgcc ccgggaaggg aagtttgtgg 60
cggaggaggt tcgtacggga ggagggggag gcgcccacgc atctggggct gactcgctct 120
ttcgcaaaac gtctgggagg agtccctggg gccacaaaac tgcctccttc ctgaggccag 180
aaggagagaa gacgtgcagg gaccccgcgc acaggagctg ccctcgcgac atgggtcacc 240
cgccgctgct gccgctgctg ctgctgctcc acacctgcgt cccagcctct tggggcctgc 300
ggtgcatgca gtgtaagacc aacggggatt gccgtgtgga agagtgcgcc ctgggacagg 360
acctctgcag gaccacgatc gtgcgcttgt gggaagaagg agaagagctg gagctggtgg 420
agaaaagctg tacccactca gagaagacca acaggaccct gagctatcgg actggcttga 480
agatcaccag ccttaccgag gttgtgtgtg ggttagactt gtgcaaccag ggcaactctg 540
gccgggctgt cacctattcc cgaagccgtt acctcgaatg catttcctgt ggctcatcag 600
acatgagctg tgagaggggc cggcaccaga gcctgcagtg ccgcagccct gaagaacagt 660
gcctggatgt ggtgacccac tggatccagg aaggtgaaga agggcgtcca aaggatgacc 720
gccacctccg tggctgtggc taccttcccg gctgcccggg ctccaatggt ttccacaaca 780
acgacacctt ccacttcctg aaatgctgca acaccaccaa atgcaacgag ggcccaatcc 840
tggagcttga aaatctgccg cagaatggcc gccagtgtta cagctgcaag gggaacagca 900
cccatggatg ctcctctgaa gagactttcc tcattgactg ccgaggcccc atgaatcaat 960
gtctggtagc caccggcact cacgaacgct cactctgggg aagctggttg ccatgtaaaa 1020
gtactactgc cctgagacca ccatgctgtg aggaagccca agctactcat gtataaatgc 1080
catgtggaga tagagcccca gatgtttcag ccatctcagc ccaggcacca gacaagtggg 1140
tgaagaagcc accttggaca tgtagcccca gcagatgtga tatagagaag aaacaggaaa 1200
cttggctata ttagtttcct agggctgcct gtgataaatt attacaaact ttataaacta 1260
acacattgtg tgcctatatc aaaacatcat ggaaggacag gcacagtggc tcatgcctgt 1320
agtcctagca ctttgggagg gtgagaaagg aagatctctt gagctcagga gttcaagatc 1380
agcctgggca acacagtgag acctcatctc cactaaaaat aaaaaaaaat tggctgg 1437

<210> 47
<211> 290
<212> PRT
<213> Homo sapiens

<400> 47
Met Gly His Pro Pro Leu Leu Pro Leu Leu Leu Leu Leu His Thr Cys
1               5                   10                  15
Val Pro Ala Ser Trp Gly Leu Arg Cys Met Gln Cys Lys Thr Asn Gly
            20                  25                  30
Asp Cys Arg Val Glu Glu Cys Ala Leu Gly Gln Asp Leu Cys Arg Thr
        35                  40                  45
Thr Ile Val Arg Leu Trp Glu Glu Gly Glu Glu Leu Glu Leu Val Glu
    50                  55                  60
Lys Ser Cys Thr His Ser Glu Lys Thr Asn Arg Thr Leu Ser Tyr Arg
65                  70                  75                  80
Thr Gly Leu Lys Ile Thr Ser Leu Thr Glu Val Val Cys Gly Leu Asp
                85                  90                  95
Leu Cys Asn Gln Gly Asn Ser Gly Arg Ala Val Thr Tyr Ser Arg Ser
            100                 105                 110
Arg Tyr Leu Glu Cys Ile Ser Cys Gly Ser Ser Asp Met Ser Cys Glu
        115                 120                 125
Arg Gly Arg His Gln Ser Leu Gln Cys Arg Ser Pro Glu Glu Gln Cys
    130                 135                 140
Leu Asp Val Val Thr His Trp Ile Gln Glu Gly Glu Glu Val Leu Glu
145                 150                 155                 160
Leu Glu Asn Leu Pro Gln Asn Gly Arg Gln Cys Tyr Ser Cys Lys Gly
                165                 170                 175
Asn Ser Thr His Gly Cys Ser Ser Glu Glu Thr Phe Leu Ile Asp Cys
            180                 185                 190
Arg Gly Pro Met Asn Gln Cys Leu Val Ala Thr Gly Thr His Glu Pro
        195                 200                 205
Lys Asn Gln Ser Tyr Met Val Arg Gly Cys Ala Thr Ala Ser Met Cys
    210                 215                 220
Gln His Ala His Leu Gly Asp Ala Phe Ser Met Asn His Ile Asp Val
225                 230                 235                 240

Ser Cys Cys Thr Lys Ser Gly Cys Asn His Pro Asp Leu Asp Val Gln
                245                 250                 255
Tyr Arg Ser Gly Ala Ala Pro Gln Pro Gly Pro Ala His Leu Ser Leu
            260                 265                 270
Thr Ile Thr Leu Leu Met Thr Ala Arg Leu Trp Gly Gly Thr Leu Leu
        275                 280                 285
Trp Thr
    290

<210> 48
<211> 1360
<212> DNA
<213> Homo sapiens

<400> 48
cagggccgag ccagcccctt caccaccagc cggccgcgcc ccgggaaggg aagtttgtgg 60
cggaggaggt tcgtacggga ggagggggag gcgcccacgc atctggggct gactcgctct 120
ttcgcaaaac gtctgggagg agtccctggg gccacaaaac tgcctccttc ctgaggccag 180
aaggagagaa gacgtgcagg gaccccgcgc acaggagctg ccctcgcgac atgggtcacc 240
cgccgctgct gccgctgctg ctgctgctcc acacctgcgt cccagcctct tggggcctgc 300
ggtgcatgca gtgtaagacc aacggggatt gccgtgtgga agagtgcgcc ctgggacagg 360
acctctgcag gaccacgatc gtgcgcttgt gggaagaagg agaagagctg gagctggtgg 420
agaaaagctg tacccactca gagaagacca acaggaccct gagctatcgg actggcttga 480
agatcaccag ccttaccgag gttgtgtgtg ggttagactt gtgcaaccag ggcaactctg 540
gccgggctgt cacctattcc cgaagccgtt acctcgaatg catttcctgt ggctcatcag 600
acatgagctg tgagaggggc cggcaccaga gcctgcagtg ccgcagccct gaagaacagt 660
gcctggatgt ggtgacccac tggatccagg aaggtgaaga agtcctggag cttgaaaatc 720
tgccgcagaa tggccgccag tgttacagct gcaaggggaa cagcacccat ggatgctcct 780
ctgaagagac tttcctcatt gactgccgag gccccatgaa tcaatgtctg gtagccaccg 840
gcactcacga accgaaaaac caaagctata tggtaagagg ctgtgcaacc gcctcaatgt 900
gccaacatgc ccacctgggt gacgccttca gcatgaacca cattgatgtc tcctgctgta 960
ctaaaagtgg ctgtaaccac ccagacctgg atgtccagta ccgcagtggg gctgctcctc 1020
agcctggccc tgcccatctc agcctcacca tcaccctgct aatgactgcc agactgtggg 1080
gaggcactct cctctggacc taaacctgaa atccccctct ctgccctggc tggatccggg 1140
ggaccccttt gcccttccct cggctcccag ccctacagac ttgctgtgtg acctcaggcc 1200
agtgtgccga cctctctggg cctcagtttt cccagctatg aaaacagcta tctcacaaag 1260
ttgtgtgaag cagaagagaa aagctggagg aaggccgtgg gccaatggga gagctcttgt 1320
tattattaat attgttgccg ctgttgtgtt gttgttatta                       1360

<210> 49
<211> 243
<212> PRT
<213> Homo sapiens

<400> 49
Met Arg Pro Gln Gly Pro Ala Ala Ser Pro Gln Arg Leu Arg Gly Leu
 1               5                  10                  15
Leu Leu Leu Leu Leu Leu Gln Leu Pro Ala Pro Ser Ser Ala Ser Glu
            20                  25                  30
Ile Pro Lys Gly Lys Gln Lys Ala Gln Leu Arg Gln Arg Glu Val Val
        35                  40                  45
Asp Leu Tyr Asn Gly Met Cys Leu Gln Gly Pro Ala Gly Val Pro Gly
    50                  55                  60
Arg Asp Gly Ser Pro Gly Ala Asn Gly Ile Pro Gly Thr Pro Gly Ile
65                  70                  75                  80
Pro Gly Arg Asp Gly Phe Lys Gly Glu Lys Gly Glu Cys Leu Arg Glu
                85                  90                  95
Ser Phe Glu Glu Ser Trp Thr Pro Asn Tyr Lys Gln Cys Ser Trp Ser
            100                 105                 110
Ser Leu Asn Tyr Gly Ile Asp Leu Gly Lys Ile Ala Glu Cys Thr Phe
        115                 120                 125
Thr Lys Met Arg Ser Asn Ser Ala Leu Arg Val Leu Phe Ser Gly Ser
    130                 135                 140
Leu Arg Leu Lys Cys Arg Asn Ala Cys Cys Gln Arg Trp Tyr Phe Thr
145                 150                 155                 160
Phe Asn Gly Ala Glu Cys Ser Gly Pro Leu Pro Ile Glu Ala Ile Ile
                165                 170                 175

Tyr Leu Asp Gln Gly Ser Pro Glu Met Asn Ser Thr Ile Asn Ile His
            180                 185                 190
Arg Thr Ser Ser Val Glu Gly Leu Cys Glu Gly Ile Gly Ala Gly Leu
        195                 200                 205
Val Asp Val Ala Ile Trp Val Gly Thr Cys Ser Asp Tyr Pro Lys Gly
    210                 215                 220
Asp Ala Ser Thr Gly Trp Asn Ser Val Ser Arg Ile Ile Ile Glu Glu
225                 230                 235                 240
Leu Pro Lys

<210> 50
<211> 1236
<212> DNA
<213> Homo sapiens

<400> 50
ctgcggcggc ctcggagcgc ggcggagcca gacgctgacc acgttcctct cctcggtctc 60
ctccgcctcc agctccgcgc tgcccggcag ccgggagcca tgcgacccca gggccccgcc 120
gcctccccgc agcggctccg cggcctcctg ctgctcctgc tgctgcagct gcccgcgccg 180
tcgagcgcct ctgagatccc caaggggaag caaaaggcgc agctccggca gagggaggtg 240
gtggacctgt ataatggaat gtgcttacaa gggccagcag gagtgcctgg tcgagacggg 300
agccctgggg ccaatggcat tccgggtaca cctgggatcc caggtcggga tggattcaaa 360
ggagaaaagg gggaatgtct gagggaaagc tttgaggagt cctggacacc caactacaag 420
cagtgttcat ggagttcatt gaattatggc atagatcttg ggaaaattgc ggagtgtaca 480
tttacaaaga tgcgttcaaa tagtgctcta agagttttgt tcagtggctc acttcggcta 540
aaatgcagaa atgcatgctg tcagcgttgg tatttcacat tcaatggagc tgaatgttca 600
ggacctcttc ccattgaagc tataatttat ttggaccaag gaagccctga aatgaattca 660
acaattaata ttcatcgcac ttcttctgtg gaaggacttt gtgaaggaat tggtgctgga 720
ttagtggatg ttgctatctg ggttggcact tgttcagatt acccaaaagg agatgcttct 780
actggatgga attcagtttc tcgcatcatt attgaagaac taccaaaata aatgctttaa 840
ttttcatttg ctacctcttt ttttattatg ccttggaatg gttcacttaa atgacatttt 900
aaataagttt atgtatacat ctgaatgaaa agcaaagcta aatatgttta cagaccaaag 960
tgtgatttca cactgttttt aaatctagca ttattcattt tgcttcaatc aaaagtggtt 1020
tcaatatttt ttttagttgg ttagaatact ttcttcatag tcacattctc tcaacctata 1080
atttggaata ttgttgtggt cttttgtttt ttctcttagt atagcatttt taaaaaaata 1140
taaaagctac caatctttgt acaatttgta aatgttaaga atttttttta tatctgttaa 1200
ataaaaatta tttccaacaa aaaaaaaaaa aaaaaa                            1236

<210> 51
<211> 366
<212> PRT
<213> Homo sapiens

<400> 51
Met Val Leu His Leu Leu Leu Phe Leu Leu Leu Thr Pro Gln Gly Gly
 1               5                  10                  15
His Ser Cys Gln Gly Leu Glu Leu Ala Arg Glu Leu Val Leu Ala Lys
            20                  25                  30
Val Arg Ala Leu Phe Leu Asp Ala Leu Gly Pro Pro Ala Val Thr Arg
        35                  40                  45
Glu Gly Gly Asp Pro Gly Val Arg Arg Leu Pro Arg Arg His Ala Leu
    50                  55                  60
Gly Gly Phe Thr His Arg Gly Ser Glu Pro Glu Glu Glu Glu Asp Val
65                  70                  75                  80
Ser Gln Ala Ile Leu Phe Pro Ala Thr Asp Ala Ser Cys Glu Asp Lys
                85                  90                  95
Ser Ala Ala Arg Gly Leu Ala Gln Glu Ala Glu Glu Gly Leu Phe Arg
            100                 105                 110
Tyr Met Phe Arg Pro Ser Gln His Thr Arg Ser Arg Gln Val Thr Ser
        115                 120                 125
Ala Gln Leu Trp Phe His Thr Gly Leu Asp Arg Gln Gly Thr Ala Ala
    130                 135                 140
Ser Asn Ser Ser Glu Pro Leu Leu Gly Leu Leu Ala Leu Ser Pro Gly
145                 150                 155                 160
Gly Pro Val Ala Val Pro Met Ser Leu Gly His Ala Pro Pro His Trp
                165                 170                 175

Ala Val Leu His Leu Ala Thr Ser Ala Leu Ser Leu Leu Thr His Pro
            180                 185                 190
Val Leu Val Leu Leu Leu Arg Cys Pro Leu Cys Thr Cys Ser Ala Arg
        195                 200                 205
Pro Glu Ala Thr Pro Phe Leu Val Ala His Thr Arg Thr Arg Pro Pro
    210                 215                 220
Ser Gly Gly Glu Arg Ala Arg Arg Ser Thr Pro Leu Met Ser Trp Pro
225                 230                 235                 240
Trp Ser Pro Ser Ala Leu Arg Leu Leu Gln Arg Pro Pro Glu Glu Pro
                245                 250                 255
Ala Ala His Ala Asn Cys His Arg Val Ala Leu Asn Ile Ser Phe Gln
            260                 265                 270
Glu Leu Gly Trp Glu Arg Trp Ile Val Tyr Pro Pro Ser Phe Ile Phe
        275                 280                 285
His Tyr Cys His Gly Gly Cys Gly Leu His Ile Pro Pro Asn Leu Ser
    290                 295                 300
Leu Pro Val Pro Gly Ala Pro Pro Thr Pro Ala Gln Pro Tyr Ser Leu
305                 310                 315                 320
Leu Pro Gly Ala Gln Pro Cys Cys Ala Ala Leu Pro Gly Thr Met Arg
                325                 330                 335
Pro Leu His Val Arg Thr Thr Ser Asp Gly Gly Tyr Ser Phe Lys Tyr
            340                 345                 350
Glu Thr Val Pro Asn Leu Leu Thr Gln His Cys Ala Cys Ile
        355                 360                 365

<210> 52
<211> 1424
<212> DNA
<213> Homo sapiens

<400> 52
gaaggactgg ggaagactgg atgagaaggg tagaagaggg tgggtgtggg atggggaggg 60
gagagtggaa aggccctggg cagaccctgg cagaaggggc acggggcagg gtgtgagttc 120
cccactagca gggccaggtg agctatggtg ctgcacctac tgctcttctt gctgctgacc 180
ccacagggtg ggcacagctg ccaggggctg gagctggccc gggaacttgt tctggccaag 240
gtgagggccc tgttcttgga tgccttgggg ccccccgcgg tgaccaggga aggtggggac 300
cctggagtca ggcggctgcc ccgaagacat gccctggggg gcttcacaca caggggctct 360
gagcccgagg aagaggagga tgtctcccaa gccatccttt tcccagccac agatgccagc 420
tgtgaggaca agtcagctgc cagagggctg gcccaggagg ctgaggaggg cctcttcaga 480
tacatgttcc ggccatccca gcatacacgc agccgccagg tgacttcagc ccagctgtgg 540
ttccacaccg ggctggacag gcagggcaca gcagcctcca atagctctga gcccctgcta 600
ggcctgctgg cactgtcacc gggaggaccc gtggctgtgc ccatgtcttt gggccatgct 660
ccccctcact gggccgtgct gcacctggcc acctctgctc tctctctgct gacccacccc 720
gtcctggtgc tgctgctgcg ctgtcccctc tgtacctgct cagcccggcc tgaggccacg 780
cccttcctgg tggcccacac tcggaccaga ccacccagtg gaggggagag agcccgacgc 840
tcaactcccc tgatgtcctg gccttggtct ccctctgctc tgcgcctgct gcagaggcct 900
ccggaggaac cggctgccca tgccaactgc cacagagtag cactgaacat ctccttccag 960
gagctgggct gggaacggtg gatcgtgtac cctcccagtt tcatcttcca ctactgtcat 1020
ggtggttgtg ggctgcacat cccaccaaac ctgtcccttc cagtccctgg ggctccccct 1080
accccagccc agccctactc cttgctgcca ggggcccagc cctgctgtgc tgctctccca 1140
gggaccatga ggcccctaca tgtccgcacc acctcggatg gaggttactc tttcaagtat 1200
gagacagtgc ccaaccttct cacgcagcac tgtgcttgta tctaagggtg gggggtcttc 1260
cttcttaatc ccatggctgg tggccacgcc cccaccatca tcagctggga ggaaaggcag 1320
agttgggaaa tagatggctc ccactcctcc ctcctttcac ttctctgcct atgggctacc 1380
ctccccaccc cacttctatc tcaataaaga acacagtgca tatg                  1424

<210> 53
<211> 407
<212> PRT
<213> Homo sapiens

<400> 53
Met Asp Gly Leu Pro Gly Arg Ala Leu Gly Ala Ala Cys Leu Leu Leu
1               5                   10                  15
Leu Ala Ala Gly Trp Leu Gly Pro Glu Ala Trp Gly Ser Pro Thr Pro
            20                  25                  30
Pro Pro Thr Pro Ala Ala Pro Pro Pro Pro Pro Pro Pro Gly Ala Pro

35                  40                  45
Gly Gly Ser Gln Asp Thr Cys Thr Ser Cys Gly Gly Phe Arg Arg Pro
    50                  55                  60
Glu Glu Leu Gly Arg Val Asp Gly Asp Phe Leu Glu Ala Val Lys Arg
65                  70                  75                  80
His Ile Leu Ser Arg Leu Gln Met Arg Gly Arg Pro Asn Ile Thr His
                85                  90                  95
Ala Val Pro Lys Ala Ala Met Val Thr Ala Leu Arg Lys Leu His Ala
            100                 105                 110
Gly Lys Val Arg Glu Asp Gly Arg Val Glu Ile Pro His Leu Asp Gly
        115                 120                 125
His Ala Ser Pro Gly Ala Asp Gly Gln Glu Arg Val Ser Glu Ile Ile
    130                 135                 140
Ser Phe Ala Glu Thr Asp Gly Leu Ala Ser Ser Arg Val Arg Leu Tyr
145                 150                 155                 160
Phe Phe Ile Ser Asn Glu Gly Asn Gln Asn Leu Phe Val Val Gln Ala
                165                 170                 175
Ser Leu Trp Leu Tyr Leu Lys Leu Leu Pro Tyr Val Leu Glu Lys Gly
            180                 185                 190
Ser Arg Arg Lys Val Arg Val Lys Val Tyr Phe Gln Glu Gln Gly His
        195                 200                 205
Gly Asp Arg Trp Asn Met Val Glu Lys Arg Val Asp Leu Lys Arg Ser
    210                 215                 220
Gly Trp His Thr Phe Pro Leu Thr Glu Ala Ile Gln Ala Leu Phe Glu
225                 230                 235                 240
Arg Gly Glu Arg Arg Leu Asn Leu Asp Val Gln Cys Asp Ser Cys Gln
                245                 250                 255
Glu Leu Ala Val Val Pro Val Phe Val Asp Pro Gly Glu Glu Ser His
            260                 265                 270
Arg Pro Phe Val Val Val Gln Ala Arg Leu Gly Asp Ser Arg His Arg
        275                 280                 285
Ile Arg Lys Arg Gly Leu Glu Cys Asp Gly Arg Thr Asn Leu Cys Cys
    290                 295                 300
Arg Gln Gln Phe Phe Ile Asp Phe Arg Leu Ile Gly Trp Asn Asp Trp
305                 310                 315                 320
Ile Ile Ala Pro Thr Gly Tyr Tyr Gly Asn Tyr Cys Glu Gly Ser Cys
                325                 330                 335
Pro Ala Tyr Leu Ala Gly Val Pro Gly Ser Ala Ser Ser Phe His Thr
            340                 345                 350
Ala Val Val Asn Gln Tyr Arg Met Arg Gly Leu Asn Pro Gly Thr Val
        355                 360                 365
Asn Ser Cys Cys Ile Pro Thr Lys Leu Ser Thr Met Ser Met Leu Tyr
    370                 375                 380
Phe Asp Asp Glu Tyr Asn Ile Val Lys Arg Asp Val Pro Asn Met Ile
385                 390                 395                 400
Val Glu Glu Cys Gly Cys Ala
                405

<210> 54
<211> 3516
<212> DNA
<213> Homo sapiens

<400> 54
ggatcctgga gacaactttg ccgtgtgacg cgccgggagg actgcagggc ccgcggccga 60
gggctcggcg ccgcctgtga gcgggcccgc gcggccggct ctcccgggca ccaagcttgc 120
tccgcgccac tgcccgccgg cccgcggcga ggacgacctg cccgtctccg ccgccggcgg 180
cccttcctgg cgcgaggcag tgagggcgag gcgctcaggt gcgagcgcgg ggccccgccg 240
cagcgcccgc cgcagcgccg cgccaagccg cgcccggctc cgctccgggg ggctccagcg 300
ccttcgcttc cgtctcagcc aagttgcgtg gacccgctct ttcgccacct tccccagccg 360
ccggccgaac cgccgctccc actgacgctg ctttcgcttc acccgaaccg ggctgcgggg 420
gcccccgacg cggaaaggat ggggagaagg ctgcagatgc cgaggcgccc cgagacgccc 480
gtgcggcagt gacccgcgac ctccgccccg cccggcgcgc ccctcgggcc cccggggccc 540
tcggcgcccc ttccctgccg cgcgggaacc cccgaggccc ggccggcccc ctccccctgc 600
gagccggcgg cagccctccc ggcgggcggg cgggcggagg cccgggcggg cgcgggcgcg 660
ggcgggggcg gggcggggcg gcgcgcccgg agcccggagc ccggccctgc gctcggctcg 720
actcggctcg cctcgcggcg ggcgccctcg tcgccagcgg cgcaccatgg acgggctgcc 780

cggtcgggcg ctgggggccg cctgccttct gctgctggcg gccggctggc tggggcctga 840
ggcctggggc tcacccacgc ccccgccgac gcctgccgcg ccgccgccac ccccgccacc 900
cggagccccg ggtggctcgc aggacacctg tacgtcgtgc ggcggcttcc ggcggccaga 960
ggagctcggc cgagtggacg gcgacttcct ggaggcggtg aagcggcaca tcttgagccg 1020
cctgcagatg cggggccggc ccaacatcac gcacgccgtg cctaaggccg ccatggtcac 1080
ggccctgcgc aagctgcacg cgggcaaggt gcgcgaggac ggccgcgtgg agatcccgca 1140
cctcgacggc cacgccagcc cgggcgccga cggccaggag cgcgtttccg aaatcatcag 1200
cttcgccgag acagatggcc tcgcctcctc ccgggtccgc ctatacttct tcatctccaa 1260
cgaaggcaac cagaacctgt ttgtggtcca ggccagcctg tggctttacc tgaaactcct 1320
gccctacgtc ctggagaagg gcagccggcg gaaggtgcgg gtcaaagtgt acttccagga 1380
gcagggccac ggtgacaggt ggaacatggt ggagaagagg gtggacctca agcgcagcgg 1440
ctggcatacc ttcccactca cggaggccat ccaggccttg tttgagcggg gcgagcggcg 1500
actcaaccta gacgtgcagt gtgacagctg ccaggagctg gccgtggtgc cggtgttcgt 1560
ggacccaggc gaagagtcgc accgaccctt tgtggtggtg caggctcggc tgggcgacag 1620
caggcaccgc attcgcaagc gaggcctgga gtgcgatggc cggaccaacc tctgttgcag 1680
gcaacagttc ttcattgact tccgcctcat cggctggaac gactggatca tagcacccac 1740
cggctactac ggcaactact gtgagggcag ctgcccagcc tacctggcag gggtccccgg 1800
ctctgcctcc tccttccaca cggctgtggt gaaccagtac cgcatgcggg gtctgaaccc 1860
cggcacggtg aactcctgct gcattcccac caagctgagc accatgtcca tgctgtactt 1920
cgatgatgag tacaacatcg tcaagcggga cgtgcccaac atgattgtgg aggagtgcgg 1980
ctgcgcctga cagtgcaagg caggggcacg gtggtggggc acggagggca gtcccgggtg 2040
ggcttcttcc agccccccgc gggaacgggg tacacggtgg gctgagtaca gtcattctgt 2100
tgggctgtgg agatagtgcc agggtgcggc ctgagatatt tttctacagc ttcatagagc 2160
aaccagtcaa aaccagagcg agaaccctca actgacatga aatactttaa aatgcacacg 2220
tagccacgca cagccagacg catcctgcca cccacacagc agcctccagg ataccagcaa 2280
atggatgcgg tgacaaatgg cagcttagct acaaatgcct gtcagtcgga gagaatgggg 2340
tgagcagcca ccattccacc agctggcccg gccacgtctc gaagttgcgc cttcccgagc 2400
acacataaaa gcacaaagac agagacgcag agagagagag agagccacgg agaggaaaag 2460
cagatgcagg ggtggggagc gcagctcggc ggaggctgcg tgtgccccgt ggcttttacc 2520
aggcctgctc tgcctggctc gatgtctgct tcttcccagc ctgggatcct tcgtgcttca 2580
aggcctgggg agcctgtcct tccatgccct tgtcgaggga aagagaccca gaaaggacac 2640
aacccgtcag agacctggga gcaggggcaa tgaccgtttg actgtttgtg gcttgggcct 2700
ctgacatgac ttatgtgtgt gtgtgttttt ggggtgggga gggagggaga gaagaggggg 2760
ctaaatttga tgctttaact gatctccaac agttgacagg tcatccttgc cagttgtata 2820
actgaaaaag gacttttcta ccaggtatga ccttttaagt gaaaatctga attgttctaa 2880
atggaaagaa aaaaagttgc aatctgtgcc cttcattggg gacattcctc taggactggt 2940
ttggggacgg gtgggaatga cccctaggca aggggatgag accgcaggag gaaatggcgg 3000
ggaggtggca ttcttgaact gctgaggatg gggggtgtcc cctcagcgga ggccaaggga 3060
ggggagcagc ctagttggtc ttggagagat ggggaaggct ttcagctgat ttgcagaagt 3120
tgcccatgtg ggcccaacca tcagggctgg ccgtggacgt ggcccctgcc cactcacctg 3180
cccgcctgcc cgcccgcccg catagcactt gcagacctgc ctgaacgcac atgacatagc 3240
acttgccgat ctgcgtgtgc ccagaagtgg cccttggccg agcgccgaac tcgctcgccc 3300
tctagatgtc caagtgccac gtgaactatg caatttaaag ggttgaccca cactagacga 3360
aactggactc gtacgactct ttttatattt tttatacttg aaatgaaatc ctttgcttct 3420
tttttaagcg aatgattgct tttaatgttt gcactgattt agttgcatga ttagtcagaa 3480
actgccattt gaaaaaaaag ttatttttat agcagc 3516

<210> 55
<211> 426
<212> PRT
<213> Homo sapiens

<400> 55
Met Pro Leu Leu Trp Leu Arg Gly Phe Leu Leu Ala Ser Cys Trp Ile
1               5                   10                  15
Ile Val Arg Ser Ser Pro Thr Pro Gly Ser Glu Gly His Ser Ala Ala
            20                  25                  30
Pro Asp Cys Pro Ser Cys Ala Leu Ala Ala Leu Pro Lys Asp Val Pro
        35                  40                  45
Asn Ser Gln Pro Glu Met Val Glu Ala Val Lys Lys His Ile Leu Asn
    50                  55                  60
Met Leu His Leu Lys Lys Arg Pro Asp Val Thr Gln Pro Val Pro Lys
65                  70                  75                  80
Ala Ala Leu Leu Asn Ala Ile Arg Lys Leu His Val Gly Lys Val Gly
                85                  90                  95
Glu Asn Gly Tyr Val Glu Ile Glu Asp Asp Ile Gly Arg Arg Ala Glu
            100                 105                 110
Met Asn Glu Leu Met Glu Gln Thr Ser Glu Ile Ile Thr Phe Ala Glu

115                 120                 125
Ser Gly Thr Ala Arg Lys Thr Leu His Phe Glu Ile Ser Lys Glu Gly
    130                 135                 140
Ser Asp Leu Ser Val Val Glu Arg Ala Glu Val Trp Leu Phe Leu Lys
145                 150                 155                 160
Val Pro Lys Ala Asn Arg Thr Arg Thr Lys Val Thr Ile Arg Leu Phe
                165                 170                 175
Gln Gln Gln Lys His Pro Gln Gly Ser Leu Asp Thr Gly Glu Glu Ala
            180                 185                 190
Glu Glu Val Gly Leu Lys Gly Glu Arg Ser Glu Leu Leu Leu Ser Glu
        195                 200                 205
Lys Val Val Asp Ala Arg Lys Ser Thr Trp His Val Phe Pro Val Ser
    210                 215                 220
Ser Ser Ile Gln Arg Leu Leu Asp Gln Gly Lys Ser Ser Leu Asp Val
225                 230                 235                 240
Arg Ile Ala Cys Glu Gln Cys Gln Glu Ser Gly Ala Ser Leu Val Leu
                245                 250                 255
Leu Gly Lys Lys Lys Lys Lys Glu Glu Glu Gly Glu Gly Lys Lys Lys
            260                 265                 270
Gly Gly Gly Glu Gly Gly Ala Gly Ala Asp Glu Glu Lys Glu Gln Ser
        275                 280                 285
His Arg Pro Phe Leu Met Leu Gln Ala Arg Gln Ser Glu Asp His Pro
    290                 295                 300
His Arg Arg Arg Arg Arg Gly Leu Glu Cys Asp Gly Lys Val Asn Ile
305                 310                 315                 320
Cys Cys Lys Lys Gln Phe Phe Val Ser Phe Lys Asp Ile Gly Trp Asn
                325                 330                 335
Asp Trp Ile Ile Ala Pro Ser Gly Tyr His Ala Asn Tyr Cys Glu Gly
            340                 345                 350
Glu Cys Pro Ser His Ile Ala Gly Thr Ser Gly Ser Ser Leu Ser Phe
        355                 360                 365
His Ser Thr Val Ile Asn His Tyr Arg Met Arg Gly His Ser Pro Phe
    370                 375                 380
Ala Asn Leu Lys Ser Cys Cys Val Pro Thr Lys Leu Arg Pro Met Ser
385                 390                 395                 400
Met Leu Tyr Tyr Asp Asp Gly Gln Asn Ile Ile Lys Lys Asp Ile Gln
                405                 410                 415
Asn Met Ile Val Glu Glu Cys Gly Cys Ser
            420                 425

<210> 56
<211> 2175
<212> DNA
<213> Homo sapiens

<400> 56
agtacagtat aaaacttcac agtgccaata ccatgaagag gagctcagac agctcttacc 60
acatgataca agagccggct ggtggaagag tggggaccag aaagagaatt tgctgaagag 120
gagaaggaaa aaaaaaacac caaaaaaaaa aataaaaaaa tccacacaca caaaaaaacc 180
tgcgcgtgag gggggaggaa aagcagggcc ttttaaaaag gcaatcacaa caacttttgc 240
tgccaggatg cccttgcttt ggctgagagg atttctgttg gcaagttgct ggattatagt 300
gaggagttcc ccccacccag gatccgaggg gcacagcgcg gcccccgact gtccgtcctg 360
tgcgctggcc gccctcccaa aggatgtacc caactctcag ccagagatgg tggaggccgt 420
caagaagcac attttaaaca tgctgcactt gaagaagaga cccgatgtca cccagccggt 480
acccaaggcg gcgcttctga acgcgatcag aaagcttcat gtgggcaaag tcggggagaa 540
cgggtatgtg gagatagagg atgacattgg aaggagggca gaaatgaatg aacttatgga 600
gcagacctcg gagatcatca cgtttgccga gtcaggaaca gccaggaaga cgctgcactt 660
cgagatttcc aaggaaggca gtgacctgtc agtggtggag cgtgcagaag tctggctctt 720
cctaaaagtc cccaaggcca acaggaccag gaccaaagtc accatccgcc tcttccagca 780
gcagaagcac ccgcagggca gcttggacac aggggaagag gccgaggaag tgggcttaaa 840
gggggagagg agtgaactgt tgctctctga aaaagtagta gacgctcgga agagcacctg 900
gcatgtcttc cctgtctcca gcagcatcca gcggttgctg gaccagggca agagctccct 960
ggacgttcgg attgcctgtg agcagtgcca ggagagtggc gccagcttgg ttctcctggg 1020
caagaagaag aagaaagaag aggaggggga agggaaaaag aagggcggag gtgaaggtgg 1080
ggcaggagca gatgaggaaa aggagcagtc gcacagacct ttcctcatgc tgcaggcccg 1140
gcagtctgaa gaccaccctc atcgccggcg tcggcggggc ttggagtgtg atggcaaggt 1200
caacatctgc tgtaagaaac agttctttgt cagtttcaag gacatcggct ggaatgactg 1260

gatcattgct ccctctggct atcatgccaa ctactgcgag ggtgagtgcc cgagccatat 1320
agcaggcacg tccgggtcct cactgtcctt ccactcaaca gtcatcaacc actaccgcat 1380
gcggggccat agcccctttg ccaacctcaa atcgtgctgt gtgcccacca agctgagacc 1440
catgtccatg ttgtactatg atgatggtca aaacatcatc aaaaaggaca ttcagaacat 1500
gatcgtggag gagtgtgggt gctcatagag ttgcccagcc cagggggaaa gggagcaaga 1560
gttgtccaga gaagacagtg gcaaaatgaa gaaattttta aggtttctga gttaaccaga 1620
aaaatagaaa ttaaaaacaa aacaaaaaaa aaaacaaaaa aaaacaaaag taaattaaaa 1680
acaaaacctg atgaaacaga tgaaggaaga tgtggaaaaa atccttagcc agggctcaga 1740
gatgaagcag tgaaagagac aggaattggg agggaaaggg agaatggtgt accctttatt 1800
tcttctgaaa tcacactgat gacatcagtt gtttaaacgg ggtattgtcc tttcccccct 1860
tgaggttccc ttgtgagcct tgaatcaacc aatctagtct gcagtagtgt ggactagaac 1920
aacccaaata gcatctagaa agccatgagt ttgaaagggc ccatcacagg cactttccta 1980
cccaattacc caggtcataa ggtatgtctg tgtgacactt atctctgtgt atatcagcat 2040
acacacacac acacacacac acacacacac acacaggcat ttccacacat tacatatata 2100
cacatactgg taaaagaaca atcgtgtgca ggtggtcaca cttccttttt ctgtaccact 2160
tttgcaacaa aacaa 2175

<210> 57
<211> 14507
<212> PRT
<213> Homo sapiens

<400> 57
Met Leu Lys Pro Ser Gly Leu Pro Gly Ser Ser Ser Pro Thr Arg Ser
1               5                   10                  15
Leu Met Thr Gly Ser Arg Ser Thr Lys Ala Thr Pro Glu Met Asp Ser
            20                  25                  30
Gly Leu Thr Gly Ala Thr Leu Ser Pro Lys Thr Ser Thr Gly Ala Ile
        35                  40                  45
Val Val Thr Glu His Thr Leu Pro Phe Thr Ser Pro Asp Lys Thr Leu
    50                  55                  60
Ala Ser Pro Thr Ser Ser Val Val Gly Arg Thr Thr Gln Ser Leu Gly
65                  70                  75                  80
Val Met Ser Ser Ala Leu Pro Glu Ser Thr Ser Arg Gly Met Thr His
                85                  90                  95
Ser Glu Gln Arg Thr Ser Pro Ser Leu Ser Pro Gln Val Asn Gly Thr
            100                 105                 110
Pro Ser Arg Asn Tyr Pro Ala Thr Ser Met Val Ser Gly Leu Ser Ser
        115                 120                 125
Pro Arg Thr Arg Thr Ser Ser Thr Glu Gly Asn Phe Thr Lys Glu Ala
    130                 135                 140
Ser Thr Tyr Thr Leu Thr Val Glu Thr Thr Ser Gly Pro Val Thr Glu
145                 150                 155                 160
Lys Tyr Thr Val Pro Thr Glu Thr Ser Thr Thr Glu Gly Asp Ser Thr
                165                 170                 175
Glu Thr Pro Trp Asp Thr Arg Tyr Ile Pro Val Lys Ile Thr Ser Pro
            180                 185                 190
Met Lys Thr Phe Ala Asp Ser Thr Ala Ser Lys Glu Asn Ala Pro Val
        195                 200                 205
Ser Met Thr Pro Ala Glu Thr Thr Val Thr Asp Ser His Thr Pro Gly
    210                 215                 220
Arg Thr Asn Pro Ser Phe Gly Thr Leu Tyr Ser Ser Phe Leu Asp Leu
225                 230                 235                 240
Ser Pro Lys Gly Thr Pro Asn Ser Arg Gly Glu Thr Ser Leu Glu Leu
                245                 250                 255
Ile Leu Ser Thr Thr Gly Tyr Pro Phe Ser Ser Pro Glu Pro Gly Ser
            260                 265                 270
Ala Gly His Ser Arg Ile Ser Thr Ser Ala Pro Leu Ser Ser Ser Ala
        275                 280                 285
Ser Val Leu Asp Asn Lys Ile Ser Glu Thr Ser Ile Phe Ser Gly Gln
    290                 295                 300
Ser Leu Thr Ser Pro Leu Ser Pro Gly Val Pro Glu Ala Arg Ala Ser
305                 310                 315                 320
Thr Met Pro Asn Ser Ala Ile Pro Phe Ser Met Thr Leu Ser Asn Ala
                325                 330                 335
Glu Thr Ser Ala Glu Arg Val Arg Ser Thr Ile Ser Ser Leu Gly Thr
            340                 345                 350
Pro Ser Ile Ser Thr Lys Gln Thr Ala Glu Thr Ile Leu Thr Phe His

        355                 360                 365
Ala Phe Ala Glu Thr Met Asp Ile Pro Ser Thr His Ile Ala Lys Thr
    370                 375                 380
Leu Ala Ser Glu Trp Leu Gly Ser Pro Gly Thr Leu Gly Gly Thr Ser
385                 390                 395                 400
Thr Ser Ala Leu Thr Thr Thr Ser Pro Ser Thr Thr Leu Val Ser Glu
                405                 410                 415
Glu Thr Asn Thr His His Ser Thr Ser Gly Lys Glu Thr Glu Gly Thr
            420                 425                 430
Leu Asn Thr Ser Met Thr Pro Leu Glu Thr Ser Ala Pro Gly Glu Glu
        435                 440                 445
Ser Glu Met Thr Ala Thr Leu Val Pro Thr Leu Gly Phe Thr Thr Leu
    450                 455                 460
Asp Ser Lys Ile Arg Ser Pro Ser Gln Val Ser Ser Ser His Pro Thr
465                 470                 475                 480
Arg Glu Leu Arg Thr Thr Gly Ser Thr Ser Gly Arg Gln Ser Ser Ser
                485                 490                 495
Thr Ala Ala His Gly Ser Ser Asp Ile Leu Arg Ala Thr Thr Ser Ser
            500                 505                 510
Thr Ser Lys Ala Ser Ser Trp Thr Ser Glu Ser Thr Ala Gln Gln Phe
        515                 520                 525
Ser Glu Pro Gln His Thr Gln Trp Val Glu Thr Ser Pro Ser Met Lys
    530                 535                 540
Thr Glu Arg Pro Pro Ala Ser Thr Ser Val Ala Ala Pro Ile Thr Thr
545                 550                 555                 560
Ser Val Pro Ser Val Val Ser Gly Phe Thr Thr Leu Lys Thr Ser Ser
                565                 570                 575
Thr Lys Gly Ile Trp Leu Glu Glu Thr Ser Ala Asp Thr Leu Ile Gly
            580                 585                 590
Glu Ser Thr Ala Gly Pro Thr Thr His Gln Phe Ala Val Pro Thr Gly
        595                 600                 605
Ile Ser Met Thr Gly Gly Ser Ser Thr Arg Gly Ser Gln Gly Thr Thr
    610                 615                 620
His Leu Leu Thr Arg Ala Thr Ala Ser Ser Glu Thr Ser Ala Asp Leu
625                 630                 635                 640
Thr Leu Ala Thr Asn Gly Val Pro Val Ser Val Ser Pro Ala Val Ser
                645                 650                 655
Lys Thr Ala Ala Gly Ser Ser Pro Pro Gly Gly Thr Lys Pro Ser Tyr
            660                 665                 670
Thr Met Val Ser Ser Val Ile Pro Glu Thr Ser Ser Leu Gln Ser Ser
        675                 680                 685
Ala Phe Arg Glu Gly Thr Ser Leu Gly Leu Thr Pro Leu Asn Thr Arg
    690                 695                 700
His Pro Phe Ser Ser Pro Glu Pro Asp Ser Ala Gly His Thr Lys Ile
705                 710                 715                 720
Ser Thr Ser Ile Pro Leu Leu Ser Ser Ala Ser Val Leu Glu Asp Lys
                725                 730                 735
Val Ser Ala Thr Ser Thr Phe Ser His His Lys Ala Thr Ser Ser Ile
            740                 745                 750
Thr Thr Gly Thr Pro Glu Ile Ser Thr Lys Thr Lys Pro Ser Ser Ala
        755                 760                 765
Val Leu Ser Ser Met Thr Leu Ser Asn Ala Ala Thr Ser Pro Glu Arg
    770                 775                 780
Val Arg Asn Ala Thr Ser Pro Leu Thr His Pro Ser Pro Ser Gly Glu
785                 790                 795                 800
Glu Thr Ala Gly Ser Val Leu Thr Leu Ser Thr Ser Ala Glu Thr Thr
                805                 810                 815
Asp Ser Pro Asn Ile His Pro Thr Gly Thr Leu Thr Ser Glu Ser Ser
            820                 825                 830
Glu Ser Pro Ser Thr Leu Ser Leu Pro Ser Val Ser Gly Val Lys Thr
        835                 840                 845
Thr Phe Ser Ser Ser Thr Pro Ser Thr His Leu Phe Thr Ser Gly Glu
    850                 855                 860
Glu Thr Glu Glu Thr Ser Asn Pro Ser Val Ser Gln Pro Glu Thr Ser
865                 870                 875                 880
Val Ser Arg Val Arg Thr Thr Leu Ala Ser Thr Ser Val Pro Thr Pro
                885                 890                 895
Val Phe Pro Thr Met Asp Thr Trp Pro Thr Arg Ser Ala Gln Phe Ser

900                 905                 910
Ser Ser His Leu Val Ser Glu Leu Arg Ala Thr Ser Ser Thr Ser Val
        915                 920                 925
Thr Asn Ser Thr Gly Ser Ala Leu Pro Lys Ile Ser His Leu Thr Gly
    930                 935                 940
Thr Ala Thr Met Ser Gln Thr Asn Arg Asp Thr Phe Asn Asp Ser Ala
945                 950                 955                 960
Ala Pro Gln Ser Thr Thr Trp Pro Glu Thr Ser Pro Arg Phe Lys Thr
                965                 970                 975
Gly Leu Pro Ser Ala Thr Thr Thr Val Ser Thr Ser Ala Thr Ser Leu
            980                 985                 990
Ser Ala Thr Val Met Val Ser Lys Phe Thr Ser Pro Ala Thr Ser Ser
        995                 1000                1005
Met Glu Ala Thr Ser Ile Arg Glu Pro Ser Thr Thr Ile Leu Thr Thr
    1010                1015                1020
Glu Thr Thr Asn Gly Pro Gly Ser Met Ala Val Ala Ser Thr Asn Ile
1025                1030                1035                1040
Pro Ile Gly Lys Gly Tyr Ile Thr Glu Gly Arg Leu Asp Thr Ser His
                1045                1050                1055
Leu Pro Ile Gly Thr Thr Ala Ser Ser Glu Thr Ser Met Asp Phe Thr
            1060                1065                1070
Met Ala Lys Glu Ser Val Ser Met Ser Val Ser Pro Ser Gln Ser Met
        1075                1080                1085
Asp Ala Ala Gly Ser Ser Thr Pro Gly Arg Thr Ser Gln Phe Val Asp
    1090                1095                1100
Thr Phe Ser Asp Asp Val Tyr His Leu Thr Ser Arg Glu Ile Thr Ile
1105                1110                1115                1120
Pro Arg Asp Gly Thr Ser Ser Ala Leu Thr Pro Gln Met Thr Ala Thr
                1125                1130                1135
His Pro Pro Ser Pro Asp Pro Gly Ser Ala Arg Ser Thr Trp Leu Gly
            1140                1145                1150
Ile Leu Ser Ser Ser Pro Ser Ser Pro Thr Pro Lys Val Thr Met Ser
        1155                1160                1165
Ser Thr Phe Ser Thr Gln Arg Val Thr Thr Ser Met Ile Met Asp Thr
    1170                1175                1180
Val Glu Thr Ser Arg Trp Asn Met Pro Asn Leu Pro Ser Thr Thr Ser
1185                1190                1195                1200
Leu Thr Pro Ser Asn Ile Pro Thr Ser Gly Ala Ile Gly Lys Ser Thr
                1205                1210                1215
Leu Val Pro Leu Asp Thr Pro Ser Pro Ala Thr Ser Leu Glu Ala Ser
            1220                1225                1230
Glu Gly Gly Leu Pro Thr Leu Ser Thr Tyr Pro Glu Ser Thr Asn Thr
        1235                1240                1245
Pro Ser Ile His Leu Gly Ala His Ala Ser Ser Glu Ser Pro Ser Thr
    1250                1255                1260
Ile Lys Leu Thr Met Ala Ser Val Val Lys Pro Gly Ser Tyr Thr Pro
1265                1270                1275                1280
Leu Thr Phe Pro Ser Ile Glu Thr His Ile His Val Ser Thr Ala Arg
                1285                1290                1295
Met Ala Tyr Ser Ser Gly Ser Ser Pro Glu Met Thr Ala Pro Gly Glu
            1300                1305                1310
Thr Asn Thr Gly Ser Thr Trp Asp Pro Thr Thr Tyr Ile Thr Thr Thr
        1315                1320                1325
Asp Pro Lys Asp Thr Ser Ser Ala Gln Val Ser Thr Pro His Ser Val
    1330                1335                1340
Arg Thr Leu Arg Thr Thr Glu Asn His Pro Lys Thr Glu Ser Ala Thr
1345                1350                1355                1360
Pro Ala Ala Tyr Ser Gly Ser Pro Lys Ile Ser Ser Ser Pro Asn Leu
                1365                1370                1375
Thr Ser Pro Ala Thr Lys Ala Trp Thr Ile Thr Asp Thr Thr Glu His
            1380                1385                1390
Ser Thr Gln Leu His Tyr Thr Lys Leu Ala Glu Lys Ser Ser Gly Phe
        1395                1400                1405
Glu Thr Gln Ser Ala Pro Gly Pro Val Ser Val Val Ile Pro Thr Ser
    1410                1415                1420
Pro Thr Ile Gly Ser Ser Thr Leu Glu Leu Thr Ser Asp Val Pro Gly
1425                1430                1435                1440
Glu Pro Leu Val Leu Ala Pro Ser Glu Gln Thr Thr Ile Thr Leu Pro

                1445                1450                1455
Met Ala Thr Trp Leu Ser Thr Ser Leu Thr Glu Glu Met Ala Ser Thr
            1460                1465                1470
Asp Leu Asp Ile Ser Ser Pro Ser Ser Pro Met Ser Thr Phe Ala Ile
        1475                1480                1485
Phe Pro Pro Met Ser Thr Pro Ser His Glu Leu Ser Lys Ser Glu Ala
    1490                1495                1500
Asp Thr Ser Ala Ile Arg Asn Thr Asp Ser Thr Thr Leu Asp Gln His
1505                1510                1515                1520
Leu Gly Ile Arg Ser Leu Gly Arg Thr Gly Asp Leu Thr Thr Val Pro
                1525                1530                1535
Ile Thr Pro Leu Thr Thr Thr Trp Thr Ser Val Ile Glu His Ser Thr
            1540                1545                1550
Gln Ala Gln Asp Thr Leu Ser Ala Thr Met Ser Pro Thr His Val Thr
        1555                1560                1565
Gln Ser Leu Lys Asp Gln Thr Ser Ile Pro Ala Ser Ala Ser Pro Ser
    1570                1575                1580
His Leu Thr Glu Val Tyr Pro Glu Leu Gly Thr Gln Gly Arg Ser Ser
1585                1590                1595                1600
Ser Glu Ala Thr Thr Phe Trp Lys Pro Ser Thr Asp Thr Leu Ser Arg
                1605                1610                1615
Glu Ile Glu Thr Gly Pro Thr Asn Ile Gln Ser Thr Pro Pro Met Asp
            1620                1625                1630
Asn Thr Thr Thr Gly Ser Ser Ser Ser Gly Val Thr Leu Gly Ile Ala
        1635                1640                1645
His Leu Pro Ile Gly Thr Ser Ser Pro Ala Glu Thr Ser Thr Asn Met
    1650                1655                1660
Ala Leu Glu Arg Arg Ser Ser Thr Ala Thr Val Ser Met Ala Gly Thr
1665                1670                1675                1680
Met Gly Leu Leu Val Thr Ser Ala Pro Gly Arg Ser Ile Ser Gln Ser
                1685                1690                1695
Leu Gly Arg Val Ser Ser Val Leu Ser Glu Ser Thr Thr Glu Gly Val
            1700                1705                1710
Thr Asp Ser Ser Lys Gly Ser Ser Pro Arg Leu Asn Thr Gln Gly Asn
        1715                1720                1725
Thr Ala Leu Ser Ser Ser Leu Glu Pro Ser Tyr Ala Glu Gly Ser Gln
    1730                1735                1740
Met Ser Thr Ser Ile Pro Leu Thr Ser Ser Pro Thr Thr Pro Asp Val
1745                1750                1755                1760
Glu Phe Ile Gly Gly Ser Thr Phe Trp Thr Lys Glu Val Thr Thr Val
                1765                1770                1775
Met Thr Ser Asp Ile Ser Lys Ser Ser Ala Arg Thr Glu Ser Ser Ser
            1780                1785                1790
Ala Thr Leu Met Ser Thr Ala Leu Gly Ser Thr Glu Asn Thr Gly Lys
        1795                1800                1805
Glu Lys Leu Arg Thr Ala Ser Met Asp Leu Pro Ser Pro Thr Pro Ser
    1810                1815                1820
Met Glu Val Thr Pro Trp Ile Ser Leu Thr Leu Ser Asn Ala Pro Asn
1825                1830                1835                1840
Thr Thr Asp Ser Leu Asp Leu Ser His Gly Val His Thr Ser Ser Ala
                1845                1850                1855
Gly Thr Leu Ala Thr Asp Arg Ser Leu Asn Thr Gly Val Thr Arg Ala
            1860                1865                1870
Ser Arg Leu Glu Asn Gly Ser Asp Thr Ser Ser Lys Ser Leu Ser Met
        1875                1880                1885
Gly Asn Ser Thr His Thr Ser Met Thr Tyr Thr Glu Lys Ser Glu Val
    1890                1895                1900
Ser Ser Ser Ile His Pro Arg Pro Glu Thr Ser Ala Pro Gly Ala Glu
1905                1910                1915                1920
Thr Thr Leu Thr Ser Thr Pro Gly Asn Arg Ala Ile Ser Leu Thr Leu
                1925                1930                1935
Pro Phe Ser Ser Ile Pro Val Glu Glu Val Ile Ser Thr Gly Ile Thr
            1940                1945                1950
Ser Gly Pro Asp Ile Asn Ser Ala Pro Met Thr His Ser Pro Ile Thr
        1955                1960                1965
Pro Pro Thr Ile Val Trp Thr Ser Thr Gly Thr Ile Glu Gln Ser Thr
    1970                1975                1980
Gln Pro Leu His Ala Val Ser Ser Glu Lys Val Ser Val Gln Thr Gln

1985                1990                1995                2000
Ser Thr Pro Tyr Val Asn Ser Val Ala Val Ser Ala Ser Pro Thr His
                2005                2010                2015
Glu Asn Ser Val Ser Ser Gly Ser Ser Thr Ser Ser Pro Tyr Ser Ser
            2020                2025                2030
Ala Ser Leu Glu Ser Leu Asp Ser Thr Ile Ser Arg Arg Asn Ala Ile
        2035                2040                2045
Thr Ser Trp Leu Trp Asp Leu Thr Thr Ser Leu Pro Thr Thr Thr Trp
    2050                2055                2060
Pro Ser Thr Ser Leu Ser Glu Ala Leu Ser Ser Gly His Ser Gly Val
2065                2070                2075                2080
Ser Asn Pro Ser Ser Thr Thr Thr Glu Phe Pro Leu Phe Ser Ala Ala
                2085                2090                2095
Ser Thr Ser Ala Ala Lys Gln Arg Asn Pro Glu Thr Glu Thr His Gly
            2100                2105                2110
Pro Gln Asn Thr Ala Ala Ser Thr Leu Asn Thr Asp Ala Ser Ser Val
        2115                2120                2125
Thr Gly Leu Ser Glu Thr Pro Val Gly Ala Ser Ile Ser Ser Glu Val
    2130                2135                2140
Pro Leu Pro Met Ala Ile Thr Ser Arg Ser Asp Val Ser Gly Leu Thr
2145                2150                2155                2160
Ser Glu Ser Thr Ala Asn Pro Ser Leu Gly Thr Ala Ser Ser Ala Gly
                2165                2170                2175
Thr Lys Leu Thr Arg Thr Ile Ser Leu Pro Thr Ser Glu Ser Leu Val
            2180                2185                2190
Ser Phe Arg Met Asn Lys Asp Pro Trp Thr Val Ser Ile Pro Leu Gly
        2195                2200                2205
Ser His Pro Thr Thr Asn Thr Glu Thr Ser Ile Pro Val Asn Ser Ala
    2210                2215                2220
Gly Pro Pro Gly Leu Ser Thr Val Ala Ser Asp Val Ile Asp Thr Pro
2225                2230                2235                2240
Ser Asp Gly Ala Glu Ser Ile Pro Thr Val Ser Phe Ser Pro Ser Pro
                2245                2250                2255
Asp Thr Glu Val Thr Thr Ile Ser His Phe Pro Glu Lys Thr Thr His
            2260                2265                2270
Ser Phe Arg Thr Ile Ser Ser Leu Thr His Glu Leu Thr Ser Arg Val
        2275                2280                2285
Thr Pro Ile Pro Gly Asp Trp Met Ser Ser Ala Met Ser Thr Lys Pro
    2290                2295                2300
Thr Gly Ala Ser Pro Ser Ile Thr Leu Gly Glu Arg Arg Thr Ile Thr
2305                2310                2315                2320
Ser Ala Ala Pro Thr Thr Ser Pro Ile Val Leu Thr Ala Ser Phe Thr
                2325                2330                2335
Glu Thr Ser Thr Val Ser Leu Asp Asn Glu Thr Thr Val Lys Thr Ser
            2340                2345                2350
Asp Ile Leu Asp Ala Arg Lys Thr Asn Glu Leu Pro Ser Asp Ser Ser
        2355                2360                2365
Ser Ser Ser Asp Leu Ile Asn Thr Ser Ile Ala Ser Ser Thr Met Asp
    2370                2375                2380
Val Thr Lys Thr Ala Ser Ile Ser Pro Thr Ser Ile Ser Gly Met Thr
2385                2390                2395                2400
Ala Ser Ser Ser Pro Ser Leu Phe Ser Ser Asp Arg Pro Gln Val Pro
                2405                2410                2415
Thr Ser Thr Thr Glu Thr Asn Thr Ala Thr Ser Pro Ser Val Ser Ser
            2420                2425                2430
Asn Thr Tyr Ser Leu Asp Gly Gly Ser Asn Val Gly Gly Thr Pro Ser
        2435                2440                2445
Thr Leu Pro Pro Phe Thr Ile Thr His Pro Val Glu Thr Ser Ser Ala
    2450                2455                2460
Leu Leu Ala Trp Ser Arg Pro Val Arg Thr Phe Ser Thr Met Val Ser
2465                2470                2475                2480
Thr Asp Thr Ala Ser Gly Glu Asn Pro Thr Ser Ser Asn Ser Val Val
                2485                2490                2495
Thr Ser Val Pro Ala Pro Gly Thr Trp Thr Ser Val Gly Ser Thr Thr
            2500                2505                2510
Asp Leu Pro Ala Met Gly Phe Leu Lys Thr Ser Pro Ala Gly Glu Ala
        2515                2520                2525
His Ser Leu Leu Ala Ser Thr Ile Glu Pro Ala Thr Ala Phe Thr Pro

2530                 2535                 2540
His Leu Ser Ala Ala Val Val Thr Gly Ser Ser Ala Thr Ser Glu Ala
2545                 2550                 2555                 2560
Ser Leu Leu Thr Thr Ser Glu Ser Lys Ala Ile His Ser Ser Pro Gln
                2565                 2570                 2575
Thr Pro Thr Thr Pro Thr Ser Gly Ala Asn Trp Glu Thr Ser Ala Thr
            2580                 2585                 2590
Pro Glu Ser Leu Leu Val Val Thr Glu Thr Ser Asp Thr Thr Leu Thr
        2595                 2600                 2605
Ser Lys Ile Leu Val Thr Asp Thr Ile Leu Phe Ser Thr Val Ser Thr
    2610                 2615                 2620
Pro Pro Ser Lys Phe Pro Ser Thr Gly Thr Leu Ser Gly Ala Ser Phe
2625                 2630                 2635                 2640
Pro Thr Leu Leu Pro Asp Thr Pro Ala Ile Pro Leu Thr Ala Thr Glu
                2645                 2650                 2655
Pro Thr Ser Ser Leu Ala Thr Ser Phe Asp Ser Thr Pro Leu Val Thr
            2660                 2665                 2670
Ile Ala Ser Asp Ser Leu Gly Thr Val Pro Glu Thr Thr Leu Thr Met
        2675                 2680                 2685
Ser Glu Thr Ser Asn Gly Asp Ala Leu Val Leu Lys Thr Val Ser Asn
    2690                 2695                 2700
Pro Asp Arg Ser Ile Pro Gly Ile Thr Ile Gln Gly Val Thr Glu Ser
2705                 2710                 2715                 2720
Pro Leu His Pro Ser Ser Thr Ser Pro Ser Lys Ile Val Ala Pro Arg
                2725                 2730                 2735
Asn Thr Thr Tyr Glu Gly Ser Ile Thr Val Ala Leu Ser Thr Leu Pro
            2740                 2745                 2750
Ala Gly Thr Thr Gly Ser Leu Val Phe Ser Gln Ser Ser Glu Asn Ser
        2755                 2760                 2765
Glu Thr Thr Ala Leu Val Asp Ser Ser Ala Gly Leu Glu Arg Ala Ser
    2770                 2775                 2780
Val Met Pro Leu Thr Thr Gly Ser Gln Gly Met Ala Ser Ser Gly Gly
2785                 2790                 2795                 2800
Ile Arg Ser Gly Ser Thr His Ser Thr Gly Thr Lys Thr Phe Ser Ser
                2805                 2810                 2815
Leu Pro Leu Thr Met Asn Pro Gly Glu Val Thr Ala Met Ser Glu Ile
            2820                 2825                 2830
Thr Thr Asn Arg Leu Thr Ala Thr Gln Ser Thr Ala Pro Lys Gly Ile
        2835                 2840                 2845
Pro Val Lys Pro Thr Ser Ala Glu Ser Gly Leu Leu Thr Pro Val Ser
    2850                 2855                 2860
Ala Ser Ser Ser Pro Ser Lys Ala Phe Ala Ser Leu Thr Thr Ala Pro
2865                 2870                 2875                 2880
Pro Thr Trp Gly Ile Pro Gln Ser Thr Leu Thr Phe Glu Phe Ser Glu
                2885                 2890                 2895
Val Pro Ser Leu Asp Thr Lys Ser Ala Ser Leu Pro Thr Pro Gly Gln
            2900                 2905                 2910
Ser Leu Asn Thr Ile Pro Asp Ser Asp Ala Ser Thr Ala Ser Ser Ser
        2915                 2920                 2925
Leu Ser Lys Ser Pro Glu Lys Asn Pro Arg Ala Arg Met Met Thr Ser
    2930                 2935                 2940
Thr Lys Ala Ile Ser Ala Ser Ser Phe Gln Ser Thr Gly Phe Thr Glu
2945                 2950                 2955                 2960
Thr Pro Glu Gly Ser Ala Ser Pro Ser Met Ala Gly His Glu Pro Arg
                2965                 2970                 2975
Val Pro Thr Ser Gly Thr Gly Asp Pro Arg Tyr Ala Ser Glu Ser Met
            2980                 2985                 2990
Ser Tyr Pro Asp Pro Ser Lys Ala Ser Ser Ala Met Thr Ser Thr Ser
        2995                 3000                 3005
Leu Ala Ser Lys Leu Thr Thr Leu Phe Ser Thr Gly Gln Ala Ala Arg
    3010                 3015                 3020
Ser Gly Ser Ser Ser Ser Pro Ile Ser Leu Ser Thr Glu Lys Glu Thr
3025                 3030                 3035                 3040
Ser Phe Leu Ser Pro Thr Ala Ser Thr Ser Arg Lys Thr Ser Leu Phe
                3045                 3050                 3055
Leu Gly Pro Ser Met Ala Arg Gln Pro Asn Ile Leu Val His Leu Gln
            3060                 3065                 3070
Thr Ser Ala Leu Thr Leu Ser Pro Thr Ser Thr Leu Asn Met Ser Gln

3075                3080                3085
Glu Glu Pro Pro Glu Leu Thr Ser Ser Gln Thr Ile Ala Glu Glu Glu
    3090                3095                3100
Gly Thr Thr Ala Glu Thr Gln Thr Leu Thr Phe Thr Pro Ser Glu Thr
3105                3110                3115                3120
Pro Thr Ser Leu Leu Pro Val Ser Ser Pro Thr Glu Pro Thr Ala Arg
                3125                3130                3135
Arg Lys Ser Ser Pro Glu Thr Trp Ala Ser Ser Ile Ser Val Pro Ala
            3140                3145                3150
Lys Thr Ser Leu Val Glu Thr Thr Asp Gly Thr Leu Val Thr Thr Ile
        3155                3160                3165
Lys Met Ser Ser Gln Ala Ala Gln Gly Asn Ser Thr Trp Pro Ala Pro
    3170                3175                3180
Ala Glu Glu Thr Gly Ser Ser Pro Ala Gly Thr Ser Pro Gly Ser Pro
3185                3190                3195                3200
Glu Met Ser Thr Thr Leu Lys Ile Met Ser Ser Lys Glu Pro Ser Ile
                3205                3210                3215
Ser Pro Glu Ile Arg Ser Thr Val Arg Asn Ser Pro Trp Lys Thr Pro
            3220                3225                3230
Glu Thr Thr Val Pro Met Glu Thr Thr Val Glu Pro Val Thr Leu Gln
        3235                3240                3245
Ser Thr Ala Leu Gly Ser Gly Ser Thr Ser Ile Ser His Leu Pro Thr
    3250                3255                3260
Gly Thr Thr Ser Pro Thr Lys Ser Pro Thr Glu Asn Met Leu Ala Thr
3265                3270                3275                3280
Glu Arg Val Ser Leu Ser Pro Ser Pro Pro Glu Ala Trp Thr Asn Leu
                3285                3290                3295
Tyr Ser Gly Thr Pro Gly Gly Thr Arg Gln Ser Leu Ala Thr Met Ser
            3300                3305                3310
Ser Val Ser Leu Glu Ser Pro Thr Ala Arg Ser Ile Thr Gly Thr Gly
        3315                3320                3325
Gln Gln Ser Ser Pro Glu Leu Val Ser Lys Thr Thr Gly Met Glu Phe
    3330                3335                3340
Ser Met Trp His Gly Ser Thr Gly Gly Thr Thr Gly Asp Thr His Val
3345                3350                3355                3360
Ser Leu Ser Thr Ser Ser Asn Ile Leu Glu Asp Pro Val Thr Ser Pro
                3365                3370                3375
Asn Ser Val Ser Ser Leu Thr Asp Lys Ser Lys His Lys Thr Glu Thr
            3380                3385                3390
Trp Val Ser Thr Thr Ala Ile Pro Ser Thr Val Leu Asn Asn Lys Ile
        3395                3400                3405
Met Ala Ala Glu Gln Gln Thr Ser Arg Ser Val Asp Glu Ala Tyr Ser
    3410                3415                3420
Ser Thr Ser Ser Trp Ser Asp Gln Thr Ser Gly Ser Asp Ile Thr Leu
3425                3430                3435                3440
Gly Ala Ser Pro Asp Val Thr Asn Thr Leu Tyr Ile Thr Ser Thr Ala
                3445                3450                3455
Gln Thr Thr Ser Leu Val Ser Leu Pro Ser Gly Asp Gln Gly Ile Thr
            3460                3465                3470
Ser Leu Thr Asn Pro Ser Gly Gly Lys Thr Ser Ser Ala Ser Ser Val
        3475                3480                3485
Thr Ser Pro Ser Ile Gly Leu Glu Thr Leu Arg Ala Asn Val Ser Ala
    3490                3495                3500
Val Lys Ser Asp Ile Ala Pro Thr Ala Gly His Leu Ser Gln Thr Ser
3505                3510                3515                3520
Ser Pro Ala Glu Val Ser Ile Leu Asp Val Thr Thr Ala Pro Thr Pro
                3525                3530                3535
Gly Ile Ser Thr Thr Ile Thr Thr Met Gly Thr Asn Ser Ile Ser Thr
            3540                3545                3550
Thr Thr Pro Asn Pro Glu Val Gly Met Ser Thr Met Asp Ser Thr Pro
        3555                3560                3565
Ala Thr Glu Arg Arg Thr Thr Ser Thr Glu His Pro Ser Thr Trp Ser
    3570                3575                3580
Ser Thr Ala Ala Ser Asp Ser Trp Thr Val Thr Asp Met Thr Ser Asn
3585                3590                3595                3600
Leu Lys Val Ala Arg Ser Pro Gly Thr Ile Ser Thr Met His Thr Thr
                3605                3610                3615
Ser Phe Leu Ala Ser Ser Thr Glu Leu Asp Ser Met Ser Thr Pro His

3620                3625                3630
Gly Arg Ile Thr Val Ile Gly Thr Ser Leu Val Thr Pro Ser Ser Asp
        3635                3640                3645
Ala Ser Ala Val Lys Thr Glu Thr Ser Thr Ser Glu Arg Thr Leu Ser
    3650                3655                3660
Pro Ser Asp Thr Thr Ala Ser Thr Pro Ile Ser Thr Phe Ser Arg Val
3665                3670                3675                3680
Gln Arg Met Ser Ile Ser Val Pro Asp Ile Leu Ser Thr Ser Trp Thr
                3685                3690                3695
Pro Ser Ser Thr Glu Ala Glu Asp Val Pro Val Ser Met Val Ser Thr
            3700                3705                3710
Asp His Ala Ser Thr Lys Thr Asp Pro Asn Thr Pro Leu Ser Thr Phe
        3715                3720                3725
Leu Phe Asp Ser Leu Ser Thr Leu Asp Trp Asp Thr Gly Arg Ser Leu
    3730                3735                3740
Ser Ser Ala Thr Ala Thr Thr Ser Ala Pro Gln Gly Ala Thr Thr Pro
3745                3750                3755                3760
Gln Glu Leu Thr Leu Glu Thr Met Ile Ser Pro Ala Thr Ser Gln Leu
                3765                3770                3775
Pro Phe Ser Ile Gly His Ile Thr Ser Ala Val Thr Pro Ala Ala Met
            3780                3785                3790
Ala Arg Ser Ser Gly Val Thr Phe Ser Arg Pro Asp Pro Thr Ser Lys
        3795                3800                3805
Lys Ala Glu Gln Thr Ser Thr Gln Leu Pro Thr Thr Thr Ser Ala His
    3810                3815                3820
Pro Gly Gln Val Pro Arg Ser Ala Ala Thr Thr Leu Asp Val Ile Pro
3825                3830                3835                3840
His Thr Ala Lys Thr Pro Asp Ala Thr Phe Gln Arg Gln Gly Gln Thr
                3845                3850                3855
Ala Leu Thr Thr Glu Ala Arg Ala Thr Ser Asp Ser Trp Asn Glu Lys
            3860                3865                3870
Glu Lys Ser Thr Pro Ser Ala Pro Trp Ile Thr Glu Met Met Asn Ser
        3875                3880                3885
Val Ser Glu Asp Thr Ile Lys Glu Val Thr Ser Ser Ser Ser Val Leu
    3890                3895                3900
Arg Thr Leu Asn Thr Leu Asp Ile Asn Leu Glu Ser Gly Thr Thr Ser
3905                3910                3915                3920
Ser Pro Ser Trp Lys Ser Ser Pro Tyr Glu Arg Ile Ala Pro Ser Glu
                3925                3930                3935
Ser Thr Thr Asp Lys Glu Ala Ile His Pro Ser Thr Asn Thr Val Glu
            3940                3945                3950
Thr Thr Gly Trp Val Thr Ser Ser Glu His Ala Ser His Ser Thr Ile
        3955                3960                3965
Pro Ala His Ser Ala Ser Ser Lys Leu Thr Ser Pro Val Val Thr Thr
    3970                3975                3980
Ser Thr Arg Glu Gln Ala Ile Val Ser Met Ser Thr Thr Thr Trp Pro
3985                3990                3995                4000
Glu Ser Thr Arg Ala Arg Thr Glu Pro Asn Ser Phe Leu Thr Ile Glu
                4005                4010                4015
Leu Arg Asp Val Ser Pro Tyr Met Asp Thr Ser Ser Thr Thr Gln Thr
            4020                4025                4030
Ser Ile Ile Ser Ser Pro Gly Ser Thr Ala Ile Thr Lys Gly Pro Arg
        4035                4040                4045
Thr Glu Ile Thr Ser Ser Lys Arg Ile Ser Ser Ser Phe Leu Ala Gln
    4050                4055                4060
Ser Met Arg Ser Ser Asp Ser Pro Ser Glu Ala Ile Thr Arg Leu Ser
4065                4070                4075                4080
Asn Phe Pro Ala Met Thr Glu Ser Gly Gly Met Ile Leu Ala Met Gln
                4085                4090                4095
Thr Ser Pro Pro Gly Ala Thr Ser Leu Ser Ala Pro Thr Leu Asp Thr
            4100                4105                4110
Ser Ala Thr Ala Ser Trp Thr Gly Thr Pro Leu Ala Thr Thr Gln Arg
        4115                4120                4125
Phe Thr Tyr Ser Glu Lys Thr Thr Leu Phe Ser Lys Gly Pro Glu Asp
    4130                4135                4140
Thr Ser Gln Pro Ser Pro Pro Ser Val Glu Glu Thr Ser Ser Ser Ser
4145                4150                4155                4160
Ser Leu Val Pro Ile His Ala Thr Thr Ser Pro Ser Asn Ile Leu Leu

4165                4170                4175
Thr Ser Gln Gly His Ser Pro Ser Ser Thr Pro Pro Val Thr Ser Val
            4180                4185                4190
Phe Leu Ser Glu Thr Ser Gly Leu Gly Lys Thr Thr Asp Met Ser Arg
        4195                4200                4205
Ile Ser Leu Glu Pro Gly Thr Ser Leu Pro Pro Asn Leu Ser Ser Thr
    4210                4215                4220
Ala Gly Glu Ala Leu Ser Thr Tyr Glu Ala Ser Arg Asp Thr Lys Ala
4225                4230                4235                4240
Ile His His Ser Ala Asp Thr Ala Val Thr Asn Met Glu Ala Thr Ser
                4245                4250                4255
Ser Glu Tyr Ser Pro Ile Pro Gly His Thr Lys Pro Ser Lys Ala Thr
            4260                4265                4270
Ser Pro Leu Val Thr Ser His Ile Met Gly Asp Ile Thr Ser Ser Thr
        4275                4280                4285
Ser Val Phe Gly Ser Ser Glu Thr Thr Glu Ile Glu Thr Val Ser Ser
    4290                4295                4300
Val Asn Gln Gly Leu Gln Glu Arg Ser Thr Ser Gln Val Ala Ser Ser
4305                4310                4315                4320
Ala Thr Glu Thr Ser Thr Val Ile Thr His Val Ser Ser Gly Asp Ala
                4325                4330                4335
Thr Thr His Val Thr Lys Thr Gln Ala Thr Phe Ser Ser Gly Thr Ser
            4340                4345                4350
Ile Ser Ser Pro His Gln Phe Ile Thr Ser Thr Asn Thr Phe Thr Asp
        4355                4360                4365
Val Ser Thr Asn Pro Ser Thr Ser Leu Ile Met Thr Glu Ser Ser Gly
    4370                4375                4380
Val Thr Ile Thr Thr Gln Thr Gly Pro Thr Gly Ala Ala Thr Gln Gly
4385                4390                4395                4400
Pro Tyr Leu Leu Asp Thr Ser Thr Met Pro Tyr Leu Thr Glu Thr Pro
                4405                4410                4415
Leu Ala Val Thr Pro Asp Phe Met Gln Ser Glu Lys Thr Thr Leu Ile
            4420                4425                4430
Ser Lys Gly Pro Lys Asp Val Ser Trp Thr Ser Pro Pro Ser Val Ala
        4435                4440                4445
Glu Thr Ser Tyr Pro Ser Ser Leu Thr Pro Phe Leu Val Thr Thr Ile
    4450                4455                4460
Pro Pro Ala Thr Ser Thr Leu Gln Gly Gln His Thr Ser Ser Pro Val
4465                4470                4475                4480
Ser Ala Thr Ser Val Leu Thr Ser Gly Leu Val Lys Thr Thr Asp Met
                4485                4490                4495
Leu Asn Thr Ser Met Glu Pro Val Thr Asn Ser Pro Gln Asn Leu Asn
            4500                4505                4510
Asn Pro Ser Asn Glu Ile Leu Ala Thr Leu Ala Ala Thr Thr Asp Ile
        4515                4520                4525
Glu Thr Ile His Pro Ser Ile Asn Lys Ala Val Thr Asn Met Gly Thr
    4530                4535                4540
Ala Ser Ser Ala His Val Leu His Ser Thr Leu Pro Val Ser Ser Glu
4545                4550                4555                4560
Pro Ser Thr Ala Thr Ser Pro Met Val Pro Ala Ser Ser Met Gly Asp
                4565                4570                4575
Ala Leu Ala Ser Ile Ser Ile Pro Gly Ser Glu Thr Thr Asp Ile Glu
            4580                4585                4590
Gly Glu Pro Thr Ser Ser Leu Thr Ala Gly Arg Lys Glu Asn Ser Thr
        4595                4600                4605
Leu Gln Glu Met Asn Ser Thr Thr Glu Ser Asn Ile Ile Leu Ser Asn
    4610                4615                4620
Val Ser Val Gly Ala Ile Thr Glu Ala Thr Lys Met Glu Val Pro Ser
4625                4630                4635                4640
Phe Asp Ala Thr Phe Ile Pro Thr Pro Ala Gln Ser Thr Lys Phe Pro
                4645                4650                4655
Asp Ile Phe Ser Val Ala Ser Ser Arg Leu Ser Asn Ser Pro Pro Met
            4660                4665                4670
Thr Ile Ser Thr His Met Thr Thr Thr Gln Thr Gly Ser Ser Gly Ala
        4675                4680                4685
Thr Ser Lys Ile Pro Leu Ala Leu Asp Thr Ser Thr Leu Glu Thr Ser
    4690                4695                4700
Ala Gly Thr Pro Ser Val Val Thr Glu Gly Phe Ala His Ser Lys Ile

4705                4710                4715                4720
Thr Thr Ala Met Asn Asn Asp Val Lys Asp Val Ser Gln Thr Asn Pro
                4725                4730                4735
Pro Phe Gln Asp Glu Ala Ser Ser Pro Ser Ser Gln Ala Pro Val Leu
            4740                4745                4750
Val Thr Thr Leu Pro Ser Ser Val Ala Phe Thr Pro Gln Trp His Ser
        4755                4760                4765
Thr Ser Ser Pro Val Ser Met Ser Ser Val Leu Thr Ser Ser Leu Val
    4770                4775                4780
Lys Thr Ala Gly Lys Val Asp Thr Ser Leu Glu Thr Val Thr Ser Ser
4785                4790                4795                4800
Pro Gln Ser Met Ser Asn Thr Leu Asp Asp Ile Ser Val Thr Ser Ala
                4805                4810                4815
Ala Thr Thr Asp Ile Glu Thr Thr His Pro Ser Ile Asn Thr Val Val
            4820                4825                4830
Thr Asn Val Gly Thr Thr Gly Ser Ala Phe Glu Ser His Ser Thr Val
        4835                4840                4845
Ser Ala Tyr Pro Glu Pro Ser Lys Val Thr Ser Pro Asn Val Thr Thr
    4850                4855                4860
Ser Thr Met Glu Asp Thr Thr Ile Ser Arg Ser Ile Pro Lys Ser Ser
4865                4870                4875                4880
Lys Thr Thr Arg Thr Glu Thr Glu Thr Thr Ser Ser Leu Thr Pro Lys
                4885                4890                4895
Leu Arg Glu Thr Ser Ile Ser Gln Glu Ile Thr Ser Ser Thr Glu Thr
            4900                4905                4910
Ser Thr Val Pro Tyr Lys Glu Leu Thr Gly Ala Thr Thr Glu Val Ser
        4915                4920                4925
Arg Thr Asp Val Thr Ser Ser Ser Ser Thr Ser Phe Pro Gly Pro Asp
    4930                4935                4940
Gln Ser Thr Val Ser Leu Asp Ile Ser Thr Glu Thr Asn Thr Arg Leu
4945                4950                4955                4960
Ser Thr Ser Pro Ile Met Thr Glu Ser Ala Glu Ile Thr Ile Thr Thr
                4965                4970                4975
Gln Thr Gly Pro His Gly Ala Thr Ser Gln Asp Thr Phe Thr Met Asp
            4980                4985                4990
Pro Ser Asn Thr Thr Pro Gln Ala Gly Ile His Ser Ala Met Thr His
        4995                5000                5005
Gly Phe Ser Gln Leu Asp Val Thr Thr Leu Met Ser Arg Ile Pro Gln
    5010                5015                5020
Asp Val Ser Trp Thr Ser Pro Pro Ser Val Asp Lys Thr Ser Ser Pro
5025                5030                5035                5040
Ser Ser Phe Leu Ser Ser Pro Ala Met Thr Thr Pro Ser Leu Ile Ser
                5045                5050                5055
Ser Thr Leu Pro Glu Asp Lys Leu Ser Ser Pro Met Thr Ser Leu Leu
            5060                5065                5070
Thr Ser Gly Leu Val Lys Ile Thr Asp Ile Leu Arg Thr Arg Leu Glu
        5075                5080                5085
Pro Val Thr Ser Ser Leu Pro Asn Phe Ser Ser Thr Ser Asp Lys Ile
    5090                5095                5100
Leu Ala Thr Ser Lys Asp Ser Lys Asp Thr Lys Glu Ile Phe Pro Ser
5105                5110                5115                5120
Ile Asn Thr Glu Glu Thr Asn Val Lys Ala Asn Asn Ser Gly His Glu
                5125                5130                5135
Ser His Ser Pro Ala Leu Ala Asp Ser Glu Thr Pro Lys Ala Thr Thr
            5140                5145                5150
Gln Met Val Ile Thr Thr Thr Val Gly Asp Pro Ala Pro Ser Thr Ser
        5155                5160                5165
Met Pro Val His Gly Ser Ser Glu Thr Thr Asn Ile Lys Arg Glu Pro
    5170                5175                5180
Thr Tyr Phe Leu Thr Pro Arg Leu Arg Glu Thr Ser Thr Ser Gln Glu
5185                5190                5195                5200
Ser Ser Phe Pro Thr Asp Thr Ser Phe Leu Leu Ser Lys Val Pro Thr
                5205                5210                5215
Gly Thr Ile Thr Glu Val Ser Ser Thr Gly Val Asn Ser Ser Ser Lys
            5220                5225                5230
Ile Ser Thr Pro Asp His Asp Lys Ser Thr Val Pro Pro Asp Thr Phe
        5235                5240                5245
Thr Gly Glu Ile Pro Arg Val Phe Thr Ser Ser Ile Lys Thr Lys Ser

5250                5255                5260
Ala Glu Met Thr Ile Thr Thr Gln Ala Ser Pro Pro Glu Ser Ala Ser
5265                5270                5275                5280
His Ser Thr Leu Pro Leu Asp Thr Ser Thr Thr Leu Ser Gln Gly Gly
                5285                5290                5295
Thr His Ser Thr Val Thr Gln Gly Phe Pro Tyr Ser Glu Val Thr Thr
            5300                5305                5310
Leu Met Gly Met Gly Pro Gly Asn Val Ser Trp Met Thr Thr Pro Pro
        5315                5320                5325
Val Glu Glu Thr Ser Ser Val Ser Ser Leu Met Ser Ser Pro Ala Met
    5330                5335                5340
Thr Ser Pro Ser Pro Val Ser Ser Thr Ser Pro Gln Ser Ile Pro Ser
5345                5350                5355                5360
Ser Pro Leu Pro Val Thr Ala Leu Pro Thr Ser Val Leu Val Thr Thr
                5365                5370                5375
Thr Asp Val Leu Gly Thr Thr Ser Pro Glu Ser Val Thr Ser Ser Pro
            5380                5385                5390
Pro Asn Leu Ser Ser Ile Thr His Glu Arg Pro Ala Thr Tyr Lys Asp
        5395                5400                5405
Thr Ala His Thr Glu Ala Ala Met His His Ser Thr Asn Thr Ala Val
    5410                5415                5420
Thr Asn Val Gly Thr Ser Gly Ser Gly His Lys Ser Gln Ser Ser Val
5425                5430                5435                5440
Leu Ala Asp Ser Glu Thr Ser Lys Ala Thr Pro Leu Met Ser Thr Thr
                5445                5450                5455
Ser Thr Leu Gly Asp Thr Ser Val Ser Thr Ser Thr Pro Asn Ile Ser
            5460                5465                5470
Gln Thr Asn Gln Ile Gln Thr Glu Pro Thr Ala Ser Leu Ser Pro Arg
        5475                5480                5485
Leu Arg Glu Ser Ser Thr Ser Glu Lys Thr Ser Ser Thr Thr Glu Thr
    5490                5495                5500
Asn Thr Ala Phe Ser Tyr Val Pro Thr Gly Ala Ile Thr Gln Ala Ser
5505                5510                5515                5520
Arg Thr Glu Ile Ser Ser Ser Arg Thr Ser Ile Ser Asp Leu Asp Arg
                5525                5530                5535
Pro Thr Ile Ala Pro Asp Ile Ser Thr Gly Met Ile Thr Arg Leu Phe
            5540                5545                5550
Thr Ser Pro Ile Met Thr Lys Ser Ala Glu Met Thr Val Thr Thr Gln
        5555                5560                5565
Thr Thr Thr Pro Gly Ala Thr Ser Gln Gly Ile Leu Pro Trp Asp Thr
    5570                5575                5580
Ser Thr Thr Leu Phe Gln Gly Gly Thr His Ser Thr Val Ser Gln Gly
5585                5590                5595                5600
Phe Pro His Ser Glu Ile Thr Thr Leu Arg Ser Arg Thr Pro Gly Asp
                5605                5610                5615
Val Ser Trp Met Thr Thr Pro Pro Val Glu Glu Thr Ser Ser Gly Phe
            5620                5625                5630
Ser Leu Met Ser Pro Ser Met Thr Ser Pro Ser Pro Val Ser Ser Thr
        5635                5640                5645
Ser Pro Glu Ser Ile Pro Ser Ser Pro Leu Pro Val Thr Ala Leu Leu
    5650                5655                5660
Thr Ser Val Leu Val Thr Thr Thr Asn Val Leu Gly Thr Thr Ser Pro
5665                5670                5675                5680
Glu Pro Val Thr Ser Ser Pro Pro Asn Leu Ser Ser Pro Thr Gln Glu
                5685                5690                5695
Arg Leu Thr Thr Tyr Lys Asp Thr Ala His Thr Glu Ala Met His Ala
            5700                5705                5710
Ser Met His Thr Asn Thr Ala Val Ala Asn Val Gly Thr Ser Ile Ser
        5715                5720                5725
Gly His Glu Ser Gln Ser Ser Val Pro Ala Asp Ser His Thr Ser Lys
    5730                5735                5740
Ala Thr Ser Pro Met Gly Ile Thr Phe Ala Met Gly Asp Thr Ser Val
5745                5750                5755                5760
Ser Thr Ser Thr Pro Ala Phe Phe Glu Thr Arg Ile Gln Thr Glu Ser
                5765                5770                5775
Thr Ser Ser Leu Ile Pro Gly Leu Arg Asp Thr Arg Thr Ser Glu Glu
            5780                5785                5790
Ile Asn Thr Val Thr Glu Thr Ser Thr Val Leu Ser Glu Val Pro Thr

5795                5800                5805
Thr Thr Thr Thr Glu Val Ser Arg Thr Glu Val Ile Thr Ser Ser Arg
    5810                5815                5820
Thr Thr Ile Ser Gly Pro Asp His Ser Lys Met Ser Pro Tyr Ile Ser
5825                5830                5835                5840
Thr Glu Thr Ile Thr Arg Leu Ser Thr Phe Pro Phe Val Thr Gly Ser
                5845                5850                5855
Thr Glu Met Ala Ile Thr Asn Gln Thr Gly Pro Ile Gly Thr Ile Ser
            5860                5865                5870
Gln Ala Thr Leu Thr Leu Asp Thr Ser Ser Thr Ala Ser Trp Glu Gly
        5875                5880                5885
Thr His Ser Pro Val Thr Gln Arg Phe Pro His Ser Glu Glu Thr Thr
    5890                5895                5900
Thr Met Ser Arg Ser Thr Lys Gly Val Ser Trp Gln Ser Pro Pro Ser
5905                5910                5915                5920
Val Glu Glu Thr Ser Ser Pro Ser Ser Pro Val Pro Leu Pro Ala Ile
                5925                5930                5935
Thr Ser His Ser Ser Leu Tyr Ser Ala Val Ser Gly Ser Ser Pro Thr
            5940                5945                5950
Ser Ala Leu Pro Val Thr Ser Leu Leu Thr Ser Gly Arg Arg Lys Thr
        5955                5960                5965
Ile Asp Met Leu Asp Thr His Ser Glu Leu Val Thr Ser Ser Leu Pro
    5970                5975                5980
Ser Ala Ser Ser Phe Ser Gly Glu Ile Leu Thr Ser Glu Ala Ser Thr
5985                5990                5995                6000
Asn Thr Glu Thr Ile His Phe Ser Glu Asn Thr Ala Glu Thr Asn Met
                6005                6010                6015
Gly Thr Thr Asn Ser Met His Lys Leu His Ser Ser Val Ser Ile His
            6020                6025                6030
Ser Gln Pro Ser Gly His Thr Pro Pro Lys Val Thr Gly Ser Met Met
        6035                6040                6045
Glu Asp Ala Ile Val Ser Thr Ser Thr Pro Gly Ser Pro Glu Thr Lys
    6050                6055                6060
Asn Val Asp Arg Asp Ser Thr Ser Pro Leu Thr Pro Glu Leu Lys Glu
6065                6070                6075                6080
Asp Ser Thr Ala Leu Val Met Asn Ser Thr Thr Glu Ser Asn Thr Val
                6085                6090                6095
Phe Ser Ser Val Ser Leu Asp Ala Ala Thr Glu Val Ser Arg Ala Glu
            6100                6105                6110
Val Thr Tyr Tyr Asp Pro Thr Phe Met Pro Ala Ser Ala Gln Ser Thr
        6115                6120                6125
Lys Ser Pro Asp Ile Ser Pro Glu Ala Ser Ser Ser His Ser Asn Ser
    6130                6135                6140
Pro Pro Leu Thr Ile Ser Thr His Lys Thr Ile Ala Thr Gln Thr Gly
6145                6150                6155                6160
Pro Ser Gly Val Thr Ser Leu Gly Gln Leu Thr Leu Asp Thr Ser Thr
                6165                6170                6175
Ile Ala Thr Ser Ala Gly Thr Pro Ser Ala Arg Thr Gln Asp Phe Val
            6180                6185                6190
Asp Ser Glu Thr Thr Ser Val Met Asn Asn Asp Leu Asn Asp Val Leu
        6195                6200                6205
Lys Thr Ser Pro Phe Ser Ala Glu Glu Ala Asn Ser Leu Ser Ser Gln
    6210                6215                6220
Ala Pro Leu Leu Val Thr Thr Ser Pro Ser Pro Val Thr Ser Thr Leu
6225                6230                6235                6240
Gln Glu His Ser Thr Ser Ser Leu Val Ser Val Thr Ser Val Pro Thr
                6245                6250                6255
Pro Thr Leu Ala Lys Ile Thr Asp Met Asp Thr Asn Leu Glu Pro Val
            6260                6265                6270
Thr Arg Ser Pro Gln Asn Leu Arg Asn Thr Leu Ala Thr Ser Glu Ala
        6275                6280                6285
Thr Thr Asp Thr His Thr Met His Pro Ser Ile Asn Thr Ala Val Ala
    6290                6295                6300
Asn Val Gly Thr Thr Ser Ser Pro Asn Glu Phe Tyr Phe Thr Val Ser
6305                6310                6315                6320
Pro Asp Ser Asp Pro Tyr Lys Ala Thr Ser Ala Val Val Ile Thr Ser
                6325                6330                6335
Thr Ser Gly Asp Ser Ile Val Ser Thr Ser Met Pro Arg Ser Ser Ala

6340                6345                6350
Met Lys Lys Ile Glu Ser Glu Thr Thr Phe Ser Leu Ile Phe Arg Leu
        6355                6360                6365
Arg Glu Thr Ser Thr Ser Gln Lys Ile Gly Ser Ser Ser Asp Thr Ser
    6370                6375                6380
Thr Val Phe Asp Lys Ala Phe Thr Ala Ala Thr Thr Glu Val Ser Arg
6385                6390                6395                6400
Thr Glu Leu Thr Ser Ser Ser Arg Thr Ser Ile Gln Gly Thr Glu Lys
                6405                6410                6415
Pro Thr Met Ser Pro Asp Thr Ser Thr Arg Ser Val Thr Met Leu Ser
            6420                6425                6430
Thr Phe Ala Gly Leu Thr Lys Ser Glu Glu Arg Thr Ile Ala Thr Gln
        6435                6440                6445
Thr Gly Pro His Arg Ala Thr Ser Gln Gly Thr Leu Thr Trp Asp Thr
    6450                6455                6460
Ser Ile Thr Thr Ser Gln Ala Gly Thr His Ser Ala Met Thr His Gly
6465                6470                6475                6480
Phe Ser Gln Leu Asp Leu Ser Thr Leu Thr Ser Arg Val Pro Glu Tyr
                6485                6490                6495
Ile Ser Gly Thr Ser Pro Pro Ser Val Glu Lys Thr Ser Ser Ser Ser
            6500                6505                6510
Ser Leu Leu Ser Leu Pro Ala Ile Thr Ser Pro Ser Pro Val Pro Thr
        6515                6520                6525
Thr Leu Pro Glu Ser Arg Pro Ser Ser Pro Val His Leu Thr Ser Leu
    6530                6535                6540
Pro Thr Ser Gly Leu Val Lys Thr Thr Asp Met Leu Ala Ser Val Ala
6545                6550                6555                6560
Ser Leu Pro Pro Asn Leu Gly Ser Thr Ser His Lys Ile Pro Thr Thr
                6565                6570                6575
Ser Glu Asp Ile Lys Asp Thr Glu Lys Met Tyr Pro Ser Thr Asn Ile
            6580                6585                6590
Ala Val Thr Asn Val Gly Thr Thr Thr Ser Glu Lys Glu Ser Tyr Ser
        6595                6600                6605
Ser Val Pro Ala Tyr Ser Glu Pro Pro Lys Val Thr Ser Pro Met Val
    6610                6615                6620
Thr Ser Phe Asn Ile Arg Asp Thr Ile Val Ser Thr Ser Met Pro Gly
6625                6630                6635                6640
Ser Ser Glu Ile Thr Arg Ile Glu Met Glu Ser Thr Phe Ser Leu Ala
                6645                6650                6655
His Gly Leu Lys Gly Thr Ser Thr Ser Gln Asp Pro Ile Val Ser Thr
            6660                6665                6670
Glu Lys Ser Ala Val Leu His Lys Leu Thr Thr Gly Ala Thr Glu Thr
        6675                6680                6685
Ser Arg Thr Glu Val Ala Ser Ser Arg Arg Thr Ser Ile Pro Gly Pro
    6690                6695                6700
Asp His Ser Thr Glu Ser Pro Asp Ile Ser Thr Glu Val Ile Pro Ser
6705                6710                6715                6720
Leu Pro Ile Ser Leu Gly Ile Thr Glu Ser Ser Asn Met Thr Ile Ile
                6725                6730                6735
Thr Arg Thr Gly Pro Pro Leu Gly Ser Thr Ser Gln Gly Thr Phe Thr
            6740                6745                6750
Leu Asp Thr Pro Thr Thr Ser Ser Arg Ala Gly Thr His Ser Met Ala
        6755                6760                6765
Thr Gln Glu Phe Pro His Ser Glu Met Thr Thr Val Met Asn Lys Asp
    6770                6775                6780
Pro Glu Ile Leu Ser Trp Thr Ile Pro Pro Ser Ile Glu Lys Thr Ser
6785                6790                6795                6800
Phe Ser Ser Ser Leu Met Pro Ser Pro Ala Met Thr Ser Pro Pro Val
                6805                6810                6815
Ser Ser Thr Leu Pro Lys Thr Ile His Thr Thr Pro Ser Pro Met Thr
            6820                6825                6830
Ser Leu Leu Thr Pro Ser Leu Val Met Thr Thr Asp Thr Leu Gly Thr
        6835                6840                6845
Ser Pro Glu Pro Thr Thr Ser Ser Pro Pro Asn Leu Ser Ser Thr Ser
    6850                6855                6860
His Glu Ile Leu Thr Thr Asp Glu Asp Thr Thr Ala Ile Glu Ala Met
6865                6870                6875                6880
His Pro Ser Thr Ser Thr Ala Ala Thr Asn Val Glu Thr Thr Ser Ser

6885                    6890                    6895
Gly His Gly Ser Gln Ser Ser Val Leu Ala Asp Ser Glu Lys Thr Lys
            6900                    6905                    6910
Ala Thr Ala Pro Met Asp Thr Thr Ser Thr Met Gly His Thr Thr Val
        6915                    6920                    6925
Ser Thr Ser Met Ser Val Ser Ser Glu Thr Thr Lys Ile Lys Arg Glu
    6930                    6935                    6940
Ser Thr Tyr Ser Leu Thr Pro Gly Leu Arg Glu Thr Ser Ile Ser Gln
6945                    6950                    6955                    6960
Asn Ala Ser Phe Ser Thr Asp Thr Ser Ile Val Leu Ser Glu Val Pro
                6965                    6970                    6975
Thr Gly Thr Thr Ala Glu Val Ser Arg Thr Glu Val Thr Ser Ser Gly
            6980                    6985                    6990
Arg Thr Ser Ile Pro Gly Pro Ser Gln Ser Thr Val Leu Pro Glu Ile
        6995                    7000                    7005
Ser Thr Arg Thr Met Thr Arg Leu Phe Ala Ser Pro Thr Met Thr Glu
    7010                    7015                    7020
Ser Ala Glu Met Thr Ile Pro Thr Gln Thr Gly Pro Ser Gly Ser Thr
7025                    7030                    7035                    7040
Ser Gln Asp Thr Leu Thr Leu Asp Thr Ser Thr Thr Lys Ser Gln Ala
                7045                    7050                    7055
Lys Thr His Ser Thr Leu Thr Gln Arg Phe Pro His Ser Glu Met Thr
            7060                    7065                    7070
Thr Leu Met Ser Arg Gly Pro Gly Asp Met Ser Trp Gln Ser Ser Pro
        7075                    7080                    7085
Ser Leu Glu Asn Pro Ser Ser Leu Pro Ser Leu Leu Ser Leu Pro Ala
    7090                    7095                    7100
Thr Thr Ser Pro Pro Pro Ile Ser Ser Thr Leu Pro Val Thr Ile Ser
7105                    7110                    7115                    7120
Ser Ser Pro Leu Pro Val Thr Ser Leu Leu Thr Ser Ser Pro Val Thr
                7125                    7130                    7135
Thr Thr Asp Met Leu His Thr Ser Pro Glu Leu Val Thr Ser Ser Pro
            7140                    7145                    7150
Pro Lys Leu Ser His Thr Ser Asp Glu Arg Leu Thr Thr Gly Lys Asp
        7155                    7160                    7165
Thr Thr Asn Thr Glu Ala Val His Pro Ser Thr Asn Thr Ala Ala Ser
    7170                    7175                    7180
Asn Val Glu Ile Pro Ser Ser Gly His Glu Ser Pro Ser Ser Ala Leu
7185                    7190                    7195                    7200
Ala Asp Ser Glu Thr Ser Lys Ala Thr Ser Pro Met Phe Ile Thr Ser
                7205                    7210                    7215
Thr Gln Glu Asp Thr Thr Val Ala Ile Ser Thr Pro His Phe Leu Glu
            7220                    7225                    7230
Thr Ser Arg Ile Gln Lys Glu Ser Ile Ser Ser Leu Ser Pro Lys Leu
        7235                    7240                    7245
Arg Glu Thr Gly Ser Ser Val Glu Thr Ser Ser Ala Ile Glu Thr Ser
    7250                    7255                    7260
Ala Val Leu Ser Glu Val Ser Ile Gly Ala Thr Thr Glu Ile Ser Arg
7265                    7270                    7275                    7280
Thr Glu Val Thr Ser Ser Ser Arg Thr Ser Ile Ser Gly Ser Ala Glu
                7285                    7290                    7295
Ser Thr Met Leu Pro Glu Ile Ser Thr Thr Arg Lys Ile Ile Lys Phe
            7300                    7305                    7310
Pro Thr Ser Pro Ile Leu Ala Glu Ser Ser Glu Met Thr Ile Lys Thr
        7315                    7320                    7325
Gln Thr Ser Pro Pro Gly Ser Thr Ser Glu Ser Thr Phe Thr Leu Asp
    7330                    7335                    7340
Thr Ser Thr Thr Pro Ser Leu Val Ile Thr His Ser Thr Met Thr Gln
7345                    7350                    7355                    7360
Arg Leu Pro His Ser Glu Ile Thr Thr Leu Val Ser Arg Gly Ala Gly
                7365                    7370                    7375
Asp Val Pro Arg Pro Ser Ser Leu Pro Val Glu Glu Thr Ser Pro Pro
            7380                    7385                    7390
Ser Ser Gln Leu Ser Leu Ser Ala Met Ile Ser Pro Ser Pro Val Ser
        7395                    7400                    7405
Ser Thr Leu Pro Ala Ser Ser His Ser Ser Ser Ala Ser Val Thr Ser
    7410                    7415                    7420
Leu Leu Thr Pro Gly Gln Val Lys Thr Thr Glu Val Leu Asp Ala Ser

7425                7430                7435                7440
Ala Glu Pro Glu Thr Ser Ser Pro Pro Ser Leu Ser Ser Thr Ser Val
                7445                7450                7455
Glu Ile Leu Ala Thr Ser Glu Val Thr Thr Asp Thr Glu Lys Ile His
            7460                7465                7470
Pro Phe Ser Asn Thr Ala Val Thr Lys Val Gly Thr Ser Ser Ser Gly
        7475                7480                7485
His Glu Ser Pro Ser Ser Val Leu Pro Asp Ser Glu Thr Thr Lys Ala
    7490                7495                7500
Thr Ser Ala Met Gly Thr Ile Ser Ile Met Gly Asp Thr Ser Val Ser
7505                7510                7515                7520
Thr Leu Thr Pro Ala Leu Ser Asn Thr Arg Lys Ile Gln Ser Glu Pro
                7525                7530                7535
Ala Ser Ser Leu Thr Thr Arg Leu Arg Glu Thr Ser Thr Ser Glu Glu
            7540                7545                7550
Thr Ser Leu Ala Thr Glu Ala Asn Thr Val Leu Ser Lys Val Ser Thr
        7555                7560                7565
Gly Ala Thr Thr Glu Val Ser Arg Thr Glu Ala Ile Ser Phe Ser Arg
    7570                7575                7580
Thr Ser Met Ser Gly Pro Glu Gln Ser Thr Met Ser Gln Asp Ile Ser
7585                7590                7595                7600
Ile Gly Thr Ile Pro Arg Ile Ser Ala Ser Ser Val Leu Thr Glu Ser
                7605                7610                7615
Ala Lys Met Thr Ile Thr Thr Gln Thr Gly Pro Ser Glu Ser Thr Leu
            7620                7625                7630
Glu Ser Thr Leu Asn Leu Asn Thr Ala Thr Thr Pro Ser Trp Val Glu
        7635                7640                7645
Thr His Ser Ile Val Ile Gln Gly Phe Pro His Pro Glu Met Thr Thr
    7650                7655                7660
Ser Met Gly Arg Gly Pro Gly Gly Val Ser Trp Pro Ser Pro Pro Phe
7665                7670                7675                7680
Val Lys Glu Thr Ser Pro Pro Ser Ser Pro Leu Ser Leu Pro Ala Val
                7685                7690                7695
Thr Ser Pro His Pro Val Ser Thr Thr Phe Leu Ala His Ile Pro Pro
            7700                7705                7710
Ser Pro Leu Pro Val Thr Ser Leu Leu Thr Ser Gly Pro Ala Thr Thr
        7715                7720                7725
Thr Asp Ile Leu Gly Thr Ser Thr Glu Pro Gly Thr Ser Ser Ser Ser
    7730                7735                7740
Ser Leu Ser Thr Thr Ser His Glu Arg Leu Thr Thr Tyr Lys Asp Thr
7745                7750                7755                7760
Ala His Thr Glu Ala Val His Pro Ser Thr Asn Thr Gly Gly Thr Asn
                7765                7770                7775
Val Ala Thr Thr Ser Ser Gly Tyr Lys Ser Gln Ser Ser Val Leu Ala
            7780                7785                7790
Asp Ser Ser Pro Met Cys Thr Thr Ser Thr Met Gly Asp Thr Ser Val
        7795                7800                7805
Leu Thr Ser Thr Pro Ala Phe Leu Glu Thr Arg Arg Ile Gln Thr Glu
    7810                7815                7820
Leu Ala Ser Ser Leu Thr Pro Gly Leu Arg Glu Ser Ser Gly Ser Glu
7825                7830                7835                7840
Gly Thr Ser Ser Gly Thr Lys Met Ser Thr Val Leu Ser Lys Val Pro
                7845                7850                7855
Thr Gly Ala Thr Thr Glu Ile Ser Lys Glu Asp Val Thr Ser Ile Pro
            7860                7865                7870
Gly Pro Ala Gln Ser Thr Ile Ser Pro Asp Ile Ser Thr Arg Thr Val
        7875                7880                7885
Ser Trp Phe Ser Thr Ser Pro Val Met Thr Glu Ser Ala Glu Ile Thr
    7890                7895                7900
Met Asn Thr His Thr Ser Pro Leu Gly Ala Thr Thr Gln Gly Thr Ser
7905                7910                7915                7920
Thr Leu Asp Thr Ser Ser Thr Thr Ser Leu Thr Met Thr His Ser Thr
                7925                7930                7935
Ile Ser Gln Gly Phe Ser His Ser Gln Met Ser Thr Leu Met Arg Arg
            7940                7945                7950
Gly Pro Glu Asp Val Ser Trp Met Ser Pro Pro Leu Leu Glu Lys Thr
        7955                7960                7965
Arg Pro Ser Phe Ser Leu Met Ser Ser Pro Ala Thr Thr Ser Pro Ser

7970                7975                7980
Pro Val Ser Ser Thr Leu Pro Glu Ser Ile Ser Ser Ser Pro Leu Pro
7985                7990                7995                8000
Val Thr Ser Leu Leu Thr Ser Gly Leu Ala Lys Thr Thr Asp Met Leu
                8005                8010                8015
His Lys Ser Ser Glu Pro Val Thr Asn Ser Pro Ala Asn Leu Ser Ser
            8020                8025                8030
Thr Ser Val Glu Ile Leu Ala Thr Ser Glu Val Thr Thr Asp Thr Glu
        8035                8040                8045
Lys Thr His Pro Ser Ser Asn Arg Thr Val Thr Asp Val Gly Thr Ser
    8050                8055                8060
Ser Ser Gly His Glu Ser Thr Ser Phe Val Leu Ala Asp Ser Gln Thr
8065                8070                8075                8080
Ser Lys Val Thr Ser Pro Met Val Ile Thr Ser Thr Met Glu Asp Thr
                8085                8090                8095
Ser Val Ser Thr Ser Thr Pro Gly Phe Phe Glu Thr Ser Arg Ile Gln
            8100                8105                8110
Thr Glu Pro Thr Ser Ser Leu Thr Leu Gly Leu Arg Lys Thr Ser Ser
        8115                8120                8125
Ser Glu Gly Thr Ser Leu Ala Thr Glu Met Ser Thr Val Leu Ser Gly
    8130                8135                8140
Val Pro Thr Gly Ala Thr Ala Glu Val Ser Arg Thr Glu Val Thr Ser
8145                8150                8155                8160
Ser Ser Arg Thr Ser Ile Ser Gly Phe Ala Gln Leu Thr Val Ser Pro
                8165                8170                8175
Glu Thr Ser Thr Glu Thr Ile Thr Arg Leu Pro Thr Ser Ser Ile Met
            8180                8185                8190
Thr Glu Ser Ala Glu Met Met Ile Lys Thr Gln Thr Asp Pro Pro Gly
        8195                8200                8205
Ser Thr Pro Glu Ser Thr His Thr Val Asp Ile Ser Thr Thr Pro Asn
    8210                8215                8220
Trp Val Glu Thr His Ser Thr Val Thr Gln Arg Phe Ser His Ser Glu
8225                8230                8235                8240
Met Thr Thr Leu Val Ser Arg Ser Pro Gly Asp Met Leu Trp Pro Ser
                8245                8250                8255
Gln Ser Ser Val Glu Glu Thr Ser Ser Ala Ser Ser Leu Leu Ser Leu
            8260                8265                8270
Pro Ala Thr Thr Ser Pro Ser Pro Val Ser Ser Thr Leu Val Glu Asp
        8275                8280                8285
Phe Pro Ser Ala Ser Leu Pro Val Thr Ser Leu Leu Asn Pro Gly Leu
    8290                8295                8300
Val Ile Thr Thr Asp Arg Met Gly Ile Ser Arg Glu Pro Gly Thr Ser
8305                8310                8315                8320
Ser Thr Ser Asn Leu Ser Ser Thr Ser His Glu Arg Leu Thr Thr Leu
                8325                8330                8335
Glu Asp Thr Val Asp Thr Glu Asp Met Gln Pro Ser Thr His Thr Ala
            8340                8345                8350
Val Thr Asn Val Arg Thr Ser Ile Ser Gly His Glu Ser Gln Ser Ser
        8355                8360                8365
Val Leu Ser Asp Ser Glu Thr Pro Lys Ala Thr Ser Pro Met Gly Thr
    8370                8375                8380
Thr Tyr Thr Met Gly Glu Thr Ser Val Ser Ile Ser Thr Ser Asp Phe
8385                8390                8395                8400
Phe Glu Thr Ser Arg Ile Gln Ile Glu Pro Thr Ser Ser Leu Thr Ser
                8405                8410                8415
Gly Leu Arg Glu Thr Ser Ser Ser Glu Arg Ile Ser Ser Ala Thr Glu
            8420                8425                8430
Gly Ser Thr Val Leu Ser Glu Val Pro Ser Gly Ala Thr Thr Glu Val
        8435                8440                8445
Ser Arg Thr Glu Val Ile Ser Ser Arg Gly Thr Ser Met Ser Gly Pro
    8450                8455                8460
Asp Gln Phe Thr Ile Ser Pro Asp Ile Ser Thr Glu Ala Ile Thr Arg
8465                8470                8475                8480
Leu Ser Thr Ser Pro Ile Met Thr Glu Ser Ala Glu Ser Ala Ile Thr
                8485                8490                8495
Ile Glu Thr Gly Ser Pro Gly Ala Thr Ser Glu Gly Thr Leu Thr Leu
            8500                8505                8510
Asp Thr Ser Thr Thr Thr Phe Trp Ser Gly Thr His Ser Thr Ala Ser

8515                8520                8525
Pro Gly Phe Ser His Ser Glu Met Thr Thr Leu Met Ser Arg Thr Pro
    8530                8535                8540
Gly Asp Val Pro Trp Pro Ser Leu Pro Ser Val Glu Glu Ala Ser Ser
8545                8550                8555                8560
Val Ser Ser Ser Leu Ser Ser Pro Ala Met Thr Ser Thr Ser Phe Phe
                8565                8570                8575
Ser Thr Leu Pro Glu Ser Ile Ser Ser Ser Pro His Pro Val Thr Ala
            8580                8585                8590
Leu Leu Thr Leu Gly Pro Val Lys Thr Thr Asp Met Leu Arg Thr Ser
        8595                8600                8605
Ser Glu Pro Glu Thr Ser Ser Pro Pro Asn Leu Ser Ser Thr Ser Ala
    8610                8615                8620
Glu Ile Leu Ala Thr Ser Glu Val Thr Lys Asp Arg Glu Lys Ile His
8625                8630                8635                8640
Pro Ser Ser Asn Thr Pro Val Val Asn Val Gly Thr Val Ile Tyr Lys
                8645                8650                8655
His Leu Ser Pro Ser Ser Val Leu Ala Asp Leu Val Thr Thr Lys Pro
            8660                8665                8670
Thr Ser Pro Met Ala Thr Thr Ser Thr Leu Gly Asn Thr Ser Val Ser
        8675                8680                8685
Thr Ser Thr Pro Ala Phe Pro Glu Thr Met Met Thr Gln Pro Thr Ser
    8690                8695                8700
Ser Leu Thr Ser Gly Leu Arg Glu Ile Ser Thr Ser Gln Glu Thr Ser
8705                8710                8715                8720
Ser Ala Thr Glu Arg Ser Ala Ser Leu Ser Gly Met Pro Thr Gly Ala
                8725                8730                8735
Thr Thr Lys Val Ser Arg Thr Glu Ala Leu Ser Leu Gly Arg Thr Ser
            8740                8745                8750
Thr Pro Gly Pro Ala Gln Ser Thr Ile Ser Pro Glu Ile Ser Thr Glu
        8755                8760                8765
Thr Ile Thr Arg Ile Ser Thr Pro Leu Thr Thr Thr Gly Ser Ala Glu
    8770                8775                8780
Met Thr Ile Thr Pro Lys Thr Gly His Ser Gly Ala Ser Ser Gln Gly
8785                8790                8795                8800
Thr Phe Thr Leu Asp Thr Ser Ser Arg Ala Ser Trp Pro Gly Thr His
                8805                8810                8815
Ser Ala Ala Thr His Arg Ser Pro His Ser Gly Met Thr Thr Pro Met
            8820                8825                8830
Ser Arg Gly Pro Glu Asp Val Ser Trp Pro Ser Arg Pro Ser Val Glu
        8835                8840                8845
Lys Thr Ser Pro Pro Ser Ser Leu Val Ser Leu Ser Ala Val Thr Ser
    8850                8855                8860
Pro Ser Pro Leu Tyr Ser Thr Pro Ser Glu Ser Ser His Ser Ser Pro
8865                8870                8875                8880
Leu Arg Val Thr Ser Leu Phe Thr Pro Val Met Met Lys Thr Thr Asp
                8885                8890                8895
Met Leu Asp Thr Ser Leu Glu Pro Val Thr Thr Ser Pro Pro Ser Met
            8900                8905                8910
Asn Ile Thr Ser Asp Glu Ser Leu Ala Thr Ser Lys Ala Thr Met Glu
        8915                8920                8925
Thr Glu Ala Ile Gln Leu Ser Glu Asn Thr Ala Val Thr Gln Met Gly
    8930                8935                8940
Thr Ile Ser Ala Arg Gln Glu Phe Tyr Ser Ser Tyr Pro Gly Leu Pro
8945                8950                8955                8960
Glu Pro Ser Lys Val Thr Ser Pro Val Val Thr Ser Ser Thr Ile Lys
                8965                8970                8975
Asp Ile Val Ser Thr Thr Ile Pro Ala Ser Ser Glu Ile Thr Arg Ile
            8980                8985                8990
Glu Met Glu Ser Thr Ser Thr Leu Thr Pro Thr Pro Arg Glu Thr Ser
        8995                9000                9005
Thr Ser Gln Glu Ile His Ser Ala Thr Lys Pro Ser Thr Val Pro Tyr
    9010                9015                9020
Lys Ala Leu Thr Ser Ala Thr Ile Glu Asp Ser Met Thr Gln Val Met
9025                9030                9035                9040
Ser Ser Ser Arg Gly Pro Ser Pro Asp Gln Ser Thr Met Ser Gln Asp
                9045                9050                9055
Ile Ser Thr Glu Val Ile Thr Arg Leu Ser Thr Ser Pro Ile Lys Thr

9060                9065                9070
Glu Ser Thr Glu Met Thr Ile Thr Thr Gln Thr Gly Ser Pro Gly Ala
        9075                9080                9085
Thr Ser Arg Gly Thr Leu Thr Leu Asp Thr Ser Thr Thr Phe Met Ser
    9090                9095                9100
Gly Thr His Ser Thr Ala Ser Gln Gly Phe Ser His Ser Gln Met Thr
9105                9110                9115                9120
Ala Leu Met Ser Arg Thr Pro Gly Asp Val Pro Trp Leu Ser His Pro
                9125                9130                9135
Ser Val Glu Glu Ala Ser Ser Ala Ser Phe Ser Leu Ser Ser Pro Val
            9140                9145                9150
Met Thr Ser Ser Ser Pro Val Ser Ser Thr Leu Pro Asp Ser Ile His
        9155                9160                9165
Ser Ser Ser Leu Pro Val Thr Ser Leu Leu Thr Ser Gly Leu Val Lys
    9170                9175                9180
Thr Thr Glu Leu Leu Gly Thr Ser Ser Glu Pro Glu Thr Ser Ser Pro
9185                9190                9195                9200
Pro Asn Leu Ser Ser Thr Ser Ala Glu Ile Leu Ala Ile Thr Glu Val
                9205                9210                9215
Thr Thr Asp Thr Glu Lys Leu Glu Met Thr Asn Val Val Thr Ser Gly
            9220                9225                9230
Tyr Thr His Glu Ser Pro Ser Ser Val Leu Ala Asp Ser Val Thr Thr
        9235                9240                9245
Lys Ala Thr Ser Ser Met Gly Ile Thr Tyr Pro Thr Gly Asp Thr Asn
    9250                9255                9260
Val Leu Thr Ser Thr Pro Ala Phe Ser Asp Thr Ser Arg Ile Gln Thr
9265                9270                9275                9280
Lys Ser Lys Leu Ser Leu Thr Pro Gly Leu Met Glu Thr Ser Ile Ser
                9285                9290                9295
Glu Glu Thr Ser Ser Ala Thr Glu Lys Ser Thr Val Leu Ser Ser Val
            9300                9305                9310
Pro Thr Gly Ala Thr Thr Glu Val Ser Arg Thr Glu Ala Ile Ser Ser
        9315                9320                9325
Ser Arg Thr Ser Ile Pro Gly Pro Ala Gln Ser Thr Met Ser Ser Asp
    9330                9335                9340
Thr Ser Met Glu Thr Ile Thr Arg Ile Ser Thr Pro Leu Thr Arg Lys
9345                9350                9355                9360
Glu Ser Thr Asp Met Ala Ile Thr Pro Lys Thr Gly Pro Ser Gly Ala
                9365                9370                9375
Thr Ser Gln Gly Thr Phe Thr Leu Asp Ser Ser Ser Thr Ala Ser Trp
            9380                9385                9390
Pro Gly Thr His Ser Ala Thr Thr Gln Arg Phe Pro Gln Ser Val Val
        9395                9400                9405
Thr Thr Pro Met Ser Arg Gly Pro Glu Asp Val Ser Trp Pro Ser Pro
    9410                9415                9420
Leu Ser Val Glu Lys Asn Ser Pro Pro Ser Ser Leu Val Ser Ser Ser
9425                9430                9435                9440
Ser Val Thr Ser Pro Ser Pro Leu Tyr Ser Thr Pro Ser Gly Ser Ser
                9445                9450                9455
His Ser Ser Pro Val Pro Val Thr Ser Leu Phe Thr Ser Ile Met Met
            9460                9465                9470
Lys Ala Thr Asp Met Leu Asp Ala Ser Leu Glu Pro Glu Thr Thr Ser
        9475                9480                9485
Ala Pro Asn Met Asn Ile Thr Ser Asp Glu Ser Leu Ala Ala Ser Lys
    9490                9495                9500
Ala Thr Thr Glu Thr Glu Ala Ile His Val Phe Glu Asn Thr Ala Ala
9505                9510                9515                9520
Ser His Val Glu Thr Thr Ser Ala Thr Glu Glu Leu Tyr Ser Ser Ser
                9525                9530                9535
Pro Gly Phe Ser Glu Pro Thr Lys Val Ile Ser Pro Val Val Thr Ser
            9540                9545                9550
Ser Ser Ile Arg Asp Asn Met Val Ser Thr Thr Met Pro Gly Ser Ser
        9555                9560                9565
Gly Ile Thr Arg Ile Glu Ile Glu Ser Met Ser Ser Leu Thr Pro Gly
    9570                9575                9580
Leu Arg Glu Thr Arg Thr Ser Gln Asp Ile Thr Ser Ser Thr Glu Thr
9585                9590                9595                9600
Ser Thr Val Leu Tyr Lys Met Pro Ser Gly Ala Thr Pro Glu Val Ser

9605                9610                9615
Arg Thr Glu Val Met Pro Ser Ser Arg Thr Ser Ile Pro Gly Pro Ala
        9620                9625                9630
Gln Ser Thr Met Ser Leu Asp Ile Ser Asp Glu Val Val Thr Arg Leu
    9635                9640                9645
Ser Thr Ser Pro Ile Met Thr Glu Ser Ala Glu Ile Thr Ile Thr Thr
9650                9655                9660
Gln Thr Gly Tyr Ser Leu Ala Thr Ser Gln Val Thr Leu Pro Leu Gly
9665                9670                9675                9680
Thr Ser Met Thr Phe Leu Ser Gly Thr His Ser Thr Met Ser Gln Gly
                9685                9690                9695
Leu Ser His Ser Glu Met Thr Asn Leu Met Ser Arg Gly Pro Glu Ser
            9700                9705                9710
Leu Ser Trp Thr Ser Pro Arg Phe Val Glu Thr Thr Arg Ser Ser Ser
        9715                9720                9725
Ser Leu Thr Ser Leu Pro Leu Thr Thr Ser Leu Ser Pro Val Ser Ser
    9730                9735                9740
Thr Leu Leu Asp Ser Ser Pro Ser Ser Pro Leu Pro Val Thr Ser Leu
9745                9750                9755                9760
Ile Leu Pro Gly Leu Val Lys Thr Thr Glu Val Leu Asp Thr Ser Ser
                9765                9770                9775
Glu Pro Lys Thr Ser Ser Ser Pro Asn Leu Ser Ser Thr Ser Val Glu
            9780                9785                9790
Ile Pro Ala Thr Ser Glu Ile Met Thr Asp Thr Glu Lys Ile His Pro
        9795                9800                9805
Ser Ser Asn Thr Ala Val Ala Lys Val Arg Thr Ser Ser Ser Val His
    9810                9815                9820
Glu Ser His Ser Ser Val Leu Ala Asp Ser Glu Thr Thr Ile Thr Ile
9825                9830                9835                9840
Pro Ser Met Gly Ile Thr Ser Ala Val Asp Asp Thr Thr Val Phe Thr
                9845                9850                9855
Ser Asn Pro Ala Phe Ser Glu Thr Arg Arg Ile Pro Thr Glu Pro Thr
            9860                9865                9870
Phe Ser Leu Thr Pro Gly Phe Arg Glu Thr Ser Thr Ser Glu Glu Thr
        9875                9880                9885
Thr Ser Ile Thr Glu Thr Ser Ala Val Leu Tyr Gly Val Pro Thr Ser
    9890                9895                9900
Ala Thr Thr Glu Val Ser Met Thr Glu Ile Met Ser Ser Asn Arg Ile
9905                9910                9915                9920
His Ile Pro Asp Ser Asp Gln Ser Thr Met Ser Pro Asp Ile Ile Thr
                9925                9930                9935
Glu Val Ile Thr Arg Leu Ser Ser Ser Ser Met Met Ser Glu Ser Thr
            9940                9945                9950
Gln Met Thr Ile Thr Thr Gln Lys Ser Ser Pro Gly Ala Thr Ala Gln
        9955                9960                9965
Ser Thr Leu Thr Leu Ala Thr Thr Thr Ala Pro Leu Ala Arg Thr His
    9970                9975                9980
Ser Thr Val Pro Pro Arg Phe Leu His Ser Glu Met Thr Thr Leu Met
9985                9990                9995                10000
Ser Arg Ser Pro Glu Asn Pro Ser Trp Lys Ser Ser Leu Phe Val Glu
                10005               10010               10015
Lys Thr Ser Ser Ser Ser Ser Leu Leu Ser Leu Pro Val Thr Thr Ser
            10020               10025               10030
Pro Ser Val Ser Ser Thr Leu Pro Gln Ser Ile Pro Ser Ser Ser Phe
        10035               10040               10045
Ser Val Thr Ser Leu Leu Thr Pro Gly Met Val Lys Thr Thr Asp Thr
    10050               10055               10060
Ser Thr Glu Pro Gly Thr Ser Leu Ser Pro Asn Leu Ser Gly Thr Ser
10065               10070               10075               10080
Val Glu Ile Leu Ala Ala Ser Glu Val Thr Thr Asp Thr Glu Lys Ile
                10085               10090               10095
His Pro Ser Ser Ser Met Ala Val Thr Asn Val Gly Thr Thr Ser Ser
            10100               10105               10110
Gly His Glu Leu Tyr Ser Ser Val Ser Ile His Ser Glu Pro Ser Lys
        10115               10120               10125
Ala Thr Tyr Pro Val Gly Thr Pro Ser Ser Met Ala Glu Thr Ser Ile
    10130               10135               10140
Ser Thr Ser Met Pro Ala Asn Phe Glu Thr Thr Gly Phe Glu Ala Glu

10145               10150               10155               10160
Pro Phe Ser His Leu Thr Ser Gly Phe Arg Lys Thr Asn Met Ser Leu
                10165               10170               10175
Asp Thr Ser Ser Val Thr Pro Thr Asn Thr Pro Ser Ser Pro Gly Ser
            10180               10185               10190
Thr His Leu Leu Gln Ser Ser Lys Thr Asp Phe Thr Ser Ser Ala Lys
        10195               10200               10205
Thr Ser Ser Pro Asp Trp Pro Pro Ala Ser Gln Tyr Thr Glu Ile Pro
    10210               10215               10220
Val Asp Ile Ile Thr Pro Phe Asn Ala Ser Pro Ser Ile Thr Glu Ser
10225               10230               10235               10240
Thr Gly Ile Thr Ser Phe Pro Glu Ser Arg Phe Thr Met Ser Val Thr
                10245               10250               10255
Glu Ser Thr His His Leu Ser Thr Asp Leu Leu Pro Ser Ala Glu Thr
            10260               10265               10270
Ile Ser Thr Gly Thr Val Met Pro Ser Leu Ser Glu Ala Met Thr Ser
        10275               10280               10285
Phe Ala Thr Thr Gly Val Pro Arg Ala Ile Ser Gly Ser Gly Ser Pro
    10290               10295               10300
Phe Ser Arg Thr Glu Ser Gly Pro Gly Asp Ala Thr Leu Ser Thr Ile
10305               10310               10315               10320
Ala Glu Ser Leu Pro Ser Ser Thr Pro Val Pro Phe Ser Ser Ser Thr
                10325               10330               10335
Phe Thr Thr Thr Asp Ser Ser Thr Ile Pro Ala Leu His Glu Ile Thr
            10340               10345               10350
Ser Ser Ser Ala Thr Pro Tyr Arg Val Asp Thr Ser Leu Gly Thr Glu
        10355               10360               10365
Ser Ser Thr Thr Glu Gly Arg Leu Val Met Val Ser Thr Leu Asp Thr
    10370               10375               10380
Ser Ser Gln Pro Gly Arg Thr Ser Ser Ser Pro Ile Leu Asp Thr Arg
10385               10390               10395               10400
Met Thr Glu Ser Val Glu Leu Gly Thr Val Thr Ser Ala Tyr Gln Val
                10405               10410               10415
Pro Ser Leu Ser Thr Arg Leu Thr Arg Thr Asp Gly Ile Met Glu His
            10420               10425               10430
Ile Thr Lys Ile Pro Asn Glu Ala Ala His Arg Gly Thr Ile Arg Pro
        10435               10440               10445
Val Lys Gly Pro Gln Thr Ser Thr Ser Pro Ala Ser Pro Lys Gly Leu
    10450               10455               10460
His Thr Gly Gly Thr Lys Arg Met Glu Thr Thr Thr Thr Ala Leu Lys
10465               10470               10475               10480
Thr Thr Thr Thr Ala Leu Lys Thr Thr Ser Arg Ala Thr Leu Thr Thr
                10485               10490               10495
Ser Val Tyr Thr Pro Thr Leu Gly Thr Leu Thr Pro Leu Asn Ala Ser
            10500               10505               10510
Met Gln Met Ala Ser Thr Ile Pro Thr Glu Met Met Ile Thr Thr Pro
        10515               10520               10525
Tyr Val Phe Pro Asp Val Pro Glu Thr Thr Ser Ser Leu Ala Thr Ser
    10530               10535               10540
Leu Gly Ala Glu Thr Ser Thr Ala Leu Pro Arg Thr Thr Pro Ser Val
10545               10550               10555               10560
Phe Asn Arg Glu Ser Glu Thr Thr Ala Ser Leu Val Ser Arg Ser Gly
                10565               10570               10575
Ala Glu Arg Ser Pro Val Ile Gln Thr Leu Asp Val Ser Ser Ser Glu
            10580               10585               10590
Pro Asp Thr Thr Ala Ser Trp Val Ile His Pro Ala Glu Thr Ile Pro
        10595               10600               10605
Thr Val Ser Lys Thr Thr Pro Asn Phe Phe His Ser Glu Leu Asp Thr
    10610               10615               10620
Val Ser Ser Thr Ala Thr Ser His Gly Ala Asp Val Ser Ser Ala Ile
10625               10630               10635               10640
Pro Thr Asn Ile Ser Pro Ser Glu Leu Asp Ala Leu Thr Pro Leu Val
                10645               10650               10655
Thr Ile Ser Gly Thr Asp Thr Ser Thr Thr Phe Pro Thr Leu Thr Lys
            10660               10665               10670
Ser Pro His Glu Thr Glu Thr Arg Thr Thr Trp Leu Thr His Pro Ala
        10675               10680               10685
Glu Thr Ser Ser Thr Ile Pro Arg Thr Ile Pro Asn Phe Ser His His

10690               10695               10700
Glu Ser Asp Ala Thr Pro Ser Ile Ala Thr Ser Pro Gly Ala Glu Thr
10705               10710               10715               10720
Ser Ser Ala Ile Pro Ile Met Thr Val Ser Pro Gly Ala Glu Asp Leu
                10725               10730               10735
Val Thr Ser Gln Val Thr Ser Ser Gly Thr Asp Arg Asn Met Thr Ile
            10740               10745               10750
Pro Thr Leu Thr Leu Ser Pro Gly Glu Pro Lys Thr Ile Ala Ser Leu
        10755               10760               10765
Val Thr His Pro Glu Ala Gln Thr Ser Ser Ala Ile Pro Thr Ser Thr
    10770               10775               10780
Ile Ser Pro Ala Val Ser Arg Leu Val Thr Ser Met Val Thr Ser Leu
10785               10790               10795               10800
Ala Ala Lys Thr Ser Thr Thr Asn Arg Ala Leu Thr Asn Ser Pro Gly
                10805               10810               10815
Glu Pro Ala Thr Thr Val Ser Leu Val Thr His Pro Ala Gln Thr Ser
            10820               10825               10830
Pro Thr Val Pro Trp Thr Thr Ser Ile Phe Phe His Ser Lys Ser Asp
        10835               10840               10845
Thr Thr Pro Ser Met Thr Thr Ser His Gly Ala Glu Ser Ser Ser Ala
    10850               10855               10860
Val Pro Thr Pro Thr Val Ser Thr Glu Val Pro Gly Val Val Thr Pro
10865               10870               10875               10880
Leu Val Thr Ser Ser Arg Ala Val Ile Ser Thr Thr Ile Pro Ile Leu
                10885               10890               10895
Thr Leu Ser Pro Gly Glu Pro Glu Thr Thr Pro Ser Met Ala Thr Ser
            10900               10905               10910
His Gly Glu Glu Ala Ser Ser Ala Ile Pro Thr Pro Thr Val Ser Pro
        10915               10920               10925
Gly Val Pro Gly Val Val Thr Ser Leu Val Thr Ser Ser Arg Ala Val
    10930               10935               10940
Thr Ser Thr Thr Ile Pro Ile Leu Thr Phe Ser Leu Gly Glu Pro Glu
10945               10950               10955               10960
Thr Thr Pro Ser Met Ala Thr Ser His Gly Thr Glu Ala Gly Ser Ala
                10965               10970               10975
Val Pro Thr Val Leu Pro Glu Val Pro Gly Met Val Thr Ser Leu Val
            10980               10985               10990
Ala Ser Ser Arg Ala Val Thr Ser Thr Thr Leu Pro Thr Leu Thr Leu
        10995               11000               11005
Ser Pro Gly Glu Pro Glu Thr Thr Pro Ser Met Ala Thr Ser His Gly
    11010               11015               11020
Ala Glu Ala Ser Ser Thr Val Pro Thr Val Ser Pro Glu Val Pro Gly
11025               11030               11035               11040
Val Val Thr Ser Leu Val Thr Ser Ser Ser Gly Val Asn Ser Thr Ser
                11045               11050               11055
Ile Pro Thr Leu Ile Leu Ser Pro Gly Glu Leu Glu Thr Thr Pro Ser
            11060               11065               11070
Met Ala Thr Ser His Gly Ala Glu Ala Ser Ser Ala Val Pro Thr Pro
        11075               11080               11085
Thr Val Ser Pro Gly Val Ser Gly Val Val Thr Pro Leu Val Thr Ser
    11090               11095               11100
Ser Arg Ala Val Thr Ser Thr Thr Ile Pro Ile Leu Thr Leu Ser Ser
11105               11110               11115               11120
Ser Glu Pro Glu Thr Thr Pro Ser Met Ala Thr Ser His Gly Val Glu
                11125               11130               11135
Ala Ser Ser Ala Val Leu Thr Val Ser Pro Glu Val Pro Gly Met Val
            11140               11145               11150
Thr Ser Leu Val Thr Ser Ser Arg Ala Val Thr Ser Thr Thr Ile Pro
        11155               11160               11165
Thr Leu Thr Ile Ser Ser Asp Glu Pro Glu Thr Thr Thr Ser Leu Val
    11170               11175               11180
Thr His Ser Glu Ala Lys Met Ile Ser Ala Ile Pro Thr Leu Ala Val
11185               11190               11195               11200
Ser Pro Thr Val Gln Gly Leu Val Thr Ser Leu Val Thr Ser Ser Gly
                11205               11210               11215
Ser Glu Thr Ser Ala Phe Ser Asn Leu Thr Val Ala Ser Ser Gln Pro
            11220               11225               11230
Glu Thr Ile Asp Ser Trp Val Ala His Pro Gly Thr Glu Ala Ser Ser

11235                   11240                   11245
Val Val Pro Thr Leu Thr Val Ser Thr Gly Glu Pro Phe Thr Asn Ile
    11250                   11255                   11260
Ser Leu Val Thr His Pro Ala Glu Ser Ser Ser Thr Leu Pro Arg Thr
11265                   11270                   11275                   11280
Thr Ser Arg Phe Ser His Ser Glu Leu Asp Thr Met Pro Ser Thr Val
                11285                   11290                   11295
Thr Ser Pro Glu Ala Glu Ser Ser Ser Ala Ile Ser Thr Thr Ile Ser
            11300                   11305                   11310
Pro Gly Ile Pro Gly Val Leu Thr Ser Leu Val Thr Ser Ser Gly Arg
        11315                   11320                   11325
Asp Ile Ser Ala Thr Phe Pro Thr Val Pro Glu Ser Pro His Glu Ser
    11330                   11335                   11340
Glu Ala Thr Ala Ser Trp Val Thr His Pro Ala Val Thr Ser Thr Thr
11345                   11350                   11355                   11360
Val Pro Arg Thr Thr Pro Asn Tyr Ser His Ser Glu Pro Asp Thr Thr
                11365                   11370                   11375
Pro Ser Ile Ala Thr Ser Pro Gly Ala Glu Ala Thr Ser Asp Phe Pro
            11380                   11385                   11390
Thr Ile Thr Val Ser Pro Asp Val Pro Asp Met Val Thr Ser Gln Val
        11395                   11400                   11405
Thr Ser Ser Gly Thr Asp Thr Ser Ile Thr Ile Pro Thr Leu Thr Leu
    11410                   11415                   11420
Ser Ser Gly Glu Pro Glu Thr Thr Thr Ser Phe Ile Thr Tyr Ser Glu
11425                   11430                   11435                   11440
Thr His Thr Ser Ser Ala Ile Pro Thr Leu Pro Val Ser Pro Gly Ala
                11445                   11450                   11455
Ser Lys Met Leu Thr Ser Leu Val Ile Ser Ser Gly Thr Asp Ser Thr
            11460                   11465                   11470
Thr Thr Phe Pro Thr Leu Thr Glu Thr Pro Tyr Glu Pro Glu Thr Thr
        11475                   11480                   11485
Ala Ile Gln Leu Ile His Pro Ala Glu Thr Asn Thr Met Val Pro Arg
    11490                   11495                   11500
Thr Thr Pro Lys Phe Ser His Ser Lys Ser Asp Thr Thr Leu Pro Val
11505                   11510                   11515                   11520
Ala Ile Thr Ser Pro Gly Pro Glu Ala Ser Ser Ala Val Ser Thr Thr
                11525                   11530                   11535
Thr Ile Ser Pro Asp Met Ser Asp Leu Val Thr Ser Leu Val Pro Ser
            11540                   11545                   11550
Ser Gly Thr Asp Thr Ser Thr Thr Phe Pro Thr Leu Ser Glu Thr Pro
        11555                   11560                   11565
Tyr Glu Pro Glu Thr Thr Ala Thr Trp Leu Thr His Pro Ala Glu Thr
    11570                   11575                   11580
Ser Thr Thr Val Ser Gly Thr Ile Pro Asn Phe Ser His Arg Gly Ser
11585                   11590                   11595                   11600
Asp Thr Ala Pro Ser Met Val Thr Ser Pro Gly Val Asp Thr Arg Ser
                11605                   11610                   11615
Gly Val Pro Thr Thr Thr Ile Pro Pro Ser Ile Pro Gly Val Val Thr
            11620                   11625                   11630
Ser Gln Val Thr Ser Ser Ala Thr Asp Thr Ser Thr Ala Ile Pro Thr
        11635                   11640                   11645
Leu Thr Pro Ser Pro Gly Glu Pro Glu Thr Thr Ala Ser Ser Ala Thr
    11650                   11655                   11660
His Pro Gly Thr Gln Thr Gly Phe Thr Val Pro Ile Arg Thr Val Pro
11665                   11670                   11675                   11680
Ser Ser Glu Pro Asp Thr Met Ala Ser Trp Val Thr His Pro Pro Gln
                11685                   11690                   11695
Thr Ser Thr Pro Val Ser Arg Thr Thr Ser Ser Phe Ser His Ser Ser
            11700                   11705                   11710
Pro Asp Ala Thr Pro Val Met Ala Thr Ser Pro Arg Thr Glu Ala Ser
        11715                   11720                   11725
Ser Ala Val Leu Thr Thr Ile Ser Pro Gly Ala Pro Glu Met Val Thr
    11730                   11735                   11740
Ser Gln Ile Thr Ser Ser Gly Ala Ala Thr Ser Thr Thr Val Pro Thr
11745                   11750                   11755                   11760
Leu Thr His Ser Pro Gly Met Pro Glu Thr Thr Ala Leu Leu Ser Thr
                11765                   11770                   11775
His Pro Arg Thr Glu Thr Ser Lys Thr Phe Pro Ala Ser Thr Val Phe

11780                11785                11790
Pro Gln Val Ser Glu Thr Thr Ala Ser Leu Thr Ile Arg Pro Gly Ala
        11795                11800                11805
Glu Thr Ser Thr Ala Leu Pro Thr Gln Thr Thr Ser Ser Leu Phe Thr
    11810                11815                11820
Leu Leu Val Thr Gly Thr Ser Arg Val Asp Leu Ser Pro Thr Ala Ser
11825                11830                11835                11840
Pro Gly Val Ser Ala Lys Thr Ala Pro Leu Ser Thr His Pro Gly Thr
                11845                11850                11855
Glu Thr Ser Thr Met Ile Pro Thr Ser Thr Leu Ser Leu Gly Leu Leu
            11860                11865                11870
Glu Thr Thr Gly Leu Leu Ala Thr Ser Ser Ser Ala Glu Thr Ser Thr
        11875                11880                11885
Ser Thr Leu Thr Leu Thr Val Ser Pro Ala Val Ser Gly Leu Ser Ser
    11890                11895                11900
Ala Ser Ile Thr Thr Asp Lys Pro Gln Thr Val Thr Ser Trp Asn Thr
11905                11910                11915                11920
Glu Thr Ser Pro Ser Val Thr Ser Val Gly Pro Pro Glu Phe Ser Arg
                11925                11930                11935
Thr Val Thr Gly Thr Thr Met Thr Leu Ile Pro Ser Glu Met Pro Thr
            11940                11945                11950
Pro Pro Lys Thr Ser His Gly Glu Gly Val Ser Pro Thr Thr Ile Leu
        11955                11960                11965
Arg Thr Thr Met Val Glu Ala Thr Asn Leu Ala Thr Thr Gly Ser Ser
    11970                11975                11980
Pro Thr Val Ala Lys Thr Thr Thr Thr Phe Asn Thr Leu Ala Gly Ser
11985                11990                11995                12000
Leu Phe Thr Pro Leu Thr Thr Pro Gly Met Ser Thr Leu Ala Ser Glu
                12005                12010                12015
Ser Val Thr Ser Arg Thr Ser Tyr Asn His Arg Ser Trp Ile Ser Thr
            12020                12025                12030
Thr Ser Ser Tyr Asn Arg Arg Tyr Trp Thr Pro Ala Thr Ser Thr Pro
        12035                12040                12045
Val Thr Ser Thr Phe Ser Pro Gly Ile Ser Thr Ser Ser Ile Pro Ser
    12050                12055                12060
Ser Thr Ala Ala Thr Val Pro Phe Met Val Pro Phe Thr Leu Asn Phe
12065                12070                12075                12080
Thr Ile Thr Asn Leu Gln Tyr Glu Glu Asp Met Arg His Pro Gly Ser
                12085                12090                12095
Arg Lys Phe Asn Ala Thr Glu Arg Glu Leu Gln Gly Leu Leu Lys Pro
            12100                12105                12110
Leu Phe Arg Asn Ser Ser Leu Glu Tyr Leu Tyr Ser Gly Cys Arg Leu
        12115                12120                12125
Ala Ser Leu Arg Pro Glu Lys Asp Ser Ser Ala Thr Ala Val Asp Ala
    12130                12135                12140
Ile Cys Thr His Arg Pro Asp Pro Glu Asp Leu Gly Leu Asp Arg Glu
12145                12150                12155                12160
Arg Leu Tyr Trp Glu Leu Ser Asn Leu Thr Asn Gly Ile Gln Glu Leu
                12165                12170                12175
Gly Pro Tyr Thr Leu Asp Arg Asn Ser Leu Tyr Val Asn Gly Phe Thr
            12180                12185                12190
His Arg Ser Ser Met Pro Thr Thr Ser Thr Pro Gly Thr Ser Thr Val
        12195                12200                12205
Asp Val Gly Thr Ser Gly Thr Pro Ser Ser Ser Pro Ser Pro Thr Thr
    12210                12215                12220
Ala Gly Pro Leu Leu Met Pro Phe Thr Leu Asn Phe Thr Ile Thr Asn
12225                12230                12235                12240
Leu Gln Tyr Glu Glu Asp Met Arg Arg Thr Gly Ser Arg Lys Phe Asn
                12245                12250                12255
Thr Met Glu Ser Val Leu Gln Gly Leu Leu Lys Pro Leu Phe Lys Asn
            12260                12265                12270
Thr Ser Val Gly Pro Leu Tyr Ser Gly Cys Arg Leu Thr Leu Leu Arg
        12275                12280                12285
Pro Glu Lys Asp Gly Ala Ala Thr Gly Val Asp Ala Ile Cys Thr His
    12290                12295                12300
Arg Leu Asp Pro Lys Ser Pro Gly Leu Asn Arg Glu Gln Leu Tyr Trp
12305                12310                12315                12320
Glu Leu Ser Lys Leu Thr Asn Asp Ile Glu Glu Leu Gly Pro Tyr Thr

12325                   12330                   12335
Leu Asp Arg Asn Ser Leu Tyr Val Asn Gly Phe Thr His Gln Ser Ser
        12340                   12345                   12350
Val Ser Thr Thr Ser Thr Pro Gly Thr Ser Thr Val Asp Leu Arg Thr
    12355                   12360                   12365
Ser Gly Thr Pro Ser Ser Leu Ser Ser Pro Thr Ile Met Ala Ala Gly
12370                   12375                   12380
Pro Leu Leu Val Pro Phe Thr Leu Asn Phe Thr Ile Thr Asn Leu Gln
12385                   12390                   12395                   12400
Tyr Gly Glu Asp Met Gly His Pro Gly Ser Arg Lys Phe Asn Thr Thr
                12405                   12410                   12415
Glu Arg Val Leu Gln Gly Leu Leu Gly Pro Ile Phe Lys Asn Thr Ser
            12420                   12425                   12430
Val Gly Pro Leu Tyr Ser Gly Cys Arg Leu Thr Ser Leu Arg Ser Glu
        12435                   12440                   12445
Lys Asp Gly Ala Ala Thr Gly Val Asp Ala Ile Cys Ile His His Leu
    12450                   12455                   12460
Asp Pro Lys Ser Pro Gly Leu Asn Arg Glu Arg Leu Tyr Trp Glu Leu
12465                   12470                   12475                   12480
Ser Gln Leu Thr Asn Gly Ile Lys Glu Leu Gly Pro Tyr Thr Leu Asp
                12485                   12490                   12495
Arg Asn Ser Leu Tyr Val Asn Gly Phe Thr His Arg Thr Ser Val Pro
            12500                   12505                   12510
Thr Ser Ser Thr Pro Gly Thr Ser Thr Val Asp Leu Gly Thr Ser Gly
        12515                   12520                   12525
Thr Pro Phe Ser Leu Pro Ser Pro Ala Thr Ala Gly Pro Leu Leu Val
    12530                   12535                   12540
Leu Phe Thr Leu Asn Phe Thr Ile Thr Asn Leu Lys Tyr Glu Glu Asp
12545                   12550                   12555                   12560
Met His Arg Pro Gly Ser Arg Lys Phe Asn Thr Thr Glu Arg Val Leu
                12565                   12570                   12575
Gln Thr Leu Leu Gly Pro Met Phe Lys Asn Thr Ser Val Gly Leu Leu
            12580                   12585                   12590
Tyr Ser Gly Cys Arg Leu Thr Leu Leu Arg Ser Glu Lys Asp Gly Ala
        12595                   12600                   12605
Ala Thr Gly Val Asp Ala Ile Cys Thr His Arg Leu Asp Pro Lys Ser
    12610                   12615                   12620
Pro Gly Val Asp Arg Glu Gln Leu Tyr Trp Glu Leu Ser Gln Leu Thr
12625                   12630                   12635                   12640
Asn Gly Ile Lys Glu Leu Gly Pro Tyr Thr Leu Asp Arg Asn Ser Leu
                12645                   12650                   12655
Tyr Val Asn Gly Phe Thr His Trp Ile Pro Val Pro Thr Ser Ser Thr
            12660                   12665                   12670
Pro Gly Thr Ser Thr Val Asp Leu Gly Ser Gly Thr Pro Ser Ser Leu
        12675                   12680                   12685
Pro Ser Pro Thr Thr Ala Gly Pro Leu Leu Val Pro Phe Thr Leu Asn
    12690                   12695                   12700
Phe Thr Ile Thr Asn Leu Lys Tyr Glu Glu Asp Met His Cys Pro Gly
12705                   12710                   12715                   12720
Ser Arg Lys Phe Asn Thr Thr Glu Arg Val Leu Gln Ser Leu Leu Gly
                12725                   12730                   12735
Pro Met Phe Lys Asn Thr Ser Val Gly Pro Leu Tyr Ser Gly Cys Arg
            12740                   12745                   12750
Leu Thr Leu Leu Arg Ser Glu Lys Asp Gly Ala Ala Thr Gly Val Asp
        12755                   12760                   12765
Ala Ile Cys Thr His Arg Leu Asp Pro Lys Ser Pro Gly Val Asp Arg
    12770                   12775                   12780
Glu Gln Leu Tyr Trp Glu Leu Ser Gln Leu Thr Asn Gly Ile Lys Glu
12785                   12790                   12795                   12800
Leu Gly Pro Tyr Thr Leu Asp Arg Asn Ser Leu Tyr Val Asn Gly Phe
                12805                   12810                   12815
Thr His Gln Thr Ser Ala Pro Asn Thr Ser Thr Pro Gly Thr Ser Thr
            12820                   12825                   12830
Val Asp Leu Gly Thr Ser Gly Thr Pro Ser Ser Leu Pro Ser Pro Thr
        12835                   12840                   12845
Ser Ala Gly Pro Leu Leu Val Pro Phe Thr Leu Asn Phe Thr Ile Thr
    12850                   12855                   12860
Asn Leu Gln Tyr Glu Glu Asp Met His His Pro Gly Ser Arg Lys Phe

12865               12870               12875               12880
Asn Thr Thr Glu Arg Val Leu Gln Gly Leu Leu Gly Pro Met Phe Lys
                12885               12890               12895
Asn Thr Ser Val Gly Leu Leu Tyr Ser Gly Cys Arg Leu Thr Leu Leu
            12900               12905               12910
Arg Pro Glu Lys Asn Gly Ala Ala Thr Gly Met Asp Ala Ile Cys Ser
        12915               12920               12925
His Arg Leu Asp Pro Lys Ser Pro Gly Leu Asn Arg Glu Gln Leu Tyr
    12930               12935               12940
Trp Glu Leu Ser Gln Leu Thr His Gly Ile Lys Glu Leu Gly Pro Tyr
12945               12950               12955               12960
Thr Leu Asp Arg Asn Ser Leu Tyr Val Asn Gly Phe Thr His Arg Ser
                12965               12970               12975
Ser Val Ala Pro Thr Ser Thr Pro Gly Thr Ser Thr Val Asp Leu Gly
            12980               12985               12990
Thr Ser Gly Thr Pro Ser Ser Leu Pro Ser Pro Thr Thr Ala Val Pro
        12995               13000               13005
Leu Leu Val Pro Phe Thr Leu Asn Phe Thr Ile Thr Asn Leu Gln Tyr
    13010               13015               13020
Gly Glu Asp Met Arg His Pro Gly Ser Arg Lys Phe Asn Thr Thr Glu
13025               13030               13035               13040
Arg Val Leu Gln Gly Leu Leu Gly Pro Leu Phe Lys Asn Ser Ser Val
                13045               13050               13055
Gly Pro Leu Tyr Ser Gly Cys Arg Leu Ile Ser Leu Arg Ser Glu Lys
            13060               13065               13070
Asp Gly Ala Ala Thr Gly Val Asp Ala Ile Cys Thr His His Leu Asn
        13075               13080               13085
Pro Gln Ser Pro Gly Leu Asp Arg Glu Gln Leu Tyr Trp Gln Leu Ser
    13090               13095               13100
Gln Met Thr Asn Gly Ile Lys Glu Leu Gly Pro Tyr Thr Leu Asp Arg
13105               13110               13115               13120
Asn Ser Leu Tyr Val Asn Gly Phe Thr His Arg Ser Ser Gly Leu Thr
                13125               13130               13135
Thr Ser Thr Pro Trp Thr Ser Thr Val Asp Leu Gly Thr Ser Gly Thr
            13140               13145               13150
Pro Ser Pro Val Pro Ser Pro Thr Thr Thr Gly Pro Leu Leu Val Pro
        13155               13160               13165
Phe Thr Leu Asn Phe Thr Ile Thr Asn Leu Gln Tyr Glu Glu Asn Met
    13170               13175               13180
Gly His Pro Gly Ser Arg Lys Phe Asn Ile Thr Glu Ser Val Leu Gln
13185               13190               13195               13200
Gly Leu Leu Lys Pro Leu Phe Lys Ser Thr Ser Val Gly Pro Leu Tyr
                13205               13210               13215
Ser Gly Cys Arg Leu Thr Leu Leu Arg Pro Glu Lys Asp Gly Val Ala
            13220               13225               13230
Thr Arg Val Asp Ala Ile Cys Thr His Arg Pro Asp Pro Lys Ile Pro
        13235               13240               13245
Gly Leu Asp Arg Gln Gln Leu Tyr Trp Glu Leu Ser Gln Leu Thr His
    13250               13255               13260
Ser Ile Thr Glu Leu Gly Pro Tyr Thr Leu Asp Arg Asp Ser Leu Tyr
13265               13270               13275               13280
Val Asn Gly Phe Thr Gln Arg Ser Ser Val Pro Thr Thr Ser Thr Pro
                13285               13290               13295
Gly Thr Phe Thr Val Gln Pro Glu Thr Ser Glu Thr Pro Ser Ser Leu
            13300               13305               13310
Pro Gly Pro Thr Ala Thr Gly Pro Val Leu Leu Pro Phe Thr Leu Asn
        13315               13320               13325
Phe Thr Ile Thr Asn Leu Gln Tyr Glu Glu Asp Met Arg Arg Pro Gly
    13330               13335               13340
Ser Arg Lys Phe Asn Thr Thr Glu Arg Val Leu Gln Gly Leu Leu Met
13345               13350               13355               13360
Pro Leu Phe Lys Asn Thr Ser Val Ser Ser Leu Tyr Ser Gly Cys Arg
                13365               13370               13375
Leu Thr Leu Leu Arg Pro Glu Lys Asp Gly Ala Ala Thr Arg Val Asp
            13380               13385               13390
Ala Val Cys Thr His Arg Pro Asp Pro Lys Ser Pro Gly Leu Asp Arg
        13395               13400               13405
Glu Arg Leu Tyr Trp Lys Leu Ser Gln Leu Thr His Gly Ile Thr Glu

13410               13415               13420
Leu Gly Pro Tyr Thr Leu Asp Arg His Ser Leu Tyr Val Asn Gly Phe
13425               13430               13435               13440
Thr His Gln Ser Ser Met Thr Thr Thr Arg Thr Pro Asp Thr Ser Thr
                13445               13450               13455
Met His Leu Ala Thr Ser Arg Thr Pro Ala Ser Leu Ser Gly Pro Met
            13460               13465               13470
Thr Ala Ser Pro Leu Leu Val Leu Phe Thr Ile Asn Phe Thr Ile Thr
        13475               13480               13485
Asn Leu Arg Tyr Glu Glu Asn Met His His Pro Gly Ser Arg Lys Phe
    13490               13495               13500
Asn Thr Thr Glu Arg Val Leu Gln Gly Leu Leu Arg Pro Val Phe Lys
13505               13510               13515               13520
Asn Thr Ser Val Gly Pro Leu Tyr Ser Gly Cys Arg Leu Thr Leu Leu
                13525               13530               13535
Arg Pro Lys Lys Asp Gly Ala Ala Thr Lys Val Asp Ala Ile Cys Thr
            13540               13545               13550
Tyr Arg Pro Asp Pro Lys Ser Pro Gly Leu Asp Arg Glu Gln Leu Tyr
        13555               13560               13565
Trp Glu Leu Ser Gln Leu Thr His Ser Ile Thr Glu Leu Gly Pro Tyr
    13570               13575               13580
Thr Leu Asp Arg Asp Ser Leu Tyr Val Asn Gly Phe Thr Gln Arg Ser
13585               13590               13595               13600
Ser Val Pro Thr Thr Ser Ile Pro Gly Thr Pro Thr Val Asp Leu Gly
                13605               13610               13615
Thr Ser Gly Thr Pro Val Ser Lys Pro Gly Pro Ser Ala Ala Ser Pro
            13620               13625               13630
Leu Leu Val Leu Phe Thr Leu Asn Phe Thr Ile Thr Asn Leu Arg Tyr
        13635               13640               13645
Glu Glu Asn Met Gln His Pro Gly Ser Arg Lys Phe Asn Thr Thr Glu
    13650               13655               13660
Arg Val Leu Gln Gly Leu Leu Arg Ser Leu Phe Lys Ser Thr Ser Val
13665               13670               13675               13680
Gly Pro Leu Tyr Ser Gly Cys Arg Leu Thr Leu Leu Arg Pro Glu Lys
                13685               13690               13695
Asp Gly Thr Ala Thr Gly Val Asp Ala Ile Cys Thr His His Pro Asp
            13700               13705               13710
Pro Lys Ser Pro Arg Leu Asp Arg Glu Gln Leu Tyr Trp Glu Leu Ser
        13715               13720               13725
Gln Leu Thr His Asn Ile Thr Glu Leu Gly Pro Tyr Ala Leu Asp Asn
    13730               13735               13740
Asp Ser Leu Phe Val Asn Gly Phe Thr His Arg Ser Ser Val Ser Thr
13745               13750               13755               13760
Thr Ser Thr Pro Gly Thr Pro Thr Val Tyr Leu Gly Ala Ser Lys Thr
                13765               13770               13775
Pro Ala Ser Ile Phe Gly Pro Ser Ala Ala Ser His Leu Leu Ile Leu
            13780               13785               13790
Phe Thr Leu Asn Phe Thr Ile Thr Asn Leu Arg Tyr Glu Glu Asn Met
        13795               13800               13805
Trp Pro Gly Ser Arg Lys Phe Asn Thr Thr Glu Arg Val Leu Gln Gly
    13810               13815               13820
Leu Leu Arg Pro Leu Phe Lys Asn Thr Ser Val Gly Pro Leu Tyr Ser
13825               13830               13835               13840
Gly Cys Arg Leu Thr Leu Leu Arg Pro Glu Lys Asp Gly Glu Ala Thr
                13845               13850               13855
Gly Val Asp Ala Ile Cys Thr His Arg Pro Asp Pro Thr Gly Pro Gly
            13860               13865               13870
Leu Asp Arg Glu Gln Leu Tyr Leu Glu Leu Ser Gln Leu Thr His Ser
        13875               13880               13885
Ile Thr Glu Leu Gly Pro Tyr Thr Leu Asp Arg Asp Ser Leu Tyr Val
    13890               13895               13900
Asn Gly Phe Thr His Arg Ser Ser Val Pro Thr Thr Ser Thr Gly Val
13905               13910               13915               13920
Val Ser Glu Glu Pro Phe Thr Leu Asn Phe Thr Ile Asn Asn Leu Arg
                13925               13930               13935
Tyr Met Ala Asp Met Gly Gln Pro Gly Ser Leu Lys Phe Asn Ile Thr
            13940               13945               13950
Asp Asn Val Met Gln His Leu Leu Ser Pro Leu Phe Gln Arg Ser Ser

13955                13960                13965
Leu Gly Ala Arg Tyr Thr Gly Cys Arg Val Ile Ala Leu Arg Ser Val
    13970                13975                13980
Lys Asn Gly Ala Glu Thr Arg Val Asp Leu Leu Cys Thr Tyr Leu Gln
13985                13990                13995                14000
Pro Leu Ser Gly Pro Gly Leu Pro Ile Lys Gln Val Phe His Glu Leu
                14005                14010                14015
Ser Gln Gln Thr His Gly Ile Thr Arg Leu Gly Pro Tyr Ser Leu Asp
            14020                14025                14030
Lys Asp Ser Leu Tyr Leu Asn Gly Tyr Asn Glu Pro Gly Pro Asp Glu
        14035                14040                14045
Pro Pro Thr Thr Pro Lys Pro Ala Thr Thr Phe Leu Pro Pro Leu Ser
    14050                14055                14060
Glu Ala Thr Thr Ala Met Gly Tyr His Leu Lys Thr Leu Thr Leu Asn
14065                14070                14075                14080
Phe Thr Ile Ser Asn Leu Gln Tyr Ser Pro Asp Met Gly Lys Gly Ser
                14085                14090                14095
Ala Thr Phe Asn Ser Thr Glu Gly Val Leu Gln His Leu Leu Arg Pro
            14100                14105                14110
Leu Phe Gln Lys Ser Ser Met Gly Pro Phe Tyr Leu Gly Cys Gln Leu
        14115                14120                14125
Ile Ser Leu Arg Pro Glu Lys Asp Gly Ala Ala Thr Gly Val Asp Thr
    14130                14135                14140
Thr Cys Thr Tyr His Pro Asp Pro Val Gly Pro Gly Leu Asp Ile Gln
14145                14150                14155                14160
Gln Leu Tyr Trp Glu Leu Ser Gln Leu Thr His Gly Val Thr Gln Leu
                14165                14170                14175
Gly Phe Tyr Val Leu Asp Arg Asp Ser Leu Phe Ile Asn Gly Tyr Ala
            14180                14185                14190
Pro Gln Asn Leu Ser Ile Arg Gly Glu Tyr Gln Ile Asn Phe His Ile
        14195                14200                14205
Val Asn Trp Asn Leu Ser Asn Pro Asp Pro Thr Ser Ser Glu Tyr Ile
    14210                14215                14220
Thr Leu Leu Arg Asp Ile Gln Asp Lys Val Thr Thr Leu Tyr Lys Gly
14225                14230                14235                14240
Ser Gln Leu His Asp Thr Phe Arg Phe Cys Leu Val Thr Asn Leu Thr
                14245                14250                14255
Met Asp Ser Val Leu Val Thr Val Lys Ala Leu Phe Ser Ser Asn Leu
            14260                14265                14270
Asp Pro Ser Leu Val Glu Gln Val Phe Leu Asp Lys Thr Leu Asn Ala
        14275                14280                14285
Ser Phe His Trp Leu Gly Ser Thr Tyr Gln Leu Val Asp Ile His Val
    14290                14295                14300
Thr Glu Met Glu Ser Ser Val Tyr Gln Pro Thr Ser Ser Ser Ser Thr
14305                14310                14315                14320
Gln His Phe Tyr Leu Asn Phe Thr Ile Thr Asn Leu Pro Tyr Ser Gln
                14325                14330                14335
Asp Lys Ala Gln Pro Gly Thr Thr Asn Tyr Gln Arg Asn Lys Arg Asn
            14340                14345                14350
Ile Glu Asp Ala Leu Asn Gln Leu Phe Arg Asn Ser Ser Ile Lys Ser
        14355                14360                14365
Tyr Phe Ser Asp Cys Gln Val Ser Thr Phe Arg Ser Val Pro Asn Arg
    14370                14375                14380
His His Thr Gly Val Asp Ser Leu Cys Asn Phe Ser Pro Leu Ala Arg
14385                14390                14395                14400
Arg Val Asp Arg Val Ala Ile Tyr Glu Glu Phe Leu Arg Met Thr Arg
                14405                14410                14415
Asn Gly Thr Gln Leu Gln Asn Phe Thr Leu Asp Arg Ser Ser Val Leu
            14420                14425                14430
Val Asp Gly Tyr Ser Pro Asn Arg Asn Glu Pro Leu Thr Gly Asn Ser
        14435                14440                14445
Asp Leu Pro Phe Trp Ala Val Ile Leu Ile Gly Leu Ala Gly Leu Leu
    14450                14455                14460
Gly Val Ile Thr Cys Leu Ile Cys Gly Val Leu Val Thr Thr Arg Arg
14465                14470                14475                14480
Arg Lys Lys Glu Gly Glu Tyr Asn Val Gln Gln Gln Cys Pro Gly Tyr
                14485                14490                14495
Tyr Gln Ser His Leu Asp Leu Glu Asp Leu Gln

            14500               14505

<210> 58
<211> 43816
<212> DNA
<213> Homo sapiens

<400> 58
aagcgttgca caattccccc aacctccata catacggcag ctcttctaga cacaggtttt 60
cccaggtcaa atgcggggac cccagccata tctcccaccc tgagaaattt tggagtttca 120
gggagctcag aagctctgca gaggccaccc tctctgaggg gattcttctt agacctccat 180
ccagaggcaa atgttgacct gtccatgctg aaaccctcag gccttcctgg gtcatcttct 240
cccacccgct ccttgatgac agggagcagg agcactaaag ccacaccaga aatggattca 300
ggactgacag gagccacctt gtcacctaag acatctacag gtgcaatcgt ggtgacagaa 360
catactctgc cctttacttc cccagataag accttggcca gtcctacatc ttcggttgtg 420
ggaagaacca cccagtcttt gggggtgatg tcctctgctc tccctgagtc aacctctaga 480
ggaatgacac actccgagca aagaaccagc ccatcgctga gtccccaggt caatggaact 540
ccctctagga actaccctgc tacaagcatg gtttcaggat tgagttcccc aaggaccagg 600
accagttcca cagaaggaaa ttttaccaaa gaagcatcta catacacact cactgtagag 660
accacaagtg gcccagtcac tgagaagtac acagtcccca ctgagacctc aacaactgaa 720
ggtgacagca cagagacccc ctgggacaca agatatattc ctgtaaaaat cacatctcca 780
atgaaaacat ttgcagattc aactgcatcc aaggaaaatg ccccagtgtc tatgactcca 840
gctgagacca cagttactga ctcacatact ccaggaagga caaacccatc atttgggaca 900
ctttattctt ccttccttga cctatcacct aaagggaccc caaattccag aggtgaaaca 960
agcctggaac tgattctatc aaccactgga tatcccttct cctctcctga acctggctct 1020
gcaggacaca gcagaataag taccagtgcg cctttgtcat catctgcttc agttctcgat 1080
aataaaatat cagagaccag catattctca ggccagagtc tcacctcccc tctgtctcct 1140
ggggtgcccg aggccagagc cagcacaatg cccaactcag ctatcccttt ttccatgaca 1200
ctaagcaatg cagaaacaag tgccgaaagg gtcagaagca caatttcctc tctggggact 1260
ccatcaatat ccacaaagca gacagcagag actatcctta ccttccatgc cttcgctgag 1320
accatggata tacccagcac ccacatagcc aagactttgg cttcagaatg gttgggaagt 1380
ccaggtaccc ttggtggcac cagcacttca gcgctgacaa ccacatctcc atctaccact 1440
ttagtctcag aggagaccaa cacccatcac tccacgagtg gaaaggaaac agaaggaact 1500
ttgaatacat ctatgactcc acttgagacc tctgctcctg gagaagagtc cgaaatgact 1560
gccaccttgg tccccactct aggttttaca actcttgaca gcaagatcag aagtccatct 1620
caggtctctt catcccaccc aacaagagag ctcagaacca caggcagcac ctctgggagg 1680
cagagttcca gcacagctgc ccacgggagc tctgacatcc tgagggcaac cacttccagc 1740
acctcaaaag catcatcatg gaccagtgaa agcacagctc agcaatttag tgaaccccag 1800
cacacacagt gggtggagac aagtcctagc atgaaaacag agagaccccc agcatcaacc 1860
agtgtggcag cccctatcac cacttctgtt ccctcagtgg tctctggctt caccaccctg 1920
aagaccagct ccacaaaagg gatttggctt gaagaaacat ctgcagacac actcatcgga 1980
gaatccacag ctggcccaac cacccatcag tttgctgttc ccactgggat ttcaatgaca 2040
ggaggcagca gcaccagggg aagccagggc acaacccacc tactcaccag agccacagca 2100
tcatctgaga catccgcaga tttgactctg gccacgaacg gtgtcccagt ctccgtgtct 2160
ccagcagtga gcaagacggc tgctggctca agtcctccag gagggacaaa gccatcatat 2220
acaatggttt cttctgtcat ccctgagaca tcatctctac agtcctcagc tttcagggaa 2280
ggaaccagcc tgggactgac tccattaaac actagacatc ccttctcttc ccctgaacca 2340
gactctgcag gacacaccaa gataagcacc agcattcctc tgttgtcatc tgcttcagtt 2400
cttgaggata aagtgtcagc gaccagcaca ttctcacacc acaaagccac ctcatctatt 2460
accacaggga ctcctgaaat ctcaacaaag acaaagccca gctcagccgt tctttcctcc 2520
atgaccctaa gcaatgcagc aacaagtcct gaaagagtca gaaatgcaac ttcccctctg 2580
actcatccat ctccatcagg ggaagagaca gcagggagtg tcctcactct cagcacctct 2640
gctgagacta cagactcacc taacatccac ccaactggga cactgacttc agaatcgtca 2700
gagagtccta gcactctcag cctcccaagt gtctctggag tcaaaaccac attttcttca 2760
tctactcctt ccactcatct atttactagt ggagaagaaa cagaggaaac ttcgaatcca 2820
tctgtgtctc aacctgagac ttctgtttcc agagtaagga ccaccttggc cagcacctct 2880
gtccctaccc cagtattccc caccatggac acctggccta cacgttcagc tcagttctct 2940
tcatcccacc tagtgagtga gctcagagct acgagcagta cctcagttac aaactcaact 3000
ggttcagctc ttcctaaaat atctcacctc actgggacgg caacaatgtc acagaccaat 3060
agagacacgt ttaatgactc tgctgcaccc caaagcacaa cttggccaga gactagtccc 3120
agattcaaga cagggttacc ttcagcaaca accactgttt caacctctgc cacttctctc 3180
tctgctactg taatggtctc taaattcact tctccagcaa ctagttccat ggaagcaact 3240
tctatcaggg aaccatcaac aaccatcctc acaacagaga ccacgaatgg cccaggctct 3300
atggctgtgg cttctaccaa catcccaatt ggaaagggct acattactga aggaagattg 3360
gacacaagcc atctgcccat tggaaccaca gcttcctctg agacatctat ggattttacc 3420

atggccaaag aaagtgtctc aatgtcagta tctccatctc agtccatgga tgctgctggc 3480
tcaagcactc caggaaggac aagccaattc gttgacacat tttctgatga tgtctatcat 3540
ttaacatcca gagaaattac aatacctaga gatggaacaa gctcagctct gactccacaa 3600
atgactgcaa ctcaccctcc atctcctgat cctggctctg ctagaagcac ctggcttggc 3660
atcttgtcct catctccttc ttctcctact cccaaagtca caatgagctc cacattttca 3720
actcagagag tcaccacaag catgataatg gacacagttg aaactagtcg gtggaacatg 3780
cccaacttac cttccacgac ttccttgaca ccaagtaata ttccaacaag tggtgccata 3840
ggaaaaagca ccctggttcc cttggacact ccatctccag ccacatcatt ggaggcatca 3900
gaagggggac ttccaaccct cagcacctac cctgaatcaa caaacacacc cagcatccac 3960
ctcggagcac acgctagttc agaaagtcca agcaccatca aacttaccat ggcttcagta 4020
gtaaaacctg gctcttacac acctctcacc ttcccctcaa tagagaccca cattcatgta 4080
tcaacagcca gaatggctta ctcttctggg tcttcacctg agatgacagc tcctggagag 4140
actaacactg gtagtacctg ggaccccacc acctacatca ccactacgga tcctaaggat 4200
acaagttcag ctcaggtctc tacaccccac tcagtgagga cactcagaac cacagaaaac 4260
catccaaaga cagagtccgc caccccagct gcttactctg gaagtcctaa aatctcaagt 4320
tcacccaatc tcaccagtcc ggccacaaaa gcatggacca tcacagacac aactgaacac 4380
tccactcaat tacattacac aaaattggca gaaaaatcat ctggatttga gacacagtca 4440
gctccaggac ctgtctctgt agtaatccct acctccccta ccattggaag cagcacattg 4500
gaactaactt ctgatgtccc aggggaaccc ctggtccttg ctcccagtga gcagaccaca 4560
atcactctcc ccatggcaac atggctgagt accagtttga cagaggaaat ggcttcaaca 4620
gaccttgata tttcaagtcc aagttcaccc atgagtacat ttgctatttt tccacctatg 4680
tccacacctt ctcatgaact ttcaaagtca gaggcagata ccagtgccat tagaaataca 4740
gattcaacaa cgttggatca gcacctagga atcaggagtt tgggcagaac tggggactta 4800
acaactgttc ctatcacccc actgacaacc acgtggacca gtgtgattga acactcaaca 4860
caagcacagg acaccctttc tgcaacgatg agtcctactc acgtgacaca gtcactcaaa 4920
gatcaaacat ctataccagc ctcagcatcc ccttcccatc ttactgaagt ctaccctgag 4980
ctcgggacac aagggagaag ctcctctgag gcaaccactt tttggaaacc atctacagac 5040
acactgtcca gagagattga gactggccca acaaacattc aatccactcc acccatggac 5100
aacacaacaa cagggagcag tagtagtgga gtcaccctgg gcatagccca ccttcccata 5160
ggaacatcct ccccagctga gacatccaca aacatggcac tggaaagaag aagttctaca 5220
gccactgtct ctatggctgg gacaatggga ctccttgtta ctagtgctcc aggaagaagc 5280
atcagccagt cattaggaag agtttcctct gtcctttctg agtcaactac tgaaggagtc 5340
acagattcta gtaagggaag cagcccaagg ctgaacacac agggaaatac agctctctcc 5400
tcctctcttg aacccagcta tgctgaagga agccagatga gcacaagcat ccctctaacc 5460
tcatctccta caactcctga tgtggaattc atagggggca gcacattttg gaccaaggag 5520
gtcaccacag ttatgacctc agacatctcc aagtcttcag caaggacaga gtccagctca 5580
gctaccctta tgtccacagc tttgggaagc actgaaaata caggaaaaga aaaactcaga 5640
actgcctcta tggatcttcc atctccaact ccatcaatgg aggtgacacc atggatttct 5700
ctcactctca gtaatgcccc caataccaca gattcacttg acctcagcca tggggtgcac 5760
accagctctg cagggacttt ggccactgac aggtcattga atactggtgt cactagagcc 5820
tccagattgg aaaacggctc tgatacctct tctaagtccc tgtctatggg aaacagcact 5880
cacacttcca tgacttacac agagaagagt gaagtgtctt cttcaatcca tccccgacct 5940
gagacctcag ctcctggagc agagaccact ttgacttcca ctcctggaaa cagggccata 6000
agcttaacat tgcctttttc atccattcca gtggaagaag tcatttctac aggcataacc 6060
tcaggaccag acatcaactc agcacccatg acacattctc ccatcacccc accaacaatt 6120
gtatggacca gtacaggcac aattgaacag tccactcaac cactacatgc agtttcttca 6180
gaaaaagttt ctgtgcagac acagtcaact ccatatgtca actctgtggc agtgtctgct 6240
tcccctaccc atgagaattc agtctcttct ggaagcagca catcctctcc atattcctca 6300
gcctcacttg aatccttgga ttccacaatc agtaggagga atgcaatcac ttcctggcta 6360
tgggacctca ctacatctct ccccactaca acttggccaa gtactagttt atctgaggca 6420
ctgtcctcag gccattctgg ggtttcaaac ccaagttcaa ctacgactga atttccactc 6480
ttttcagctg catccacatc tgctgctaag caaagaaatc cagaaacaga gacccatggt 6540
ccccagaata cagccgcgag tactttgaac actgatgcat cctcggtcac aggtctttct 6600
gagactcctg tgggggcaag tatcagctct gaagtccctc ttccaatggc cataacttct 6660
agatcagatg tttctggcct tacatctgag agtactgcta acccgagttt aggcacagcc 6720
tcttcagcag ggaccaaatt aactaggaca atatccctgc ccacttcaga gtctttggtt 6780
tcctttagaa tgaacaagga tccatggaca gtgtcaatcc ctttggggtc ccatccaact 6840
actaatacag aaacaagcat cccagtaaac agcgcaggtc cacctggctt gtccacagta 6900
gcatcagatg taattgacac accttcagat ggggctgaga gtattcccac tgtctccttt 6960
tccccctccc ctgatactga agtgacaact atctcacatt tcccagaaaa gacaactcat 7020
tcatttagaa ccatttcatc tctcactcat gagttgactt caagagtgac acctattcct 7080
ggggattgga tgagttcagc tatgtctaca aagcccacag gagccagtcc ctccattaca 7140
ctgggagaga gaaggacaat cacctctgct gctccaacca cttcccccat agttctcact 7200
gctagtttca cagagaccag cacagtttca ctggataatg aaactacagt aaaaacctca 7260
gatatccttg acgcacggaa aacaaatgag ctcccctcag atagcagttc ttcttctgat 7320
ctgatcaaca cctccatagc ttcttcaact atggatgtca ctaaaacagc ctccatcagt 7380
cccactagca tctcaggaat gacagcaagt tcctccccat ctctcttctc ttcagataga 7440
ccccaggttc ccacatctac aacagagaca aatacagcca cctctccatc tgtttccagt 7500

aacacctatt ctcttgatgg gggctccaat gtgggtggca ctccatccac tttaccaccc 7560
tttacaatca cccaccctgt cgagacaagc tcggccctat tagcctggtc tagaccagta 7620
agaactttca gcaccatggt cagcactgac actgcctccg gagaaaatcc tacctctagc 7680
aattctgtgg tgacttctgt tccagcacca ggtacatgga ccagtgtagg cagtactact 7740
gacttacctg ccatgggctt tctcaagaca agtcctgcag gagaggcaca ctcacttcta 7800
gcatcaacta ttgaaccagc cactgccttc actccccatc tctcagcagc agtggtcact 7860
ggatccagtg ctacatcaga agccagtctt ctcactacga gtgaaagcaa agccattcat 7920
tcttcaccac agaccccaac tacacccacc tctggagcaa actgggaaac ttcagctact 7980
cctgagagcc ttttggtagt cactgagact tcagacacaa cacttacctc aaagattttg 8040
gtcacagata ccatcttgtt ttcaactgtg tccacgccac cttctaaatt tccaagtacg 8100
gggactctgt ctggagcttc cttccctact ttactcccgg acactccagc catccctctc 8160
actgccactg agccaacaag ttcattagct acatcctttg attccacccc actggtgact 8220
atagcttcgg atagtcttgg cacagtccca gagactaccc tgaccatgtc agagacctca 8280
aatggtgatg cactggttct taagacagta agtaacccag ataggagcat ccctggaatc 8340
actatccaag gagtaacaga aagtccactc catccttctt ccacttcccc ctctaagatt 8400
gttgctccac ggaatacaac ctatgaaggt tcgatcacag tggcactttc tactttgcct 8460
gcgggaacta ctggttccct tgtattcagt cagagttctg aaaactcaga gacaacggct 8520
ttggtagact catcagctgg gcttgagagg gcatctgtga tgccactaac cacaggaagc 8580
cagggtatgg ctagctctgg aggaatcaga agtgggtcca ctcactcaac tggaaccaaa 8640
acattttctt ctctccctct gaccatgaac ccaggtgagg ttacagccat gtctgaaatc 8700
accacgaaca gactgacagc tactcaatca acagcaccca aagggatacc tgtgaagccc 8760
accagtgctg agtcaggcct cctaacacct gtctctgcct cctcaagccc atcaaaggcc 8820
tttgcctcac tgactacagc tcccccaact tgggggatcc cacagtctac cttgacattt 8880
gagttttctg aggtcccaag tttggatact aagtccgctt ctttaccaac tcctggacag 8940
tccctgaaca ccattccaga ctcagatgca agcacagcat cttcctcact gtccaagtct 9000
ccagaaaaaa acccaagggc aaggatgatg acttccacaa aggccataag tgcaagctca 9060
tttcaatcaa caggttttac tgaaacccct gagggatctg cctccccttc tatggcaggg 9120
catgaaccca gagtccccac ttcaggaaca ggggacccta gatatgcctc agagagcatg 9180
tcttatccag acccaagcaa ggcatcatca gctatgacat cgacctctct tgcatcaaaa 9240
ctcacaactc tcttcagcac aggtcaagca gcaaggtctg gttctagttc ctctcccata 9300
agcctatcca ctgagaaaga aacaagcttc ctttccccca ctgcatccac ctccagaaag 9360
acttcactat ttcttgggcc ttccatggca aggcagccca acatattggt gcatcttcag 9420
acttcagctc tgacactttc tccaacatcc actctaaata tgtcccagga ggagcctcct 9480
gagttaacct caagccagac cattgcagaa gaagagggaa caacagctga aacacagacg 9540
ttaaccttca caccatctga gaccccaaca tccttgttac ctgtctcttc tcccacagaa 9600
cccacagcca gaagaaagag ttctccagaa acatgggcaa gctctatttc agttcctgcc 9660
aagacctcct tggttgaaac aactgatgga acgctagtga ccaccataaa gatgtcaagc 9720
caggcagcac aaggaaattc cacgtggcct gccccagcag aggagacggg gagcagtcca 9780
gcaggcacat ccccaggaag cccagaaatg tctaccactc tcaaaatcat gagctccaag 9840
gaacccagca tcagcccaga gatcaggtcc actgtgagaa attctccttg gaagactcca 9900
gaaacaactg ttcccatgga gaccacagtg gaaccagtca cccttcagtc cacagcccta 9960
ggaagtggca gcaccagcat ctctcacctg cccacaggaa ccacatcacc aaccaagtca 10020
ccaacagaaa atatgttggc tacagaaagg gtctccctct ccccatcccc acctgaggct 10080
tggaccaacc tttattctgg aactccagga gggaccaggc agtcactggc cacaatgtcc 10140
tctgtctccc tagagtcacc aactgctaga agcatcacag ggactggtca gcaaagcagt 10200
ccagaactgg tttcaaagac aactggaatg gaattctcta tgtggcatgg ctctactgga 10260
gggaccacag gggacacaca tgtctctctg agcacatctt ccaatatcct tgaagaccct 10320
gtaaccagcc caaactctgt gagctcattg acagataaat ccaaacataa aaccgagaca 10380
tgggtaagca ccacagccat tccctccact gtcctgaata ataagataat ggcagctgaa 10440
caacagacaa gtcgatctgt ggatgaggct tattcatcaa ctagttcttg gtcagatcag 10500
acatctggga gtgacatcac ccttggtgca tctcctgatg tcacaaacac attatacatc 10560
acctccacag cacaaaccac ctcactagtg tctctgccct ctggagacca aggcattaca 10620
agcctcacca atccctcagg aggaaaaaca agctctgcgt catctgtcac atctccttca 10680
atagggcttg agactctgag ggccaatgta agtgcagtga aaagtgacat tgcccctact 10740
gctgggcatc tatctcagac ttcatctcct gcggaagtga gcatcctgga cgtaaccaca 10800
gctcctactc caggtatctc caccaccatc accaccatgg gaaccaactc aatctcaact 10860
accacaccca acccagaagt gggtatgagt accatggaca gcaccccggc cacagagagg 10920
cgcacaactt ctacagaaca cccttccacc tggtcttcca cagctgcatc agattcctgg 10980
actgtcacag acatgacttc aaacttgaaa gttgcaagat ctcctggaac aatttccaca 11040
atgcatacaa cttcattctt agcctcaagc actgaattag actccatgtc tactccccat 11100
ggccgtataa ctgtcattgg aaccagcctg gtcactccat cctctgatgc ttcagctgta 11160
aagacagaga ccagtacaag tgaaagaaca ttgagtcctt cagacacaac tgcatctact 11220
cccatctcaa ctttttctcg tgtccagagg atgagcatct cagttcctga cattttaagt 11280
acaagttgga ctcccagtag tacagaagca gaagatgtgc ctgtttcaat ggtttctaca 11340
gatcatgcta gtacaaagac tgacccaaat acgcccctgt ccacttttct gtttgattct 11400
ctgtccactc ttgactggga cactgggaga tctctgtcat cagccacagc cactacctca 11460
gctcctcagg gggccacaac tccccaggaa ctcactttgg aaaccatgat cagcccagct 11520
acctcacagt tgcccttctc tatagggcac attacaagtg cagtcacacc agctgcaatg 11580

gcaaggagct ctggagttac tttttcaaga ccagatccca caagcaaaaa ggcagagcag 11640
acttccactc agcttcccac caccacttct gcacatccag ggcaggtgcc cagatcagca 11700
gcaacaactc tggatgtgat cccacacaca gcaaaaactc cagatgcaac ttttcagaga 11760
caagggcaga cagctcttac aacagaggca agagctacat ctgactcctg gaatgagaaa 11820
gaaaaatcaa ccccaagtgc accttggatc actgagatga tgaattctgt ctcagaagat 11880
accatcaagg aggttaccag ctcctccagt gtattaagga ccctgaatac gctggacata 11940
aacttggaat ctgggacgac ttcatcccca agttggaaaa gcagcccata tgagagaatt 12000
gccccttctg agtccaccac agacaaagag gcaattcacc cttctacaaa cacagtagag 12060
accacaggct gggtcacaag ttccgaacat gcttctcatt ccactatccc agcccactca 12120
gcgtcatcca aactcacatc tccagtggtt acaacctcca ccagggaaca agcaatagtt 12180
tctatgtcaa caaccacatg gccagagtct acaagggcta gaacagagcc taattccttc 12240
ttgactattg aactgaggga cgtcagccct tacatggaca ccagctcaac cacacaaaca 12300
agtattatct cttccccagg ttccactgcg atcaccaagg ggcctagaac agaaattacc 12360
tcctctaaga gaatatccag ctcattcctt gcccagtcta tgaggtcgtc agacagcccc 12420
tcagaagcca tcaccaggct gtctaacttt cctgccatga cagaatctgg aggaatgatc 12480
cttgctatgc aaacaagtcc acctggcgct acatcactaa gtgcacctac tttggataca 12540
tcagccacag cctcctggac agggactcca ctggctacga ctcagagatt tacatactca 12600
gagaagacca ctctctttag caaaggtcct gaggatacat cacagccaag ccctccctct 12660
gtggaagaaa ccagctcttc ctcttccctg gtacctatcc atgctacaac ctcgccttcc 12720
aatattttgt tgacatcaca agggcacagt ccctcctcta ctccacctgt gacctcagtt 12780
ttcttgtctg agacctctgg cctggggaag accacagaca tgtcgaggat aagcttggaa 12840
cctggcacaa gtttacctcc caatttgagc agtacagcag gtgaggcgtt atccacttat 12900
gaagcctcca gagatacaaa ggcaattcat cattctgcag acacagcagt gacgaatatg 12960
gaggcaacca gttctgaata ttctcctatc ccaggccata caaagccatc caaagccaca 13020
tctccattgg ttacctccca catcatgggg gacatcactt cttccacatc agtatttggc 13080
tcctccgaga ccacagagat tgagacagtg tcctctgtga accagggact tcaggagaga 13140
agcacatccc aggtggccag ctctgctaca gagacaagca ctgtcattac ccatgtgtct 13200
agtggtgatg ctactactca tgtcaccaag acacaagcca ctttctctag cggaacatcc 13260
atctcaagcc ctcatcagtt tataacttct accaacacat ttacagatgt gagcaccaac 13320
ccctccacct ctctgataat gacagaatct tcaggagtga ccatcaccac ccaaacaggt 13380
cctactggag ctgcaacaca gggtccatat ctcttggaca catcaaccat gccttacttg 13440
acagagactc cattagctgt gactccagat tttatgcaat cagagaagac cactctcata 13500
agcaaaggtc ccaaggatgt gtcctggaca agccctccct ctgtggcaga aaccagctat 13560
ccctcttccc tgacaccttt cttggtcaca accatacctc ctgccacttc cacgttacaa 13620
gggcaacata catcctctcc tgtttctgcg acttcagttc ttacctctgg actggtgaag 13680
accacagata tgttgaacac aagcatggaa cctgtgacca attcacctca aaatttgaac 13740
aatccatcaa atgagatact ggccactttg gcagccacca cagatataga gactattcat 13800
ccttccataa acaaagcagt gaccaatatg gggactgcca gttcagcaca tgtactgcat 13860
tccactctcc cagtcagctc agaaccatct acagccacat ctccaatggt tcctgcctcc 13920
agcatggggg acgctcttgc ttctatatca atacctggtt ctgagaccac agacattgag 13980
ggagagccaa catcctccct gactgctgga cgaaaagaga acagcaccct ccaggagatg 14040
aactcaacta cagagtcaaa catcatcctc tccaatgtgt ctgtgggggc tattactgaa 14100
gccacaaaaa tggaagtccc ctcttttgat gcaacattca taccaactcc tgctcagtca 14160
acaaagttcc cagatatttt ctcagtagcc agcagtagac tttcaaactc tcctcccatg 14220
acaatatcta cccacatgac caccacccag acagggtctt ctggagctac atcaaagatt 14280
ccacttgcct tagacacatc aaccttggaa acctcagcag ggactccatc agtggtgact 14340
gaggggtttg cccactcaaa aataaccact gcaatgaaca atgatgtcaa ggacgtgtca 14400
cagacaaacc ctccctttca ggatgaagcc agctctccct cttctcaagc acctgtcctt 14460
gtcacaacct taccttcttc tgttgctttc acaccgcaat ggcacagtac ctcctctcct 14520
gtttctatgt cctcagttct tacttcttca ctggtaaaga ccgcaggcaa ggtggataca 14580
agcttagaaa cagtgaccag ttcacctcaa agtatgagca acactttgga tgacatatcg 14640
gtcacttcag cagccaccac agatatagag acaacgcatc cttccataaa cacagtagtt 14700
accaatgtgg ggaccaccgg ttcagcattt gaatcacatt ctactgtctc agcttaccca 14760
gagccatcta aagtcacatc tccaaatgtt accacctcca ccatggaaga caccacaatt 14820
tccagatcaa tacctaaatc ctctaagact acaagaactg agactgagac aacttcctcc 14880
ctgactccta aactgaggga gaccagcatc tcccaggaga tcacctcgtc cacagagaca 14940
agcactgttc cttacaaaga gctcactggt gccactaccg aggtatccag gacagatgtc 15000
acttcctcta gcagtacatc cttccctggc cctgatcagt ccacagtgtc actagacatc 15060
tccacagaaa ccaacaccag gctgtctacc tccccaataa tgacagaatc tgcagaaata 15120
accatcacca cccaaacagg tcctcatggg gctacatcac aggatacttt taccatggac 15180
ccatcaaata caacccccca ggcagggatc cactcagcta tgactcatgg attttcacaa 15240
ttggatgtga ccactcttat gagcagaatt ccacaggatg tatcatggac aagtcctccc 15300
tctgtggata aaaccagctc cccctcttcc tttctgtcct cacctgcaat gaccacacct 15360
tccctgattt cttctacctt accagaggat aagctctcct ctcctatgac ttcacttctc 15420
acctctggcc tagtgaagat tacagacata ttacgtacac gcttggaacc tgtgaccagc 15480
tcacttccaa atttcagcag cacctcagat aagatactgg ccacttctaa agacagtaaa 15540
gacacaaagg aaatttttcc ttctataaac acagaagaga ccaatgtgaa agccaacaac 15600
tctggacatg aatcccattc ccctgcactg gctgactcag agacacccaa agccacaact 15660

caaatggtta tcaccaccac tgtgggagat ccagctcctt ccacatcaat gccagtgcat 15720
ggttcctctg agactacaaa cattaagaga gagccaacat atttcttgac tcctagactg 15780
agagagacca gtacctctca ggagtccagc tttcccacgg acacaagttt tctactttcc 15840
aaagtcccca ctggtactat tactgaggtc tccagtacag gggtcaactc ttctagcaaa 15900
atttccaccc cagaccatga taagtccaca gtgccacctg acaccttcac aggagagatc 15960
cccagggtct tcacctcctc tattaagaca aaatctgcag aaatgacgat caccacccaa 16020
gcaagtcctc ctgagtctgc atcgcacagt acccttccct tggacacatc aaccacactt 16080
tcccagggag ggactcattc aactgtgact cagggattcc catactcaga ggtgaccact 16140
ctcatgggca tgggtcctgg gaatgtgtca tggatgacaa ctccccctgt ggaagaaacc 16200
agctctgtgt cttccctgat gtcttcacct gccatgacat ccccttctcc tgtttcctcc 16260
acatcaccac agagcatccc ctcctctcct cttcctgtga ctgcacttcc tacttctgtt 16320
ctggtgacaa ccacagatgt gttgggcaca acaagcccag agtctgtaac cagttcacct 16380
ccaaatttga gcagcatcac tcatgagaga ccggccactt acaaagacac tgcacacaca 16440
gaagccgcca tgcatcattc cacaaacacc gcagtgacca atgtagggac ttccgggtct 16500
ggacataaat cacaatcctc tgtcctagct gactcagaga catcgaaagc cacacctctg 16560
atgagtacca cctccaccct gggggacaca agtgtttcca catcaactcc taatatctct 16620
cagactaacc aaattcaaac agagccaaca gcatccctga gccctagact gagggagagc 16680
agcacgtctg agaagaccag ctcaacaaca gagacaaata ctgccttttc ttatgtgccc 16740
acaggtgcta ttactcaggc ctccagaaca gaaatctcct ctagcagaac atccatctca 16800
gaccttgatc ggcccacaat agcacccgac atctccacag gaatgatcac caggctcttc 16860
acctccccca tcatgacaaa atctgcagaa atgaccgtca ccactcaaac aactactcct 16920
ggggctacat cacagggtat ccttccctgg gacacatcaa ccacactttt ccagggaggg 16980
actcattcaa ccgtgtctca gggattccca cactcagaga taaccactct tcggagcaga 17040
acccctggag atgtgtcatg gatgacaact ccccctgtgg aagaaaccag ctctgggttt 17100
tccctgatgt caccttccat gacatcccct tctcctgttt cctccacatc accagagagc 17160
atcccctcct ctcctctccc tgtgactgca cttcttactt ctgttctggt gacaaccaca 17220
aatgtattgg gcacaacaag cccagagccc gtaacgagtt cacctccaaa tttaagcagc 17280
cccacacagg agagactgac cacttacaaa gacactgcgc acacagaagc catgcatgct 17340
tccatgcata caaacactgc agtggccaac gtggggacct ccatttctgg acatgaatca 17400
caatcttctg tcccagctga ttcacacaca tccaaagcca catctccaat gggtatcacc 17460
ttcgccatgg gggatacaag tgtttctaca tcaactcctg ccttctttga gactagaatt 17520
cagactgaat caacatcctc tttgattcct ggattaaggg acaccaggac gtctgaggag 17580
atcaacactg tgacagagac cagcactgtc ctttcagaag tgcccactac tactactact 17640
gaggtctcca ggacagaagt tatcacttcc agcagaacaa ccatctcagg gcctgatcat 17700
tccaaaatgt caccctacat ctccacagaa accatcacca ggctctccac ttttcctttt 17760
gtaacaggat ccacagaaat ggccatcacc aaccaaacag gtcctatagg gactatctca 17820
caggctaccc ttaccctgga cacatcaagc acagcttcct gggaagggac tcactcacct 17880
gtgactcaga gatttccaca ctcagaggag accactacta tgagcagaag tactaagggc 17940
gtgtcatggc aaagccctcc ctctgtggaa gaaaccagtt ctccttcttc cccagtgcct 18000
ttacctgcaa taacctcaca ttcatctctt tattccgcag tatcaggaag tagccccact 18060
tctgctctcc ctgtgacttc ccttctcacc tctggcagga ggaagaccat agacatgttg 18120
gacacacact cagaacttgt gaccagctcc ttaccaagtg caagtagctt ctcaggtgag 18180
atactcactt ctgaagcctc cacaaataca gagacaattc acttttcaga gaacacagca 18240
gaaaccaata tggggaccac caattctatg cataaactac attcctctgt ctcaatccac 18300
tcccagccat ccggacacac acctccaaag gttactggat ctatgatgga ggacgctatt 18360
gtttccacat caacacctgg ttctcctgag actaaaaatg ttgacagaga ctcaacatcc 18420
cctctgactc ctgaactgaa agaggacagc accgccctgg tgatgaactc aactacagag 18480
tcaaacactg ttttctccag tgtgtccctg gatgctgcta ctgaggtctc cagggcagaa 18540
gtcacctact atgatcctac attcatgcca gcttctgctc agtcaacaaa gtccccagac 18600
atttcacctg aagccagcag cagtcattct aactctcctc ccttgacaat atctacacac 18660
aagaccatcg ccacacaaac aggtccttct ggggtgacat ctcttggcca actgaccctg 18720
gacacatcaa ccatagccac ctcagcagga actccatcag ccagaactca ggattttgta 18780
gattcagaaa caaccagtgt catgaacaat gatctcaatg atgtgttgaa gacaagccct 18840
ttctctgcag aagaagccaa ctctctctct tctcaggcac ctctccttgt gacaacctca 18900
ccttctcctg taacttccac attgcaagag cacagtacct cctctcttgt ttctgtgacc 18960
tcagtaccca cccctacact ggcgaagatc acagacatgg acacaaactt agaacctgtg 19020
actcgttcac ctcaaaattt aaggaacacc ttggccactt cagaagccac cacagataca 19080
cacacaatgc atccttctat aaacacagca gtggccaatg tggggaccac cagttcacca 19140
aatgaattct attttactgt ctcacctgac tcagacccat ataaagccac atccgcagta 19200
gttatcactt ccacctcggg ggactcaata gtttccacat caatgcctag atcctctgcg 19260
atgaaaaaga ttgagtctga gacaactttc tccctgatat ttagactgag ggagactagc 19320
acctcccaga aaattggctc atcctcagac acaagcacgg tctttgacaa agcattcact 19380
gctgctacta ctgaggtctc cagaacagaa ctcacctcct ctagcagaac atccatccaa 19440
ggcactgaaa agcccacaat gtcaccggac acctccacaa gatctgtcac catgctttct 19500
acttttgctg gcctgacaaa atccgaagaa aggaccattg ccacccaaac aggtcctcat 19560
agggcgacat cacagggtac ccttacctgg gacacatcaa tcacaacctc acaggcaggg 19620
acccactcag ctatgactca tggattttca caattagatt tgtccactct tacgagtaga 19680
gttcctgagt acatatcagg gacaagccca ccctctgtgg aaaaaaccag ctcttcctct 19740

tcccttctgt ctttaccagc aataacctca ccgtcccctg tacctactac attaccagaa 19800
agtaggccgt cttctcctgt tcatctgact tcactcccca cctctggcct agtgaagacc 19860
acagatatgc tggcatctgt ggccagttta cctccaaact tgggcagcac ctcacataag 19920
ataccgacta cttcagaaga cattaaagat acagagaaaa tgtatccttc cacaaacata 19980
gcagtaacca atgtggggac caccacttct gaaaaggaat cttattcgtc tgtcccagcc 20040
tactcagaac cacccaaagt cacctctcca atggttacct ctttcaacat aagggacacc 20100
attgtttcca catccatgcc tggctcctct gagattacaa ggattgagat ggagtcaaca 20160
ttctccctgg ctcatgggct gaagggaacc agcacctccc aggaccccat cgtatccaca 20220
gagaaaagtg ctgtccttca caagttgacc actggtgcta ctgagacctc taggacagaa 20280
gttgcctctt ctagaagaac atccattcca ggccctgatc attccacaga gtcaccagac 20340
atctccactg aagtgatccc cagcctgcct atctcccttg gcattacaga atcttcaaat 20400
atgaccatca tcactcgaac aggtcctcct cttggctcta catcacaggg cacatttacc 20460
ttggacacac caactacatc ctccagggca ggaacacact cgatggcgac tcaggaattt 20520
ccacactcag aaatgaccac tgtcatgaac aaggaccctg agattctatc atggacaatc 20580
cctccttcta tagagaaaac cagcttctcc tcttccctga tgccttcacc agccatgact 20640
tcacctcctg tttcctcaac attaccaaag accattcaca ccactccttc tcctatgacc 20700
tcactgctca cccctagcct agtgatgacc acagacacat tgggcacaag cccagaacct 20760
acaaccagtt cacctccaaa tttgagcagt acctcacatg agatactgac aacagatgaa 20820
gacaccacag ctatagaagc catgcatcct tccacaagca cagcagcgac taatgtggaa 20880
accaccagtt ctggacatgg gtcacaatcc tctgtcctag ctgactcaga aaaaaccaag 20940
gccacagctc caatggatac cacctccacc atggggcata caactgtttc cacatcaatg 21000
tctgtttcct ctgagactac aaaaattaag agagagtcaa catattcctt gactcctgga 21060
ctgagagaga ccagcatttc ccaaaatgcc agcttttcca ctgacacaag tattgttctt 21120
tcagaagtcc ccactggtac tactgctgag gtctccagga cagaagtcac ctcctctggt 21180
agaacatcca tccctggccc ttctcagtcc acagttttgc cagaaatatc cacaagaaca 21240
atgacaaggc tctttgcctc gcccaccatg acagaatcag cagaaatgac catccccact 21300
caaacaggtc cttctgggtc tacctcacag gataccctta ccttggacac atccaccaca 21360
aagtcccagg caaagactca ttcaactttg actcagagat ttccacactc agagatgacc 21420
actctcatga gcagaggtcc tggagatatg tcatggcaaa gctctccctc tctggaaaat 21480
cccagctctc tcccttccct gctgtcttta cctgccacaa cctcacctcc tcccatttcc 21540
tccacattac cagtgactat ctcctcctct cctcttcctg tgacttcact tctcacctct 21600
agcccggtaa cgaccacaga catgttacac acaagcccag aacttgtaac cagttcacct 21660
ccaaagctga gccacacttc agatgagaga ctgaccactg gcaaggacac cacaaataca 21720
gaagctgtgc atccttccac aaacacagca gcgtccaatg tggagattcc cagctctgga 21780
catgaatccc cttcctctgc cttagctgac tcagagacat ccaaagccac atcaccaatg 21840
tttattacct ccacccagga ggatacaact gttgccatat caacccctca cttcttggag 21900
actagcagaa ttcagaaaga gtcaatttcc tccctgagcc ctaaattgag ggagacaggc 21960
agttctgtgg agacaagctc agccatagag acaagtgctg tcctttctga agtgtccatt 22020
ggtgctacta ctgagatctc caggacagaa gtcacctcct ctagcagaac atccatctct 22080
ggttctgctg agtccacaat gttgccagaa atatccacca caagaaaaat cattaagttc 22140
cctacttccc ccatcctggc agaatcatca gaaatgacca tcaagaccca aacaagtcct 22200
cctgggtcta catcagagag tacctttaca ttagacacat caaccactcc ctccttggta 22260
ataacccatt cgactatgac tcagagattg ccacactcag agataaccac tcttgtgagt 22320
agaggtgctg gggatgtgcc acggcccagc tctctccctg tggaagaaac aagccctcca 22380
tcttcccagc tgtctttatc tgccatgatc tcaccttctc ctgtttcttc cacattacca 22440
gcaagtagcc actcctcttc tgcttctgtg acttcacttc tcacaccagg ccaagtgaag 22500
actactgagg tgttggacgc aagtgcagaa cctgaaacca gttcacctcc aagtttgagc 22560
agcacctcag ttgaaatact ggccacctct gaagtcacca cagatacgga gaaaattcat 22620
cctttctcaa acacggcagt aaccaaagtt ggaacttcca gttctggaca tgaatcccct 22680
tcctctgtcc tacctgactc agagacaacc aaagccacat cggcaatggg taccatctcc 22740
attatggggg atacaagtgt ttctacatta actcctgcct tatctaacac taggaaaatt 22800
cagtcagagc cagcttcctc actgaccacc agattgaggg agaccagcac ctctgaagag 22860
accagcttag ccacagaagc aaacactgtt ctttctaaag tgtccactgg tgctactact 22920
gaggtctcca ggacagaagc catctccttt agcagaacat ccatgtcagg ccctgagcag 22980
tccacaatgt cacaagacat ctccatagga accatcccca ggatttctgc ctcctctgtc 23040
ctgacagaat ctgcaaaaat gaccatcaca acccaaacag gtccttcgga gtctacacta 23100
gaaagtaccc ttaatttgaa cacagcaacc acaccctctt gggtggaaac ccactctata 23160
gtaattcagg gatttccaca cccagagatg accacttcca tgggcagagg tcctggaggt 23220
gtgtcatggc ctagccctcc ctttgtgaaa gaaaccagcc ctccatcctc cccgctgtct 23280
ttacctgccg tgacctcacc tcatcctgtt tccaccacat tcctagcaca tatccccccc 23340
tctccccttc ctgtgacttc acttctcacc tctggcccgg cgacaaccac agatatcttg 23400
ggtacaagca cagaacctgg aaccagttca tcttcaagtt tgagcaccac ctcccatgag 23460
agactgacca cttacaaaga cactgcacat acagaagccg tgcatccttc cacaaacaca 23520
ggagggacca atgtggcaac caccagctct ggatataaat cacagtcctc tgtcctagct 23580
gactcatctc caatgtgtac cacctccacc atgggggata caagtgttct cacatcaact 23640
cctgccttcc ttgagactag gaggattcag acagagctag cttcctccct gacccctgga 23700
ttgagggagt ccagcggctc tgaagggacc agctcaggca ccaagatgag cactgtcctc 23760
tctaaagtgc ccactggtgc tactactgag atctccaagg aagacgtcac ctccatccca 23820

ggtcccgctc aatccacaat atcaccagac atctccacaa gaaccgtcag ctggttctct 23880
acatcccctg tcatgacaga atcagcagaa ataaccatga acacccatac aagtccttta 23940
ggggccacaa cacaaggcac cagtactttg gacacgtcaa gcacaacctc tttgacaatg 24000
acacactcaa ctatatctca aggattttca cactcacaga tgagcactct tatgaggagg 24060
ggtcctgagg atgtatcatg gatgagccct ccccttctgg aaaaaactag accttccttt 24120
tctctgatgt cttcaccagc cacaacttca ccttctcctg tttcctccac attaccagag 24180
agcatctctt cctctcctct tcctgtgact tcactcctca cgtctggctt ggcaaaaact 24240
acagatatgt tgcacaaaag ctcagaacct gtaaccaact cacctgcaaa tttgagcagc 24300
acctcagttg aaatactggc cacctctgaa gtcaccacag atacagagaa aactcatcct 24360
tcttcaaaca gaacagtgac cgatgtgggg acctccagtt ctggacatga atccacttcc 24420
tttgtcctag ctgactcaca gacatccaaa gtcacatctc caatggttat tacctccacc 24480
atggaggata cgagtgtctc cacatcaact cctggctttt ttgagactag cagaattcag 24540
acagaaccaa catcctccct gacccttgga ctgagaaaga ccagcagctc tgaggggacc 24600
agcttagcca cagagatgag cactgtcctt tctggagtgc ccactggtgc cactgctgaa 24660
gtctccagga cagaagtcac ctcctctagc agaacatcca tctcaggctt tgctcagctc 24720
acagtgtcac cagagacttc cacagaaacc atcaccagac tccctacctc cagcataatg 24780
acagaatcag cagaaatgat gatcaagaca caaacagatc ctcctgggtc tacaccagag 24840
agtactcata ctgtggacat atcaacaaca cccaactggg tagaaaccca ctcgactgtg 24900
actcagagat tttcacactc agagatgacc actcttgtga gcagaagccc tggtgatatg 24960
ttatggccta gtcaatcctc tgtggaagaa accagctctg cctcttccct gctgtctctg 25020
cctgccacga cctcaccttc tcctgtttcc tctacattag tagaggattt cccttccgct 25080
tctcttcctg tgacttctct tctcaaccct ggcctggtga taaccacaga caggatgggc 25140
ataagcagag aacctggaac cagttccact tcaaatttga gcagcacctc ccatgagaga 25200
ctgaccactt tggaagacac tgtagataca gaagacatgc agccttccac acacacagca 25260
gtgaccaacg tgaggacctc catttctgga catgaatcac aatcttctgt cctatctgac 25320
tcagagacac ccaaagccac atctccaatg ggtaccacct acaccatggg ggaaacgagt 25380
gtttccatat ccacttctga cttctttgag accagcagaa ttcagataga accaacatcc 25440
tccctgactt ctggattgag ggagaccagc agctctgaga ggatcagctc agccacagag 25500
ggaagcactg tcctttctga agtgcccagt ggtgctacca ctgaggtctc caggacagaa 25560
gtgatatcct ctaggggaac atccatgtca gggcctgatc agttcaccat atcaccagac 25620
atctctactg aagcgatcac caggctttct acttccccca ttatgacaga atcagcagaa 25680
agtgccatca ctattgagac aggttctcct ggggctacat cagagggtac cctcaccttg 25740
gacacctcaa caacaacctt ttggtcaggg acccactcaa ctgcatctcc aggattttca 25800
cactcagaga tgaccactct tatgagtaga actcctggag atgtgccatg gccgagcctt 25860
ccctctgtgg aagaagccag ctctgtctct tcctcactgt cttcacctgc catgacctca 25920
acttcttttt tctccacatt accagagagc atctcctcct ctcctcatcc tgtgactgca 25980
cttctcaccc ttggcccagt gaagaccaca gacatgttgc gcacaagctc agaacctgaa 26040
accagttcac ctccaaattt gagcagcacc tcagctgaaa tattagccac gtctgaagtc 26100
accaaagata gagagaaaat tcatccctcc tcaaacacac ctgtagtcaa tgtagggact 26160
gtgatttata aacatctatc cccttcctct gttttggctg acttagtgac aacaaaaccc 26220
acatctccaa tggctaccac ctccactctg gggaatacaa gtgtttccac atcaactcct 26280
gccttcccag aaactatgat gacacagcca acttcctccc tgacttctgg attaagggag 26340
atcagtacct ctcaagagac cagctcagca acagagagaa gtgcttctct ttctggaatg 26400
cccactggtg ctactactaa ggtctccaga acagaagccc tctccttagg cagaacatcc 26460
accccaggtc ctgctcaatc cacaatatca ccagaaatct ccacggaaac catcactaga 26520
atttctactc ccctcaccac gacaggatca gcagaaatga ccatcacccc caaaacaggt 26580
cattctgggg catcctcaca aggtaccttt accttggaca catcaagcag agcctcctgg 26640
ccaggaactc actcagctgc aactcacaga tctccacact cagggatgac cactcctatg 26700
agcagaggtc ctgaggatgt gtcatggcca agccgcccat cagtggaaaa aactagccct 26760
ccatcttccc tggtgtcttt atctgcagta acctcacctt cgccacttta ttccacacca 26820
tctgagagta gccactcatc tcctctccgg gtgacttctc ttttcacccc tgtcatgatg 26880
aagaccacag acatgttgga cacaagcttg gaacctgtga ccacttcacc tcccagtatg 26940
aatatcacct cagatgagag tctggccact tctaaagcca ccatggagac agaggcaatt 27000
cagctttcag aaaacacagc tgtgactcag atgggcacca tcagcgctag acaagaattc 27060
tattcctctt atccaggcct cccagagcca tccaaagtga catctccagt ggtcacctct 27120
tccaccataa aagacattgt ttctacaacc atacctgctt cctctgagat aacaagaatt 27180
gagatggagt caacatccac cctgaccccc acaccaaggg agaccagcac ctcccaggag 27240
atccactcag ccacaaagcc aagcactgtt ccttacaagg cactcactag tgccacgatt 27300
gaggactcca tgacacaagt catgtcctct agcagaggac ctagccctga tcagtccaca 27360
atgtcacaag acatatccac tgaagtgatc accaggctct ctacctcccc catcaagaca 27420
gaatctacag aaatgaccat taccacccaa acaggttctc ctggggctac atcaaggggt 27480
acccttacct tggacacttc aacaactttt atgtcaggga cccactcaac tgcatctcaa 27540
ggattttcac actcacagat gaccgctctt atgagtagaa ctcctggaga tgtgccatgg 27600
ctaagccatc cctctgtgga agaagccagc tctgcctctt tctcactgtc ttcacctgtc 27660
atgacctcat cttctcccgt ttcttccaca ttaccagaca gcatccactc ttcttcgctt 27720
cctgtgacat cacttctcac ctcagggctg gtgaagacca cagagctgtt gggcacaagc 27780
tcagaacctg aaaccagttc acccccaaat ttgagcagca cctcagctga aatactggcc 27840
atcactgaag tcactacaga tacagagaaa ctggagatga ccaatgtggt aacctcaggt 27900

tatacacatg aatctccttc ctctgtccta gctgactcag tgacaacaaa ggccacatct 27960
tcaatgggta tcacctaccc cacaggagat acaaatgttc tcacatcaac ccctgccttc 28020
tctgacacca gtaggattca aacaaagtca aagctctcac tgactcctgg gttgatggag 28080
accagcatct ctgaagagac cagctctgcc acagaaaaaa gcactgtcct ttctagtgtg 28140
cccactggtg ctactactga ggtctccagg acagaagcca tctcttctag cagaacatcc 28200
atcccaggcc ctgctcaatc cacaatgtca tcagacacct ccatggaaac catcactaga 28260
atttctaccc ccctcacaag gaaagaatca acagacatgg ccatcacccc caaaacaggt 28320
ccttctgggg ctacctcgca gggtaccttt accttggact catcaagcac agcctcctgg 28380
ccaggaactc actcagctac aactcagaga tttccacagt cagtggtgac aactcctatg 28440
agcagaggtc ctgaggatgt gtcatggcca agcccgctgt ctgtggaaaa aaacagccct 28500
ccatcttccc tggtatcttc atcttcagta acctcacctt cgccacttta ttccacacca 28560
tctgggagta gccactcctc tcctgtccct gtcacttctc ttttcacctc tatcatgatg 28620
aaggccacag acatgttgga tgcaagtttg gaacctgaga ccacttcagc tcccaatatg 28680
aatatcacct cagatgagag tctggccgct tctaaagcca ccacggagac agaggcaatt 28740
cacgtttttg aaaatacagc agcgtcccat gtggaaacca ccagtgctac agaggaactc 28800
tattcctctt ccccaggctt ctcagagcca acaaaagtga tatctccagt ggtcacctct 28860
tcctctataa gagacaacat ggtttccaca acaatgcctg gctcctctgg cattacaagg 28920
attgagatag agtcaatgtc atctctgacc cctggactga gggagaccag aacctcccag 28980
gacatcacct catccacaga gacaagcact gtcctttaca agatgccctc tggtgccact 29040
cctgaggtct ccaggacaga agttatgccc tctagcagaa catccattcc tggccctgct 29100
cagtccacaa tgtcactaga catctccgat gaagttgtca ccaggctgtc tacctctccc 29160
atcatgacag aatctgcaga aataaccatc accacccaaa caggttattc tctggctaca 29220
tcccaggtta cccttccctt gggcacctca atgacctttt tgtcagggac ccactcaact 29280
atgtctcaag gactttcaca ctcagagatg accaatctta tgagcagggg tcctgaaagt 29340
ctgtcatgga cgagccctcg ctttgtggaa acaactagat cttcctcttc tctgacatca 29400
ttacctctca cgacctcact ttctcctgtg tcctccacat tactagacag tagcccctcc 29460
tctcctcttc ctgtgacttc acttatcctc ccaggcctgg tgaagactac agaagtgttg 29520
gatacaagct cagagcctaa aaccagttca tctccaaatt tgagcagcac ctcagttgaa 29580
ataccggcca cctctgaaat catgacagat acagagaaaa ttcatccttc ctcaaacaca 29640
gcggtggcca aagtgaggac ctccagttct gttcatgaat ctcattcctc tgtcctagct 29700
gactcagaaa caaccataac cataccttca atgggtatca cctccgctgt ggacgatacc 29760
actgttttca catcaaatcc tgccttctct gagactagga ggattccgac agagccaaca 29820
ttctcattga ctcctggatt cagggagact agcacctctg aagagaccac ctcaatcaca 29880
gaaacaagtg cagtccttta tggagtgccc actagtgcta ctactgaagt ctccatgaca 29940
gaaatcatgt cctctaatag aatacacatc cctgactctg atcagtccac gatgtctcca 30000
gacatcatca ctgaagtgat caccaggctc tcttcctcat ccatgatgtc agaatcaaca 30060
caaatgacca tcaccaccca aaaaagttct cctggggcta cagcacagag tactcttacc 30120
ttggccacaa caacagcccc cttggcaagg acccactcaa ctgttcctcc tagattttta 30180
cactcagaga tgacaactct tatgagtagg agtcctgaaa atccatcatg gaagagctct 30240
ctctttgtgg aaaaaactag ctcttcatct tctctgttgt ccttacctgt cacgacctca 30300
ccttctgttt cttccacatt accgcagagt atcccttcct cctctttttc tgtgacttca 30360
ctcctcaccc caggcatggt gaagactaca gacacaagca cagaacctgg aaccagttta 30420
tctccaaatc tgagtggcac ctcagttgaa atactggctg cctctgaagt caccacagat 30480
acagagaaaa ttcatccttc ttcaagcatg gcagtgacca atgtgggaac caccagttct 30540
ggacatgaac tatattcctc tgtttcaatc cactcggagc catccaaggc tacataccca 30600
gtgggtactc cctcttccat ggctgaaacc tctatttcca catcaatgcc tgctaatttt 30660
gagaccacag gatttgaggc tgagccattt tctcatttga cttctggatt taggaagaca 30720
aacatgtccc tggacaccag ctcagtcaca ccaacaaata caccttcttc tcctgggtcc 30780
actcaccttt tacagagttc caagactgat ttcacctctt ctgcaaaaac atcatcccca 30840
gactggcctc cagcctcaca gtatactgaa attccagtgg acataatcac cccctttaat 30900
gcttctccat ctattacgga gtccactggg ataacctcct tcccagaatc caggtttact 30960
atgtctgtaa cagaaagtac tcatcatctg agtacagatt tgctgccttc agctgagact 31020
atttccactg gcacagtgat gccttctcta tcagaggcca tgacttcatt tgccaccact 31080
ggagttccac gagccatctc aggttcaggt agtccattct ctaggacaga gtcaggccct 31140
ggggatgcta ctctgtccac cattgcagag agcctgcctt catccactcc tgtgccattc 31200
tcctcttcaa ccttcactac cactgattct tcaaccatcc cagccctcca tgagataact 31260
tcctcttcag ctaccccata tagagtggac accagtcttg ggacagagag cagcactact 31320
gaaggacgct tggttatggt cagtactttg gacacttcaa gccaaccagg caggacatct 31380
tcatcaccca ttttggatac cagaatgaca gagagcgttg agctgggaac agtgacaagt 31440
gcttatcaag ttccttcact ctcaacacgg ttgacaagaa ctgatggcat tatggaacac 31500
atcacaaaaa tacccaatga agcagcacac agaggtacca taagaccagt caaaggccct 31560
cagacatcca cttcgcctgc cagtcctaaa ggactacaca caggagggac aaaaagaatg 31620
gagaccacca ccacagctct gaagaccacc accacagctc tgaagaccac ttccagagcc 31680
accttgacca ccagtgtcta tactcccact ttgggaacac tgactcccct caatgcatca 31740
atgcaaatgg ccagcacaat ccccacagaa atgatgatca caaccccata tgttttccct 31800
gatgttccag aaacgacatc ctcattggct accagcctgg gagcagaaac cagcacagct 31860
cttcccagga caaccccatc tgttttcaat agagaatcag agaccacagc ctcactggtc 31920
tctcgttctg gggcagagag aagtccggtt attcaaactc tagatgtttc ttctagtgag 31980

ccagatacaa cagcttcatg ggttatccat cctgcagaga ccatcccaac tgtttccaag 32040
acaaccccca attttttcca cagtgaatta gacactgtat cttccacagc caccagtcat 32100
ggggcagacg tcagctcagc cattccaaca aatatctcac ctagtgaact agatgcactg 32160
accccactgg tcactatttc ggggacagat actagtacaa cattcccaac actgactaag 32220
tccccacatg aaacagagac aagaaccaca tggctcactc atcctgcaga gaccagctca 32280
actattccca gaacaatccc caatttttct catcatgaat cagatgccac accttcaata 32340
gccaccagtc ctggggcaga aaccagttca gctattccaa ttatgactgt ctcacctggt 32400
gcagaagatc tggtgacctc acaggtcact agttctggga cagacagaaa tatgactatt 32460
ccaactttga ctctttctcc tggtgaacca aagacgatag cctcattagt cacccatcct 32520
gaagcacaga caagttcggc cattccaact tcaactatct cgcctgctgt atcacggttg 32580
gtgacctcaa tggtcaccag tttggcggca aagacaagta caactaatcg agctctgaca 32640
aactcccctg gtgaaccagc tacaacagtt tcattggtca cgcatcctgc acagaccagc 32700
ccaacagttc cctggacaac ttccattttt ttccatagta aatcagacac cacaccttca 32760
atgaccacca gtcatggggc agaatccagt tcagctgttc caactccaac tgtttcaact 32820
gaggtaccag gagtagtgac ccctttggtc accagttcta gggcagtgat cagtacaact 32880
attccaattc tgactctttc tcctggtgaa ccagagacca caccttcaat ggccaccagt 32940
catggggaag aagccagttc tgctattcca actccaactg tttcacctgg ggtaccagga 33000
gtggtgacct ctctggtcac tagttctagg gcagtgacta gtacaactat tccaattctg 33060
actttttctc ttggtgaacc agagaccaca ccttcaatgg ccaccagtca tgggacagaa 33120
gctggctcag ctgttccaac tgttttacct gaggtaccag gaatggtgac ctctctggtt 33180
gctagttcta gggcagtaac cagtacaact cttccaactc tgactctttc tcctggtgaa 33240
ccagagacca caccttcaat ggccaccagt catggggcag aagccagctc aactgttcca 33300
actgtttcac ctgaggtacc aggagtggtg acctctctgg tcactagttc tagtggagta 33360
aacagtacaa gtattccaac tctgattctt tctcctggtg aactagaaac cacaccttca 33420
atggccacca gtcatggggc agaagccagc tcagctgttc caactccaac tgtttcacct 33480
ggggtatcag gagtggtgac ccctctggtc actagttcca gggcagtgac cagtacaact 33540
attccaattc taactctttc ttctagtgag ccagagacca caccttcaat ggccaccagt 33600
catggggtag aagccagctc agctgttcta actgtttcac ctgaggtacc aggaatggtg 33660
acctctctgg tcactagttc tagagcagta accagtacaa ctattccaac tctgactatt 33720
tcttctgatg aaccagagac cacaacttca ttggtcaccc attctgaggc aaagatgatt 33780
tcagccattc caactttagc tgtctcccct actgtacaag ggctggtgac ttcactggtc 33840
actagttctg ggtcagagac cagtgcgttt tcaaatctaa ctgttgcctc aagtcaacca 33900
gagaccatag actcatgggt cgctcatcct gggacagaag caagttctgt tgttccaact 33960
ttgactgtct ccactggtga gccgtttaca aatatctcat tggtcaccca tcctgcagag 34020
agtagctcaa ctcttcccag gacaacctca aggttttccc acagtgaatt agacactatg 34080
ccttctacag tcaccagtcc tgaggcagaa tccagctcag ccatttcaac aactatttca 34140
cctggtatac caggtgtgct gacatcactg gtcactagct ctgggagaga catcagtgca 34200
acttttccaa cagtgcctga gtccccacat gaatcagagg caacagcctc atgggttact 34260
catcctgcag tcaccagcac aacagttccc aggacaaccc ctaattattc tcatagtgaa 34320
ccagacacca caccatcaat agccaccagt cctggggcag aagccacttc agattttcca 34380
acaataactg tctcacctga tgtaccagat atggtaacct cacaggtcac tagttctggg 34440
acagacacca gtataactat tccaactctg actctttctt ctggtgagcc agagaccaca 34500
acctcattta tcacctattc tgagacacac acaagttcag ccattccaac tctccctgtc 34560
tcccctggtg catcaaagat gctgacctca ctggtcatca gttctgggac agacagcact 34620
acaactttcc caacactgac ggagacccca tatgaaccag agacaacagc catacagctc 34680
attcatcctg cagagaccaa cacaatggtt cccaggacaa ctcccaagtt ttcccatagt 34740
aagtcagaca ccacactccc agtagccatc accagtcctg ggccagaagc cagttcagct 34800
gtttcaacga caactatctc acctgatatg tcagatctgg tgacctcact ggtccctagt 34860
tctgggacag acaccagtac aaccttccca acattgagtg agaccccata tgaaccagag 34920
actacagcca cgtggctcac tcatcctgca gaaaccagca caacggtttc tgggacaatt 34980
cccaactttt cccatagggg atcagacact gcaccctcaa tggtcaccag tcctggagta 35040
gacacgaggt caggtgttcc aactacaacc atcccaccca gtataccagg ggtagtgacc 35100
tcacaggtca ctagttctgc aacagacact agtacagcta ttccaacttt gactccttct 35160
cctggtgaac cagagaccac agcctcatca gctacccatc ctgggacaca gactggcttc 35220
actgttccaa ttcggactgt tccctctagt gagccagata caatggcttc ctgggtcact 35280
catcctccac agaccagcac acctgtttcc agaacaacct ccagtttttc ccatagtagt 35340
ccagatgcca cacctgtaat ggccaccagt cctaggacag aagccagttc agctgtactg 35400
acaacaatct cacctggtgc accagagatg gtgacttcac agatcactag ttctggggca 35460
gcaaccagta caactgttcc aactttgact cattctcctg gtatgccaga gaccacagcc 35520
ttattgagca cccatcccag aacagagaca agtaaaacat ttcctgcttc aactgtgttt 35580
cctcaagtat cagagaccac agcctcactc accattagac ctggtgcaga gactagcaca 35640
gctctcccaa ctcagacaac atcctctctc ttcaccctac ttgtaactgg aaccagcaga 35700
gttgatctaa gtccaactgc ttcacctggt gtttctgcaa aaacagcccc actttccacc 35760
catccaggga cagaaaccag cacaatgatt ccaacttcaa ctctttccct tggtttacta 35820
gagactacag gcttactggc caccagctct tcagcagaga ccagcacgag tactctaact 35880
ctgactgttt cccctgctgt ctctgggctt tccagtgcct ctataacaac tgataagccc 35940
caaactgtga cctcctggaa cacagaaacc tcaccatctg taacttcagt tggaccccca 36000
gaattttcca ggactgtcac aggcaccact atgaccttga taccatcaga gatgccaaca 36060

ccacctaaaa ccagtcatgg agaaggagtg agtccaacca ctatcttgag aactacaatg 36120
gttgaagcca ctaatttagc taccacaggt tccagtccca ctgtggccaa gacaacaacc 36180
accttcaata cactggctgg aagcctcttt actcctctga ccacacctgg gatgtccacc 36240
ttggcctctg agagtgtgac ctcaagaaca agttataacc atcggtcctg gatctccacc 36300
accagcagtt ataaccgtcg gtactggacc cctgccacca gcactccagt gacttctaca 36360
ttctccccag ggatttccac atcctccatc cccagctcca cagcagccac agtcccattc 36420
atggtgccat tcaccctcaa cttcaccatc accaacctgc agtacgagga ggacatgcgg 36480
caccctggtt ccaggaagtt caacgccaca gagagagaac tgcagggtct gctcaaaccc 36540
ttgttcagga atagcagtct ggaatacctc tattcaggct gcagactagc ctcactcagg 36600
ccagagaagg atagctcagc cacggcagtg gatgccatct gcacacatcg ccctgaccct 36660
gaagacctcg gactggacag agagcgactg tactgggagc tgagcaatct gacaaatggc 36720
atccaggagc tgggccccta caccctggac cggaacagtc tctatgtcaa tggtttcacc 36780
catcgaagct ctatgcccac caccagcact cctgggacct ccacagtgga tgtgggaacc 36840
tcagggactc catcctccag ccccagcccc acgactgctg gccctctcct gatgccgttc 36900
accctcaact tcaccatcac caacctgcag tacgaggagg acatgcgtcg cactggctcc 36960
aggaagttca acaccatgga gagtgtcctg cagggtctgc tcaagccctt gttcaagaac 37020
accagtgttg gccctctgta ctctggctgc agattgacct tgctcaggcc cgagaaagat 37080
ggggcagcca ctggagtgga tgccatctgc acccaccgcc ttgaccccaa aagccctgga 37140
ctcaacaggg agcagctgta ctgggagcta agcaaactga ccaatgacat tgaagagctg 37200
ggcccctaca ccctggacag gaacagtctc tatgtcaatg gtttcaccca tcagagctct 37260
gtgtccacca ccagcactcc tgggacctcc acagtggatc tcagaacctc agggactcca 37320
tcctccctct ccagccccac aattatggct gctggccctc tcctggtacc attcaccctc 37380
aacttcacca tcaccaacct gcagtatggg gaggacatgg gtcaccctgg ctccaggaag 37440
ttcaacacca cagagagggt cctgcagggt ctgcttggtc ccatattcaa gaacaccagt 37500
gttggccctc tgtactctgg ctgcagactg acctctctca ggtctgagaa ggatggagca 37560
gccactggag tggatgccat ctgcatccat catcttgacc ccaaaagccc tggactcaac 37620
agagagcggc tgtactggga gctgagccaa ctgaccaatg gcatcaaaga gctgggcccc 37680
tacaccctgg acaggaacag tctctatgtc aatggtttca cccatcggac ctctgtgccc 37740
accagcagca ctcctgggac ctccacagtg gaccttggaa cctcagggac tccattctcc 37800
ctcccaagcc ccgcaactgc tggccctctc ctggtgctgt tcaccctcaa cttcaccatc 37860
accaacctga agtatgagga ggacatgcat cgccctggct ccaggaagtt caacaccact 37920
gagagggtcc tgcagactct gcttggtcct atgttcaaga acaccagtgt tggccttctg 37980
tactctggct gcagactgac cttgctcagg tccgagaagg atggagcagc cactggagtg 38040
gatgccatct gcacccaccg tcttgacccc aaaagccctg gagtggacag ggagcagcta 38100
tactgggagc tgagccagct gaccaatggc atcaaagagc tgggccccta caccctggac 38160
aggaacagtc tctatgtcaa tggtttcacc cattggatcc ctgtgcccac cagcagcact 38220
cctgggacct ccacagtgga ccttgggtca gggactccat cctccctccc cagccccaca 38280
actgctggcc ctctcctggt gccgttcacc ctcaacttca ccatcaccaa cctgaagtac 38340
gaggaggaca tgcattgccc tggctccagg aagttcaaca ccacagagag agtcctgcag 38400
agtctgcttg gtcccatgtt caagaacacc agtgttggcc ctctgtactc tggctgcaga 38460
ctgaccttgc tcaggtccga gaaggatgga gcagccactg gagtggatgc catctgcacc 38520
caccgtcttg accccaaaag ccctggagtg gacagggagc agctatactg ggagctgagc 38580
cagctgacca atggcatcaa agagctgggt ccctacaccc tggacagaaa cagtctctat 38640
gtcaatggtt tcacccatca gacctctgcg cccaacacca gcactcctgg gacctccaca 38700
gtggaccttg ggacctcagg gactccatcc tccctcccca gccctacatc tgctggccct 38760
ctcctggtgc cattcaccct caacttcacc atcaccaacc tgcagtacga ggaggacatg 38820
catcacccag gctccaggaa gttcaacacc acggagcggg tcctgcaggg tctgcttggt 38880
cccatgttca agaacaccag tgtcggcctt ctgtactctg gctgcagact gaccttgctc 38940
aggcctgaga agaatggggc agccactgga atggatgcca tctgcagcca ccgtcttgac 39000
cccaaaagcc ctggactcaa cagagagcag ctgtactggg agctgagcca gctgacccat 39060
ggcatcaaag agctgggccc ctacaccctg gacaggaaca gtctctatgt caatggtttc 39120
acccatcgga gctctgtggc ccccaccagc actcctggga cctccacagt ggaccttggg 39180
acctcaggga ctccatcctc cctccccagc cccacaacag ctgttcctct cctggtgccg 39240
ttcaccctca actttaccat caccaatctg cagtatgggg aggacatgcg tcaccctggc 39300
tccaggaagt tcaacaccac agagagggtc ctgcagggtc tgcttggtcc cttgttcaag 39360
aactccagtg tcggccctct gtactctggc tgcagactga tctctctcag gtctgagaag 39420
gatggggcag ccactggagt ggatgccatc tgcacccacc accttaaccc tcaaagccct 39480
ggactggaca gggagcagct gtactggcag ctgagccaga tgaccaatgg catcaaagag 39540
ctgggcccct acaccctgga ccggaacagt ctctacgtca atggtttcac ccatcggagc 39600
tctgggctca ccaccagcac tccttggact tccacagttg accttggaac ctcagggact 39660
ccatcccccg tccccagccc cacaaccacc ggccctctcc tggtgccatt cacactcaac 39720
ttcaccatca ctaacctaca gtatgaggag aacatgggtc accctggctc caggaagttc 39780
aacatcacgg agagtgttct gcagggtctg ctcaagccct tgttcaagag caccagtgtt 39840
ggccctctgt attctggctg cagactgacc ttgctcaggc ctgagaagga tggagtagcc 39900
accagagtgg acgccatctg cacccaccgc cctgacccca aaatccctgg gctagacaga 39960
cagcagctat actgggagct gagccagctg acccacagca tcactgagct gggaccctac 40020
accctggata gggacagtct ctatgtcaat ggtttcaccc agcggagctc tgtgcccacc 40080
accagcactc ctgggacttt cacagtacag ccggaaacct ctgagactcc atcatccctc 40140

cctggcccca cagccactgg ccctgtcctg ctgccattca ccctcaattt taccatcact 40200
aacctgcagt atgaggagga catgcgtcgc cctggctcca ggaagttcaa caccacggag 40260
agggtccttc agggtctgct tatgcccttg ttcaagaaca ccagtgtcag ctctctgtac 40320
tctggttgca gactgacctt gctcaggcct gagaaggatg gggcagccac cagagtggat 40380
gctgtctgca cccatcgtcc tgaccccaaa agccctggac tggacagaga gcggctgtac 40440
tggaagctga gccagctgac ccacggcatc actgagctgg gcccctacac cctggacagg 40500
cacagtctct atgtcaatgg tttcacccat cagagctcta tgacgaccac cagaactcct 40560
gatacctcca caatgcacct ggcaacctcg agaactccag cctccctgtc tggacccatg 40620
accgccagcc ctctcctggt gctattcaca attaacttca ccatcactaa cctgcggtat 40680
gaggagaaca tgcatcaccc tggctctaga aagtttaaca ccacggagag agtccttcag 40740
ggtctgctca ggcctgtgtt caagaacacc agtgttggcc ctctgtactc tggctgcaga 40800
ctgaccttgc tcaggcccaa gaaggatggg gcagccacca aagtggatgc catctgcacc 40860
taccgccctg atcccaaaag ccctggactg gacagagagc agctatactg ggagctgagc 40920
cagctgaccc acagcatcac tgagctgggc ccctacaccc tggacaggga cagtctctat 40980
gtcaatggtt tcacacagcg gagctctgtg cccaccacta gcattcctgg gacccccaca 41040
gtggacctgg gaacatctgg gactccagtt tctaaacctg gtccctcggc tgccagccct 41100
ctcctggtgc tattcactct caacttcacc atcaccaacc tgcggtatga ggagaacatg 41160
cagcaccctg gctccaggaa gttcaacacc acggagaggg tccttcaggg cctgctcagg 41220
tccctgttca agagcaccag tgttggccct ctgtactctg gctgcagact gactttgctc 41280
aggcctgaaa aggatgggac agccactgga gtggatgcca tctgcaccca ccaccctgac 41340
cccaaaagcc ctaggctgga cagagagcag ctgtattggg agctgagcca gctgacccac 41400
aatatcactg agctgggccc ctatgccctg gacaacgaca gcctctttgt caatggtttc 41460
actcatcgga gctctgtgtc caccaccagc actcctggga cccccacagt gtatctggga 41520
gcatctaaga ctccagcctc gatatttggc ccttcagctg ccagccatct cctgatacta 41580
ttcaccctca acttcaccat cactaacctg cggtatgagg agaacatgtg gcctggctcc 41640
aggaagttca acactacaga gagggtcctt cagggcctgc taaggccctt gttcaagaac 41700
accagtgttg gccctctgta ctctggctgc aggctgacct tgctcaggcc agagaaagat 41760
ggggaagcca ccggagtgga tgccatctgc acccaccgcc ctgaccccac aggccctggg 41820
ctggacagag agcagctgta tttggagctg agccagctga cccacagcat cactgagctg 41880
ggcccctaca cactggacag ggacagtctc tatgtcaatg gtttcaccca tcggagctct 41940
gtacccacca ccagcaccgg ggtggtcagc gaggagccat tcacactgaa cttcaccatc 42000
aacaacctgc gctacatggc ggacatgggc caacccggct ccctcaagtt caacatcaca 42060
gacaacgtca tgcagcacct gctcagtcct ttgttccaga ggagcagcct gggtgcacgg 42120
tacacaggct gcagggtcat cgcactaagg tctgtgaaga acggtgctga gacacgggtg 42180
gacctcctct gcacctacct gcagcccctc agcggcccag gtctgcctat caagcaggtg 42240
ttccatgagc tgagccagca gacccatggc atcacccggc tgggccccta ctctctggac 42300
aaagacagcc tctaccttaa cggttacaat gaacctggtc cagatgagcc tcctacaact 42360
cccaagccag ccaccacatt cctgcctcct ctgtcagaag ccacaacagc catggggtac 42420
cacctgaaga ccctcacact caacttcacc atctccaatc tccagtattc accagatatg 42480
ggcaagggct cagctacatt caactccacc gagggggtcc ttcagcacct gctcagaccc 42540
ttgttccaga agagcagcat gggccccttc tacttgggtt gccaactgat ctccctcagg 42600
cctgagaagg atggggcagc cactggtgtg gacaccacct gcacctacca ccctgaccct 42660
gtgggccccg ggctggacat acagcagctt tactgggagc tgagtcagct gacccatggt 42720
gtcacccaac tgggcttcta tgtcctggac agggatagcc tcttcatcaa tggctatgca 42780
ccccagaatt tatcaatccg gggcgagtac cagataaatt tccacattgt caactggaac 42840
ctcagtaatc cagaccccac atcctcagag tacatcaccc tgctgaggga catccaggac 42900
aaggtcacca cactctacaa aggcagtcaa ctacatgaca cattccgctt ctgcctggtc 42960
accaacttga cgatggactc cgtgttggtc actgtcaagg cattgttctc ctccaatttg 43020
gaccccagcc tggtggagca agtctttcta gataagaccc tgaatgcctc attccattgg 43080
ctgggctcca cctaccagtt ggtggacatc catgtgacag aaatggagtc atcagtttat 43140
caaccaacaa gcagctccag cacccagcac ttctacctga atttcaccat caccaaccta 43200
ccatattccc aggacaaagc ccagccaggc accaccaatt accagaggaa caaaaggaat 43260
attgaggatg cgctcaacca actcttccga aacagcagca tcaagagtta tttttctgac 43320
tgtcaagttt caacattcag gtctgtcccc aacaggcacc acaccggggt ggactccctg 43380
tgtaacttct cgccactggc tcggagagta gacagagttg ccatctatga ggaatttctg 43440
cggatgaccc ggaatggtac ccagctgcag aacttcaccc tggacaggag cagtgtcctt 43500
gtggatgggt attctcccaa cagaaatgag cccttaactg ggaattctga ccttcccttc 43560
tgggctgtca tcctcatcgg cttggcagga ctcctgggag tcatcacatg cctgatctgc 43620
ggtgtcctgg tgaccacccg ccggcggaag aaggaaggag aatacaacgt ccagcaacag 43680
tgcccaggct actaccagtc acacctagac ctggaggatc tgcaatgact ggaacttgcc 43740
ggtgcctggg gtgcctttcc cccagccagg gtccaaagaa gcttggctgg ggcagaaata 43800
aaccatattg gtcgga 43816

<210> 59
<211> 267
<212> PRT

<213> Homo sapiens

<400> 59
Met Arg Leu Thr Val Leu Cys Ala Val Cys Leu Leu Pro Gly Ser Leu
1               5                   10                  15
Ala Leu Pro Leu Pro Gln Glu Ala Gly Gly Met Ser Glu Leu Gln Trp
            20                  25                  30
Glu Gln Ala Gln Asp Tyr Leu Lys Arg Phe Tyr Leu Tyr Asp Ser Glu
        35                  40                  45
Thr Lys Asn Ala Asn Ser Leu Glu Ala Lys Leu Lys Glu Met Gln Lys
    50                  55                  60
Phe Phe Gly Leu Pro Ile Thr Gly Met Leu Asn Ser Arg Val Ile Glu
65                  70                  75                  80
Ile Met Gln Lys Pro Arg Cys Gly Val Pro Asp Val Ala Glu Tyr Ser
                85                  90                  95
Leu Phe Pro Asn Ser Pro Lys Trp Thr Ser Lys Val Val Thr Tyr Arg
            100                 105                 110
Ile Val Ser Tyr Thr Arg Asp Leu Pro His Ile Thr Val Asp Arg Leu
        115                 120                 125
Val Ser Lys Ala Leu Asn Met Trp Gly Lys Glu Ile Pro Leu His Phe
    130                 135                 140
Arg Lys Val Val Trp Gly Thr Ala Asp Ile Met Ile Gly Phe Ala Arg
145                 150                 155                 160
Gly Ala His Gly Asp Ser Tyr Pro Phe Asp Gly Pro Gly Asn Thr Leu
                165                 170                 175
Ala His Ala Phe Ala Pro Gly Thr Gly Leu Gly Gly Asp Ala His Phe
            180                 185                 190
Asp Glu Asp Glu Arg Trp Thr Asp Gly Ser Ser Leu Gly Ile Asn Phe
        195                 200                 205
Leu Tyr Ala Ala Thr His Glu Leu Gly His Ser Leu Gly Met Gly His
    210                 215                 220
Ser Ser Asp Pro Asn Ala Val Met Tyr Pro Thr Tyr Gly Asn Gly Asp
225                 230                 235                 240
Pro Gln Asn Phe Lys Leu Ser Gln Asp Asp Ile Lys Gly Ile Gln Lys
                245                 250                 255
Leu Tyr Gly Lys Arg Ser Asn Ser Arg Lys Lys
            260                 265

<210> 60
<211> 1147
<212> DNA
<213> Homo sapiens

<400> 60
accaaatcaa ccataggtcc aagaacaatt gtctctggac ggcagctatg cgactcaccg 60
tgctgtgtgc tgtgtgcctg ctgcctggca gcctggccct gccgctgcct caggaggcgg 120
gaggcatgag tgagctacag tgggaacagg ctcaggacta tctcaagaga ttttatctct 180
atgactcaga aacaaaaaat gccaacagtt tagaagccaa actcaaggag atgcaaaaat 240
tctttggcct acctataact ggaatgttaa actcccgcgt catagaaata atgcagaagc 300
ccagatgtgg agtgccagat gttgcagaat actcactatt tccaaatagc ccaaaatgga 360
cttccaaagt ggtcacctac aggatcgtat catatactcg agacttaccg catattacag 420
tggatcgatt agtgtcaaag gctttaaaca tgtggggcaa agagatcccc ctgcatttca 480
ggaaagttgt atggggaact gctgacatca tgattggctt tgcgcgagga gctcatgggg 540
actcctaccc atttgatggg ccaggaaaca cgctggctca tgcctttgcg cctgggacag 600
gtctcggagg agatgctcac ttcgatgagg atgaacgctg gacggatggt agcagtctag 660
ggattaactt cctgtatgct gcaactcatg aacttggcca ttctttgggt atgggacatt 720
cctctgatcc taatgcagtg atgtatccaa cctatggaaa tggagatccc caaaatttta 780
aactttccca ggatgatatt aaaggcattc agaaactata tggaaagaga agtaattcaa 840
gaaagaaata gaaacttcag gcagaacatc cattcattca ttcattggat tgtatatcat 900
tgttgcacaa tcagaattga taagcactgt tcctccactc catttagcaa ttatgtcacc 960
cttttttatt gcagttggtt tttgaatgtc tttcactcct tttaaggata aactccttta 1020
tggtgtgact gtgtcttatt catctatact tgcagtgggt agatgtcaat aaatgttaca 1080
tacacaaata aataaaatgt ttattccatg gtaaatttaa aaaaaaaaaa aaaaaaaaaa 1140
aaaaaaa                                                           1147

<210> 61
<211> 227

<212> PRT
<213> Homo sapiens

<400> 61
Met Asn Ile Lys Gly Ser Pro Trp Lys Gly Ser Leu Leu Leu Leu Leu
1               5                   10                  15
Val Ser Asn Leu Leu Leu Cys Gln Ser Val Ala Pro Leu Pro Ile Cys
            20                  25                  30
Pro Gly Gly Ala Ala Arg Cys Gln Val Thr Leu Arg Asp Leu Phe Asp
        35                  40                  45
Arg Ala Val Val Leu Ser His Tyr Ile His Asn Leu Ser Ser Glu Met
    50                  55                  60
Phe Ser Glu Phe Asp Lys Arg Tyr Thr His Gly Arg Gly Phe Ile Thr
65                  70                  75                  80
Lys Ala Ile Asn Ser Cys His Thr Ser Ser Leu Ala Thr Pro Glu Asp
                85                  90                  95
Lys Glu Gln Ala Gln Gln Met Asn Gln Lys Asp Phe Leu Ser Leu Ile
            100                 105                 110
Val Ser Ile Leu Arg Ser Trp Asn Glu Pro Leu Tyr His Leu Val Thr
        115                 120                 125
Glu Val Arg Gly Met Gln Glu Ala Pro Glu Ala Ile Leu Ser Lys Ala
    130                 135                 140
Val Glu Ile Glu Glu Gln Thr Lys Arg Leu Leu Glu Gly Met Glu Leu
145                 150                 155                 160
Ile Val Ser Gln Val His Pro Glu Thr Lys Glu Asn Glu Ile Tyr Pro
                165                 170                 175
Val Trp Ser Gly Leu Pro Ser Leu Gln Met Ala Asp Glu Glu Ser Arg
            180                 185                 190
Leu Ser Ala Tyr Tyr Asn Leu Leu His Cys Leu Arg Arg Asp Ser His
        195                 200                 205
Lys Ile Asp Asn Tyr Leu Lys Leu Leu Lys Cys Arg Ile Ile His Asn
    210                 215                 220
Asn Asn Cys
225


<210> 62
<211> 1388
<212> DNA
<213> Homo sapiens

<400> 62
gggatcctta ttctatatct cttggtattt agtgtaaaaa ttttaaaatc tttacctagc 60
aatcttgagg aagaaacttg ataactgata atacatgaga ttgttaccta agtgaaatat 120
aatcctatat attcaacaaa ctttagagaa ataagataaa ttttaaagta aatgacttct 180
gtagttttat agatcctcca aaccaatcta gtctcagatc tcaccttcat catttctctc 240
atttcctttt ggcctaatta atcaaaatcc ttcctagaat gttcatttct ggccagtatg 300
tcttcctgaa tatgaataag aaataaaata ccatttgatg tttgaaatta tgggggtaat 360
ctcaatgacg gaaatagatg accaggaaaa gggaaacgaa tgcctgattc attatattca 420
tgaagatatc aaaggtttat aaagccaata tctgggaaag agaaaaccgt gagacttcca 480
gatcttctct ggtgaagtgt gtttcctgca acgatcacga acatgaacat caaaggatcg 540
ccatggaaag ggtccctcct gctgctgctg gtgtcaaacc tgctcctgtg ccagagcgtg 600
gcccccttgc ccatctgtcc cggcggggct gcccgatgcc aggtgaccct tcgagacctg 660
tttgaccgcg ccgtcgtcct gtcccactac atccataacc tctcctcaga aatgttcagc 720
gaattcgata aacggtatac ccatggccgg gggttcatta ccaaggccat caacagctgc 780
cacacttctt cccttgccac ccccgaagac aaggagcaag cccaacagat gaatcaaaaa 840
gactttctga gcctgatagt cagcatattg cgatcctgga atgagcctct gtatcatctg 900
gtcacggaag tacgtggtat gcaagaagcc ccggaggcta tcctatccaa agctgtagag 960
attgaggagc aaaccaaacg gcttctagag ggcatggagc tgatagtcag ccaggttcat 1020
cctgaaacca aagaaaatga gatctaccct gtctggtcgg gacttccatc cctgcagatg 1080
gctgatgaag agtctcgcct ttctgcttat tataacctgc tccactgcct acgcagggat 1140
tcacataaaa tcgacaatta tctcaagctc ctgaagtgcc gaatcatcca caacaacaac 1200
tgctaagccc acatccattt catctatttc tgagaaggtc cttaatgatc cgttccattg 1260
caagcttctt ttagttgtat ctcttttgaa tccatgcttg ggtgtaacag gtctcctctt 1320
aaaaaataaa aactgactcc ttagagacat caaaatccaa aaaaaaaaaa aaaaaaaaaa 1380
aaaaaaaa                                                          1388

<210> 63

<211> 132
<212> PRT
<213> Homo sapiens

<400> 63
Met Lys Ser Ser Gly Leu Phe Pro Phe Leu Val Leu Leu Ala Leu Gly
1               5                   10                  15
Thr Leu Ala Pro Trp Ala Val Glu Gly Ser Gly Lys Ser Phe Lys Ala
            20                  25                  30
Gly Val Cys Pro Pro Lys Lys Ser Ala Gln Cys Leu Arg Tyr Lys Lys
        35                  40                  45
Pro Glu Cys Gln Ser Asp Trp Gln Cys Pro Gly Lys Lys Arg Cys Cys
    50                  55                  60
Pro Asp Thr Cys Gly Ile Lys Cys Leu Asp Pro Val Asp Thr Pro Asn
65                  70                  75                  80
Pro Thr Arg Arg Lys Pro Gly Lys Cys Pro Val Thr Tyr Gly Gln Cys
                85                  90                  95
Leu Met Leu Asn Pro Pro Asn Phe Cys Glu Met Asp Gly Gln Cys Lys
            100                 105                 110
Arg Asp Leu Lys Cys Cys Met Gly Met Cys Gly Lys Ser Cys Val Ser
        115                 120                 125
Pro Val Lys Ala
    130


<210> 64
<211> 598
<212> DNA
<213> Homo sapiens

<400> 64
cagagtcact cctgccttca ccatgaagtc cagcggcctc ttccccttcc tggtgctgct 60
tgccctggga actctggcac cttgggctgt ggaaggctct ggaaagtcct tcaaagctgg 120
agtctgtcct cctaagaaat ctgcccagtg ccttagatac aagaaacctg agtgccagag 180
tgactggcag tgtccaggga agaagagatg ttgtcctgac acttgtggca tcaaatgcct 240
ggatcctgtt gacaccccaa acccaacaag gaggaagcct gggaagtgcc cagtgactta 300
tggccaatgt ttgatgctta acccccccaa tttctgtgag atggatggcc agtgcaagcg 360
tgacttgaag tgttgcatgg gcatgtgtgg gaaatcctgc gtttcccctg tgaaagcttg 420
attcctgcca tatggaggag gctctggagt cctgctctgt gtggtccagg tcctttccac 480
cctgagactt ggctccacca ctgatatcct cctttgggga aaggcttggc acacagcagg 540
ctttcaagaa gtgccagttg atcaatgaat aaataaacga gcctatttct ctttgcac   598